(19) Europäisches Patentamt
European Patent Office
Office européen des brevets

(11) EP 2 010 289 B1

(12) EUROPEAN PATENT SPECIFICATION

(45) Date of publication and mention of the grant of the patent:
**30.11.2011 Bulletin 2011/48**

(21) Application number: **07760766.1**

(22) Date of filing: **17.04.2007**

(51) Int Cl.:
***A61P 1/18*** (2006.01) ***A61P 3/06*** (2006.01)
***A61P 3/10*** (2006.01) ***A61K 31/55*** (2006.01)
***C07D 223/16*** (2006.01) ***C07D 417/06*** (2006.01)
***C07D 409/06*** (2006.01) ***C07D 413/06*** (2006.01)
***C07D 403/06*** (2006.01) ***C07D 405/06*** (2006.01)
***C07D 401/06*** (2006.01)

(86) International application number:
**PCT/US2007/066772**

(87) International publication number:
**WO 2007/121432 (25.10.2007 Gazette 2007/43)**

(54) **BENZOAZEPIN-OXY-ACETIC ACID DERIVATIVES AS PPAR-DELTA AGONISTS USED FOR THE INCREASE OF HDL-C, LOWER LDL-C AND LOWER CHOLESTEROL**

BENZOAZEPIN-OXY-ESSIGSÄUREDERIVATE ALS PPAR-DELTA-AGONISTEN ZUR ERHÖHUNG VON HDL-C, SENKUNG VON LDL-C SOWIE CHOLESTERINSENKUNG

DERIVES DE L'ACIDE BENZOAZEPINE-OXYACETIQUE UTILISES EN TANT QU'AGONISTES DELTA PPAR UTILISES POUR AUGMENTER LE HDL-C, ABAISSER LE LDL-C ET ABAISSER LE CHOLESTEROL

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **18.04.2006 US 793001 P**

(43) Date of publication of application:
**07.01.2009 Bulletin 2009/02**

(73) Proprietor: **Janssen Pharmaceutica NV**
**2340 Beerse (BE)**

(72) Inventors:
- **GEE-HONG, Kuo**
  **Scotch Plains, NJ 07076 (US)**
- **ZHANG, Yan**
  **Fort Washington, PA 19034 (US)**
- **SHEN, Lan**
  **Thousand Oaks, CA 91362 (US)**
- **LU, Songfeng**
  **Raritan, NJ 08869 (US)**
- **DEMAREST, Keith T.**
  **Flemington, NJ 08822 (US)**
- **PELTON, Patricia**
  **Long Valley, NJ 07853 (US)**

(74) Representative: **Warner, James Alexander et al**
**Carpmaels & Ransford**
**One Southampton Row**
**London**
**WC1B 5HA (GB)**

(56) References cited:
**EP-A1- 1 424 330**
**EP-A1- 1 445 258**
**WO-A1-2005/041959**
**US-A1- 2005 124 698**
**US-A1- 2006 058 382**

EP 2 010 289 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

STATEMENT REGARDING FEDERALLY SPONSORED RESEARCH OR DEVELOPMENT

The research and development of the invention described below was not federally sponsored.

BACKGROUND OF THE INVENTION

The peroxisome proliferator-activated receptors (PPARs) are considered to be metabolic sensors regulating the expression of genes involved in glucose and lipid homeostasis. They are members of nuclear receptor superfamily of RXR heterodimers and are ligand-activated transcription factors. Agonists of the PPARα (*e.g.*, Gemfibrozil) and PPARγ (e.g., Avandia®) subtypes are used for the treatment of dyslipidemias and diabetes, respectively. Each receptor has a distinct tissue distribution with PPARα showing highest expression in liver, PPARγ in adipose tissue and PPARδ having the widest distribution being ubiquitously expressed in adult rat (Braissant et al., 1996) and in humans, expression was found in many different tissues involved in lipid metabolism including liver, kidney, abdominal adipose and skeletal muscle (Auboeuf et al., 1997).

Recently, potent ligands for PPARδ have been published allowing a better understanding of its function in lipid metabolism (Barak et al, 2002; Oliver et al., 2001; Tanaka et al, 2003; Wang et al., 2003). The main effect of these compounds in db/db mice (Leibowitz et al., 2000) and obese rhesus monkeys (Oliver et al., 2001) was an increase of high density lipoprotein cholesterol (HDL-C) and a decrease in triglycerides with little effect on glucose (although insulin levels were decreased in monkeys). HDL-C serves to remove cholesterol from peripheral cells through a process called reverse cholesterol transport. The first and rate-limiting step, which is a transfer of cellular cholesterol and phospholipids to the apolipoprotein A-I component of HDL, is mediated by the ATP binding cassette transporter A1 (ABCA1) (Lawn et al., 1999). PPARδ activation appears to increase HDL-C through transcriptional regulation of ABCA1 (Oliver et al., 2001). Therefore, by inducing ABCA1 mRNA in macrophages, PPARδ agonists could increase HDL-C levels in patients and remove excess cholesterol from lipid-laden macrophages, one of the major players in atherosclerotic lesion development. This would be an alternative therapy to the statin drugs, which show little effect on HDL-C and mainly decrease LDL-C or the fibrates, the only marketed PPARα agonists, having low potency and inducing only modest HDL-C elevations. In addition, like the fibrates, PPARδ agonists have the potential to also reduce triglycerides, an additional risk factor for cardiovascular disease.

WO 2005/041959 discloses phenoxyacetic acids, specifically 4-((phenoxyalkyl)thio)-phenoxyacetic acids and analogs, useful as PPAR dual agonists.

EP 1 445 258 discloses benzisoxazole compounds that are PPAR delta activators, and EP 1 424 330 discloses PPAR delta activators.

Examples of known PPAR delta agonists variously useful for hyperlipidemia, diabetes, or atherosclerosis include L-165041 (Leibowitz et al., 2000) and GW501516 (Oliver et al., 2001). There is a continuing need for new PPAR delta agonists. There is a further need for new PPAR delta agonists that increase HDL-C, lower LDL-C, and/or lower cholesterol. There is a further need for new PPAR delta agonists for the treatment of diabetes, nephropathy, neuropathy, retinopathy, polycystic ovary syndrome, hypertension, ischemia, stroke, irritable bowel disorder, inflammation, cataract, cardiovascular diseases, Metabolic X Syndrome, hyper-LDL-cholesterolemia, dyslipidemia (including hypertriglyceridemia, hypercholesterolemia, mixed hyperlipidemia; and hypo-HDL-cholesterolemia), atherosclerosis, obesity, and other disorders related to lipid metabolism and energy homeostasis complications thereof.

SUMMARY OF THE INVENTION

The present invention is directed to a compound of Formula (I):

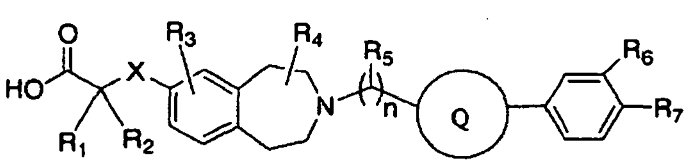

**Formula (I)**

wherein:

X is a covalent bond, O, or S;

$R_1$ and $R_2$ are independently selected from the group consisting of H, $C_{1-8}$alkyl, and substituted $C_{1-8}$alkyl, or $R_1$, $R_2$ and the carbon atom to which they are attached together may form $C_{3-7}$cycloalkyl;

$R_3$ is H;

$R_4$ and $R_5$ are independently selected from the group consisting of H, halo, $C_{1-8}$alkyl, $C_{3-7}$cycloalkyl, $C_{3-7}$cycloalkyl-$C_{1-4}$alkyl, $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{6-10}$aryl substituted $C_{1-4}$alkyl, cyano substituted $C_{1-4}$alkyl, and hydroxy substituted $C_{1-4}$alkyl;

$R_6$ and $R_7$ are independently selected from the group consisting of H, halo, $C_{1-3}$alkyl, halo substituted $C_{1-3}$alkyl, $C_{1-3}$alkoxy, and halo substituted $C_{1-3}$alkoxy;

n is 1; and

Q is selected from the group consisting of

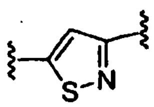, 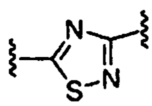, 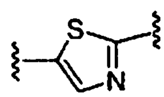, 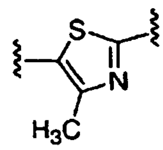,
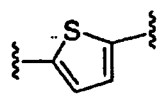, 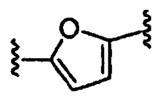, 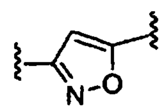, 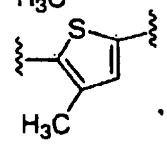,
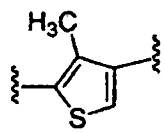, 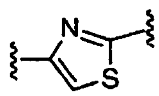, 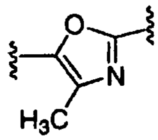, 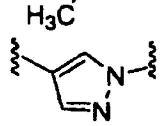,
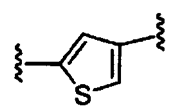, 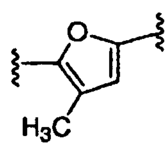, and 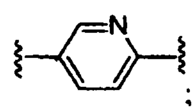;

and enantiomers, diastereomers, tautomers, solvates, or pharmaceutically acceptable salts thereof.

The present invention is further directed to pharmaceutical compositions containing one or more compounds of Formula (I), and to to use of such compounds and compositions to treat a condition directly or indirectly mediated by PPAR delta.

DETAILED DESCRIPTION OF THE INVENTION

As used herein, the following underlined terms are intended to have the following meanings:

"$C_{a-b}$" (where *a* and *b* are integers) refers to a radical containing from *a* to *b* carbon atoms inclusive. For example, $C_{1-3}$ denotes a radical containing 1, 2 or 3 carbon atoms.

"Alkyl" refers to a saturated or unsaturated, branched, straight-chain or cyclic monovalent hydrocarbon radical derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl, cycloprop-1-en-1-yl; cycloprop-2-en-1-yl, prop-1-yn-1-yl, prop-2-yn-1-yl, etc.; butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, etc.; and the like. Where specific levels of saturation are intended, the nomenclature "alkanyl", "alkenyl" and/or "alkynyl" is used, as defined below. In preferred embodiments, the alkyl groups are ($C_1$-$C_6$) alkyl, with ($C_1$-$C_3$) being particularly preferred. Cyclic alkyl can be, for example, $C_{3-10}$alkyl; preferably, cycloalkyl is

$C_{3-7}$cycloalkyl.

"Alkanyl" refers to a saturated branched, straight-chain or cyclic monovalent hydrocarbon radical derived by the removal of one hydrogen atom from a single carbon atom of a parent alkane. Typical alkanyl groups include, but are not limited to, methanyl; ethanyl; propanyls such as propan-1-yl, propan-2-yl, cyclopropan-1-yl, etc.; butyanyls such as butan-1-yl, butan-2-yl, 2-methyl-propan-1-yl, 2-methyl-propan-2-yl, cyclobutan-1-yl, etc.; and the like. In preferred embodiments, the alkanyl groups are ($C_{1-8}$) alkanyl, with ($C_{1-3}$) being particularly preferred.

"Alkenyl" refers to an unsaturated branched, straight-chain or cyclic monovalent hydrocarbon radical having at least one carbon-carbon double bond derived by the removal of one hydrogen atom from a single carbon atom of a parent alkene. The radical may be in either the cis or trans conformation about the double bond(s). Typical alkenyl groups include, but are not limited to, ethenyl; propenyls such as prop-1-en-1-yl, prop-1-en-2-yl, prop-2-en-1-yl, prop-2-en-2-yl, cycloprop-1-en-1-yl; cycloprop-2-en-1-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, etc.; and the like.

"Alkynyl" refers to an unsaturated branched, straight-chain or cyclic monovalent hydrocarbon radical having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl, etc.; butynyls such as but-1-yn-1-yl, but-1-yn-3-yl, but-3-yn-1-yl, etc.; and the like.

"Heteroalkyl" and "heteroalkanyl" refer to alkyl or alkanyl radicals, respectively, in which one or more carbon atoms (and any necessary associated hydrogen atoms) are independently replaced with the same or different heteroatoms (including any necessary hydrogen or other atoms). Typical heteroatoms to replace the carbon atom(s) include, but are not limited to, N, P, O, S, Si, etc. Preferred heteroatoms are O, N and S. Thus, heteroalkanyl radicals can contain one or more of the same or different heteroatomic groups, including, by way of example and not limitation, epoxy (-O-), epidioxy (-O-O-), thioether (-S-), epidithio (-SS-), epoxythio (-O-S-), epoxyimino (-O-NR'-), imino (-NR'-), biimino (-NR'-NR'-), azino (=N-N=), azo (-N=N-), azoxy (-N-O-N-), azimino (-NR'-N=N-), phosphano (-PH-), $\lambda^4$-sulfano (-$SH_2$-), sulfonyl (-$S(O)_2$-), and the like, where each R' is independently hydrogen or ($C_1$-$C_6$) alkyl.

"Aryl" refers to a monovalent aromatic hydrocarbon radical derived by the removal of one hydrogen atom from a single carbon atom of a parent aromatic ring system. Typical aryl groups include, but are not limited to, radicals derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene, and the like. In preferred embodiments, the aryl group is ($C_{5-20}$) aryl, with ($C_{5-10}$) being particularly preferred. Particularly preferred aryl groups are phenyl and naphthyl groups.

"Arylalkyl" refers to an acyclic alkyl group in which one of the hydrogen atoms bonded to a carbon atom, typically a terminal carbon atom, is replaced with an aryl radical. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the Like. Where specific alkyl moieties are intended, the nomenclature arylalkanyl, arylakenyl and/or arylalkynyl is used. In preferred embodiments, the arylalkyl group is ($C_{6-26}$) arylalkyl, *e.g.*, the alkanyl, alkenyl or alkynyl moiety of the arylalkyl group is ($C_{1-6}$) and the aryl moiety is ($C_{5-20}$). In particularly preferred embodiments the arylalkyl group is ($C_{6-13}$), *e.g.*, the alkanyl, alkenyl or alkynyl moiety of the arylalkyl group is ($C_{1-3}$) and the aryl moiety is ($C_{5-10}$). Even more preferred arylalkyl groups are phenylalkanyls.

"Alkanyloxy" refers to a saturated branched, straight-chain or cyclic monovalent hydrocarbon alcohol radical derived by the removal of the hydrogen atom from the hydroxide oxygen of the alcohol. Typical alkanyloxy groups include, but are not limited to, methanyloxy; ethanyloxy; propanyloxy groups such as propan-1-yloxy ($CH_3CH_2CH_2O$-), propan-2-yloxy ($(CH_3)_2CHO$-), cyclopropan-1-yloxy, etc.; butanyloxy groups such as butan-1-yloxy, butan-2-yloxy, 2-methyl-propan-1-yloxy, 2-methyl-propan-2-yloxy, cyclobutan-1-yloxy, etc.; and the like. In preferred embodiments, the alkanyloxy groups are ($C_{1-8}$) alkanyloxy groups, with ($C_{1-3}$) being particularly preferred.

"Heteroaryl" refers to a monovalent heteroaromatic radical derived by the removal of one hydrogen atom from a single atom of a parent heteroaromatic ring system. Typical heteroaryl groups include, but are not limited to, radicais derived from carbazole, imidazole, indazole, indole, indoline, indolizine, isoindole, isoindoline, isoquinoline, isothiazole, isoxazole, naphthyridine, oxadiazole, oxazole, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like. In preferred embodiments, the heteroaryl group is a 5-20 membered heteroaryl, with 5-10 membered heteroaryl being particularly preferred.

"Cycloheteroalkyl" refers to a saturated or unsaturated monocyclic or bicyclic alkyl radical in which one carbon atom is replaced with N, O or S. In certain specified embodiments the cycloheteroalkyl may contain up to four heteroatoms independently selected from the group consisting of N, O and S. Typical cycloheteroalkyl moieties include, but are not limited to, radicals derived from imidazolidine, morpholine, piperazine, piperidine, pyrazolidine, pyrrolidine, quinuclidine, and the like. In preferred embodiments, the cycloheteroalkyl is a 3-6 membered cycloheteroalkyl.

"Cycloheteroalkanyl" refers to a saturated monocyclic or bicyclic alkanyl radical in which one carbon atom is replaced with N, O or S. In certain specified embodiments the cycloheteroalkanyl may contain up to four heteroatoms independently selected from the group consisting of N, O and S. Typical cycloheteroalkanyl moieties include, but are not limited to, radicals derived from imidazolidine, morpholine, piperazine, piperidine, pyrazolidine, pyrrolidine, quinuclidine, and the like. In preferred embodiments, the cycloheteroalkanyl is a 3-6 membered cycloheteroalkanyl.

"Cycloheteroalkenyl" refers to a saturated monocyclic or bicyclic alkenyl radical in which one carbon atom is replaced with N, O or S. In certain specified embodiments the cycloheteroalkenyl may contain up to four heteroatoms independently selected from the group consisting of N, O and S. Typical cycloheteroalkenyl moieties include, but are not limited to, radicals derived from imidazoline, pyrazoline, pyrroline, indoline, pyran, and the like. In preferred embodiments, the cycloheteroalkanyl is a 3-6 membered cycloheteroalkanyl.

The term "substituted" refers to a radical in which one or more hydrogen atoms are each independently replaced with the same or different substituent(s). Typical substituents include, but are not limited to, -X, -R, $-O^-$, =O -OR, -O-OR, -SR, $-S^-$, =S, -NRR, =NR, $-CX_3$, -CN, -OCN, -SCN, -NCO, -NCS, -NO, $-NO_2$, $=N_2$, $-N_3$, -NHOH, $-S(O)_2O^-$, $-S(O)_2OH$, $-S(O)_2R$, $-P(O)(O^-)_2$, $-P(O)(OH)_2$, -C(O)R, -C(O)X, -C(S)R, -C(S)X, -C(O)OR, $-C(O)O^-$, -C(S)OR, -C(O)SR, -C(S)SR, -C(O)NRR, -C(S)NRR and -C(NR)NRR, where each X is independently a halogen (preferably -F, -Cl or -Br) and each R is independently -H, alkyl, alkanyl, alkenyl, alkynyl, alkylidene, alkylidyne, aryl, arylalkyl, arylheteroalkyl, heteroaryl, heteroarylalkyl or heteroaryl-heteroalkyl, as defined herein. Preferred substituents include hydroxy, halogen, $C_{1-8}$alkyl, $C_{1-8}$alkanyloxy, fluorinated alkanyloxy, fluorinated alkyl, $C_{1-8}$alkylthio, $C_{3-8}$cycloalkyl, $C_{3-8}$cycloalkanyloxy, nitro, amino, $C_{1-8}$alkylamino, $C_{1-8}$dialkylamino, $C_{3-8}$cycloalkylamino, cyano, carboxy, $C_{1-7}$alkanyloxycarbonyl, $C_{1-7}$alkylcarbonyloxy, formyl, carbamoyl, phenyl, aroyl, carbamoyl, amidino, $C_{1-8}$alkylamino)carbonyl, (arylamino)carbonyl and aryl($C_{1-8}$alkyl) carbonyl.

With reference to substituents, the term "independently" means that when more than one of such substituent is possible, such substituents may be the same or different from each other.

In any structure that contains the symbol 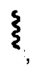, that symbol designates the location(s) of the open valence(s) where the partial structure attaches to the rest of the molecule.

Throughout this disclosure, the terminal portion of the designated side chain is described first, followed by the adjacent functionality toward the point of attachment. Thus, for example, a "phenyl$C_{1-6}$alkanylaminocarbonyl$C_{1-6}$alkyl" substituent refers to a group of the formula:

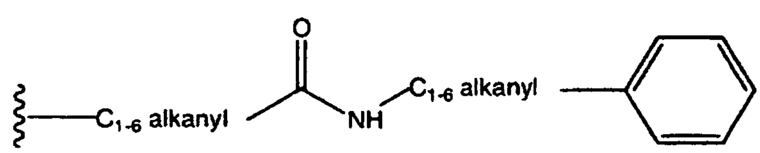

The present invention is directed to compositions comprising a compound of Formula (I) for uses as PPAR delta agonists:
wherein:

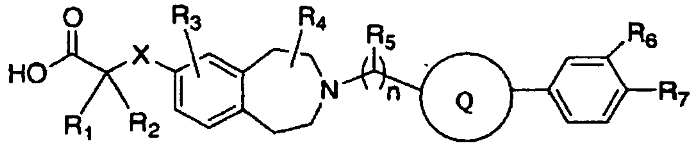

**Formula (I)**

wherein:

| | |
|---|---|
| X | is a covalent bond, O, or S; |
| $R_1$ | and $R_2$ are independently selected from the group consisting of H, $C_{1-8}$alkyl, and substituted $C_{1-8}$alkyl, or $R_1$, $R_2$ and the carbon atom to which they are attached together may form $C_{3-7}$cycloalkyl; |
| $R_3$ is | H; |
| $R_4$ | and $R_5$ are independently selected from the group consisting of H, halo, $C_{1-8}$alkyl, $C_{3-7}$cycloalkyl, $C_{3-7}$cycloalkyl-$C_{1-4}$alkyl, $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{6-10}$aryl substituted $C_{1-4}$alkyl, cyano substituted $C_{1-4}$alkyl, and hydroxy substituted $C_{1-4}$alkyl; |
| $R_6$ and $R_7$ | are independently selected from the group consisting of H, halo, $C_{1-3}$alkyl, halo substituted $C_{1-3}$alkyl, $C_{1-3}$alkoxy, and halo substituted $C_{1-3}$alkoxy; |
| n | is 1; and |
| Q | is selected from the group consisting of |

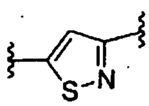 , 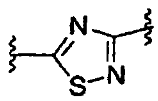 , 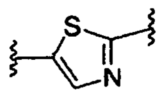 , 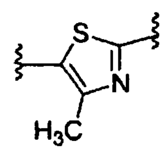 ,

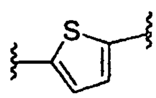 , 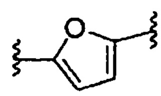 , 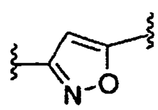 , 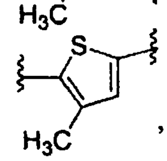 ,

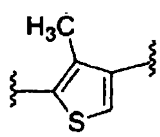 , 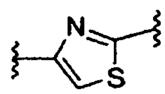 , 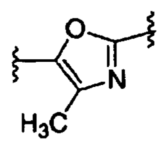 , 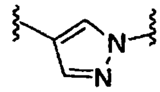 ,

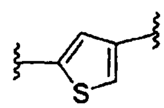 , 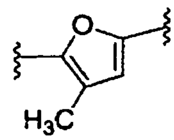 , and 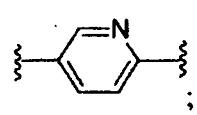 ;

and enantiomers, diastereomers, tautomers, solvates, or pharmaceutically acceptable salts thereof.

In particular, the present invention is directed to a compound of Formula (I) wherein:

X is O; or

$R_1$ and $R_2$ are independently selected from the group consisting of H and $C_{1-8}$alkyl, or $R_1$, $R_2$ and the carbon atom to which they are attached together may form $C_{3-5}$cycloalkyl, and more particularly $R_1$ and $R_2$ are independently selected from the group consisting of H and $CH_3$, or $R_1$, $R_2$ and the carbon atom to which they are attached together may form

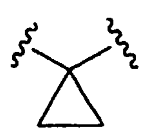 ;

or

$R_4$ and $R_5$ are independently selected from the group consisting of H and $C_{1-8}$alkyl, and more particularly $R_5$ is H,

$CH_3$, or $-CH_2CH_3$; or

$R_6$ and $R_7$ are independently selected from the group consisting of H, halo, halo substituted $C_{1-3}$alkyl, $C_{1-3}$alkoxy, and halo substituted $C_{1-3}$alkoxy, and more particularly $R_6$ is H and $R_7$ is selected from the group consisting of halo, halo substituted $C_{1-3}$alkyl, and halo substituted $C_{1-3}$alkoxy, and more particularly $R_7$ is selected from the group consisting of F, $CF_3$, and $-O-CF_3$; or

Q is selected from the group consisting of

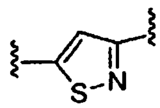, 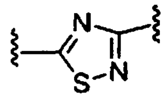, 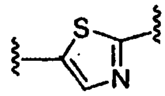, 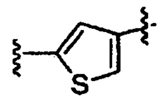,

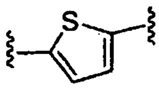, 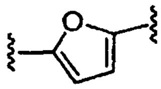, 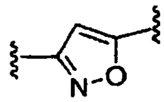, 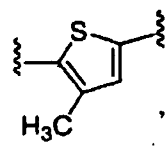,

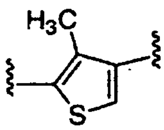, 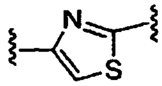, 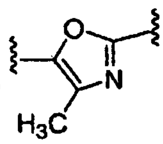

and more particularly Q is selected from the group consisting of

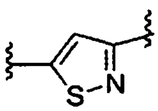, 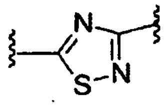, 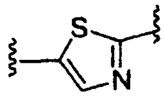, 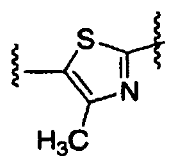,

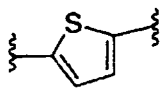, 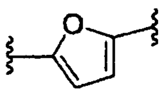, 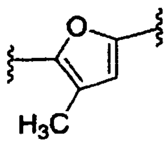 and 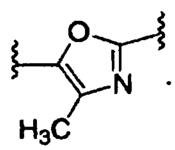.

More particularly, the present invention is directed to a compound of Formula (I) as shown above wherein:

X is O;

| | |
|---|---|
| $R_1$, $R_2$, $R_4$, and $R_5$ | are independently selected from the group consisting of H and $C_{1-3}$alkyl, or $R_1$, $R_2$ and the carbon atom to which they are attached together may form $C_{3-5}$cycloalkyl; and |
| $R_6$ and $R_7$ | are independently selected from the group consisting of H, halo, $C_{1-3}$alkoxy, halo substituted $C_{1-3}$alkyl, and halo substituted $C_{1-3}$alkoxy; |

and enantiomers, diastereomers, tautomers, solvates, or pharmaceutically acceptable salts thereof.

In another aspect, the present invention is directed to pharmaceutical compositions containing one or more compounds, salts or solvates of Formula (I) as described herein admixed with a pharmaceutically acceptable carrier, excipient or diluent, wherein the compositions can be used to treat a condition directly or indirectly mediated by PPAR delta.

The present invention is also directed to compounds for use in a method of treating or preventing a disease or condition in a subject, particularly a mammal and more particularly a human, which disease or condition is affected

by the modulation of PPAR delta.

Therefore, in yet another aspect, the present invention is directed to compounds of formula ID a method of treating or preventing a disease or condition in a mammal which disease or condition is affected by the modulation of PPAR delta receptors, which method comprises administering to a mammal in need of such treatment or prevention a therapeutically effective amount of a compound, salt or solvate of Formula (I) as described herein. More particularly, the therapeutically effective amount comprises a dose range of from about 0.1 mg to about 15,000 mg. More particularly, the therapeutically effective amount comprises a dose range of from about 50 mg to about 1000 mg. More particularly, the therapeutically effective amount comprises a dose range of from about 100 mg to about 1000 mg.

In a further aspect, the present invention is directed to compounds for use in a method for treating or preventing a disease or condition selected from the group consisting of diabetes, nephropathy, neuropathy, retinopathy, polycystic ovary syndrome, hypertension, ischemia, stroke, irritable bowel disorder, inflammation, cataract, cardiovascular diseases, Metabolic X Syndrome, hyper-LDL-cholesterolemia, dyslipidemia (including hypertriglyceridemia, hypercholesterolemia, mixed hyperlipidemia, and hypo-HDL-cholesterolemia), atherosclerosis, and obesity, said method comprising the step of administering to a mammal in need of such treatment a therapeutically effective amount of a compound, salt or solvate of Formula (I). More particularly, the therapeutically effective amount comprises a dose range of from about 0.1 mg to about 15,000 mg. More particularly, the therapeutically effective amount comprises a dose range of from about 50 mg to about 1000 mg. More particularly, the therapeutically effective amount comprises a dose range of from about 100 mg to about 1000 mg.

In still a further aspect, the present invention is directed to a kit comprising in one or more containers an amount of the composition of Formula (I) effective to treat or prevent a disease or condition selected from the group consisting of diabetes, nephropathy, neuropathy, retinopathy, polycystic ovary syndrome, hypertension, ischemia, stroke, irritable bowel disorder, inflammation, cataract, cardiovascular diseases, Metabolic X Syndrome, hyper-LDL-cholesterolemia, dyslipidemia (including hypertriglyceridemia, hypercholesterolemia, mixed hyperlipidemia, and hypo-HDL-cholesterolemia), atherosclerosis, and obesity. More particularly, the therapeutically effective amount comprises a dose range of from about 0.1 mg to about 15,000 mg. More particularly, the therapeutically effective amount comprises a dose range of from about 50 mg to about 1000 mg. More particularly, the therapeutically effective amount comprises a dose range of from about 100 mg to about 1000 mg.

In another embodiment, the present invention is directed to a compound of Formula (Ia)

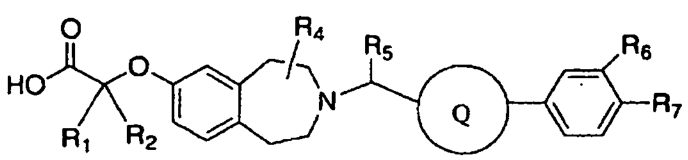

**Formula (Ia)**

wherein

| | |
|---|---|
| $R_1$ and $R_2$ | are independently selected from the group consisting of H and $C_{1-8}$alkyl, or $R_1$, $R_2$ and the carbon atom to which they are attached together may form $C_{3-5}$cycloalkyl; |
| $R_4$ and $R_5$ | are independently selected from the group consisting of H and $C_{1-8}$alkyl; |
| $R_6$ and $R_7$ | are independently selected from the group consisting of H, $C_{1-3}$alkyl, halo, and halo substituted $C_{1-3}$alkyl; and |
| Q | is selected from the group consisting of |

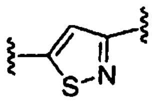, 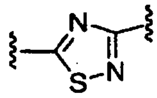, 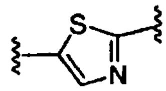, 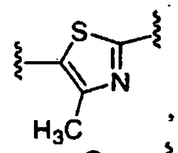,

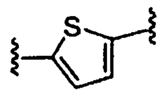, 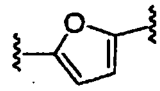, 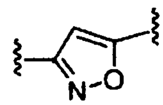, 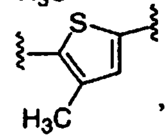,

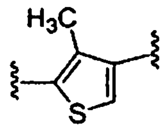, 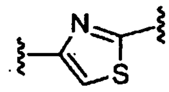, 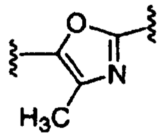, 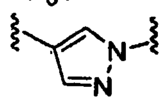,

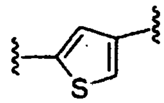, 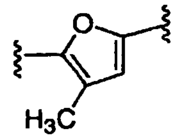, and 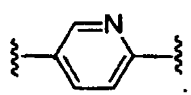;

and enantiomers, diastereomers, tautomers, solvates, or pharmaceutically acceptable salts thereof.

In yet another embodiment, the present invention is directed to a compound of Formula (Ib)

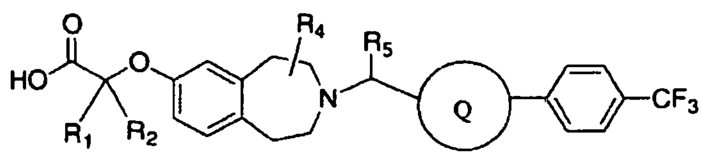

**Formula (Ib)**

wherein

$R_1$ and $R_2$ are independently selected from the group consisting of H and $CH_3$, or $R_1$, $R_2$ and the carbon atom to which they are attached together may form

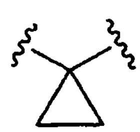;

$R_4$ and $R_5$ are independently selected from the group consisting of H, $CH_3$, and $-CH_2CH_3$; and

Q is selected from the group consisting of

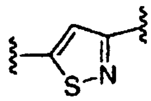, 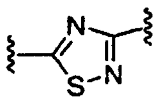, 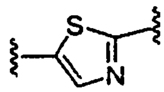, 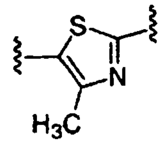, 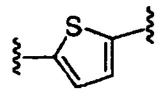, 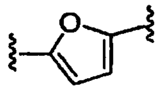, 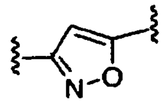, 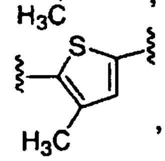, 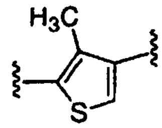, 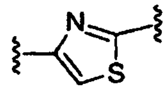, 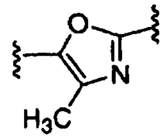, 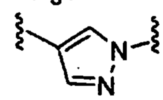, 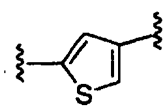, 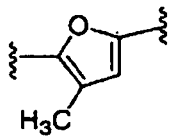, and 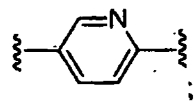;

and enantiomers, diastereomers, tautomers, solvates, or pharmaceutically acceptable salts thereof.

In yet another embodiment, the present invention is directed to a compound of Formula (Ic)

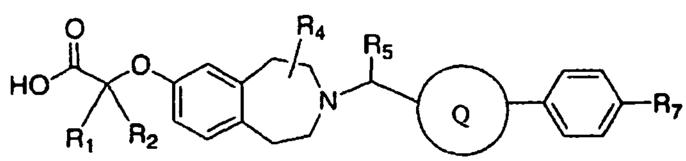

**Formula (Ic)**

$R_1$, $R_2$, and $R_4$, are independently selected from the group consisting of H and $CH_3$, or $R_1$, $R_2$ and the carbon atom to which they are attached together may form

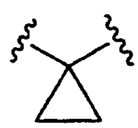;

$R_5$ is selected from the group consisting of H, $CH_3$, and $-CH_2CH_3$;

$R_7$ is halo or halo substituted $C_{1-3}$alkyl; and

Q is selected from the group consisting of

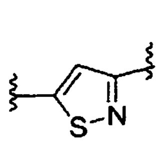, 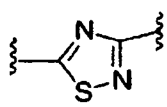, 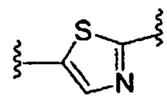, 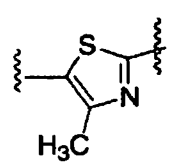, 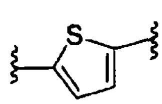, 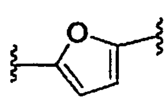, 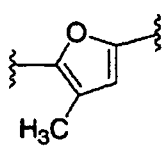 and 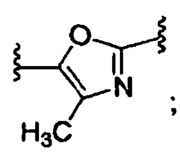;

and enantiomers, diastereomers, tautomers, solvates, or pharmaceutically acceptable salts thereof.

In particular, the present invention is directed to compounds of Formula (Ic) hereinabove wherein:

(a) $R_1$ or $R_2$ is H;
(b) $R_1$ and $R_2$ are both H;
(c) $R_1$ or $R_2$ is $CH_3$;
(d) $R_1$ and $R_2$ are both $CH_3$;
(e) $R_1$, $R_2$ and the carbon atom to which they are attached together form

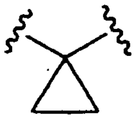

(f) $R_4$ is H or $CH_3$;
(g) $R_5$ is H, $CH_3$, or $-CH_2CH_3$;
(h) $R_1$ or $R_2$ is H and $R_4$ is H or $CH_3$;
(i) $R_1$ and $R_2$ are both H and $R_4$ is H or $CH_3$;
(j) $R_1$ or $R_2$ is H and $R_5$ is H, $CH_3$, or $-CH_2CH_3$;
(k) $R_1$ and $R_2$ are both H and $R_5$ is H, $CH_3$, or $-CH_2CH_3$;
(l) $R_1$ or $R_2$ is $CH_3$ and $R_5$ is H, $CH_3$, or $-CH_2CH_3$;
(m) $R_1$ and $R_2$ are both $CH_3$ and $R_5$ is H, $CH_3$, or $-CH_2CH_3$;
(n) $R_1$ or $R_2$ is H, $R_4$ is H, and $R_5$ is H, $CH_3$, or $-CH_2CH_3$;
(o) $R_1$ and $R_2$ are both H, $R_4$ is H, and $R_5$ is H, $CH_3$, or $-CH_2CH_3$;
(p) $R_1$ or $R_2$ is $CH_3$, $R_4$ is H, and $R_5$ is H, $CH_3$, or $-CH_2CH_3$;
(q) $R_1$ and $R_2$ are both $CH_3$, $R_4$ is H, and $R_5$ is H, $CH_3$, or $-CH_2CH_3$;
(r) $R_1$, $R_2$ and the carbon atom to which they are attached together form

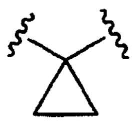,

$R_4$ is H, and $R_5$ is H, $CH_3$, or $-CH_2CH_3$;
(s) $R_7$ is $CF_3$;
(t) $R_7$ is Cl;
(u) $R_1$ or $R_2$ is H, $R_7$ is $CF_3$, and $R_4$ is H or $CH_3$;
(v) $R_1$ and $R_2$ are both H, $R_7$ is $CF_3$, and $R_4$ is H or $CH_3$;
(w) $R_1$ or $R_2$ is H, $R_7$ is $CF_3$, and $R_5$ is H, $CH_3$, or $-CH_2CH_3$;
(x) $R_1$ and $R_2$ are both H, $R_7$ is $CF_3$, and $R_5$ is H, $CH_3$, or $-CH_2CH_3$;
(y) $R_1$ or $R_2$ is $CH_3$, $R_7$ is $CF_3$, and $R_5$ is H, $CH_3$, or $-CH_2CH_3$;
(z) $R_1$ and $R_2$ are both $CH_3$, $R_7$ is $CF_3$, and $R_5$ is H, $CH_3$, or $-CH_2CH_3$;
(aa) $R_1$ or $R_2$ is H, $R_4$ is H, $R_7$ is $CF_3$, and $R_5$ is H, $CH_3$, or $-CH_2CH_3$;
(bb) $R_1$ and $R_2$ are both H, $R_4$ is H, $R_7$ is $CF_3$, and $R_5$ is H, $CH_3$, or - $CH_2CH_3$;
(cc) $R_1$ or $R_2$ is $CH_3$, $R_4$ is H, $R_7$ is $CF_3$, and $R_5$ is H, $CH_3$, or $-CH_2CH_3$;
(dd) $R_1$ and $R_2$ are both $CH_3$, $R_4$ is H, $R_7$ is $CF_3$, and $R_5$ is H, $CH_3$, or - $CH_2CH_3$;
(ee) $R_1$, $R_2$ and the carbon atom to which they are attached together form

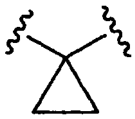,

$R_4$ is H, $R_7$ is $CF_3$, and $R_5$ is H, $CH_3$, or $-CH_2CH_3$;
(ff) Q is selected from the group consisting of

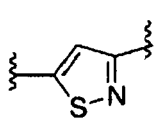 , 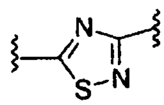 , 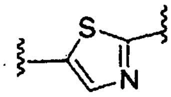 , 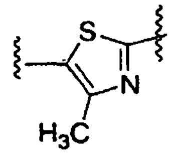 ,

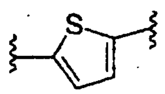 , 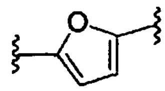 , 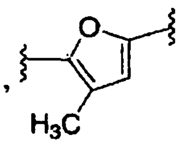 and 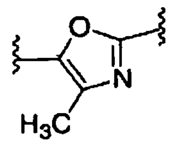 ;

(gg) $R_1$ or $R_2$ is H and Q is selected from the group consisting of

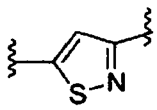 , 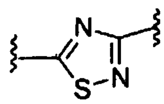 , 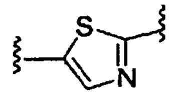 , 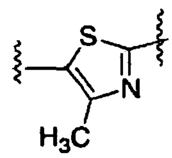 ,

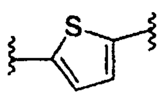 , 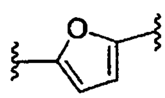 , 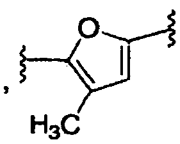 and 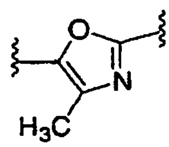 ;

(hh) $R_1$ and $R_2$ are both H and Q is selected from the group consisting of

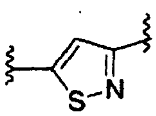 , 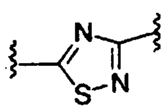 , 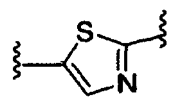 , 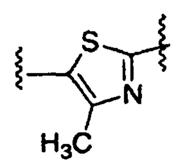 ,

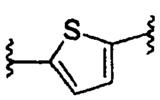 , 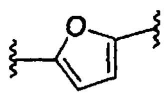 , 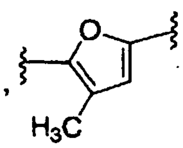 and 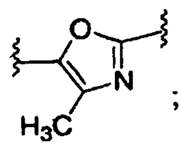 ;

(ii) $R_1$ or $R_2$ is $CH_3$ and Q is selected from the group consisting of

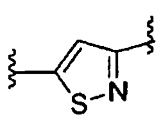 , 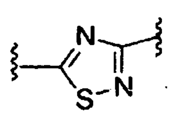 , 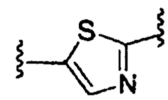 , 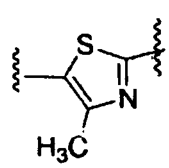 ,

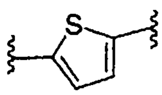, 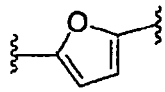, 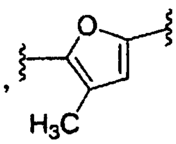 and 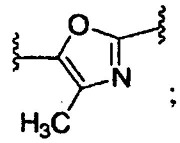;

<u>(jj)</u> $R_1$ and $R_2$ are both $CH_3$ and Q is selected from the group consisting of

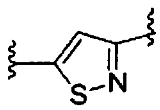, 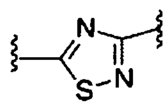, 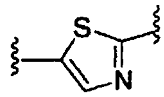, 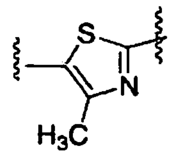,

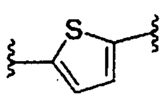, 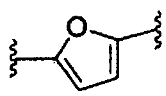, 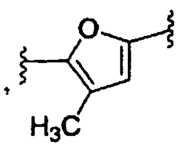 and 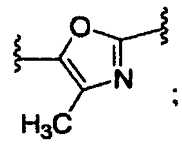;

<u>(kk)</u> $R_4$ is H or $CH_3$ and Q is selected from the group consisting of

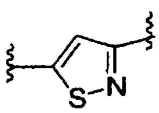, 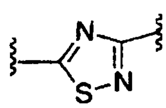, 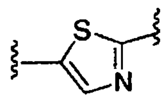, 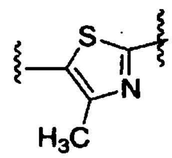,

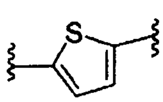, 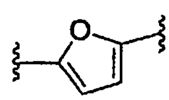, 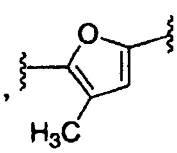 and 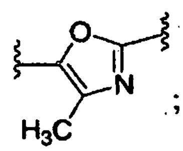;

<u>(ll)</u> $R_5$ is H, $CH_3$, or $-CH_2CH_3$ and Q is selected from the group consisting of

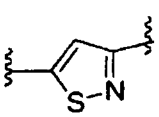, 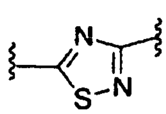, 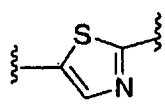, 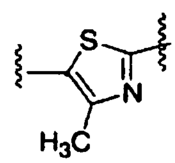,

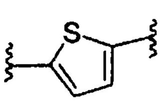, 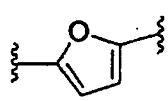, 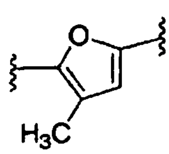 and 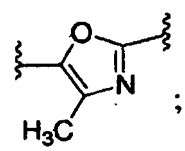;

<u>(mm)</u> $R_1$ or $R_2$ is H and $R_4$ is H or $CH_3$ and Q is selected from the group consisting of

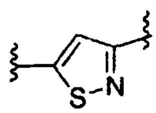, 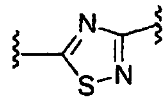, 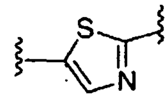, 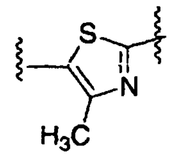,

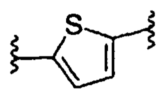, 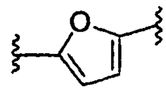, 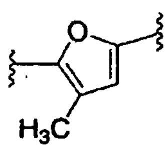 and 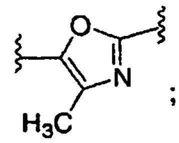;

(nn) $R_1$ and $R_2$ are both H and $R_4$ is H or $CH_3$ and Q is selected from the group consisting of

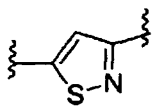, 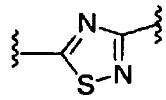, 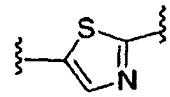, 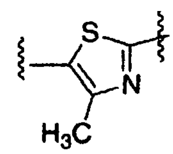,

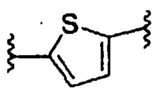, 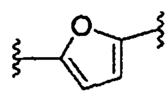, 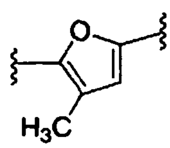 and 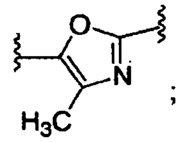;

(oo) $R_1$ or $R_2$ is H and $R_5$ is H, $CH_3$, or $-CH_2CH_3$ and Q is selected from the group consisting of

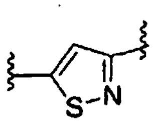, 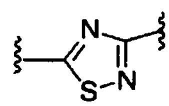, 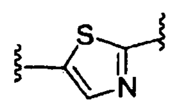, 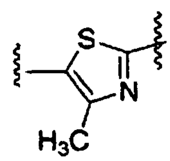,

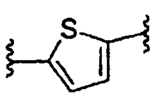, 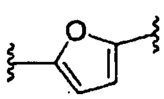, 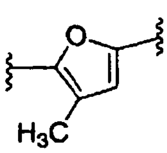 and 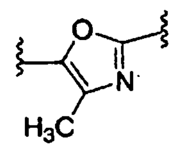;

(pp) $R_1$ and $R_2$ are both H and $R_5$ is H, $CH_3$, or $-CH_2CH_3$ and Q is selected from the group consisting of

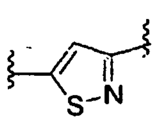, 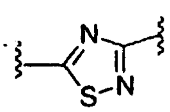, 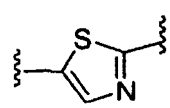, 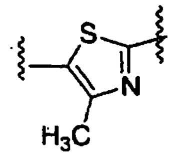,

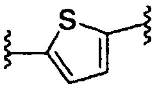, 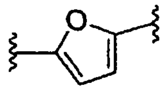, 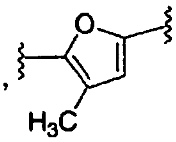 and 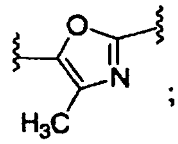 ;

(gg) $R_1$ or $R_2$ is $CH_3$ and $R_5$ is H, $CH_3$, or $-CH_2CH_3$ and Q is selected from the group consisting of

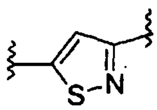, 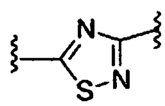, 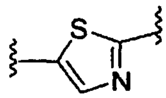, 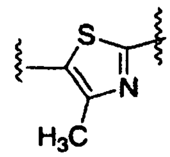,

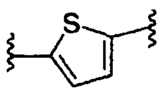, 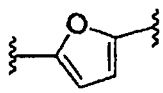, 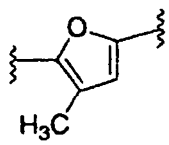 and 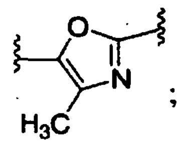 ;

(rr) $R_1$ and $R_2$ are both $CH_3$, $R_5$ is H, $CH_3$, or $-CH_2CH_3$, and Q is selected from the group consisting of

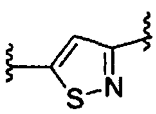, 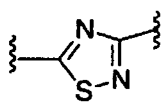, 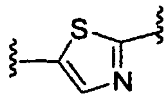, 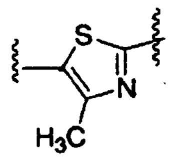,

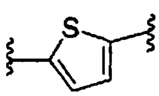, 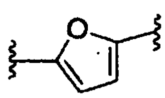, 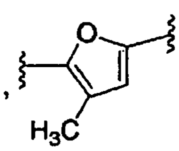 and 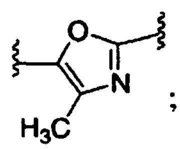 ;

(ss) $R_1$ or $R_2$ is H, $R_4$ is H, $R_5$ is H, $CH_3$, or $-CH_2CH_3$, and Q is selected from the group consisting of

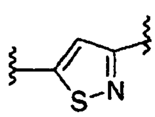, 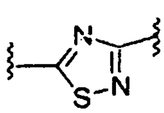, 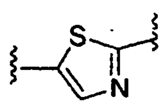, 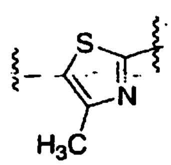,

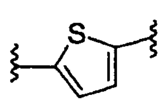, 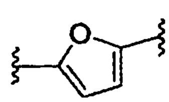, 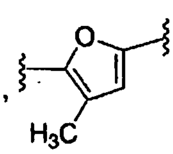 and 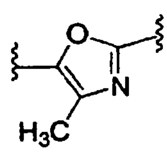 ;

(tt) $R_1$ and $R_2$ are both H, $R_4$ is H, $R_5$ is H, $CH_3$, or $-CH_2CH_3$, and Q is selected from the group consisting of

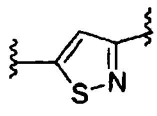, 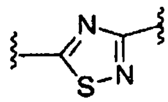, 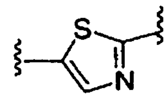, 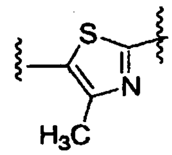,

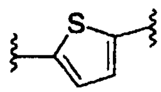, 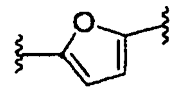, 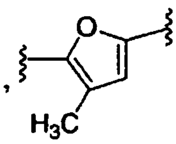 and 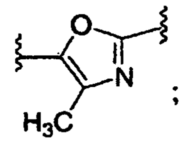;

(uu) $R_1$ or $R_2$ is $CH_3$, $R_4$ is H, $R_5$ is H, $CH_3$, or $-CH_2CH_3$, and Q is selected from the group consisting of

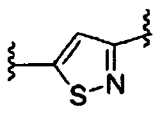, 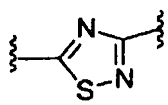, 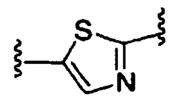, 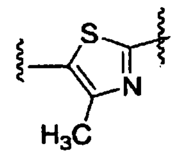,

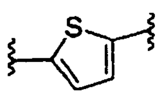, 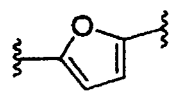, 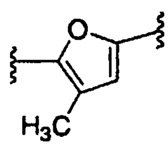 and 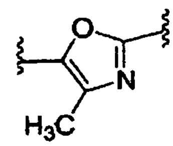;

(vv) $R_1$ and $R_2$ are both $CH_3$, $R_4$ is H, $R_5$ is H, $CH_3$, or $-CH_2CH_3$, and Q is selected from the group consisting of

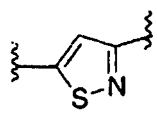, 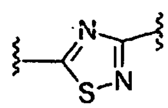, 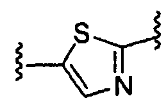, 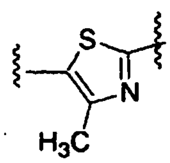,

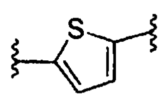, 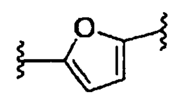, 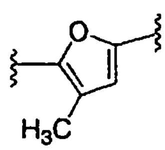 and 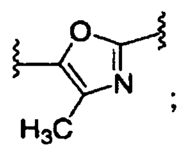;

(ww) $R_1$, $R_2$ and the carbon atom to which they are attached together form

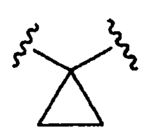,

$R_4$ is H, $R_5$ is H, $CH_3$, or $-CH_2CH_3$, and Q is selected from the group consisting of

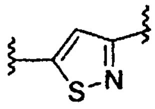 , 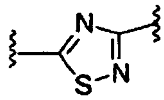 , 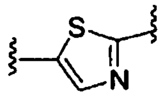 , 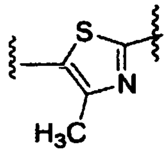 ,

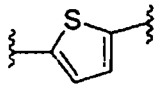 , 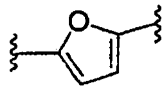 , 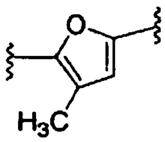 and 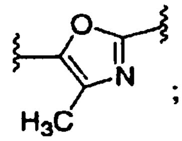 ;

$R_7$ is $CF_3$ and Q is selected from the group consisting of

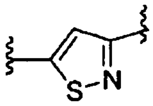 , 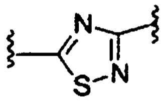 , 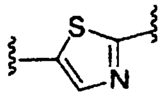 , 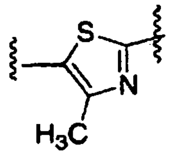 ,

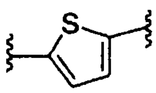 , 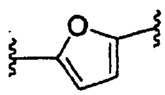 , 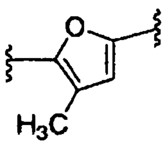 and 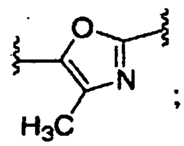 ;

(xx) $R_7$ is Cl and Q is selected from the group consisting of

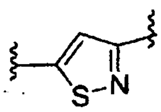 , 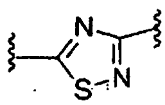 , 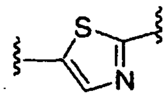 , 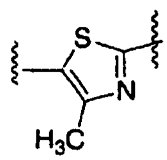 ,

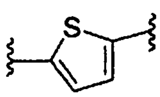 , 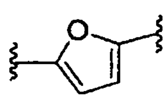 , 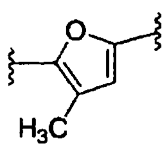 and 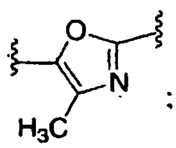 ;

(yy) $R_1$ or $R_2$ is H, $R_7$ is $CF_3$, $R_4$ is H or $CH_3$, and Q is selected from the group consisting of

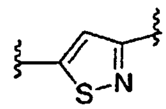, 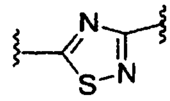, 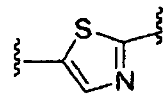, 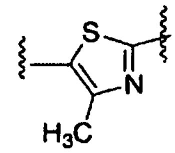,

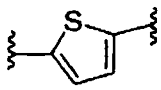, 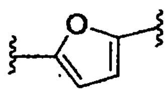, 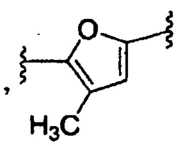 and 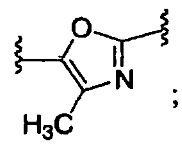;

(zz) $R_1$ and $R_2$ are both H, $R_7$ is $CF_3$, $R_4$ is H or $CH_3$, and Q is selected from the group consisting of

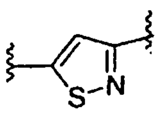, 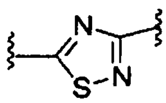, 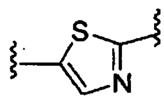, 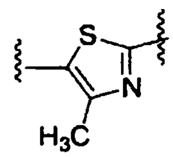,

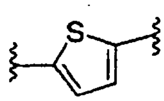, 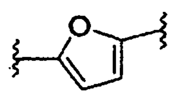, 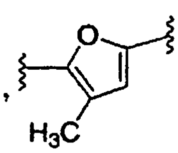 and 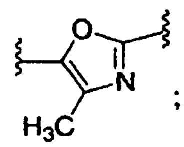;

(aaa) $R_1$ or $R_2$ is H, $R_7$ is $CF_3$, $R_5$ is H, $CH_3$, or $-CH_2CH_3$, and Q is selected from the group consisting of

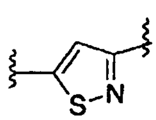, 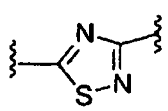, 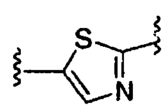, 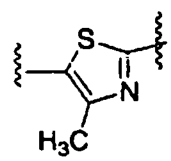,

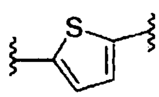, 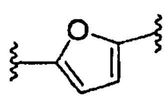, 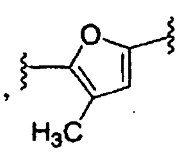 and 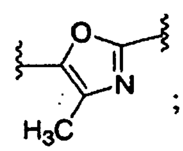;

(bbb) $R_1$ and $R_2$ are both H, $R_7$ is $CF_3$, $R_5$ is H, $CH_3$, or $-CH_2CH_3$, and Q is selected from the group consisting of

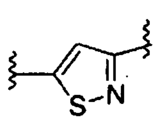, 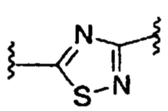, 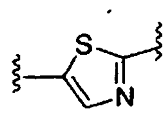, 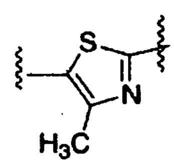,

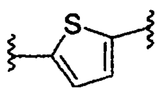, 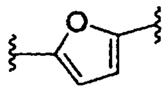, 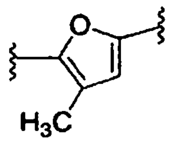 and 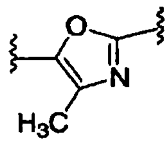;

(ccc) $R_1$ or $R_2$ is $CH_3$, $R_7$ is $CF_3$, $R_5$ is H, $CH_3$, or $-CH_2CH_3$, and Q is selected from the group consisting of

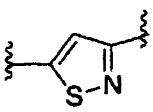, 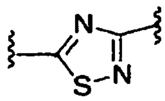, 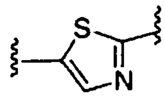, 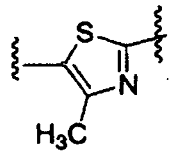,

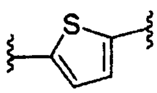, 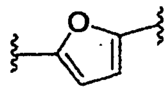, 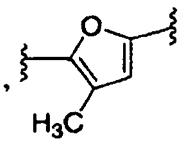 and 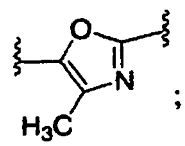;

(ddd) $R_1$ and $R_2$ are both $CH_3$, $R_7$ is $CF_3$, $R_5$ is H, $CH_3$, or $-CH_2CH_3$, and Q is selected from the group consisting of

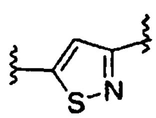, 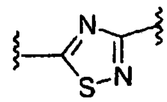, 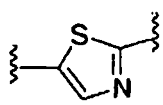, 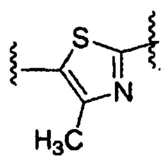,

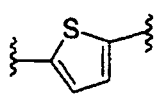, 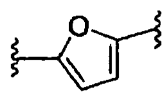, 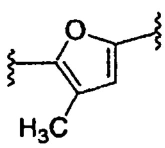 and 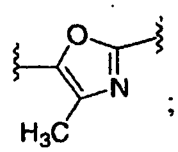;

$R_1$ or $R_2$ is H, $R_4$ is H, $R_7$ is $CF_3$, $R_5$ is H, $CH_3$, or $-CH_2CH_3$, and Q is selected from the group consisting of

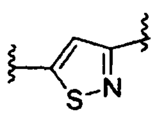, 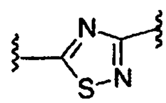, 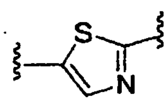, 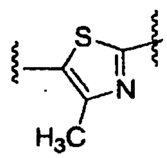,

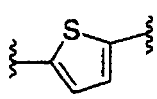, 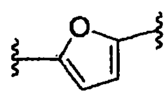, 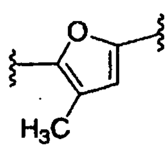 and 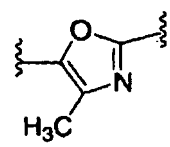;

(eee) $R_1$ and $R_2$ are both H, $R_4$ is H, $R_7$ is $CF_3$, $R_5$ is H, $CH_3$, or $-CH_2CH_3$, and Q is selected from the group consisting of

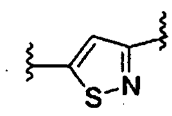, 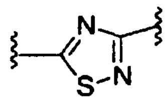, 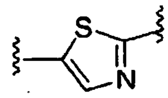, 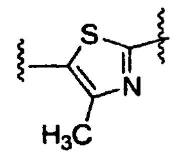,

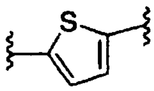, 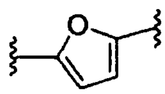, 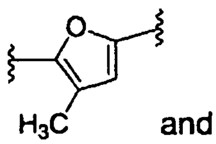 and 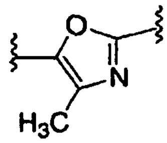;

(fff) $R_1$ or $R_2$ is $CH_3$, $R_4$ is H, $R_7$ is $CF_3$, $R_5$ is H, $CH_3$, or $-CH_2CH_3$, and Q is selected from the group consisting of

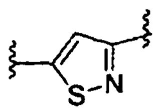, 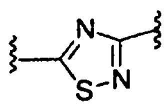, 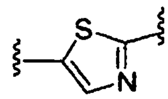, 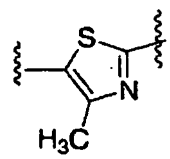,

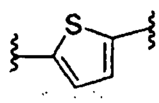, 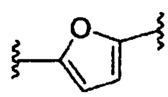, 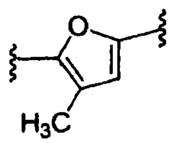 and 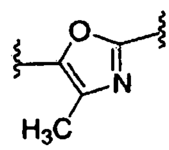;

or

(a) $R_1$ and $R_2$ are both $CH_3$, $R_4$ is H, $R_7$ is $CF_3$, $R_5$ is H, $CH_3$, or $-CH_2CH_3$, and Q is selected from the group consisting of

and enantiomers, diastereomers, tautomers, solvates, or pharmaceutically acceptable salts thereof.

In yet another embodiment, the present invention is directed to a compound selected from the group consisting of

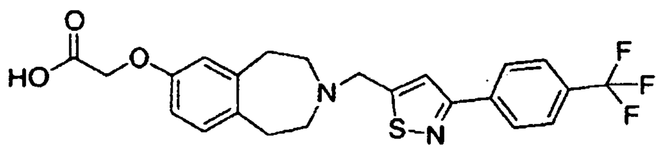;

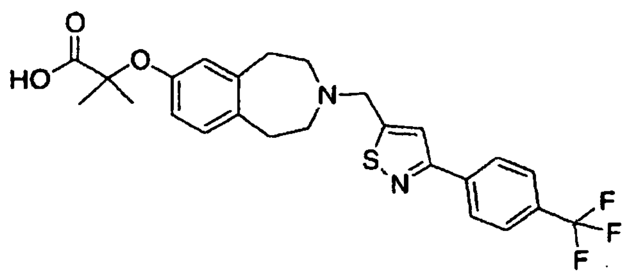;

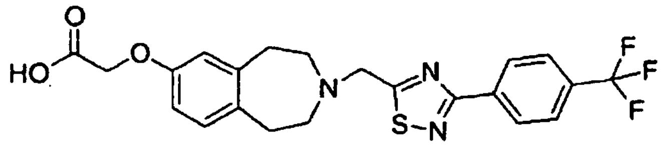

;

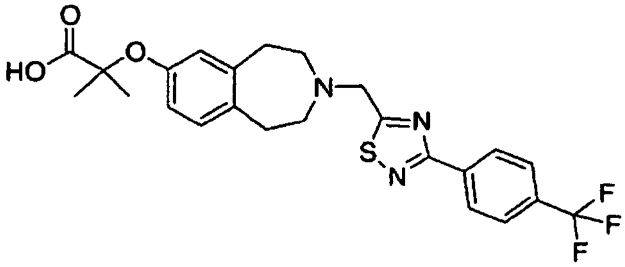

;

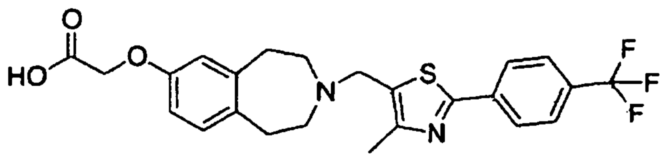

;

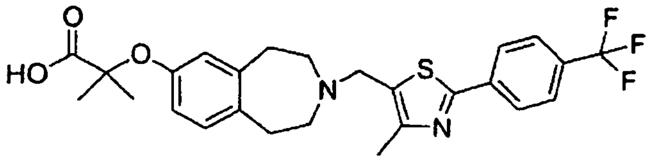

;

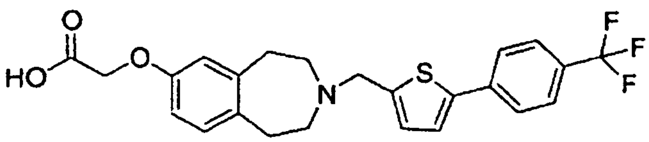

;

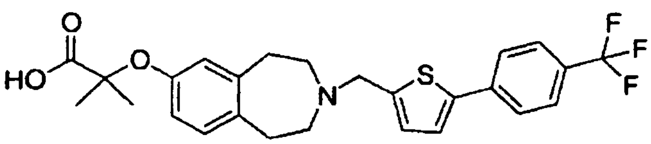

;

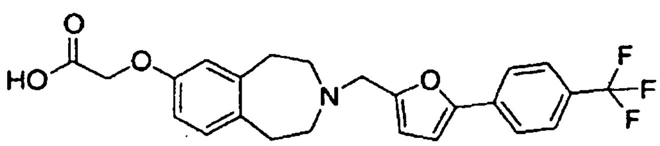

;

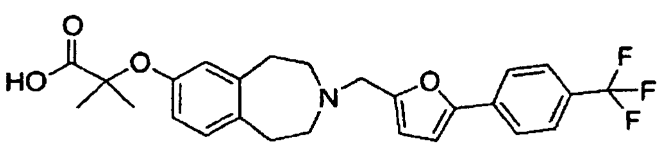

;

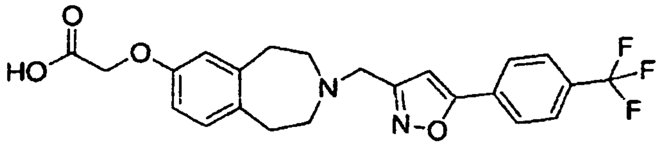

;

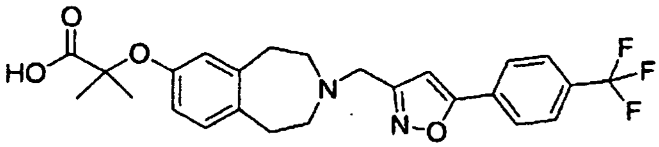

;

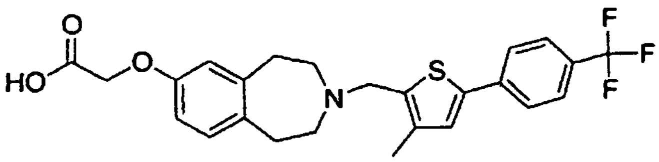

;

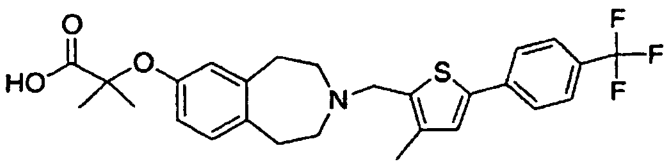

;

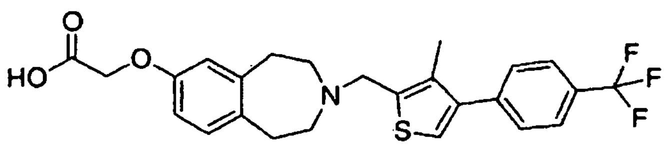

;

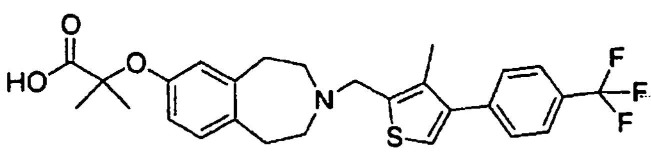

;

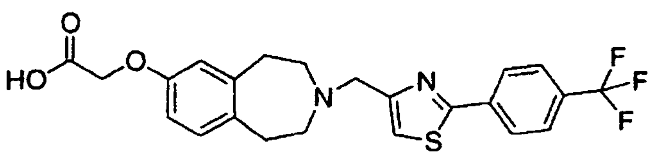

;

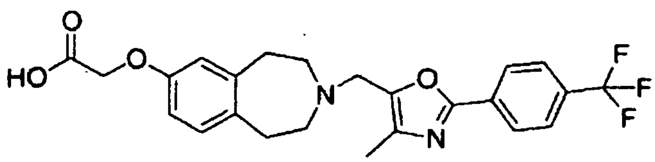

;

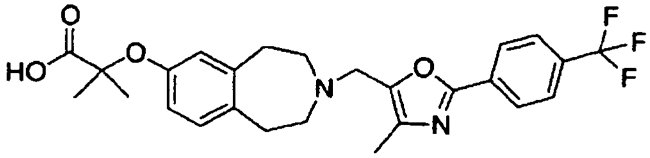
;

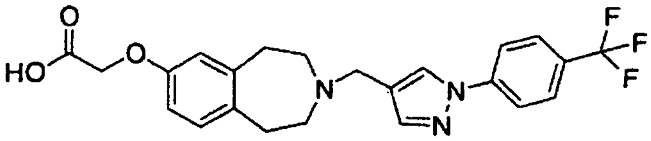
;

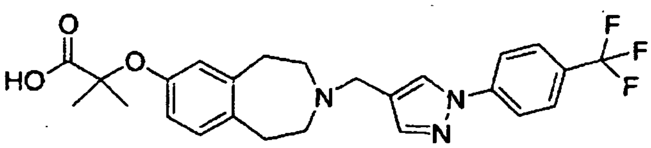
;

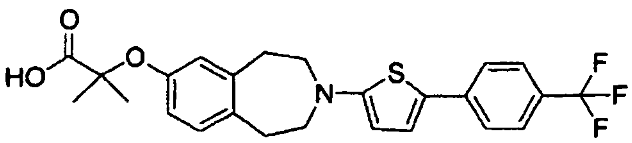
;

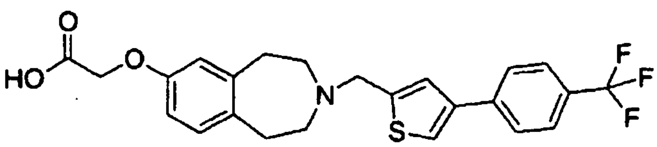
;

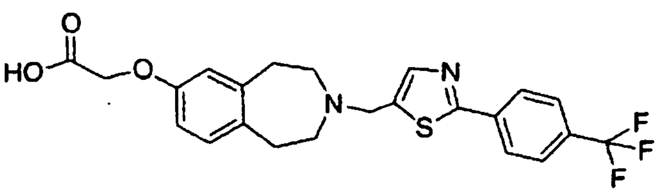
;

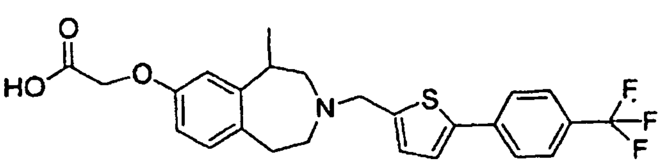
;

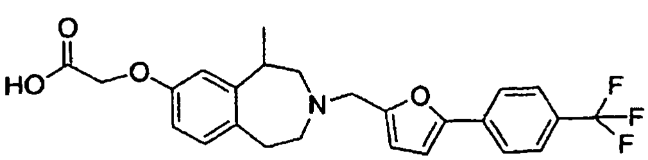
;

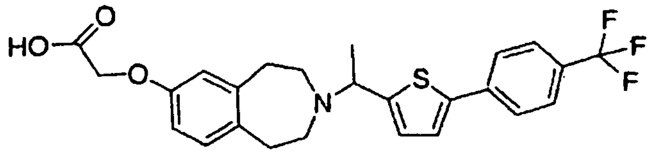
;

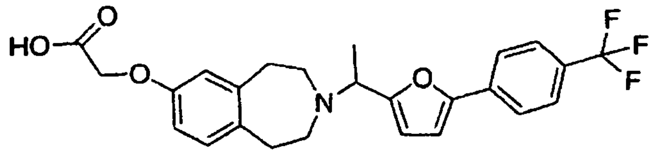
;

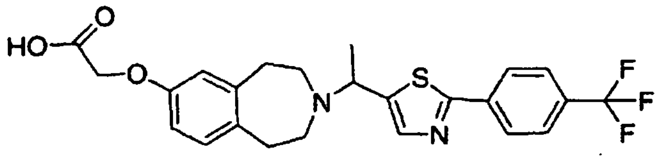
;

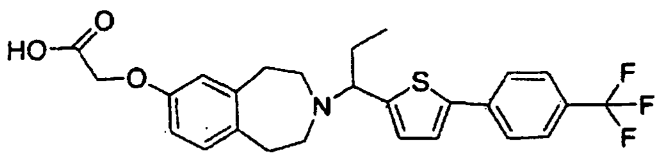
;

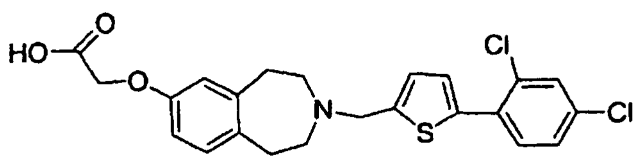
;

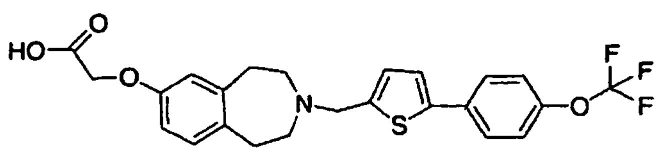
;

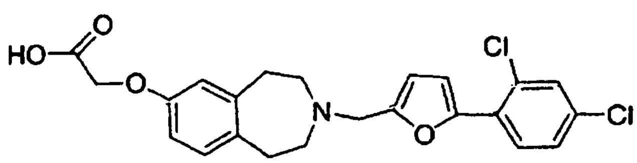
;

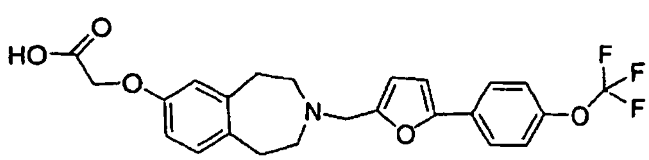
;

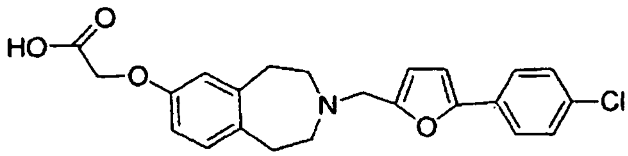
;

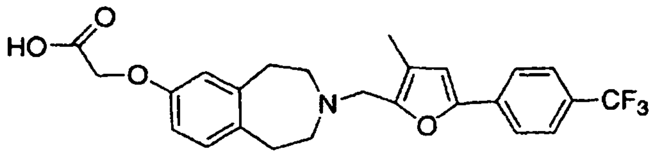
;

and

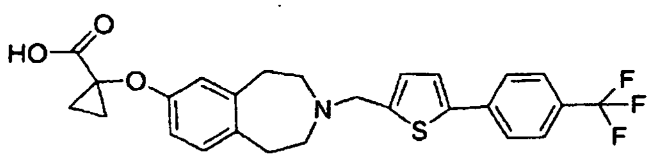
;

and enantiomers, diastereomers, tautomers, solvates, or pharmaceutically acceptable salts thereof. Specifically, the compound of formula (I) is

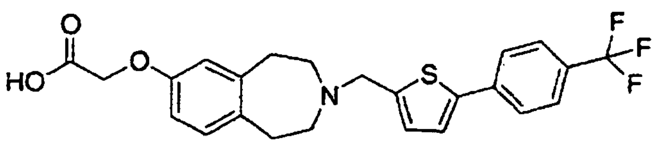
.

The present invention is further directed to a compound of Formula (II)

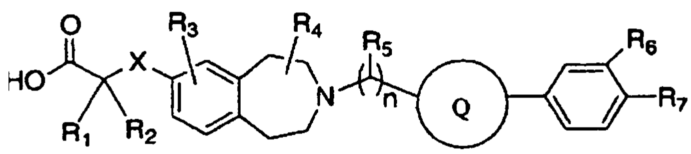

**Formula (II)**

wherein:

| | |
|---|---|
| X | is a covalent bond, O, or S; |
| $R_1$ and $R_2$ | are independently selected from the group consisting of H, $C_{1-8}$alkyl, and substituted $C_{1-8}$alkyl, or $R_1$, $R_2$ and the carbon atom to which they are attached together may form $C_{3-7}$cycloalkyl; |
| $R_3$ is | H; |
| $R_4$ and $R_5$ | are independently selected from the group consisting of H, halo, $C_{1-8}$alkyl, $C_{3-7}$cycloalkyl, $C_{3-7}$cycloalkyl$_{1-4}$alkyl, $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{6-10}$aryl substituted $C_{1-4}$alkyl, heteroaryl substituted $C_{1-4}$alkyl, cyano substituted $C_{1-4}$alkyl, and hydroxy substituted $C_{1-4}$alkyl; |
| $R_6$ and $R_7$ | are independently selected from the group consisting of H, halo, $C_{1-3}$alkyl, halo substituted $C_{1-3}$alkyl, |

$C_{1-3}$alkoxy, and halo substituted $C_{1-3}$alkoxy;

n is 1; and

Q is $C_{6-10}$aryl;

and enantiomers, diastereomers, tautomers, solvates, or pharmaceutically acceptable salts thereof.

The present invention is also directed to a compound of Formula (III)

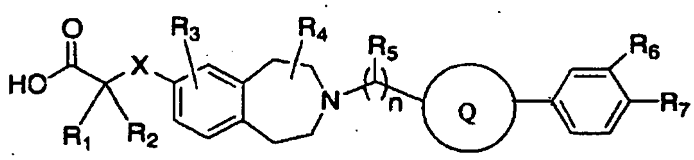

Formula (III)

wherein:

X is a covalent bond, O, or S;

$R_1$ and $R_2$ are independently selected from the group-consisting of H, $C_{1-8}$alkyl, and substituted $C_{1-8}$alkyl, or $R_1$, $R_2$ and the carbon atom to which they are attached together may form $C_{3-7}$cycloalkyl;

$R_3$ is H;

$R_4$ and $R_5$ are independently selected from the group consisting of H, halo, $C_{1-8}$alkyl, $C_{3-7}$cycloalkyl, $C_{3-7}$cycloalkyl-$C_{1-4}$alkyl, $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{6-10}$aryl substituted $C_{1-4}$alkyl, heteroaryl substituted $C_{1-4}$alkyl, cyano substituted $C_{1-4}$alkyl, and hydroxy substituted $C_{1-4}$alkyl;

$R_6$ and $R_7$ are independently selected from the group consisting of H, halo, $C_{1-3}$alkyl, halo substituted $C_{1-3}$alkyl, $C_{1-3}$alkoxy, and halo substituted $C_{1-3}$alkoxy;

n is 1 or 2; and

Q is selected from the group consisting of

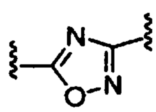 , 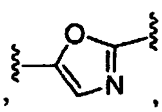 , 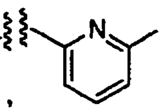 , 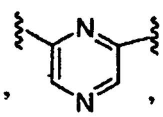 , and 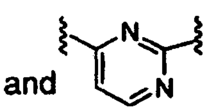 ;

and enantiomers, diastereomers, tautomers, solvates, or pharmaceutically acceptable salts thereof.

The present invention is also directed to a compound of Formula (IV)

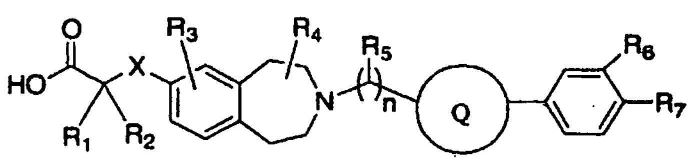

Formula (IV)

wherein:

X is a covalent bond, O or S;

$R_1$ and $R_2$ are independently selected from the group consisting of H, $C_{1-8}$alkyl, and substituted $C_{1-8}$alkyl, or $R_1$, $R_2$ and the carbon atom to which they are attached together may form $C_{3-7}$cycloalkyl;

$R_3$ is H;

$R_4$ and $R_5$ are independently selected from the group consisting of H, halo, $C_{1-8}$alkyl, $C_{3-7}$cycloalkyl, $C_{3-7}$cycloalkyl-

$C_{1-4}$alkyl $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{8-10}$aryl substituted $C_{1-4}$alkyl, cyano substituted $C_{1-4}$alkyl, and hydroxy substituted $C_{1-4}$alkyl;

$R_6$ and $R_7$ are independently selected from the group consisting of H, halo, $C_{1-3}$alkyl, halo substituted $C_{1-3}$alkyl, $C_{1-3}$alkoxy, and halo substituted $C_{1-3}$alkoxy;

n is 2; and

Q is selected from the group consisting of

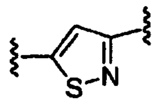 , 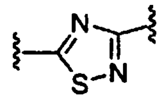 , 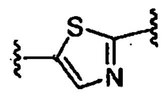 , 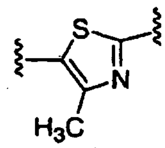 ,

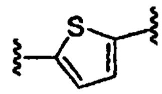 , 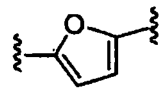 , 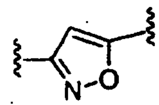 , 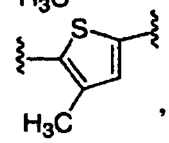 ,

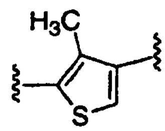 , 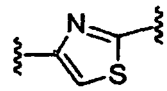 , 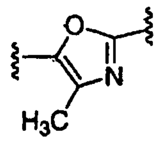 , 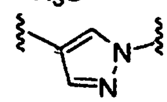 ,

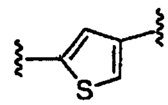 , 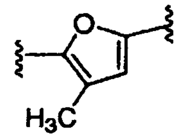 , and 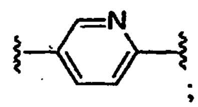 ;

and enantiomers, diastereomers, tautomers, solvates, or pharmaceutically acceptable salts thereof.

In yet another embodiment, the present invention is directed to a compound selected from the group consisting of

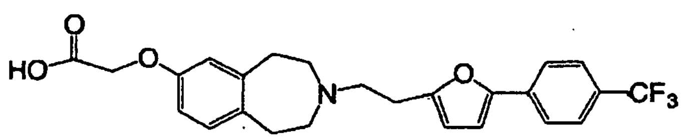
,

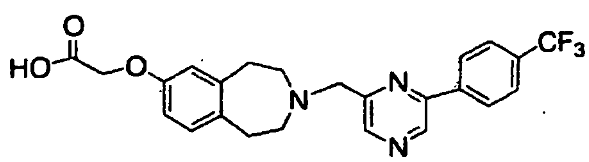
,

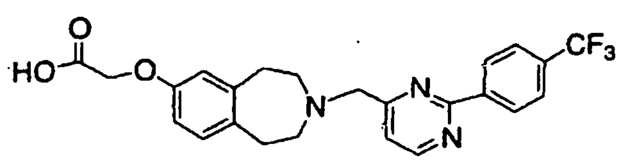
,

and

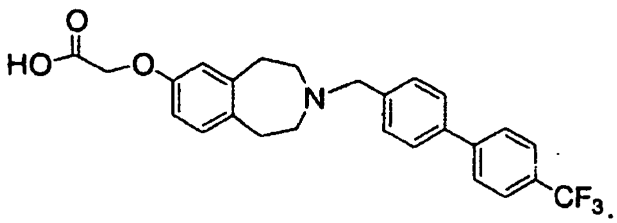

and enantiomers, diastereomers, tautomers, solvates, or pharmaceutically acceptable salts thereof.

The compounds of the present invention may also be present in the form of pharmaceutically acceptable salts. For use in medicine, the salts of the compounds of this invention refer to non-toxic "pharmaceutically acceptable salts" (Ref. International J. Pharm., 1986, 33, 201-217; J. Pharm.Sci., 1997 (Jan), 66, 1, 1). Other salts well known to those in the art may, however, be useful in the preparation of compounds according to this invention or of their pharmaceutically acceptable salts. Representative organic or inorganic acids include, but are not limited to, hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthatenesutfonic, *p*-toluenesulfonic, cyclohexanesulfamic, salicylic, saccharinic or trifluoroacetic acid. Representative organic or inorganic bases include, but are not limited to, basic or cationic salts such as benzathine, chloroprocaine, choline, diethanolamine, ethylenediamine, meglumine, procaine, aluminum, calcium, lithium, magnesium, potassium, sodium and zinc.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-d-tartaric acid and/or (+)-di-p-toluoyl-l-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

Thus, another embodiment of the present invention is a composition comprising the dextrorotatory enantiomer of a compound of Formula (I), wherein said composition is substantially free from the levorotatory isomer of said compound. In the present context, substantially free means less than 25 %, preferably less than 10 %, more preferably less than 5 %, even more preferably less than 2 % and even more preferably less than 1 % of the levorotatory isomer calculated as.

$$\%\,levorotatory = \frac{(mass\ levorotatory)}{(mass\ dextrorotatory) + (mass\ levorotatory)} \times 100$$

Another embodiment of the present invention is a composition comprising the levorotatory enantiomer of a compound of formula (I) wherein said composition is substantially free from the dextrorotatory isomer of said compound. In the present context, substantially free from means less than 25 %, preferably less than 10 %, more preferably less than 5 %, even more preferably less than 2 % and even more preferably less than 1 % of the dextrorotatory isomer calculated as

$$\%\,dextrorotatory = \frac{(mass\ dextrorotatory)}{(mass\ dextrorotatory) + (mass\ levorotatory)} \times 100$$

For example, the present invention is also directed to a compound selected from the group consisting of

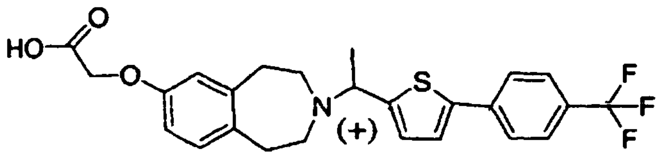

,

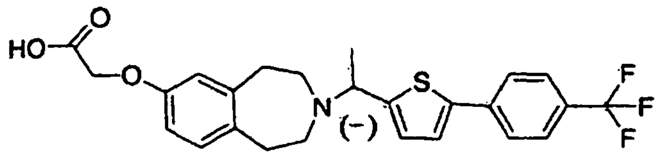

,

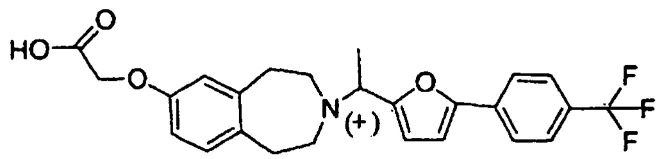

,

and

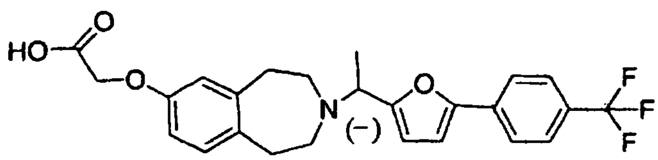

.

wherin the compound is substantially free from the corresponding other enantiomer.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

Even though the compounds of the present invention (including their pharmaceutically acceptable salts and pharmaceutically acceptable solvates) can be administered alone, they will generally be administered in admixture with a pharmaceutical carrier, excipient or diluent selected with regard to the intended route of administration and standard pharmaceutical or veterinary practice. Thus, the present invention is directed to pharmaceutical and veterinary compositions comprising compounds of Formula (I) and one or more pharmaceutically acceptable carriers, excipients or diluents.

By way of example, in the pharmaceutical compositions of the present invention, the compounds of the present invention may be admixed with any suitable binder(s), lubricant(s), suspending agent(s), coating agent(s), and/or solubilising agent(s).

Tablets or capsules of the compounds may be administered singly or two or more at a time, as appropriate. It is also possible to administer the compounds in sustained release formulations.

Alternatively, the compounds of the general Formula (I) can be administered by inhalation or in the form of a suppository or pessary, or they may be applied topically in the form of a lotion, solution, cream, ointment or dusting powder. An alternative means of transdermal administration is by use of a skin patch. For example, they can be incorporated into a cream consisting of an aqueous emulsion of polyethylene glycols or liquid paraffin. They can also be incorporated, at a concentration of between 1 and 10% by weight, into an ointment consisting of a white wax or white soft paraffin base together with such stabilizers and preservatives as may be required.

For some applications, preferably the compositions are administered morally in the form of tablets containing excipients such as starch or lactose, or in capsules or ovules either alone or in admixture with excipients, or in the form

of elixirs, solutions or suspensions containing flavoring or coloring agents.

The compositions (as well as the compounds alone) can also be injected parenterally, for example intracavernosally, intravenously, intramuscularly or subcutaneously. In this case, the compositions will comprise a suitable carrier or diluent.

For parenteral administration, the compositions are best used in the form of a sterile aqueous solution which may contain other substances, for example enough salts or monosaccharides to make the solution isotonic with blood.

For buccal or sublingual administration the compositions may be administered in the form of tablets or lozenges which can be formulated in a conventional manner.

By way of further example, pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate the major site of absorption. For parenteral administration, the carrier will usually consist of sterile water and other ingredients may be added to increase solubility or preservation. Injectable suspensions or solutions may also be prepared utilizing aqueous carriers along with appropriate additives.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those skilled in that art.

It is also apparent to one skilled in the art that the therapeutically effective dose for active compounds of the invention or a pharmaceutical composition thereof will vary according to the desired effect. Therefore, optimal dosages to be administered may be readily determined and will vary with the particular compound used, the mode of administration, the strength of the preparation, and the advancement of the disease condition. In addition, factors associated with the particular subject being treated, including subject age, weight, diet and time of administration, will result in the need to adjust the dose to an appropriate therapeutic level. The above dosages are thus exemplary of the average case. There can, of course, be individual instances where higher or lower dosage ranges are merited, and such are within the scope of this invention.

Compounds of this invention may be administered in any of the foregoing compositions and dosage regimens or by means of those compositions and dosage regimens established in the art whenever use of the compounds of the invention is required for a subject in need thereof.

The invention also provides a pharmaceutical pack or kit comprising one or more containers filled with one or more of the ingredients of the pharmaceutical and veterinary compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

For oral administration, a pharmaceutical composition is preferably provided in the form of tablets containing 0.01, 10.0, 50.0, 100, 150, 200, 250, and 500 milligrams of the active ingredient for the symptomatic adjustment of the dosage to the subject to be treated.

Examples of condition intended to be within the scope of the present invention include, but are not limited to, diabetes, nephropathy, neuropathy, retinopathy, polycystic ovary syndrome, hypertension, ischemia, stroke, irritable bowel disorder, inflammation, cataract, cardiovascular diseases, metabolic X Syndrome, hyper-LDL-cholesterolemia, dyslipidemia (including hypertriglyceridemia, hypercholesterolemia, mixed hyperlipidemia, and hypo-HDL-cholesterolemia), atherosclerosis, obesity, and other disorders related to lipid metabolism and energy homeostasis complications thereof.

Compounds of the present invention are also useful as PPAR delta agonists for treating, preventing, or inhibiting the progression of, a condition directly or indirectly mediated by PPAR delta.

The compounds of the present invention are partcularly useful in treating diabetes, nephropathy, neuropathy, retinopathy, polycystic ovary syndrome, hypertension, ischemia, stroke, irritable bowel disorder, inflammation, cataract, cardiovascular diseases, Metabolic X Syndrome, hyper-LDL-cholesterolemia, dyslipidemia (including hypertriglyceridemia, hypercholesterolemia, mixed hyperlipidemia, and hypo-HDL-cholesterolemia), atherosclerosis, obesity, and other disorders related to lipid metabolism and energy homeostasis complications thereof.

In regard to compounds for use in treatment of the disases or conditions such as those listed above, a therapeutically effective dose can be determined by persons skilled in the art by the use of established animal models. Such a dose would likely fall in the range of from about 0.01 mg to about 15,000 mg of active ingredient administered 1 to 4

times per day for an average (70 kg) human.

GENERAL SYNTHETIC METHODS

Representative compounds of the present invention can be synthesized in accordance with the general synthetic methods described below as well as the illustrative examples that follow. Since the schemes are an illustration, the invention should not be construed as being limited by the chemical reactions and conditions expressed. The preparation of the various starting materials used in the schemes is well within the skill of persons versed in the art.

**Scheme 1. General methods for the synthesis of Compounds of formula (Id)**

Method 1.

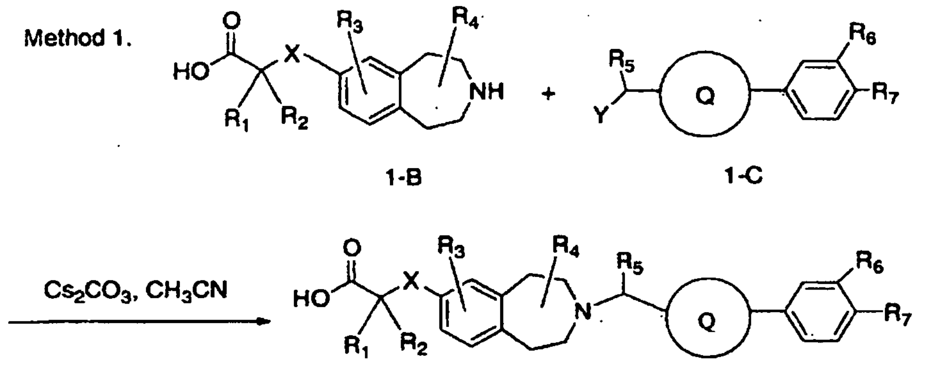


1-B 1-C

Id

Method 2.

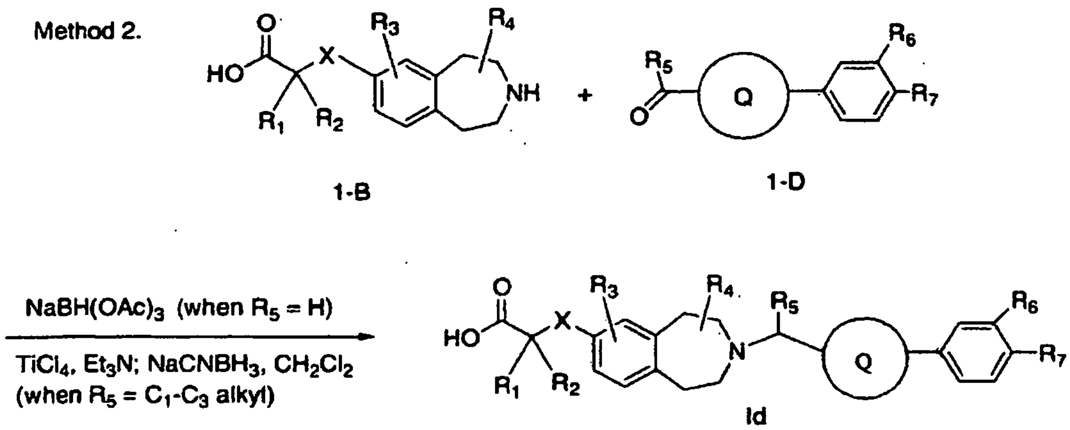


1-B 1-D

Id

Method 3.

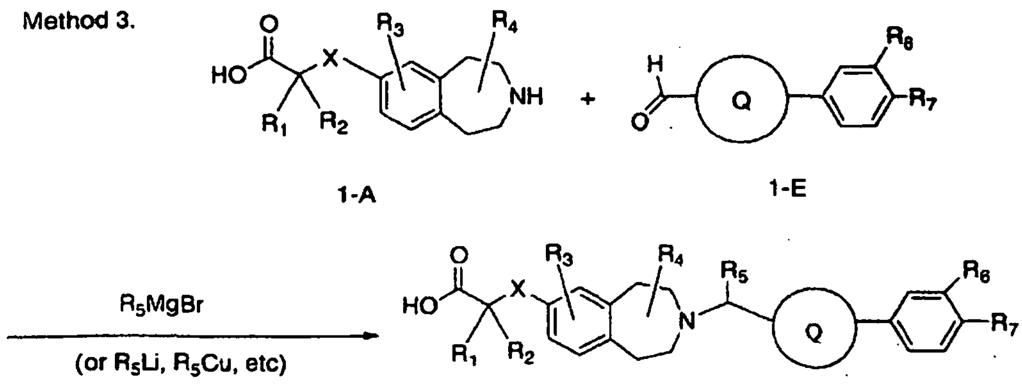


1-A

1-E

Id

Scheme 1, wherein $R_1$, $R_2$, $R_3$, $R_4$, $R_5$, $R_6$, $R_7$, X and Q are as described above, describes several general methods for the synthesis of compounds of Formula **Id**. For example, in Method 1, alkylation of the substituted benzoazepine 1-B with a compound of Formula 1-C where Y can be a leaving group such as Br, Cl, I, mesylate, etc. under a basic condition, such as $Cs_2CO_3$ in $CH_3CN$, can generate the corresponding compound of **Id**. In Method 2, reductive amination of 1-B with aryl aldehyde 1-D (e.g. $R_5$ = H) by using $NaBH(OAc)_3$ will generate **Id**; or reaction of 1-B with aryl ketone (e.g. $R_5$ = $C_1$-$C_3$ alkyl) to give the shiff-base followed by reduction with $NaCNBH_3$ will generate **Id**. In Method 3, reaction of 1-B with aryl aldehyde 1-E to give the shiff-base followed by reaction with organo-alkyl reagents such as Grignard reagents, $CH_3Li$ or organo-cupper reagent, will also provide **Id**.

## Scheme 2. General methods for the synthesis of Compounds of formula (Ie)

Method 1:

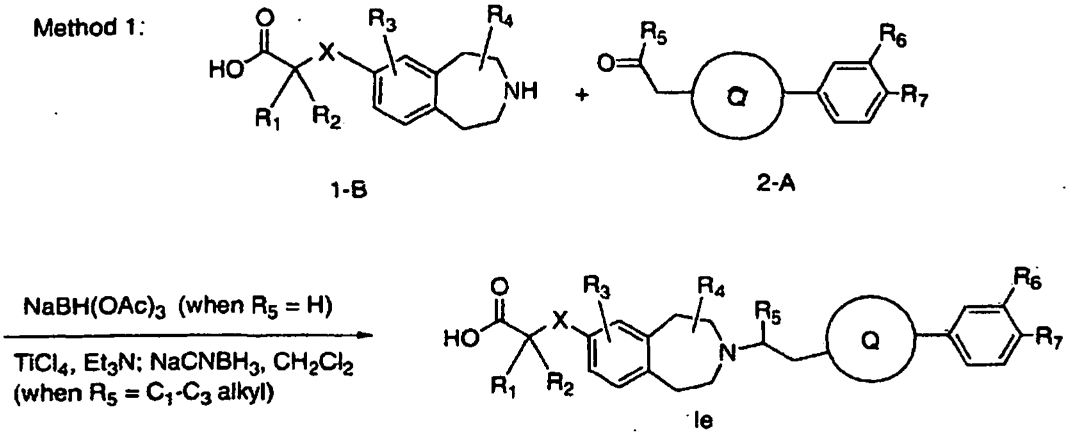


1-B

2-A

Ie

Method 2.

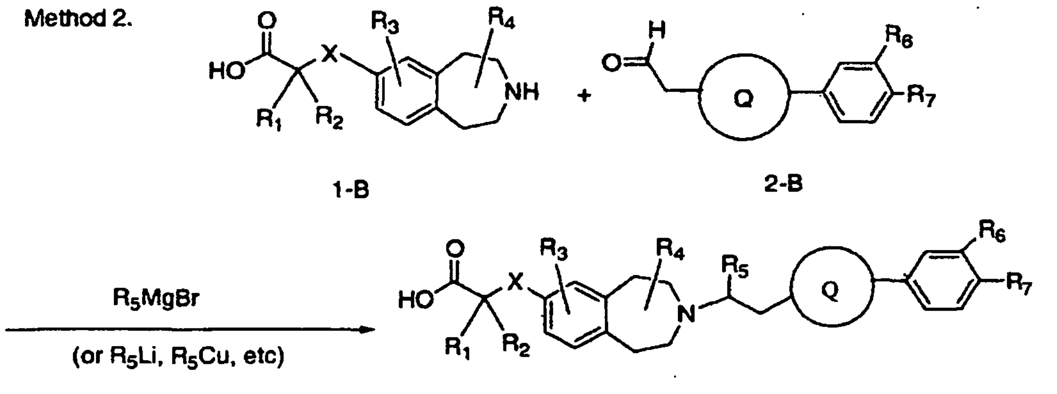


1-B

2-B

Ie

Scheme 2, wherein $R_1$, $R_2$, $R_3$, $R_4$, $R_5$, $R_6$, $R_7$, X and Q are as described above, describes several general methods for the synthesis of compounds of Formula **Ie**. For examples, in Method 1, reductive amination of 1-B with aryl aldehyde 2-A (e.g. $R_5$ = H) by using $NaBH(OAc)_3$ will generate **Ie**; or reaction of 1-B with aryl ketone (e.g. $R_5$ = $C_1$-$C_3$ alkyl) to give the shiff-base followed by reduction with $NaCNBH_3$ will generate **Ie**. In Method 2, reaction of 1-B with aryl aldehyde 2-B to give the shiff-base followed by reaction with organo-alkyl reagents such as Grignard reagents, $CH_3Li$ or organo-cupper reagent, will also provide **Ie**.

Compounds of Formula (I) that are chiral may be separated into their enantiomers by chromatography on a chiral stationary phase. Alternatively, the basic compounds of the present invention may be converted to diastereomeric salts by mixture with a chiral acid and resolved into their enantiomers by fractional crystallization.

It is generally preferred that the respective product of each process step be separated from other components of the reaction mixture and subjected to purification before its use as a starting material in a subsequent step. Separation techniques typically include evaporation, extraction, precipitation and filtration. Purification techniques typically include column chromatography (Still, W. C. et. al., J. Org. Chem. 1978, 43, 2921), thin-layer chromatography, crystallization and distillation. The structures of the final products, intermediates and starting materials are confirmed by spectroscopic, spectrometric and analytical methods including nuclear magnetic resonance (NMR), mass spectrometry (MS) and liquid chromatography (HPLC). In the descriptions for the preparation of compounds of this invention, ethyl ether, tetrahydrofuran and dioxane are common examples of an ethereal solvent; benzene, toluene, hexanes and heptanes are typical hydrocarbon solvents and dichloromethane and dichloroethane are representative halogenated hydrocarbon solvents. In those cases where the product is isolated as the acid addition salt the free base may be obtained by techniques known to those skilled in the art. In those cases in which the product is isolated as an acid addition salt, the salt-may contain one or more equivalents of the acid. Enantiomers of the compounds of the present invention may be separated using chiral HPLC.

Abbreviations

| | |
|---|---|
| Ac = | $CH_3C(O)$- |
| Aq = | aqueous |
| Cpd = | Compound |
| con = | concentration |
| DCE = | dichloroethane |
| DMF = | *N,N*-dimethylformamide |
| DMSO = | dimethylsulfoxide |
| dppf = | diphenylphosphinoferrocene |
| Et = | ethyl |
| EtOAc = | ethyl acetate |
| h or hr = | hour(s) |
| HATU = | N-[(dimethylamino)(3H-1,2,3-triazolo(4,5-b)pyridin.e-3-yloxy)methylene]-N-methylmethanaminium hexafluorophosphate |
| LAH = | lithium aluminum hydride |
| Me = | methyl |
| min = | minute(s) |
| Ph = | phenyl |
| PPA = | polyphosphoric acid |
| psi = | pascal per square inch |
| t-Boc = | tert-butoxycarbonyl |
| t-Bu = | tert-butyl |
| TEA = | triethylamine |
| TFA = | trifluoroacetic acid |
| THF = | tetrahydrofuran |
| TLC = | (thin layer chromatography) |
| $LiN(TMS)_2$ = | Lithium bis(trimethylsilyl)amide |
| Tol = | toluene |

<u>EXAMPLES</u>

<u>Example A</u>

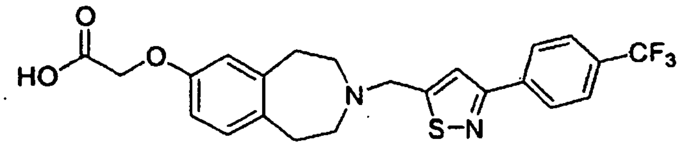

**Compound 1: {3-[3-(4-Trifluoromethyl-phenyl)-isothiazol-5-ylmethyl]-2,3,4,5-tetrahydro-1*H*-benzo[*d*]azepin-7-yloxy}-acetic acid**

The title compound was made according to Schemes A1 and A2.

## Scheme A1

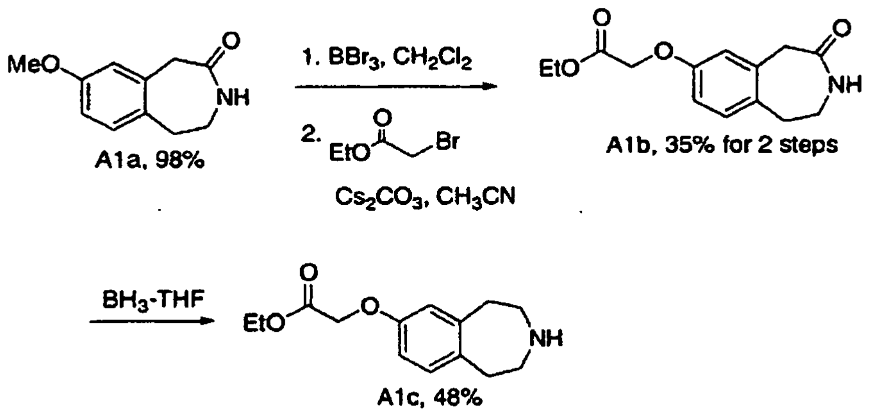


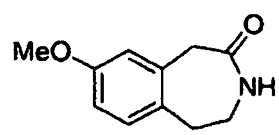

**A1a**

Cpd A1 a can be prepared according to published procedures (US patent No. 4,659,706 and Eur. Pat. Appl. 204349).

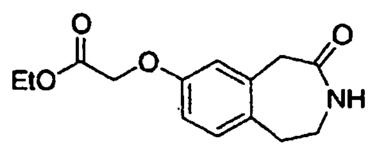

**A1b**
**(4-Oxo-2,3,4,5-tetrahydro-1*H*-benzo[*d*]azepin-7-yloxy)-acetic acid ethyl ester**

To a solution of A1 a (725 mg, 3.8 mmol) in $CH_2Cl_2$ (10 mL) at -78 ˚C was added $BBr_3$ (1 M in $CH_2Cl_2$, 11.4 mL, 11.4 mmol). The reaction mixture was allowed to warm to room temperature and stirred at room temperature for 5 h. MeOH (5 mL) was added slowly to quench the reaction. The reaction mixture was then concentrated to give a yellow solid.

A mixture of the above crude phenol, ethyl bromoacetate (950 mg, 5.69 mmol) and $Cs_2CO_3$ (2.47 g, 7.58 mmol) in $CH_3CN$ (15 mL) was stirred at 80˚C for 20 h. After cooling to room temperature, the mixture was partitioned between EtOAc and $H_2O$ and the aqueous layer was extracted with EtOAc. The combined organic phases were dried ($Na_2SO_4$) concentrated and purified by column chromatography to give 350 mg (35%) of Cpd A1 b as a white solid: $^1H$ NMR (300 MHz, $CDCl_3$) δ 7.04 (d, *J* = 8.4 Hz, 1 H), 7.77 (dd, *J* = 8.4, 2.7 Hz, 1 H), 6.69 (d. *J* = 2.5 Hz, 1 H), 5.83 (bs, 1 H), 4.59 (s, 2 H), 4.27 (q, *J* = 7.1 Hz, 2 H), 3.80-(s, 2 H), 3.58 - 3.52 (m, 2 H), 3.06 (t, *J* = 6.0 Hz, 2 H), 1.30 (t, *J* = 7.1 Hz, 3 H);

MS (ES) m/z: 286 ($M+Na^+$).

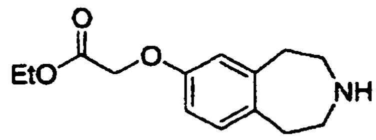

**A1c**

**(2,3,4,5-Tetrahydro-1*H*-benzo[*d*]azepin-7-yloxy)-acetic acid ethyl ester**

1 M borane-THF solution (1 mL, 1.02 mmol) was added dropwise to an ice-cooled and stirred solution of A1 b (90 mg, 0.342 mmol) in THF (5 mL). The stirring was continued at 0 °C for 1 h and then room temperature for 20 min. The solution was cooled back to 0 °C and 1 N HCl (2 mL) was added slowly to destroy the excess borane. After stirring at room temperature for 15 min, the solution was concentrated to remove THF. The aqueous solution was washed with EtOAc and then basified with $Na_2CO_3$ until PH > 10 and then extracted with EtOAc. The combined organic phases were dried ($Na_2SO_4$), concentrated and vacuum dried to give 43 mg (48%) of A1c as a yellow jelly oil: $^1H$ NMR (300 MHz, $CDCl_3$) δ 6.99 (d, *J*= 8.4 Hz, 1 H), 6.70 (s, 1 H), 6.60 (d, *J* = 8.4 Hz, 1 H), 4.59 (s, 2 H), 4.28 (q, *J* = 7.1 Hz, 2 H), 2.97 (bm, 4 H), 2.84 (bm, 4 H), 2.67 (s, 1 H), 1.30 (t, *J* = 7.1 Hz, 3 H); MS (ES) m/z: 250 ($M+H^+$).

## Scheme A2

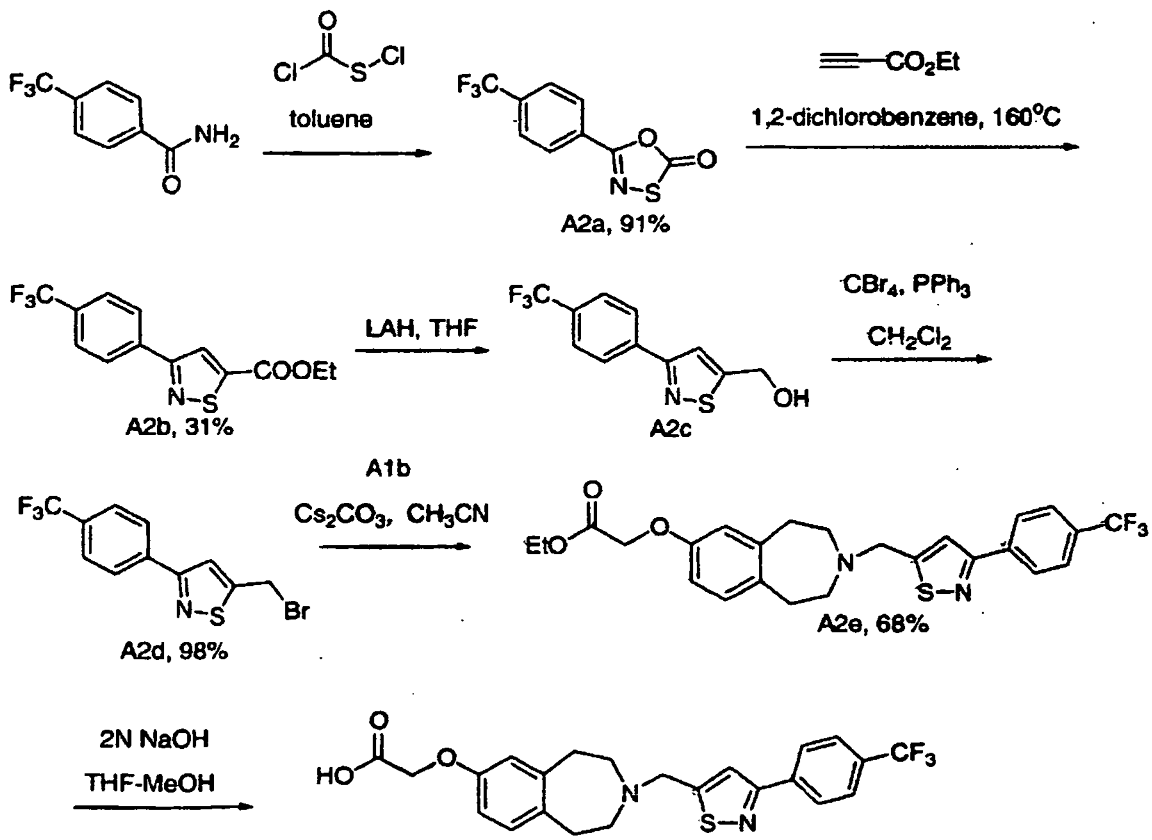

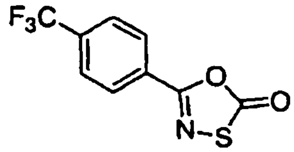

A2a

**5-(4-Trifluoromethyl-phenyl)-[1,3,4]oxathiazol-2-one**

The reaction mixture of 4-trifluoromethylbenzamide (11.3 g, 59.8 mmol), chlorocarbonylsulfenyl chloride (10.1 mL, 119.6 mmol) in toluene (120 mL) was heated at 80 °C for 15 h, cooled and concentrated. The solid was transferred to a sintered funnel, washed with a small amount of ethanol and dried under vacuum to give 13.4 g (91%) of A2a as white crystals: $^1H$ NMR (300 MHz, $CDCl_3$) δ 8.11 (d, *J* = 8.2 Hz, 2 H), 7.77 (d, *J* = 8.3 Hz, 2 H).

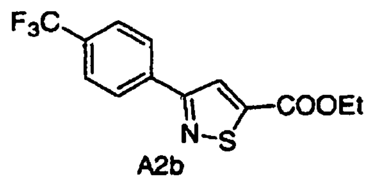

A2b

**3-(4-Trifluoromethyl-phenyl)-isothiazole-5-carboxylic acid ethyl ester**

The reaction mixture of A2a (608 mg, 2.46 mmol) and ethyl propiolate (726 mg, 7.41 mmol) in chlorobenzene (10 mL) was heated at 135°C for 20 h. TLC showed some of the starting material A2a still remained. More ethyl propiolate (726 mg, 7.41 mmol) and 1,2-dichlorobenzene (10 mL) were added and the solution was heated at 160 °C for 7 h. After cooling to room temperature, the reaction mixture was purified by column chromatography to give 229 mg (31%) of A2b as a white solid: $^1H$ NMR (300 MHz, $CDCl_3$) δ 8.15 (s, 1 H), 8.09 (d, *J* = 8.1 Hz, 2 H), 7.73 (d, *J* = 8.2 Hz, 2 H), 4.44 (q, *J* = 7.1 Hz, 2 H), 1.43 (t, *J*= 7.1 Hz, 3 H); MS (ES) m/z: 302 ($M+H^+$).

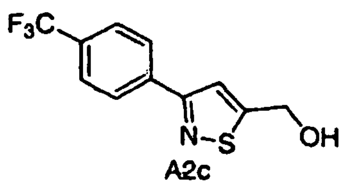

A2c

**[3-(4-Trifluoromethyl-phenyl)-isothiazol-5-yl]-methanol**

To the solution of A2b (104 mg, 0.345 mmol) in THF (2 mL) at -78°C was added 1.0 M $LiAlH_4$ (0.21 mL, 0.21 mmol) in THF. After stirring at-78 °C for 30 min, water was slowly added and the mixture was allowed to warm up to room temperature. The precipitated solid was filtered and rinsed with $CH_2Cl_2$. The filtrate was washed with saturated $NH_4Cl$, and the aqueous solution was back extracted with $CH_2Cl_2$. The combined organic phases were dried and concentrated to give 98 mg of crude A2c as a yellow solid: $^1H$ NMR (300 MHz, $CDCl_3$) δ 8.04 (d, *J*= 8.3 Hz, 2 H), 7.70 (d, *J*= 8.4 Hz, 2 H), 7.51 (s, 1 H), 5.06 (s, 2 H); MS (ES) m/z: 260 ($M+H^+$).

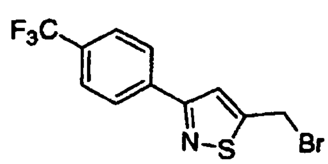

A2d

**5-Bromomethyl-3-(4-trifluoromethyl-phenyl)-isothiazole**

To the solution of A2c (1.0 g, 3.86 mmol) in $CH_2Cl_2$ (25 mL) at 0 °C was added $PPh_3$ (1.3 g, 5.01 mmol) and $CBr_4$ (1.7 g, 5.01 mmol). The reaction mixture was allowed to warm up to room temperature and stirred for 4 h at room

temperature. The reaction mixture was concentrated and purified through column chromatography to get 1.36 g (98%) A2d as a white solid:

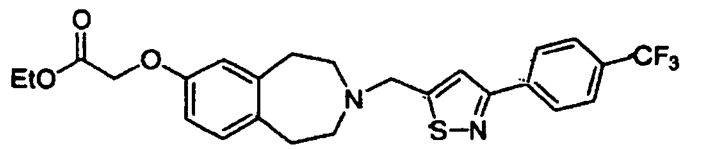

A2e

**{3-[3-(4-Trifluoromethyl-phenyl)-isothiazol-5-ylmethyl]-2,3,4,5-tetrahydro-1*H*-benzo[*d*]azepin-7-yloxy}-acetic acid ethyl ester**

A mixture of A1e (15 mg, 0.060 mmol), A2d (23 mg, 0.072 mmol) and $Et_3N$ (20 mg, 0.18 mmol) in $CH_3CN$ (1 mL) was stirred for 5 hours. EtOAc and $H_2O$ were added and the aqueous layer was extracted with EtOAc. The combined organic phases were dried ($Na_2SO_4$), concentrated and purified through column chromatography to get 20 mg (68%) Cpd A2e as a white solid: $^1H$ NMR (300 MHz, $CDCl_3$) δ 8.05 (d, *J* = 8.4 Hz, 2 H), 7.69 (d, *J* = 8.4 Hz, 2 H), 7.45 (s, 1 H), 7.00 (d, *J* = 8.2 Hz, 1 H), 6.70 (d, *J* = 2.5 Hz, 1 H), 6.62 (dd, *J* = 8.2, 2.5 Hz, 1 H), 4.59 (s, 2 H), 4.27 (q, J = 7.1 Hz, 2 H), 3.98 (s, 2 H), 2.90 (m, 4 H), 2.70 (m, 4 H), 1.30 (t, *J*= 7.1 Hz, 3 H); MS (ES) m/z: 491 ($M+H^+$).

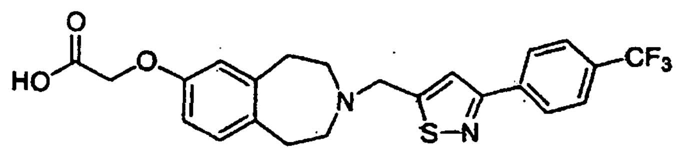

Cpd 1

**{3-[3-(4-Trifluoromethyl-phenyl)-isothiazol-5-ylmethyl]-2,3,4,5-tetrahydro-1*H*-benzo[*d*]azepin-7-yloxy}-acetic acid**

A mixture of A2e (20 mg, 0.041 mmol) and 2 M NaOH (41 μL, 0.082 mmol) in THF-MeOH (0.6 mL-0.2 mL) was stirred under $N_2$ for 2 h and concentrated. $CH_2Cl_2$ and water were added, and the mixture was acidified with concentrated HCl. The organic phase was separated and the aqueous phase was extracted with $CH_2Cl_2$. The combined organic layers were dried ($Na_2SO_4$), concentrated, and purified by column chromatography to give 15 mg (80%) of Cpd 1 as a white solid: $^1H$ NMR (300 MHz, $CDCl_3$) δ 8.05 (d, *J* = 8.0 Hz, 2 H), 7.90 (s, 1 H), 7.71 (d, *J* = 8.4 Hz, 2 H), 6.95 (d, J=8.3 Hz, 1 H), 6.75 (dd, *J* = 8.1, 2.6 Hz, 1 H), 6.65 (d, *J* = 2.6 Hz, 1 H), 4.65 (s, 2 H), 4.38 (s, 2 H), 3.07 (m, 4 H), 2.93 (m, 4 H); MS (ES) m/z: 463 ($M+H^+$).

## Example B

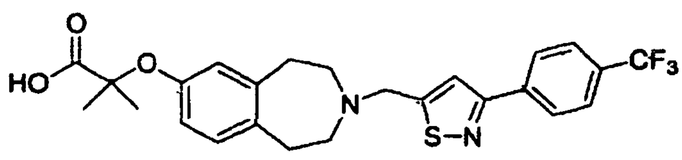

**Compound 2: 2-Methyl-2-{3-[3-(4-trifluoromethyl-phenyl)-isothiazol-5-ylmethyl]-2,3,4,5-tetrahydro-1*H*-benzo[*d*]azepin-7-yloxy}-propionic acid**

The title compound was made according to Scheme B.

## Scheme B

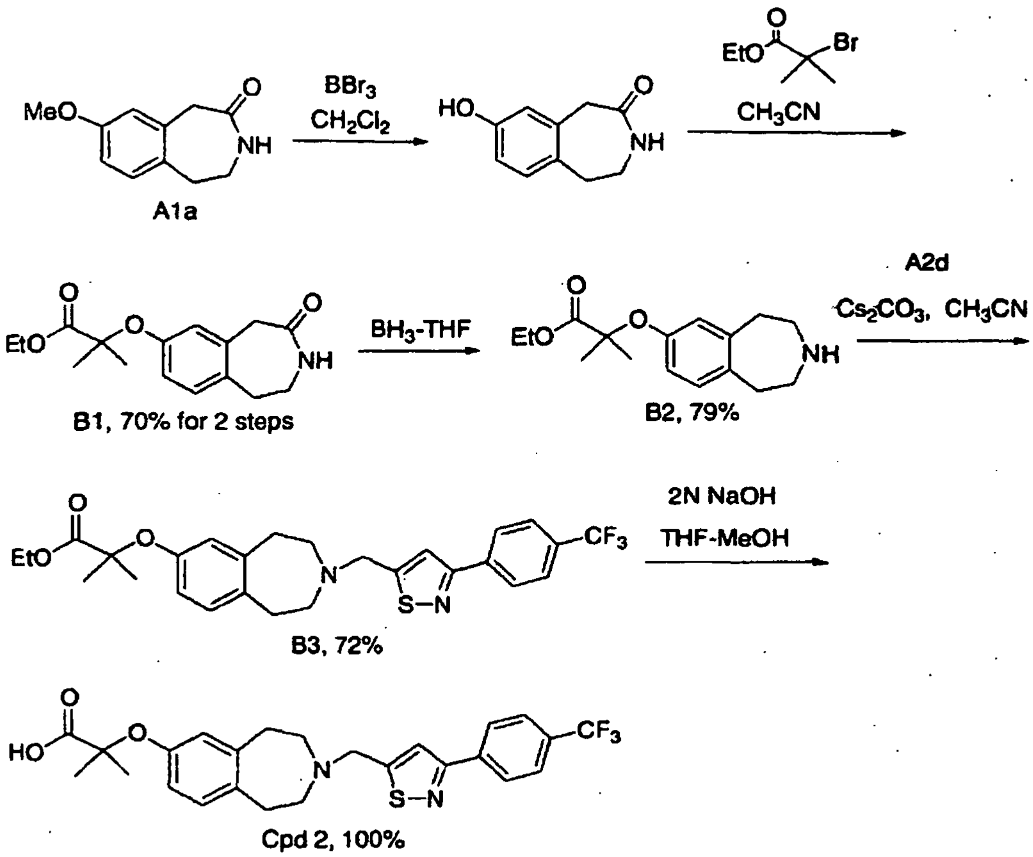


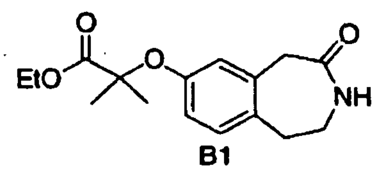

**B1**

**2-Methyl-2-(4-oxo-2,3,4,5-tetrahydro-1*H*-benzo[*d*]azepin-7-yloxy)-propionic acid ethyl ester**

To a solution of A1 a (4.0 g, 20.9 mmol) in $CH_2Cl_2$ (20 mL) at -78 °C was added $BBr_3$ (1 M in $CH_2Cl_2$, 62.8 mL, 62.8 mmol). The mixture was allowed to warm to room temperature and stirred at room temperature for 5 h. MeOH (5 mL) was added slowly to quench the reaction. The reaction mixture was then concentrated to give a yellow solid.

A mixture of the above crude phenol, ethyl bromoisobutyrate (6.1 g, 31.4 mmol) and $Cs_2CO_3$ (20.8 g, 63.8 mmol)) in $CH_3CN$ (200 mL) was stirred at 80 °C for 20 h. After cooling to room temperature the mixture was partitioned between EtOAc and $H_2O$ and the aqueous layer was extracted with EtOAc. The combined organic phases were dried ($Na_2SO_4$), concentrated and purified by column chromatography to give 4.2 g (70%) of B1 as a white solid: $^1H$ NMR (300 MHz, $CD_3OD$) δ 7.03 (d, *J* = 8.4 Hz, 1 H), 6.68 (dd, *J* = 8.3, 2.6 Hz, 1 H), 6.63 (d, *J* = 2.6 Hz, 1 H), 4.21 (q, *J* = 7.1 Hz, 2 H), 3.76 (s, 2 H), 3.57 - 3.52 (m, 2 H), 3.04 (t, *J* = 6.0 Hz, 2 H), 1.53 (s, 6 H), 1.24 (t, *J* = 7.1 Hz, 3 H); MS (ES) m/z: 314 ($M+Na^+$).

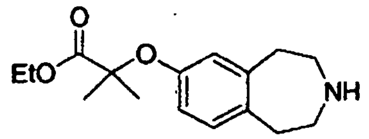

B2

**2-Methyl-2-(2,3,4,5-tetrahydro-1*H*-benzo[*d*]azepin-7-yloxy)-propionic acid ethyl ester**

The 1 M borane-THF solution (20.5 mL, 20.5 mmol) was added dropwise to an ice-cooled and stirred solution of B1 (2.0 g, 6.85 mmol) in THF (20 mL). The stirring was continued at room temperature for 1 h. The solution was cooled back to 0 °C and 1 N HCl (25 mL) was added slowly to destroy the excess borane. After stirring at room temperature for 15 min, the solution was concentrated to remove THF. The aqueous solution was washed with EtOAc, basified with $Na_2CO_3$ until PH > 10 and then extracted with EtOAc. The combined organic phases were dried ($Na_2SO_4$) and concentrated to give 1.5 g (79%) of B2 as a colorless jelly oil: $^1$H NMR (300 MHz, $CDCl_3$) δ 6.95 (d, *J* = 8.4 Hz, 1 H), 6.64 (d, *J* = 2.5 Hz, 1 H), 6.53 (dd, *J* = 8.4, 2.6 Hz, 1 H), 4.22 (q, *J* = 7.1 Hz, 2 H), 2.99 (m, 2 H), 2.88 (m, 2 H), 1.59 (s, 6 H), 1.25 (t, *J*= 7.1 Hz, 3 H); MS (ES) m/z: 278 (M+H$^+$).

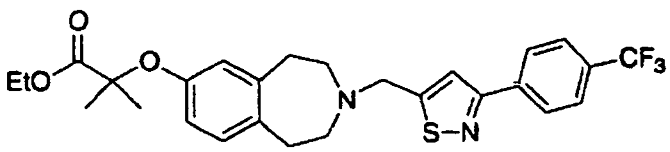

B3

**2-Methyl-2-{3-[3-(4-trifluoromethyl-phenyl)-isothiazol-5-ylmethyl]-2,3,4,5-tetrahydro-1*H*-benzo[*d*]azepin-7-yloxy}-propionic acid ethyl ester**

Cpd B3 was prepared using same procedure as for cpd A2e by replacing A1 b with B2 (white solid, 72%): $^1$H NMR (300 MHz, $CDCl_3$) δ 8.05 (d, *J* = 8.4 Hz, 2 H), 7.69 (d, *J* = 8.4 Hz, 2 H), 7.55 (s, 1 H), 6.93 (d, *J* = 8.2 Hz, 1 H), 6.64 (d, *J* = 2.5 Hz, 1 H), 6.57 (dd, *J* = 8.2, 2.5 Hz, 1 H), 4.23 (q, *J* = 7.1 Hz, 2 H), 4.05 (s, 2 H), 2.92 (m, 4 H), 2.80 (m, 4 H), 1.57 (s, 6 H), 1.25 (t, *J* = 7.1 Hz, 3 H); MS (ES) m/z: 519 (M+H$^+$).

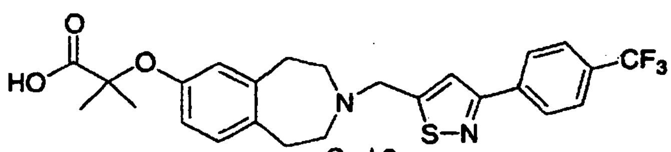

Cpd 2

**2-Methyl-2-{3-[3-(4-trifluoromethyl-phenyl)-isothiazol-5-ylmethyl]-2,3,4,5-tetrahydro-1*H*-benzo[*d*]azepin-7-yloxy}-propionic acid**

Cpd 2 was prepared using similar procedure as for cpd 1. Cpd 2 was obtained as a white solid (100%): $^1$H NMR (300 MHz, $CD_3OD$) δ 8.21 (d, *J*= 8.1 Hz, 2 H), 8.06 (s, 1 H), 7.80 (d, *J* = 8.2 Hz, 2 H), 7.09 (d, *J* = 8.2 Hz, 1 H), 6.78 (d, *J* = 2.5 Hz, 1 H), 6.72 (dd, J = 8.2, 2.5 Hz, 1 H), 4.74.(s, 2 H), 3.33 (m, 4 H), 3.11 (m, 4 H), 1.54 (s, 6 H); MS (ES) m/z: 491 (M+H$^+$).

<u>Example C</u>

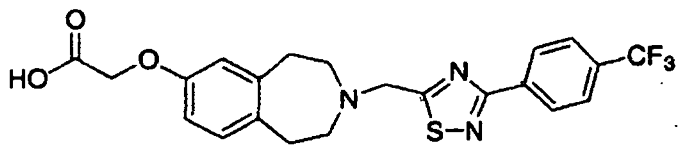

**Compound 3: {3-[3-(4-Trifluoromethyl-phenyl)-[1,2,4]thiadiazol-5-ylmethyl]-2,3,4,5-tetrahydro-1*H*-benzo[*d*]azepin-7-yloxy}-acetic acid**

The title compound was made according to Scheme C.

## Scheme C

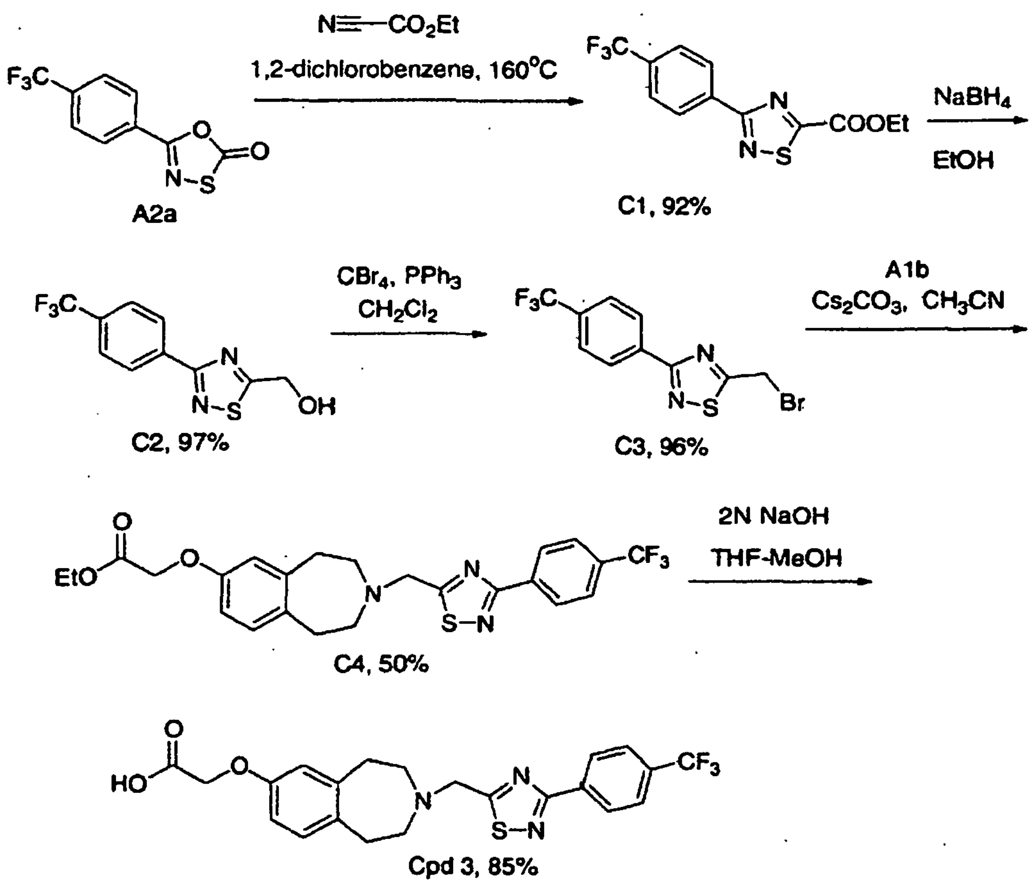


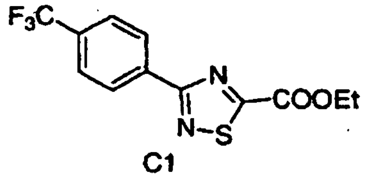

**C1**

**3-(4-Trifluoromethyl-phenyl)-[1,2,4]thiadiazole-5-carboxylic acid ethyl ester**

A reaction mixture of A2a (448 mg, 1.81 mmol) and ethyl cyanoformate (722 mg, 7.29 mmol) in 1,2-dichlorobenzene (7 mL) was heated at 160 °C for 20 h. After cooling to room temperature, the reaction mixture was purified by column chromatography to give 505 mg (92%) of C1 as a yellow solid: $^1$H NMR (300 MHz, $CDCl_3$) δ 8.50 (d, *J*= 8.1 Hz, 2 H), 7.76-(d, *J* = 8.2 Hz, 2 H), 4.57 (q, *J* = 7.1 Hz, 2 H), 1.49 (t, *J* = 7.1 Hz, 3 H); MS (ES) m/z: 303 ($M+H^+$).

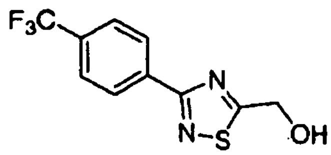

C2

**[3-(4-Trifluoromethyl-phenyl)-[1,2,4]thiadiazol-5-yl]-methanol**

To a solution of C1 (200 mg, 0.662 mmol) in EtCH (10 mL) at room temperature was added $NaBH_4$ (64 mg, 1.7 mmol). After stirring for 2 h, a few drops of water were added to quench excess of hydride: EtOH was evaporated, and the residue was partitioned between $CH_2Cl_2$ and water. The organic phase was dried ($Na_2SO_4$) and concentrated to provide 167 mg (97%) of C2 as off-white crystals: $^1$H NMR (300 MHz, $CDCl_3$) δ 8.40 (d, *J* = 8.1 Hz, 2 H), 7.74 (d, *J* = 8.2 Hz, 2 H), 5.20 (s, 2 H), 2.65 (br, 1 H); MS (ES) m/z: 261 ($M+H^+$).

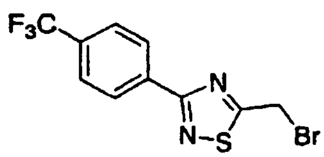

C3

**5-Bromomethyl-3-(4-trifluoromethyl-phenyl)-[1,2,4]thiadiazole**

Cpd C3 was prepared according to a similar procedure as for cpd A2d. Cpd C3 was obtained as a white solid (96%): $^1$H NMR (300 MHz, $CDCl_3$) δ 8.40 (d, *J* = 8.1 Hz, 2 H), 7.74 (d, *J* = 8.3 Hz, 2 H), 4.83 (s, 2H); MS (ES) m/z: 321 ($M-H^+$).

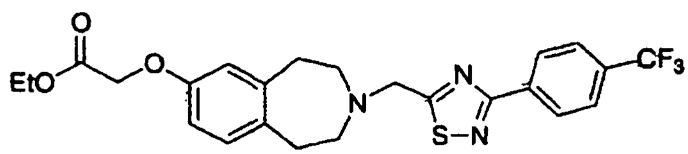

C4

**{3-[3-(4-Trifluoromethyl-phenyl)-[1,2,4]thiadiazol-5-ylmethyl]-2,3,4,5-tetrahydro-1*H*-benzo[*d*]azepin-7-yloxy}-acetic acid ethyl ester**

Cpd C4 was prepared according to a similar procedure as for cpd A2e. Cpd C4 was obtained as a white solid (50%): $^1$H NMR (300 MHz, $CDCl_3$) δ 8.40 (d, *J* = 8.1 Hz, 2 H), 7.73 (d, *J* = 8.3 Hz, 2 H), 7.02 (d, *J* = 8.2 Hz, 1 H), 6.72 (d, *J* = 2.6 Hz, 1 H), 6.65 (dd, *J* = 8.2, 2.7 Hz, 1 H), 4.59 (s, 2. H), 4.27 (q, *J* = 7.1 Hz, 2 H), 4.11 (s, 2 H), 2.95 - 2.83 (m, 8 H), 1.30 (t, *J* = 7.1 Hz, 3 H); MS (ES) m/z: 492 ($M+H^+$).

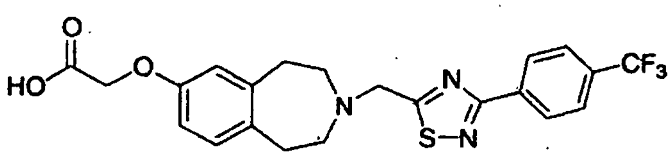

Cpd 3

**{3-[3-(4-Trifluoromethyl-phenyl)-[1,2,4]thiadiazol-5-ylmethyl]-2,3,4,5-tetrahydro-1*H*-benzo[*d*]azepin-7-yloxy}-acetic acid**

Cpd 3 was prepared according to a similar procedure as forced 1. Cpd 3 was obtained as a white solid (85%): $^1H$ NMR (300 MHz, $CD_3OD$ δ 8.48 (d, $J$ = 7.7 Hz, 2 H), 7.83 (d, $J$ = 6.8 Hz, 2 H), 7.07 (d, $J$ = 8.5 Hz, 1 H), 6.79 (s, 1 H), 6.73 (d, $J$ = 7.1 Hz, 1 H), 4.63 (s, 2 H), 4.58 (s, 2 H), 322 (m, 4 H), 3.07 (m, 4 H); MS (ES) m/z: 464 ($M+H^+$).

Example D

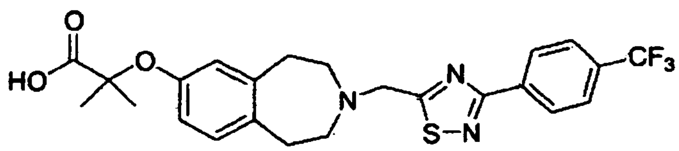

**Compound: 2-Methyl-2-{3-[3-(4-trifluoromethyl-phenyl)-[1,2,4]thiadiazol-5-ylmethyl]-2,3,4,5-tetrahydro-1*H*-benzo[*d*]azepin-7-yloxy}-propionic acid**

The title compound was made according to Scheme D.

**Scheme D**

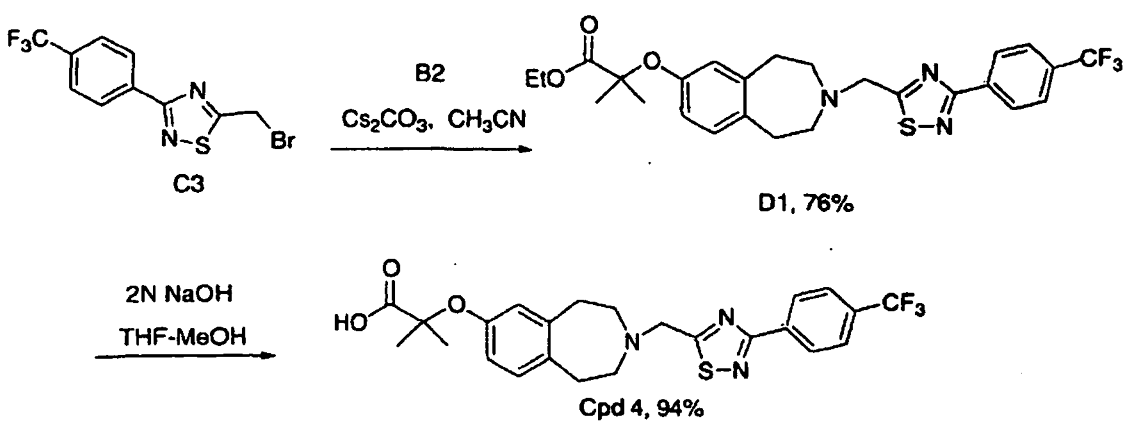


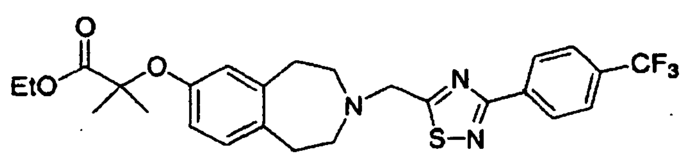

D1

**2-Methyl-2-{3-[3-(4-trifluoromethyl-phenyl)-[1,2,4]thiadiazol-5-ylmethyl]-2,3,4,5-tetrahydro-1*H*-benzo[*d*]azepin-7-yloxy}-propionic acid ethyl ester**

Cpd D1 was prepared according to a similar procedure as for cpd B3. Cpd B3 was obtained as a white solid (76%): $^1H$ NMR (300 MHz, $CDCl_3$) δ 8.39 (d, $J$ = 8.2 Hz, 2 H), 7.72 (d, $J$ = 8.4 Hz, 2 H), 6.95 (d, $J$ = 8.2 Hz, 1 H), 6.66 (d, $J$ = 2.5 Hz, 1 H), 6.58 (dd, $J$ = 8.1, 2.6 Hz, 1 H), 4.24 (q, $J$ = 7.1 Hz, 2 H), 4.12 (s, 2 H), 2.91 (m, 4 H), 2.88 (m, 4 H), 1.58 (s, 6 H), 1.26 (t, $J$ = 7.1 Hz, 3 H); MS (ES) m/z: 520 ($M+H^+$).

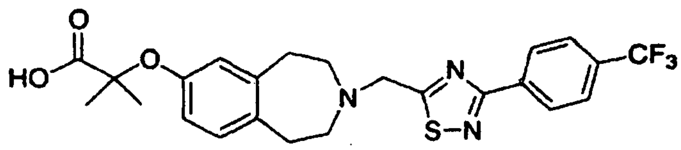

Cpd 4

**2-Methyl-2-{3-[3-(4-trifluoromethyl-phenyl)-[1,2,4]thiadiazol-5-ylmethyl]-2,3,4,5-tetrahydro-1*H*-benzo[*d*]azepin-7-yloxy}-propionic acid**

Cpd 4 was prepared according to a similar procedure as forces 2. Cpd 4 was obtained as a white solid (94%): $^1H$ NMR (300 MHz, $CD_3OD$) δ 8.50 (d, *J* = 8.2 Hz, 2 H), 7.84 (d, *J* = 8.3 Hz, 2 H), 7.09 (d, *J* = 8.2 Hz, 1 H), 6.78 (d, *J* = 2.5 Hz, 1 H), 6.72 (dd, *J* = 8.2, 2.6 Hz. 1 H), 4.84 (s, 2 H), 3.46 (m, 4 H), 3:16 (m, 4 H), 1.55 (s, 6 H); MS (ES) m/z: 492 ($M+H^+$).

Example E

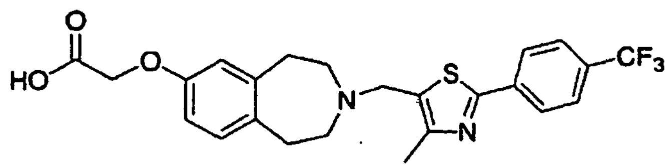

**Compound 5: {3-[4-Methyl-2-(4-trifluoromethyl-phenyl)-thiazol-5-ylmethyl]-2,3,4,5-tetrahydro-1*H*-benzo[*d*]azepin-7-yloxy}-acetic acid**

The title compound was made according to Scheme E.

## Scheme E

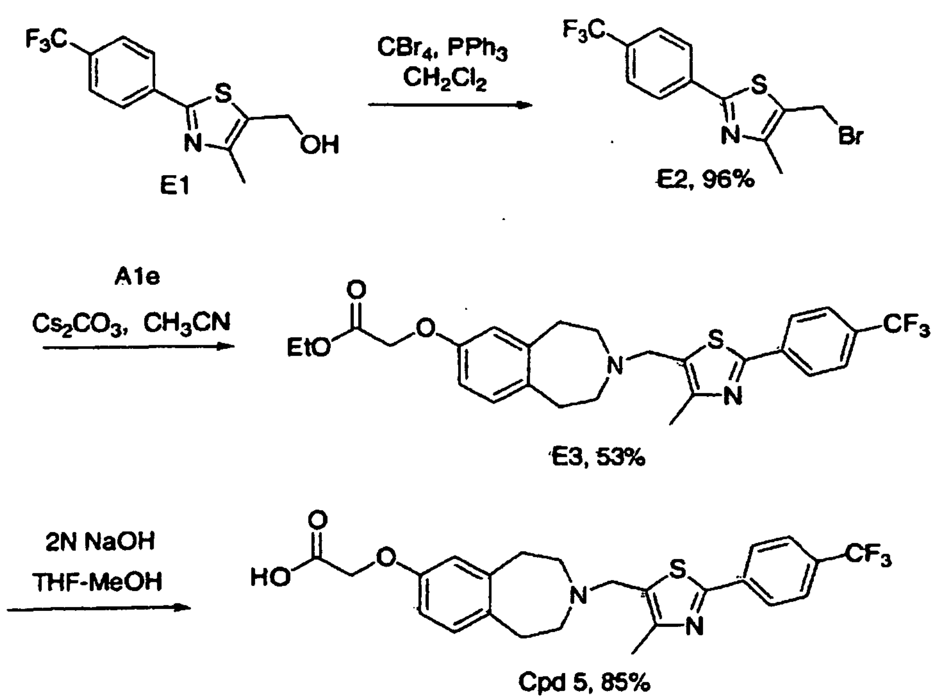

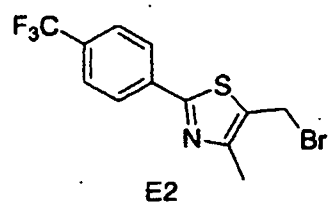

E2

5-Bromomethyl-4-methyl-2-(4-trifluoromethyl-phenyl)-thiazole

Cpd E2 was prepared from cpd E1 (Bioorg & Med. Chem. Lett., 2003, 13 (9), 1517-1521) following a similar procedure as for cpd A2d. Cpd E2 was obtained as a white solid (96%): $^1H$ NMR (300 MHz, $CDCl_3$) δ 8.01 (d, *J* = 8.7 Hz, 2 H), 7.68 (d, *J* = 8.6 Hz, 2 H), 4.72 (s, 2 H), 2.48 (s, 3 H).

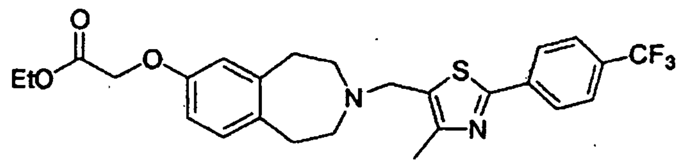

E3

{3-[4-Methyl-2-(4-trifluoromethyl-phenyl)-thiazol-5-ylmethyl]-2,3,4,5-tetrahydro-1*H*-benzo[*d*]azepin-7-yloxy}-acetic acid ethyl ester

Cpd E3 was prepared according to a similar procedure as for cpd A2e. Cpd E3 was obtained as a white solid (53%): $^1H$ NMR (300 MHz, $CDCl_3$) δ 8.02 (d, *J* = 8.2 Hz, 2 H), 7.68 (d, *J* = 8.3 Hz, 2 H), 7.00 (d, *J* = 8.2 Hz, 1 H), 6.70 (d, *J* = 2.5 Hz, 1 H), 6.62 (dd, *J* = 8.2, 2.5 Hz, 1 H), 4.59 (s, 2 H), 4.27 (q, *J* = 7.1 Hz, 2 H), 3.76 (s, 2 H), 2.90 - 2.83 (m, 4 H), 2.72 - 2.64 (m, 4 H), 2.42 (s, 3 H), 1.30 (t, *J* = 7.1 Hz, 3 H); MS (ES) m/z: 505 ($M+H^+$).

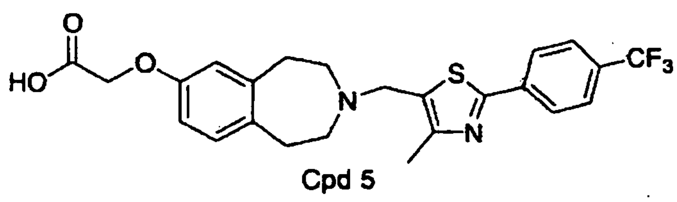

Cpd 5

{3-[4-Methyl-2-(4-trifluoromethyl-phenyl)-thiazol-5-ylmethyl]-2,3,4,5-tetrahydro-1*H*-benzo[*d*]azepin-7-yloxy}-acetic acid

Cpd 5 was prepared according to a similar procedure as for cpd 1. Cpd 1 was obtained as a white solid (85%): $^1H$ NMR (300 MHz, $CDCl_3$) δ 8.01 (d, *J* = 8.2 Hz, 2 H), 7.68 (d, *J* = 8.4 Hz, 2 H), 6.97(d, *J* = 8.3 Hz, 1 H), 6.71 (dd, *J* = 8.3, 2.5 Hz, 1 H), 6.66 (d, *J*=2.4 Hz, 1 H), 4.63 (s, 2 H), 4.52 (s, 2 H), 3.40 - 2.85 (m, 8 H), 2.49 (s, 3 H); MS (ES) m/z: 477 ($M+H^+$).

<u>Example F</u>

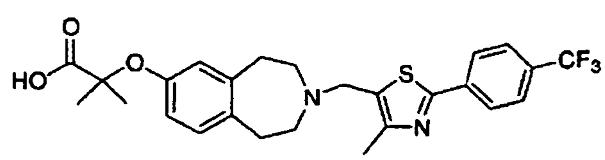

Compound 6: 2-Methyl-2-{3-[4-methyl-2-(4-trifluoromethyl-phenyl)-thiazol-5-ylmethyl]-2,3,4,5-tetrahydro-1*H*-benzo[*d*]azepin-7-yloxy}-propionic acid

The title compound was made according to Scheme F.

**Scheme F**

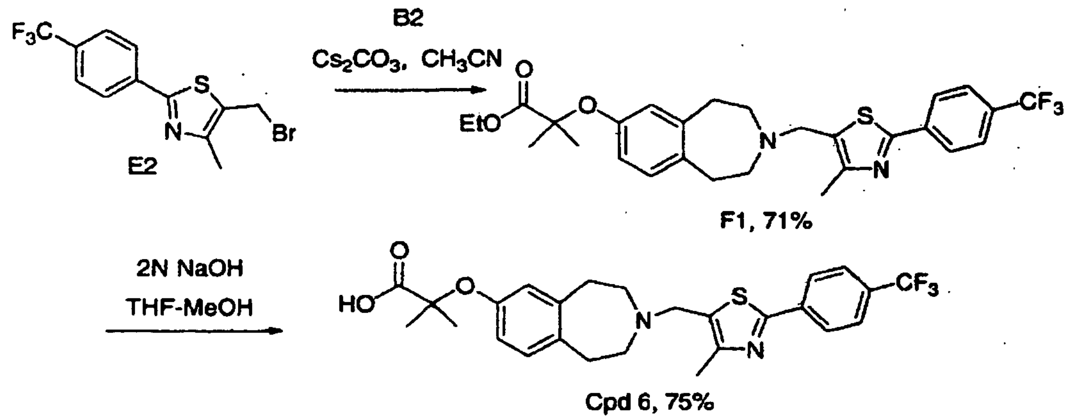


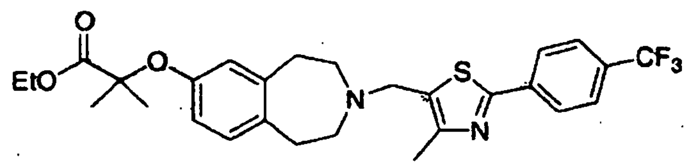

F1

**2-Methyl-2-{3-[4-methyl-2-(4-trifluoromethyl-phenyl)-thiazol-5-ylmethyl]-2,3,4,5-tetrahydro-1*H*-benzo[*d*]azepin-7-yloxy}-propionic acid ethyl ester**

Cpd F1 was prepared using a similar procedure as for cpd B3. Cpd F1 was obtained as a white solid (71%): $^1H$ NMR (300 MHz, $CDCl_3$) δ 8.02 (d, *J* = 8.2 Hz, 2 H), 7.65 (d, *J* = 8.3 Hz, 2 H), 6.93 (d, *J* = 8.2 Hz, 1 H), 6.63 (d, *J* = 2.4 Hz, 1 H), 6.56 (dd, *J* = 8.1, 2.5 Hz, 1 H), 4.23 (q, *J* = 7.1 Hz, 2 H). 3.75 (s, 2 H), 2.85 (m, 4 H), 2.68 (m, 4 H), 2.47 (s, 3 H), 1.57 (s, 6 H), 1,25 (t, *J* = 7.1 Hz, 3 H); MS (ES) m/z: 533 ($M+H^+$).

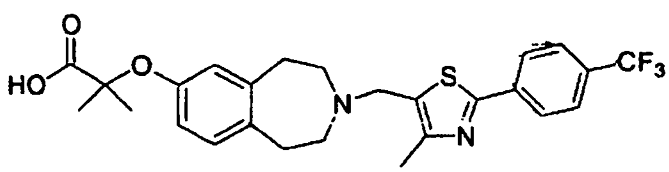

Cpd 6

**2-Methyl-2-{3-[4-methyl-2-(4-trifluoromethyl-phenyl)-thiazol-5-ylmethyl]-2,3,4,5-tetrahydro-1*H*-benzo[*d*]azepin-7-yloxy}-propionic acid**

Cpd 6 was prepared using a similar procedure as for cpd 2. Cpd 6 was obtained as a white solid (75%): $^1H$ NMR (300 MHz, $CD_3OD$) δ 8.13 (d, *J* = 8.2 Hz, 2 H), 7.80 (d, *J* = 8.3 Hz, 2 H), 7.04 (d, *J* = 8.2 Hz, 1 H), 6.75 (d, *J* = 2.4 Hz, 1 H), 6.71 (dd, *J* = 8.2, 2.5 Hz, 1 H), 4.50 (s, 2 H), 3.31 - 3.24 (m; 4 H), 3.01 - 2.97 (m, 4 H), 2.52 (s, 3 H), 1.56 (s, 6 H); MS (ES) m/z: 505 ($M+H^+$).

Example G

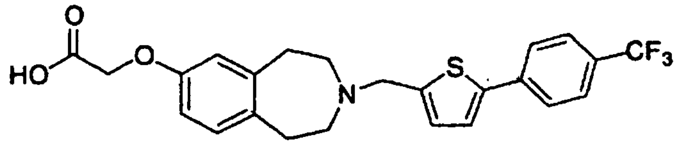

**Compound 7: {3-[5-(4-Trifluoromethyl-phenyl)-thiophen-2-ylmethyl]-2,3,4,5-tetrahydro-1*H*-benzo[*d*]azepin-7-yloxy}-acetic acid**

The title compound was made according to Schemes G1 or G2.

## Scheme G1

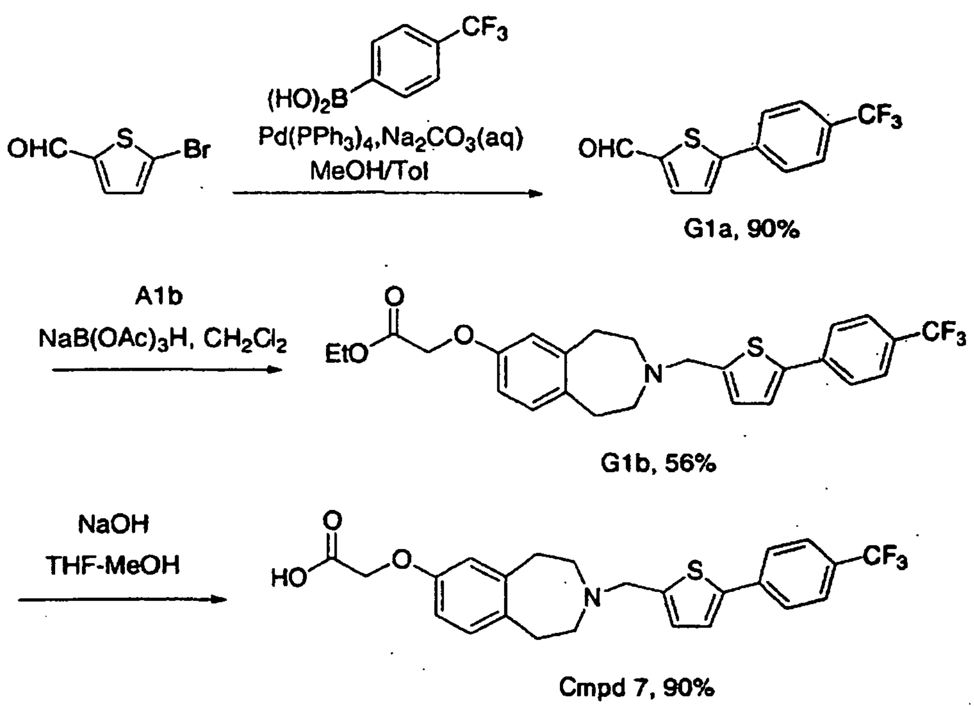


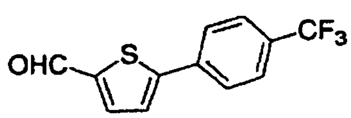

**G1a**
**5-(4-Trifluoromethyl-phenyl)-thiophene-2-carbaldehyde**

A mixture of 5-bromothiophene-2-carboxyaldehyde mg, 10.5 mmol), 4-trifluoromethyl-benzeneboronic acid (2.19 g, 11.5 mmol), $Pd(PPh_4)_3$ (605 mg, 0.52 mmol) and 2N $Na_2CO_3$ (21 mL, 42 mmol) in toluene/MeOH (30 mL/15 mL) was degassed with $N_2$ and then stirred at 80°C for 18 h. After cooling to room temperature, the reaction mixture was partitioned between EtOAc and $H_2O$ and the aqueous layer was extracted with EtOAc. The combined organic phases were dried ($Na_2SO_4$), concentrated and purified by column chromatography to give G 1 a (2.4 g, 90%) as a white solid: $^1H$ NMR (300 MHz, $CDCl_3$) δ 9.92 (s, 1 H), 7.79 - 7.77 (m, 3 H), 7.70 (d, *J* = 8.4 Hz, 2 H), 7.48 (d, *J* = 3.9 Hz, 1 H); MS (ES) m/z: 279 (M+Na$^+$).

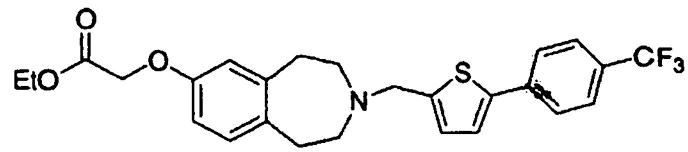

G1b

{3-[5-(4-Trifluoromethyl-phenyl)-thiophen-2-ylmethyl]-2,3,4,5-tetrahydro-1*H*-benzo[*d*]azepin-7-yloxy}-acetic acid ethyl ester

A mixture of A1b (100 mg, 0.401 mmol) and G1a (113 mg, 0.442 mmol) in dichloromethane (4 mL) was stirred at room temperature for 1.5 h. $Na(OAc)_3BH$ (170 mg, 0.803 mmol) was added and the resulting mixture was then stirred for 17 h. Saturated $NaHCO_3$ was added and the solution was extracted with $CH_2Cl_2$. The combined organic phases were dried ($Na_2SO_4$), concentrated and purified by column chromatography to give cpd G1b (110 mg, 56%) as a white solid: $^1H$ NMR (300 MHz, $CDCl_3$) δ 7.67 (d, *J* = 8.2 Hz, 2 H), 7.60 (d, *J* = 8.3 Hz, 2 H), 7.23 (d, *J* = 3.6 Hz, 1 H), 6.99 (d, *J* = 8.2 Hz, 1 H), 6.89 (d, *J* = 3.6 Hz, 1 H), 6.69 (d, *J* = 2.7 Hz, 1 H), 6.62 (dd, *J* = 8.2, 2.7 Hz, 1 H), 4.58 (s, 2 H), 4.26 (q, *J* = 7.1 Hz, 2 H), 3.85 (s, 2 H), 2.91 -2.87 (m, 4 H), 2.71 - 2.67 (m, 4 H), 1.29 (t, *J* = 7.1 Hz, 3 H); MS (ES) m/z: 490 ($M+H^+$).

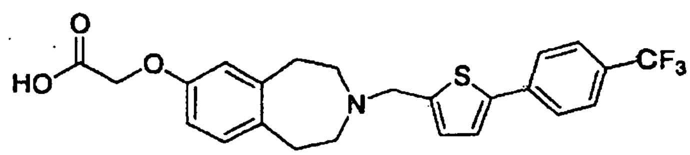

Cpd 7

{3-[5-(4-Trifluoromethyl-phenyl)-thiophen-2-ylmethyl]-2,3,4,5-tetrahydro-1*H*-benzo[*d*]azepin-7-yloxy}-acetic acid

Cpd 7 was prepared using a similar procedure, as for cpd 1. Cpd 7 was obtained as a white solid (90%): $^1H$ NMR (300 MHz, $CD_3OD$) δ 7.85 (d, *J* = 8.2 Hz, 2 H), 7.72 (d, *J* = 8.4 Hz, 2 H). 7.56 (d. *J* = 3.7 Hz, 1 H), 7.36 (d, *J* = 3.7 Hz, 1 H), 7.10 (d, *J* = 8.2 Hz, 1 H), 6.80 (d, *J* = 2.5 Hz, 1 H), 6.76 (dd, *J* = 8.2, 2.5 Hz, 1 H), 4.63 (s, 2 H), 4.58 (s, 2 H), 3.35 - 3.28 (m, 4 H), 3.13 - 3.07 (m, 4 H); MS (ES) m/z: 462 ($M+H^+$).

## Scheme G2

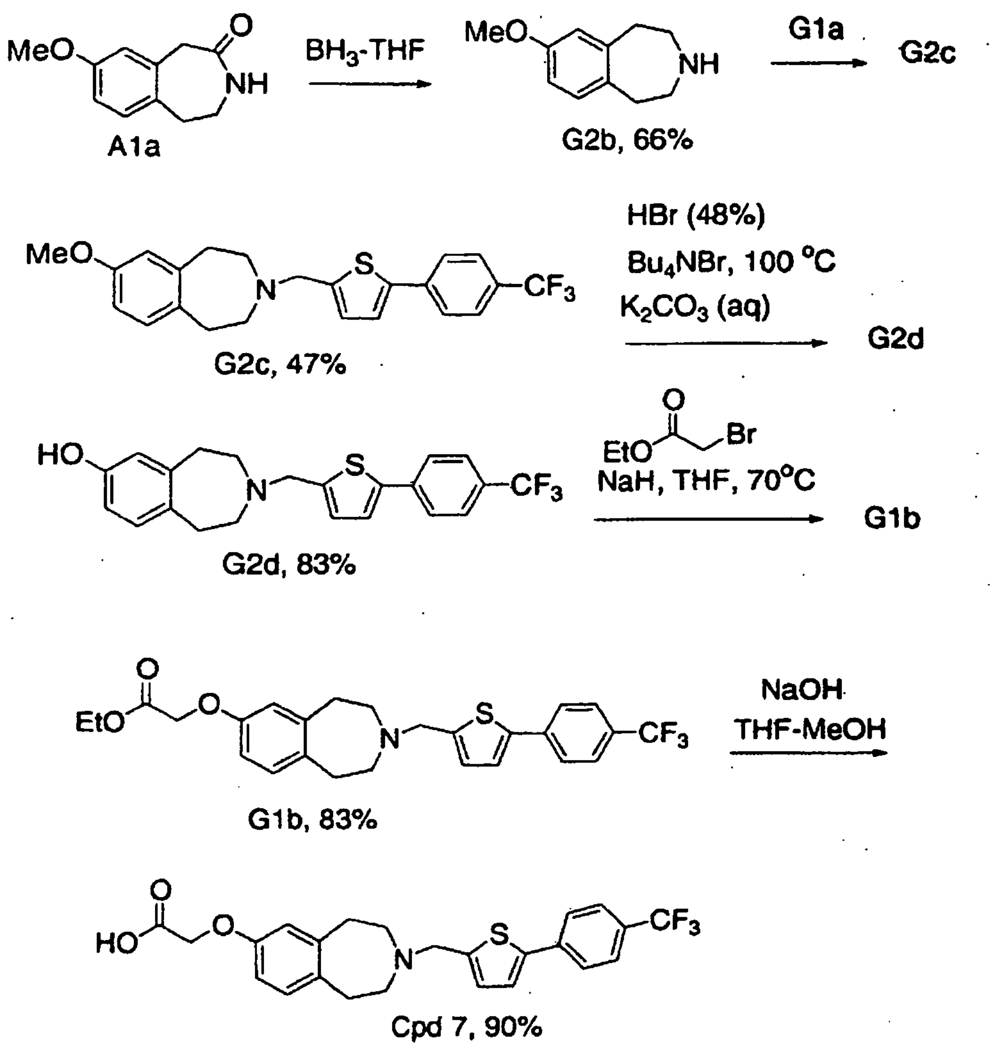


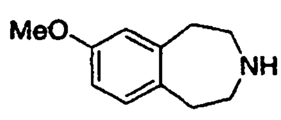

**G2b**

**7-Methoxy-2,3,4,5-tetrahydro-1*H*-benzo[*d*]azepine**

1 M borane-THF solution (1 mL, 1.02 mmol) was added dropwise to an ice-cooled and stirred solution of A1a (1.91 g, 10 mmol, in THF (50 mL). A1a was prepared according to published procedures ( Eur. Pat. Appl. 204349). The ice bath was removed and the solution was heated at reflux for 3 h. Upon cooling back to 0 ˚C, MeOH (2 mL) was added and the reaction mixture was stirred at room temperature for 35 min and concentrated. The white solid residue was treated with 6 N HCl (50 mL) and the mixture was heated at reflux for 1 h and then room temperature overnight. The aqueous solution was washed with $Et_2O$ and then basified with 5 N NaOH until PH > 10 and extracted with EtOAc. The combined organic phases were washed with brine, dried ($Na_2SO_4$), concentrated and vacuum dried to give G2b (1.17 g, 66%) as a clear yellow oil: $^1H$ NMR (300 MHz, $CDCl_3$) δ 7.01 (d, J= 8.1 Hz, 1 H), 6.70 - 6.60 (m, 2 H), 3.78 (s, 3 H), 3.01 - 2.83 (m, 8 H), 2.34 (br, 1 H).

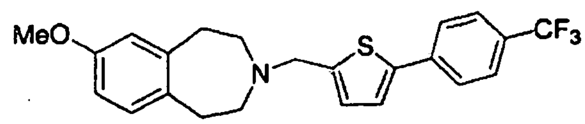

G2c

**7-Methoxy-3-[5-(4-trifluoromethyl-phenyl)-thiophen-2-ylmethyl]-2,3,4,5-tetrahydro-1*H*-benzo[*d*]azepine**

To a solution of G2b (2.02 g, 11.4 mmol) and G1a (2.92 g, 11.4 mmol) in dichloromethane (50 mL) was added AcOH (0.65 mL, 11.4 mmol). The reaction mixture was stirred for 1.5 h. $Na(OAc)_3BH$ (3.62 g, 17.1 mmol) was added and the reaction mixture was then stirred for another 20 h. 2 N NaOH was added (PH ~ 11) and the solution was extracted with EtOAc. The combined organic phases were dried ($Na2SO_4$), concentrated and purified by column chromatography to give G2c (2.23 g, 47%) as a yellow solid: $^1$H NMR (300 MHz, $CDCl_3$) δ 7.68 (d, *J* = 8.2 Hz, 2 H), 7.61 (d, *J* = 8.5 Hz, 2 H), 7.24 (d, *J* = 3.8 Hz, 1 H), 7.01 (d, *J* = 3.8 Hz, 1 H), 6.91 (m, 1 H), 6.66 - 6.62 (m, 2 H), 3.88 (s, 2 H), 3.77 (s, 3 H), 2.91 (m, 4 H), 2.72 (m, 4 H); MS (ES) m/z: 418 ($M+H^+$).

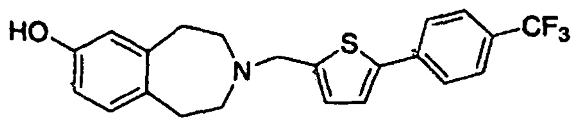

G2d

**3-[5-(4-Trifluoromethyl-phenyl)-thiophen-2-ylmethyl]-2,3,4,5-tetrahydro-1*H*-benzo[*d*]azepin-7-ol**

A mixture of G2c (2.21 g, 5.30 mmol), HBr (48%, 6.0 mL, 53.0 mmol) and $Bu_4NBr$ (171 mg, 0.53 mmol) in AcOH (6 mL) was stirred at 100 °C for 16.5 h. Saturated $K_2CO_3$ was added till PH - 10 and the solution extracted with EtOAc. The combined organic phases were dried ($Na_2SO_4$) and concentrated to give G2d (1.77 g, 83%) as a biege solid: $^1$H NMR (400 MHz, $CDCl_3$) δ 7.66 (d, *J* = 8.4 Hz, 2 H), 7.61 (d, *J* = 8.4 Hz, 2 H), 7.25 (d, *J* = 3.6 Hz, 1 H), 6.97 (d, *J* = 3.3 Hz, 1 H), 6.94 (d, *J* = 7.7 Hz, 1 H), 6.60 - 8.57 (m, 2 H), 4.01 (s, 2 H), 2.94 (m, 4 H), 2.82 (m, 4 H); MS (ES) m/z: 404 ($M+H^+$).

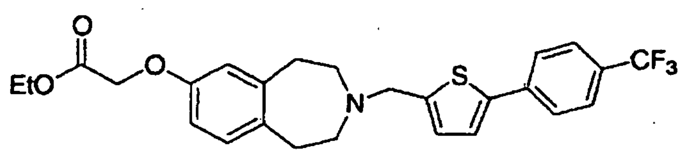

G1b

**{3-[5-(4-Trifluoromethyl-phenyl)-thiophen-2-ylmethyl]-2,3,4,5-tetrahydro-1*H*-benzo[*d*]azepin-7-yloxy}-acetic acid ethyl ester**

To a solution of NaH (60% in mineral oil, 317 mg, 7.93 mmol) in THF (12 mL) was added G2d (1.07 g, 2.64 mmol) in THF (5 mL) followed by ethyl bromoacetate (0.35 mL, 3.17 mmol). After stirring at reflux for 1 h, the reaction mixture was cooled, quenched with saturated $NH_4Cl$ solutions and partitioned between ether and water. The organic phase was dried ($Na_2SO_4$), concentrated and purified by column chromatography to give G1b (1.02 g, 79%), which was further converted to Compound 7 as described above.

Example H

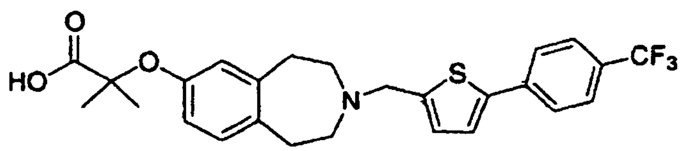

**Compound 8: 2-Methyl-2-{3-[5-(4-trifluoromethyl-phenyl)-thiophen-2-ylmethyl]-2,3,4,5-tetrahydro-1*H*-benzo[*d*]azepin-7-yloxy}-propionic acid**

The title compound was made according to Scheme H.

## Scheme H

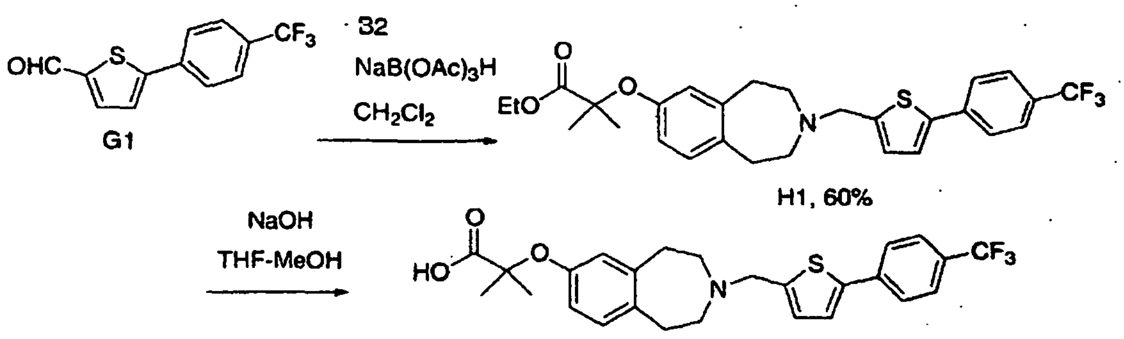


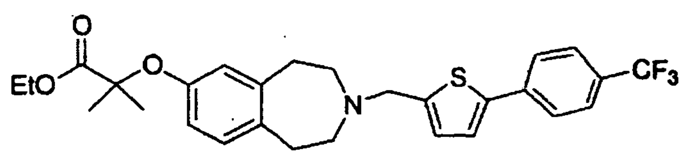

**H1**

**2-Methyl-2-{3-[5-(4-trifluoromethyl-phenyl)-thiophen-2-ylmethyl]-2,3,4,5-tetrahydro-1*H*-benzo[*d*]azepin-7-yloxy}-propionic acid ethyl ester**

Cpd H1 was prepared according to a similar procedure as for cpd G2. Cpd H1 was obtained as a white solid (60%): $^1$H NMR (300 MHz, $CDCl_3$) δ 7.67 (d, *J* = 8.4 Hz, 2 H), 7.60 (d, *J* = 8.5 Hz, 2 H), 7.24 (d, *J* = 3.6 Hz, 1 H), 6.94 - 6.91 (m, 2 H), 6.62 (d, *J* = 2.5 Hz, 1 H), 6.55 (dd, *J* = 8.2, 2.6 Hz, 1 H), 4.23 (q, *J* = 7.1 Hz, 2 H), 3.90 (s, 2 H), 2.90 (m, 4 H), 2.74 (m, 4 H), 1.57 (s, 6 H), 1.24 (t, *J* = 7.1 Hz, 3 H); MS (ES) m/z: 518 ($M+H^+$).

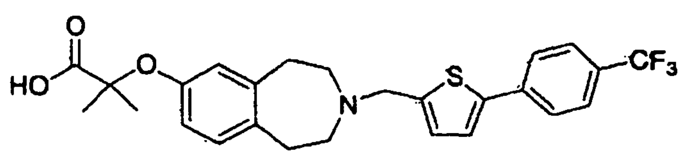

**Cpd 8**

**2-Methyl-2-{3-[5-(4-trifluoromethyl-phenyl)-thiophen-2-ylmethyl]-2,3,4,5-tetrahydro-1*H*-benzo[*d*]azepin-7-yloxy}-propionic acid**

Cpd 8 was prepared according to a similar procedure as forced 2. Cpd 8 was obtained as a white solid (61%): $^1$H NMR (300 MHz, $CD_3OD$) δ 7.86 (d, *J* = 7.9 Hz, 2 H), 7.73 (d, *J* = 8.3 Hz, 2 H), 7.58 .(d, *J* = 2.7 Hz, 1 H), 7.38 (d, *J* = 3.7 Hz, 1 H), 7.11 (d, *J* = 3.7 Hz, 1 H), 6.80 (d, *J* = 2.5 Hz, 1 H), 6.73 (dd, *J* = 8.2, 2.5 Hz, 1 H), 4.68 (s, 2 H), 3.21 - 3.00 (m, 8 H), 1.54 (s,6 H); MS (ES) m/z: 490 ($M+H^+$).

Example I

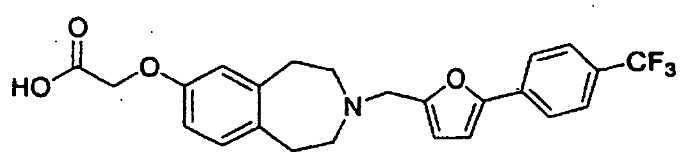

**Compound 9: {3-[5-(4-Trifluoromethyl-phenyl)-thiophen-2-ylmethyl]-2,3,4,5-tetrahydro-1*H*-benzo[*d*]azepin-7-yloxy}-acetic acid**

The title compound was made according to Scheme 1.

## Scheme I

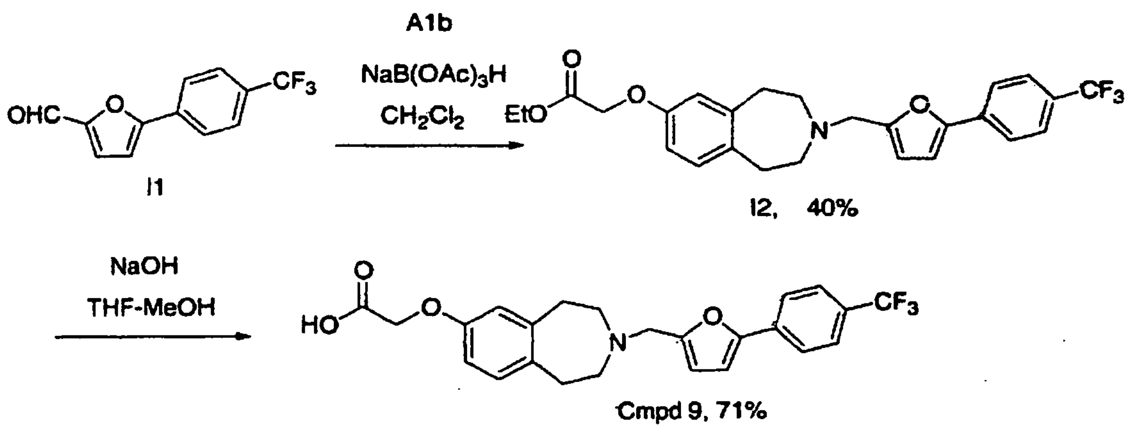


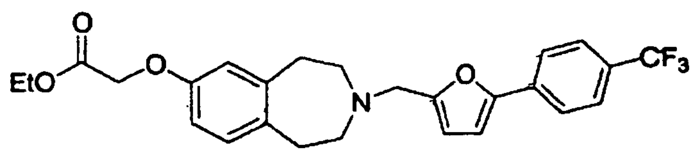

I2

**{3-[5-(4-Trifluoromethyl-phenyl)-furan-2-ylmethyl]-2,3,4,5-tetrahydro-1*H*-benzo[*d*]azepin-7-yloxy}-acetic acid ethyl ester**

Cud 12 was prepared from cpd I1 (Bioorg. & Med. Chem. Lett, 2003, 13(13), 2159-2161) using a similar procedure as for cpd G2. Cpd I2 was obtained as a white solid (40%): $^1$H NMR (300 MHz, $CDCl_3$) δ 7.72 (d, *J* = 8.2 Hz, 2 H), 7.60 (d, *J* = 8.5 Hz, 2 H), 6.98 (d, *J* = 8.2 Hz, I H), 6.68 (m, 2 H), 6.60 (dd, *J* = 8.2, 2.6 Hz, 1 H), 6.30 (d, *J* = 3.3 Hz, 1 H), 4.56 (s, 2 H), 4.26 (q, *J* = 7.1 Hz, 2 H), 3.79 (s, 2 H), 2.92 - 2.87 (m, 4 H), 2.73 - 2.67 (m, 4 H), 1.28 (t, *J* = 7.1 Hz, 3 H); MS (ES) m/z: 474 (M+H$^+$).

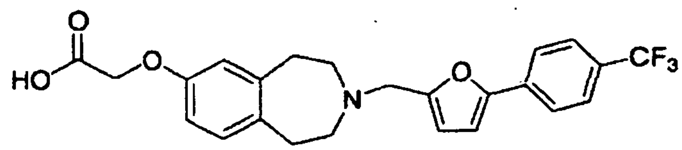

Cpd 9

**{3-[5-(4-Trifluoromethyl-phenyl)-thiophen-2-ylmethyl]-2,3,4,5-tetrahydro-1*H*-benzo[*d*]azepin-7-yloxy}-acetic acid**

Cpd 9 was prepared according to a similar procedure as for cpd 1. Cpd 9 was obtained as a white solid (71%): $^1$H NMR (300 MHz, $CD_3OD$ δ 7.89 (d, *J* = 8.2 Hz, 2 H), 7.69 (d, *J* = 8.3 Hz, 2 H), 6.98 - 6.94 (m, 2 H), 6.75 - 6.69 (m, 3 H), 4.44 (s, 2 H), 4.33 (s, 2 H), 3.14 (m, 4 H), 2.82 (m, 4 H); MS (ES) m/z: 446 ($M+H^+$).

Example J

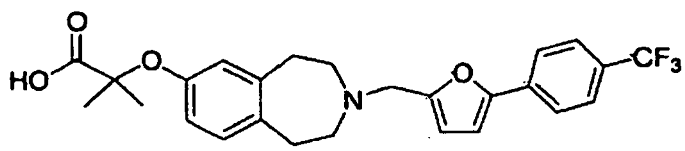

**Compound 10: 2-Methyl-2-{3-[5-(4-trifluoromethyl-phenyl)-furan-2-ylmethyl]-2,3,4,5-tetrahydro-1*H*-benzo[*d*]azepin-7-yloxy}-propionic acid**

The title compound was made according to Scheme J.

## Scheme J

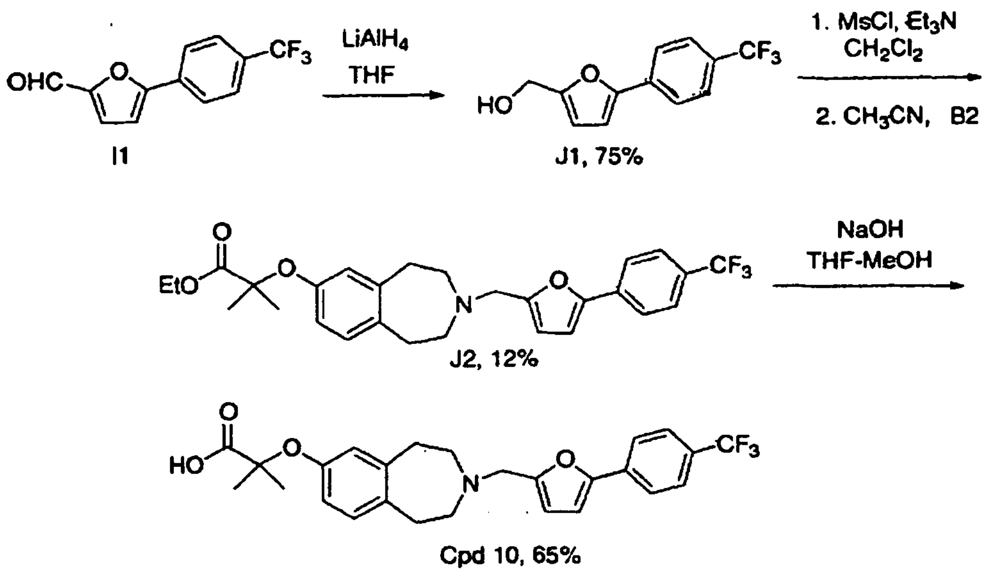


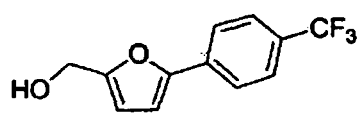

**J1**

**[5-(4-Trifluoromethyl-phenyl)-furan-2-yl]-methanol**

Cpd J1 was prepared according to a similar procedure as for cpd E1. Cpd J1 was obtained as a white solid (75%): $^1$H NMR-1300 MHz, $CDCl_3$) δ 7.76 (d, *J* = 8.4 Hz, 2 H), 7.62 (d, *J* = 8.5 Hz, 2 H), 6.73 (d, *J* = 3.3 Hz, 1 H), 6.42 (d, *J* = 3.2 Hz, 1 H), 4.69 (d, *J* = 5.9 Hz, 2 H), 1.75 (t, *J* = 6.0 Hz, 1 H); MS (ES) m/z: 225 (M-OH).

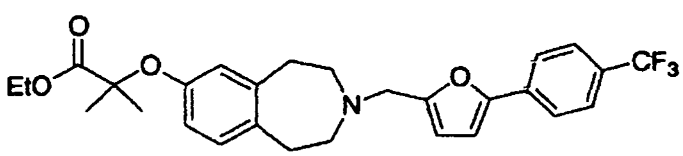

**J2**

**2-Methyl-2-{3-[5-(4-trifluoromethyl-phenyl)-furan-2-ylmethyl]-2,3,4,5-tetrahydro-1*H*-benzo[*d*]azepin-7-yloxy}-propionic acid ethyl ester**

A mixture of J1 (80 mg, 0.33 mmol), methanesulfonyl chloride (38 mg, 0.33 mmol) and triethylamine (230 μL, 1.65 mmol) in $CH_2Cl_2$ (2 mL) was stirred at room temperature for 1.5 h. Cpd B2 (50 mg, 0.165 mmol) in $CH_3CN$ (1 mL) was added and the solution was stirred overnight under $N_2$. The resulting mixture was concentrated and purified by column chromatography (EtOAc/hexane) to give 10 mg (12%, 2 steps) of J2 as a white solid: $^1$H NMR (300 MHz, $CDCl_3$) δ 7.72 (d, *J* = 8.2 Hz, 2 H), 7.60 (d, *J* = 8.4 Hz, 2 H), 6.91 (d, *J* = 8.2 Hz, 1 H), 6.69 (d, *J* = 3.3 Hz, 1 H), 6.61 (d, *J* = 2.3 Hz, 1 H), 6.54 (dd, *J* = 8.1, 2.2 Hz, 1 H), 6.30 (d, *J* = 3.2 Hz, 1 H), 4.21 (q, *J* = 7.1 Hz, 2 H), 3.79 (s, 2 H), 2.87 (m, 4 H), 2.71 (m, 4 H), 1.56 (s, 6 H), 1.23 (t, *J* = 7.1 Hz, 3 H); MS (ES) m/z: 502 (M+H$^+$).

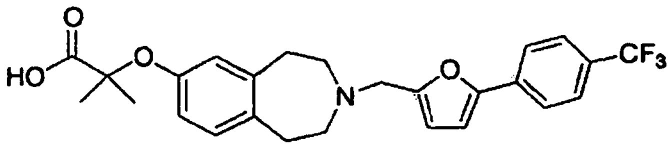

Cpd 10

2-Methyl-2-{3-[5-(4-trifluoromethyl-phenyl)-furan-2-ylmethyl]-2,3,4,5-tetrahydro-1*H*-benzo[*d*]azepin-7-yloxy}-propionic acid

Cpd 10 was prepared using a similar procedure as for cpd 2. Cpd 2 was obtained as a white solid (65%): $^1$H NMR (300 MHz, $CD_3OD$) δ 7.94 (d, *J* = 8.1 Hz, 2 H), 7.73 (d, *J* = 8.2 Hz, 2 H), 7.10-7.05 (m, 2 H), 6.87 (d. *J* = 3.2 Hz, 1 H), 6.78 (d, *J* = 2.5 Hz, 1 H), 6.72 (dd, *J* = 8.1, 2.5 Hz, 1 H), 4.54 (s, 2 H), 3.40 (m, 4 H), 3.11 (m, 4 H), 1.54 (s, 6 H); MS (ES) m/z: 474 ($M+H^+$).

Example K

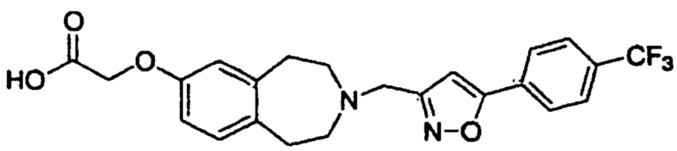

Compound 11: {3-[5-(4-Trifluoromethyl-phenyl)-isoxazol-3-ylmethyl]-2,3,4,5-tetrahydro-1*H*-benzo[*d*]azepin-7-yloxy}-acetic acid

The title compound was made according to Schemes K1 and K2.

## Scheme K1

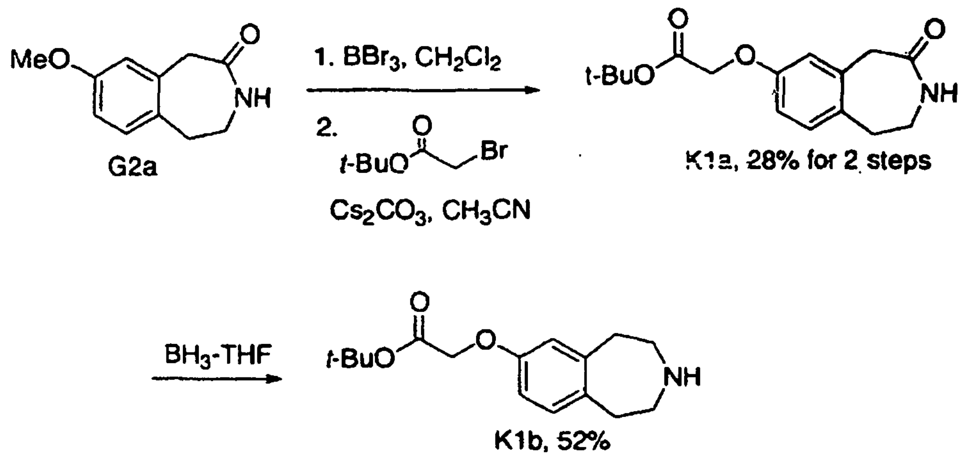


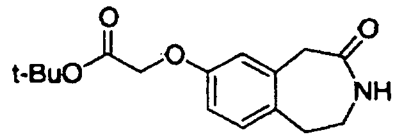

**K1a**

**(4-Oxo-2,3,4,5-tetrahydro-1*H*-benzo[*d*]azepin-7-yloxy)-acetic acid *tert*-butyl ester**

Cpd K1 a was prepared according to a similar procedure as for cpd A1b. Cpd K1a was obtained as a white solid (28% over 2 steps): $^1H$ NMR (300 MHz, $CDCl_3$) δ 7.03 (d, *J* = 8.4 Hz, 1 H), 6.76 (dd, *J* = 8.4,2.7 Hz, 1 H), 6.67 (d, *J* = 2.6 Hz, 1 H), 5.79 (brs, 1 H), 4.48 (s, 2 H), 3.79 (s, 2 H), 3.58 - 3.52 (m, 2 H), 3.06 (t, *J* = 6.6. Hz, 2 H), 1.49 (s, 9 H); MS (ES) m/z: 314 ($M+Na^+$).

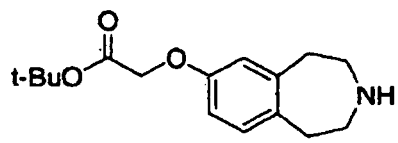

**K1b**

**(2,3,4,5-Tetrahydro-1*H*-benzo[*d*]azepin-7-yloxy)-acetic acid *tert*-butyl ester**

Cpd K1 b was prepared using a similar procedure as for cpd A1 c. Cpd K1b was obtained as a yellow oil (52%): $^1H$ NMR (300 MHz, $CDCl_3$) δ 6.98 (d, *J* = 8.3 Hz, 1 H), 6.68 (d, *J* = 2.6 Hz, 1 H), 6.63 (dd, *J* = 8.2, 2.6 Hz, 1 H), 4.48 (s, 2 H), 2.93 - 2.84 (m, 8 H), 1.49 (s, 9 H); MS (ES) m/z: 278 ($M+H^+$).

## Scheme K2

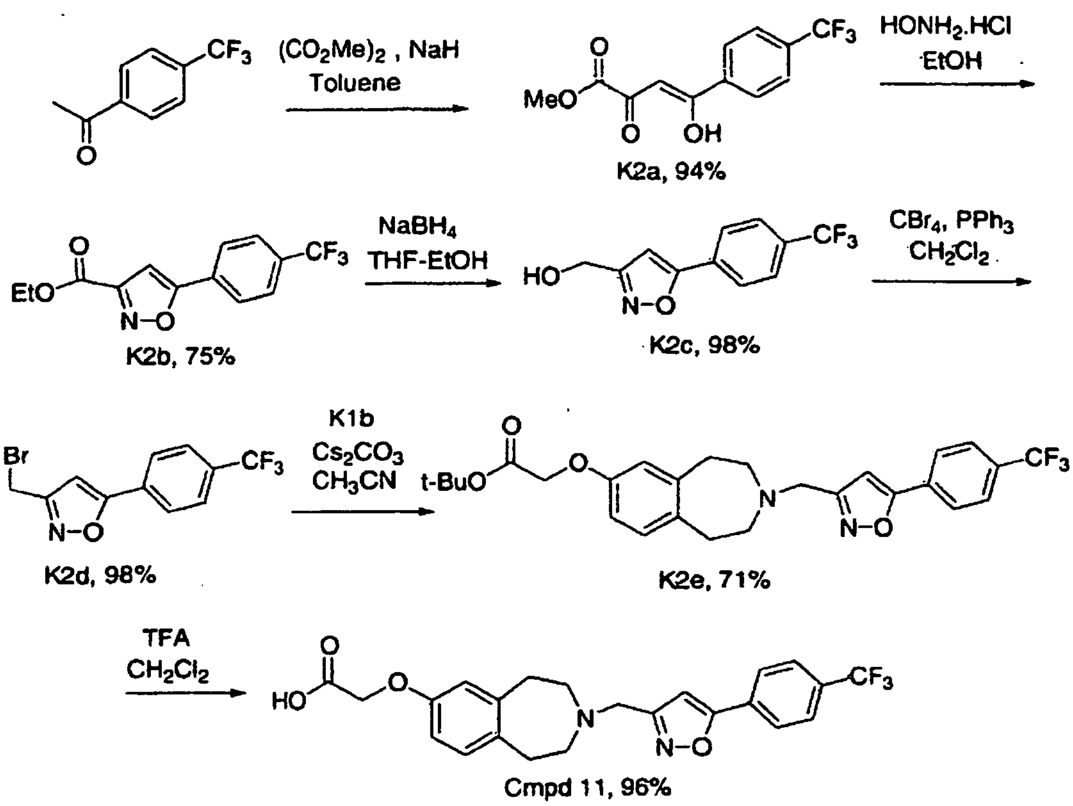


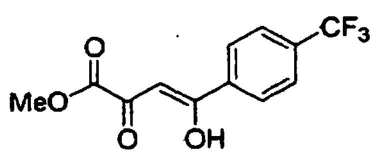

**K2a**

**4-Hydroxy-2-oxo-4-(4-trifluoromethyl-phenyl)-but-3-enoic acid methyl ester**

To a solution of 1-(4-trifluoromethyl-phenyl)ethanone (2.0 g, 10.6 mmol) and dimethyl oxylate (1.63 g, 13.8 mmol) in toluene 450 mL) at 0 °C was added NaH portionwise (60%, 636 mg, 15.9 mmol). The mixture was stirred at room temperature for 1 h and then 60 °C for 2 h. After cooling to room temperature, $H_2O$ was added slowly to quench the reaction. The mixture was acidified with 1 N HCl and extracted with EtOAc. The combined organic phases were dried ($Na_2SO_4$), concentrated and purified by column chromatography to give 2.74 g (94%) of K2a as a white solid: $^1H$ NMR (300 MHz, $CDCl_3$) δ 8.11 (d, *J* = 8.3 Hz, 2 H), 7.77 (d, *J* = 8.3 Hz, 2 H), 7.10 (s, 2 H), 3.97 (s, 3 H); MS (ES) m/z: 297 ($M+Na^+$).

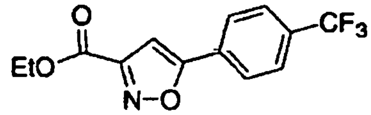

**K2b**

**5-(4-Trifluoromethyl-phenyl)-isoxazole-3-carboxylic acid ethyl ester**

To a solution of K2a (2.7 g, 9.85 mmol) in EtCH (40 mL) was added hydroxylamine hydrogen chloride (2.05 g, 29.5 mmol). The mixture was stirred at room temperature for 1 h and then 80 °C for 2 h. After cooling to room temperature, the precipitate was filtered and washed with ETOH. The white solid was dried under vacuum to give 2.0 g (75%) of K2b: $^1H$ NMR (300 MHz, $CDCl_3$) δ 7.94 (d, *J* = 8.2 Hz, 2 H), 7.76 (d, *J* = 8.3 Hz, 2 H), 7.03 (s, 1 H), 4.49 (d, *J* = 7.1 Hz, 2 H), 1.45 (d, *J* = 7.1 Hz, 3 H); MS (ES) m/z: 286 ($M+H^+$).

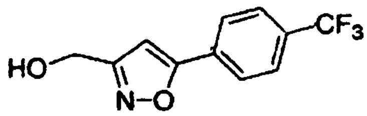

K2c
[5-(4-Trifluoromethyl-phenyl)-isoxazol-3-yl]-methanol

Cpd K2c was prepared according to a similar procedure as for cpd C2. Cpd K2c was obtained as a white solid (98%): $^1H$ NMR (300 MHz, $CDCl_3$) δ 7.90 (d, *J* = 8.2 Hz, 2 H), 7.73 (d, *J* = 8.5 Hz, 2 H), 6.70 (s, 1 H), 4.84 (s, 2 H); MS (ES) m/z: 244 ($M+H^+$).

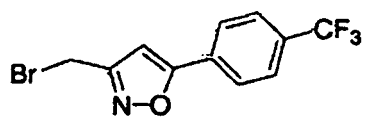

K2d
3-Bromomethyl-5-(4-trifluoromethyl-phenyl)-isoxazole

Cpd K2d was prepared according to a similar procedure as for cpd A2d. Cpd K2d was obtained as a white solid (98%); $^1H$ NMR (300 MHz, $CDCl_3$) δ 7.91 (d, *J* = 8.3 Hz, 2 H), 7.75 (d, *J* = 8.3 Hz, 2 H), 6.73 (s, 2 H), 4.49 (s, 2 H); MS (ES) m/z: 306 ($M+H^+$).

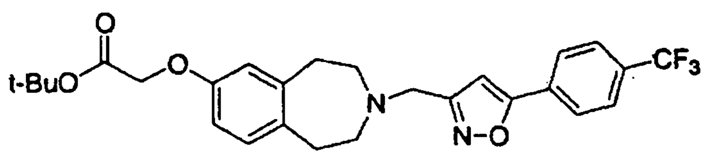

K2e
{3-[5-(4-Trifluoromethyl-phenyl)-isoxazol-3-ylmethyl]-2,3,4,5-tetrahydro-1*H*-benzo[*d*]azepin-7-yloxy}-acetic acid *tert*-butyl ester

Cpd K2e was prepared according to a similar procedure as for cpd A2e. Cpd K2e was obtained as a white solid (71%): $^1H$ NMR (300 MHz, $CDCl_3$) δ 7.90 (d, *J* = 8.4 Hz, 2 H), 7.72 (d, *J* = 8.4 Hz, 2 H), 6.98 (d, *J* = 8.2 Hz, 1 H), 6.69 (s, 1 H), 6.68 (d, *J* = 2.6 Hz, 1 H), 6.61 (dd, *J* = 8.2, 2.6 Hz, 1 H), 4.47 (s, 2 H), 3.79 (s, 2 H), 2.90 - 2.86 (m, 4 H), 2.72 - 2.67 (m, 4 H), 1.48 (s, 9 H); MS (ES) m/z: 503 ($M+H^+$).

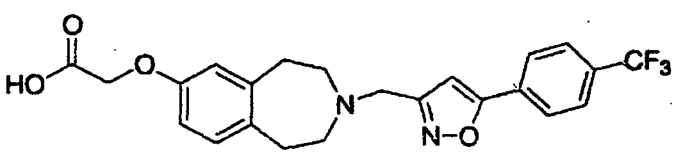

Cmpd 11
{3-[5-(4-Trifluoromethyl-phenyl)-isoxazol-3-ylmethyl]-2,3,4,5-tetrahydro-1*H*-benzo[*d*]azepin-7-yloxy}-acetic acid

To a solution of K2e (38.5 mg, 0.076 mmol) in $CH_2Cl_2$ (1 mL) was added trifluoroacetic acid (0.1 mL). The mixture was stirred at room temperature for 15 h, concentrated and purified by column chromatography to give 30 mg (96%) of compound 11 as a white solid: $^1H$ NMR (300 MHz, $CD_3OD$) δ 8.08 (d, *J* = 8.2 Hz, 2 H), 7.86 (d, *J* = 8.3 Hz, 2

H), 7.17 - 7.13(m, 2 H), 6.85 (d, *J* = 2.5 Hz, 1 H), 6.78 (dd, *J* = 8.2, 2.6 Hz, 1 H), 4.64 (s, 4 H), 3.5 (m, 4 H), 3.18 (m, 4 H); MS (ES) m/z: 447 (M+H+).

Example L

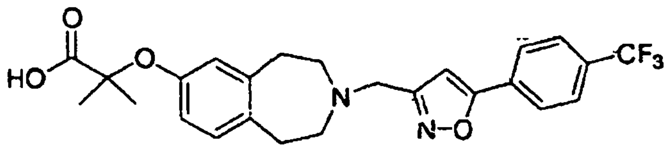

**Compound 12: 2-Methyl-2-{3-[5-(4-trifluoromethyl-phenyl)-isoxazol-3-ylmethyl]-2,3,4,5-tetrahydro-1*H*-benzo[*d*]azepin-7-yloxy}-propionic acid**

The title compound was made according to Scheme L.

**Scheme L**

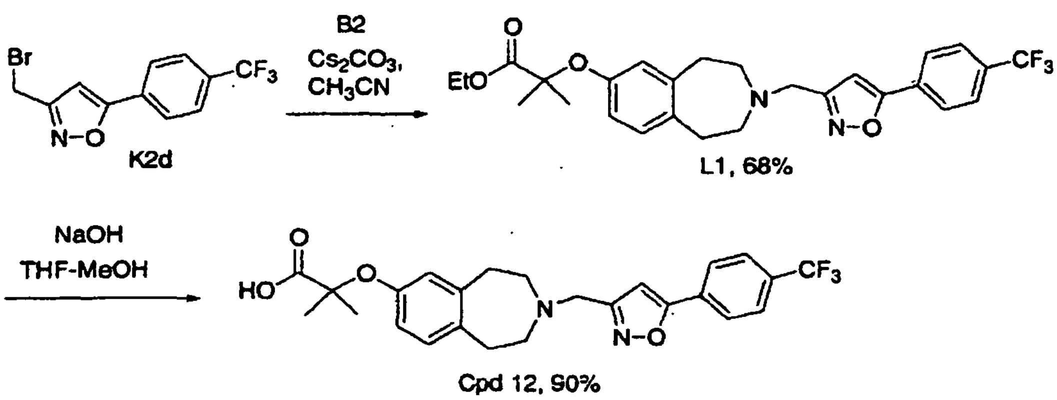


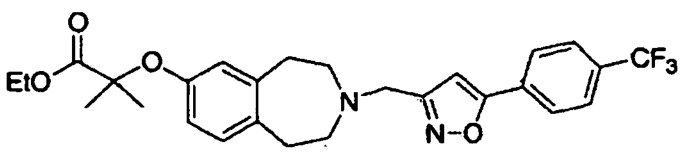

L1

**2-Methyl-2-{3-[5-(4-trifluoromethyl-phenyl)-isoxazol-3-ylmethyl]-2,3,4,5-tetrahydro-1*H*-benzo[*d*]azepin-7-yloxy}-propionic acid ethyl ester**

Cpd L1 was prepared following the same procedure as for cpd B3. Cpd L1 was obtained as a white solid (68%): $^1$H NMR (300 MHz, $CDCl_3$) δ 7.90 (d, *J* = 8.3 Hz, 2 H), 7.72 (d, *J* = 8.4 Hz, 2 H), 6.93 (d, *J* = 8.2 Hz, 1 H), 6.71 (s, 1 H), 6.63 (d, *J* = 2.5 Hz, 1 H), 6.55 (dd, *J* = 8.1, 2.6 Hz, 1 H), 4.23 (q, *J* = 7.1 Hz, 2 H), 3.81 (s, 2 H), 2.88 - 2.86 (m, 4 H), 2.73 - 2.70 (m, 4 H), 1.57 (s, 6 H), 1.25 (t, *J* = 7.1 Hz, 3 H); MS (ES) m/z: 503 (M+H+).

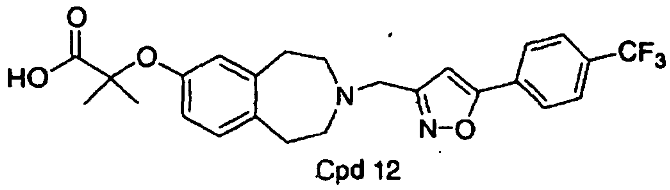

Cpd 12

**2-Methyl-2-{3-[5-(4-trifluoromethyl-phenyl)-isoxazol-3-ylmethyl]-2,3,4,5-tetrahydro-1*H*-benzo[*d*]azepin-7-yloxy}-propionic acid**

Cpd 12 was prepared following the same procedure as for cpd 2. Cpd 12 was obtained as a white solid (90%): $^1H$ NMR (300 MHz, $CD_3OD$) δ 8.08 (d, *J* = 8.0 Hz, 2 H), 7.86 (d, *J* = 8.4 Hz, 2 H), 7.16 (s, 1H), 7.11 (d, *J* = 8.3 Hz, 1 H), 6.80 (d, *J* = 2.5 Hz, 1 H), 6.73 (dd, *J* = 8.2, 2.6 Hz, 1 H), 4.62 (s, 2 H), 3.54 (m, 4 H), 3.16 (m, 4 H), 1.55 (s, 6 H); MS (ES) m/z: 475 ($M+H^+$).

Example M

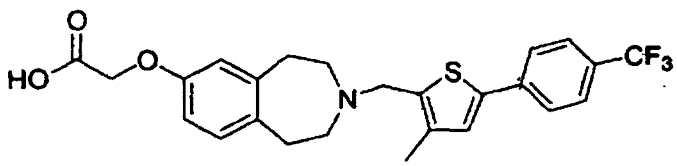

**Compound 13: {3-[3-Methyl-5-(4-trifluoromethyl-phenyl)-thiophen-2-ylmethyl]-2,3,4,5-tetrahydro-1*H*-benzo[*d*]azepin-7-yloxy}-acetic acid**

The title compound was made according to Scheme M.

**Scheme M**

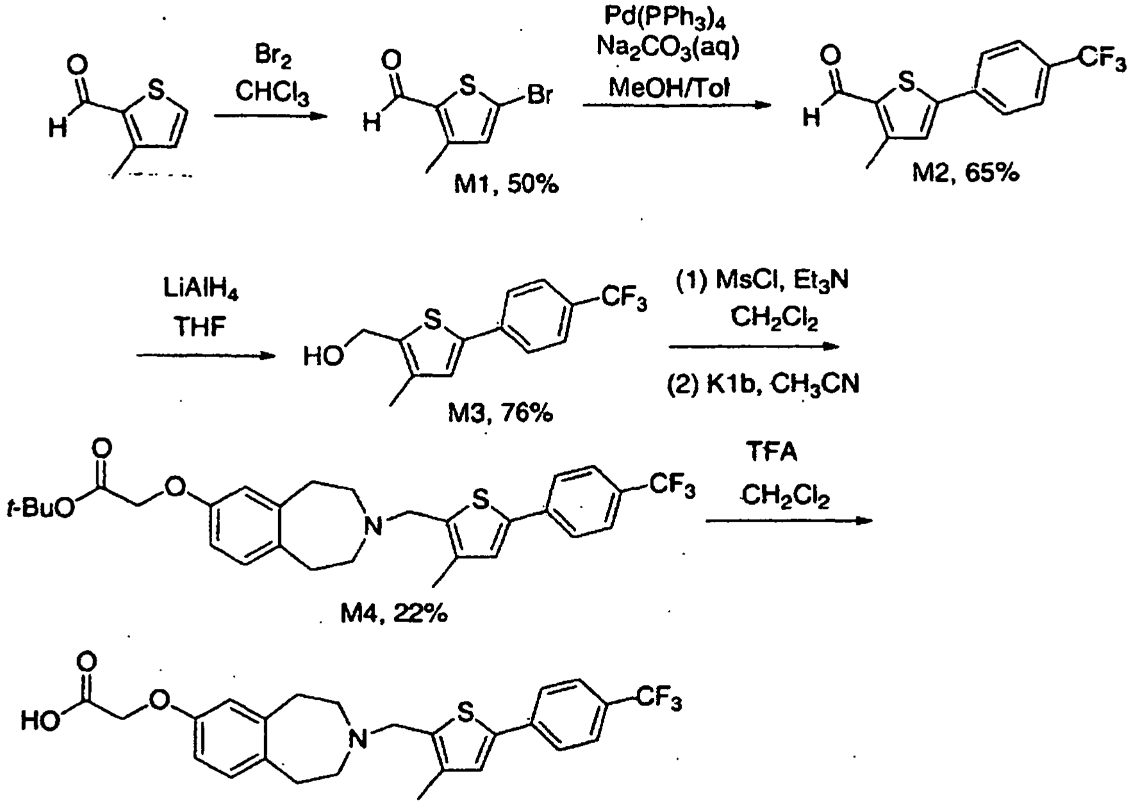


Cpd 13, 71%

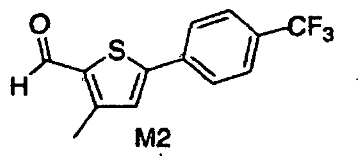

M2

**3-Methyl-5-(4-trifluoromethyl-phenyl)-thiophene-2-carbaldehyde**

Cpd M2 was prepared from cpd M1 (*J. Chem. Soc.*, Perkin Trans 2, **1972**, 1866) following the same procedure as for cpd G1a. Cpd M2 was obtained as a white crystalline solid (65%): $^1$H NMR (300 MHz, $CDCl_3$) δ 10.05 (s, 1 H), 7.75 (d, *J* = 8.5 Hz, 2 H), 7.67 (d, *J* = 8.5 Hz, 2 H), 7.26 (s, 1 H), 2.61 (s, 3 H); MS (ES) m/z: 293 (M+Na$^+$).

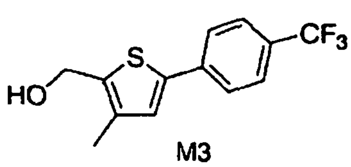

M3

**[3-Methyl-5-(4-trifluoromethyl-phenyl)-thiophen-2-yl]-methanol**

Cpd M3 was prepared according to the same procedure as for cpd E1. Cpd M3 was obtained as a white solid (76%): $^1$H NMR (300 MHz, $CDCl_3$) δ7.65 (d, *J* = 8.6 Hz, 2 H), 7.60 (d, *J* = 8.5 Hz, 2 H), 7.14 (s, 1 H), 4.79 (s, 2 H), 2.27 (s, 3 H), 1.68 (brs, 1 H); MS (ES) m/z: 255 (M-OH).

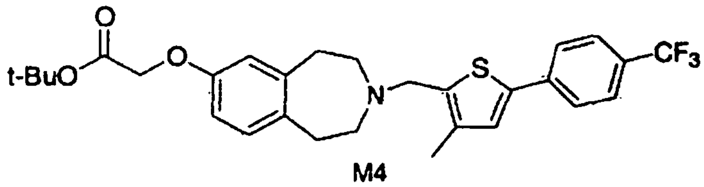

**M4**

**{3-[3-Methyl-5-(4-trifluoromethyl-phenyl)-thiophen-2-ylmethyl]-2,3,4,5-tetrah ydro-1*H*-benzo[*d*]azepin-7-yloxy}-acetic acid *tert*-butyl ester**

Cpd M4 was prepared following the same procedure as for cpd J2. Cpd M4 was obtained as a white solid (22%): $^1H$ NMR (300 MHz, $CDCl_3$) δ 7.05 (d, *J* = 8.2 Hz, 2 H), 7.58 (d, *J* = 8.4 Hz, 2 H), 7.11 (s, 1 H), 6.99 (d, *J* = 8.2 Hz, 1 H), 6.68 (d, *J* = 2.6 Hz, 1 H), 6.61 (dd, *J* = 8.2, 2.7 Hz, 1 H), 4.48 (s, 2 H), 3.74 (s, 2 H), 2.88 (m, 4 H), 2.69 (m, 4 H), 2.20 (s, 3 H), 1.48 (s, 9 H); MS (ES) m/z: 532 ($M+H^+$).

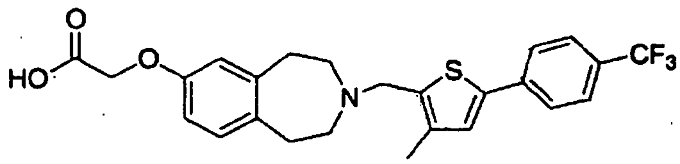

**Cpd 13**

**{3-[3-Methyl-5-(4-trifluoromethyl-phenyl)-thiophen-2-ylmethyl]-2,3,4,5-tetrah ydro-1*H*-benzo[*d*]azepin-7-yloxy}-acetic acid**

Cpd 13 was prepared following the same procedure as for cpd 11. Cpd 13 was obtained as a white solid (71%): $^1H$ NMR (300 MHz, $CD_3OD$) δ 7.79 (d, *J* = 8.2 Hz, 2 H), 7.67 (d, *J* = 8.4 Hz, 2 H), 7.36 (s, 1 H), 6.98 (d, *J* = 9.0 Hz, 1 H), 6.73 - 6.70 (m, 2 H), 4.45 (s, 2 H), 4.36 (s, 2 H), 3.17 (m, 4 H), 2.84 (m, 4 H), 2.29 (s, 3 H); MS (ES) m/z: 476 ($M+H^+$).

Example N

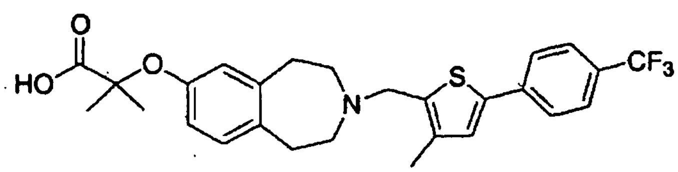

**Compound 14: 2-Methyl-2-{3-[3-methyl-5-(4-trifluoromethyl-phenyl)-thiophen-2-ylmethyl]-2,3,4,5-tetrahydro-1*H*-benzo[*d*]azepin-7-yloxy}-propionic acid**

The title compound was made according to Scheme N.

## Scheme N

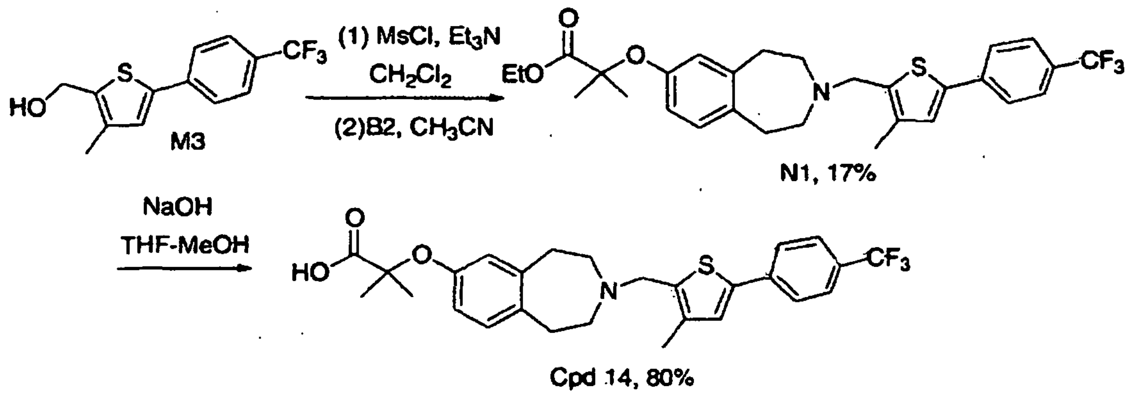


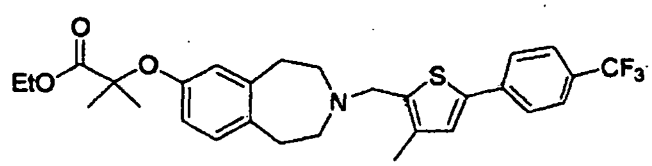

**N1**

**2-Methyl-2-{3-[3-methyl-5-(4-trifluoromethyl-phenyl)-thiophen-2-ylmethyl]-2,3,4,5-tetrahydro-1*H*-benzo[*d*]azepin-7-yloxy}-propionic acid ethyl ester**

Cpd N1 was prepared following the same procedure as forced M4. Cpd N1 was obtained as a white solid (17%): $^1$H NMR (300 MHz, $CDCl_3$) δ 7.65 (d, *J* = 8.2 Hz, 2 H), 7.59 (d, *J* = 8.3 Hz, 2 H), 7.10 (s, 1 H), 6.92 (d, *J* = 8.2 Hz, 1 H), 6.62 (d, *J* = 2.6 Hz, 1 H), 6.55 (dd, *J* = 8.2, 2.7 Hz, 1 H), 4.22 (q, *J*= 7.1 Hz, 2 H), 3.72 (s, 2 H), 2.86 (m, 4 H), 2.70 (m, 4 H), 2.20 (s, 3H), 1.58 (s, 6 H), 1.25 (t, *J* = 7.1 Hz, 3 H); MS (ES) m/z: 532 ($M+H^+$).

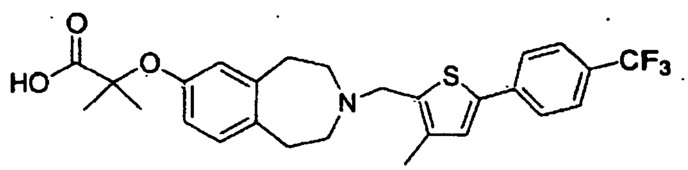

**Cpd 14**

**2-Methyl-2-{3-[3-methyl-5-(4-trifluoromethyl-phenyl)-thiophen-2-ylmethyl]-2,3,4,5-tetrahydro-1*H*-benzo[*d*]azepin-7-yloxy}-propionic acid**

Cpd 14 was prepared following the same procedure as for cpd 2. Cpd 14 was obtained as a white solid (80%): $^1$H NMR (300 MHz, $CD_3OD$) δ 7.84 (d, *J* = 8.2 Hz, 2 H), 7.71 (d, *J* = 8.3 Hz, 2 H), 7.45 (s, 1 H), 7.11 (d, *J* = 8.3 Hz, 1 H), 6.80 (d, *J* = 2.5 Hz, 1 H), 6.74 (dd, *J* = 8.2, 2.6 Hz, 1 H), 4.80 (s, 2 H), 3.29 (m, 4 H), 3.13 (m, 4 H), 2.35 (s, 3 H), 1.55 (s, 6 H); MS (ES) m/z: 504 ($M+H^+$).

Example O

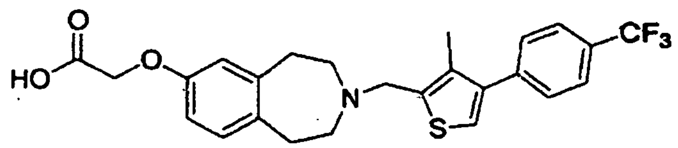

**Compound 15: {3-[3-Methyl-4-(4-trifluoromethyl-phenyl)-thiophen-2-ylmethyl]-2,3,4,5-tetrahydro-1*H*-benzo[*d*]azepin-7-yloxy}-acetic acid**

The title compound was made according to Scheme O.

## Scheme O

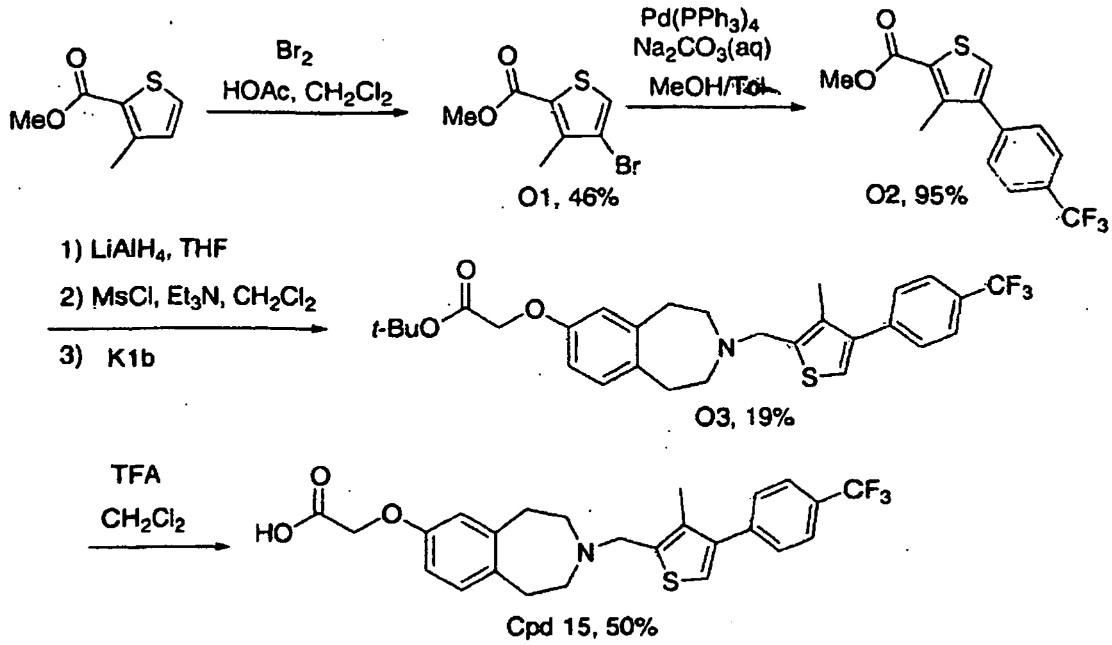


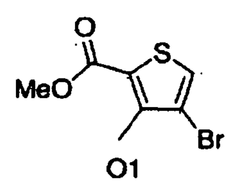

**O1**

**4-Bromo-3-methyl-thiophene-2-carboxylic acid methyl ester**

To a solution of 3-methylthiophene-2-carboxylic acid methyl ester (2.0 g, 12.8 mmol) in $CH_2Cl_2$ (10 mL) and acetic acid (10 mL) was added $Br_2$ (0.79 mL, 15.3 mmol). The mixture was stirred at room temperature for 20 h and then partitioned between $CH_2Cl_2$ and $H_2O$. The aqueous layer was extracted with $CH_2Cl_2$. The combined organic phases were washed with saturated sodium thiosulfate solution, dried ($Na_2SO_4$), concentrated and purified by column chromatography to give 1.4 g (46%) of O1 as a white solid: $^1$H NMR (300 MHz, $CD_3OD$) δ 7.41 (s, 1 H), 3.88 (s, 3 H), 2.52 (s, 3 H).

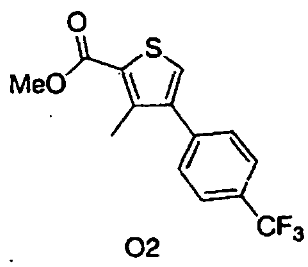

O2

3-Methyl-4-(4-trifluoromethyl-phenyl)-thiophene-2-carboxylic acid methyl ester

Cpd 02 was prepared following the same procedure as for cpd G1. Cpd 02 was obtained as a white solid (95%): $^1H$ NMR (300 MHz, $CDCl_3$) δ 7.71 (d, *J* = 8.2 Hz, 2 H), 7.65 (d, *J* = 8.2 Hz, 2 H), 7.40 (s, 1 H), 3.90 (s, 3 H), 2.51 (s, 3 H).

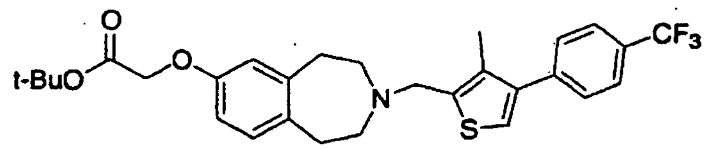

O3

{3-[3-Methyl-4-(4-trifluoromethyl-phenyl)-thiophen-2-ylmethyl]-2,3,4,5-tetrahydro-1*H*-benzo[*d*]azepin-7-yloxy}-acetic acid *tert*-butyl ester

To the solution of 02 (590 mg, 2.0 mmol) in THF (10 mL) at 0 °C was added 1.0 M $LiAlH_4$ (2.16 mL, 2.16 mmol) in THF. The mixture was allowed to warm up to room temperature and stirred at room temperature for 1 h. Water was slowly added and the precipitated solid was filtered and rinsed with $CH_2Cl_2$. The filtrate was washed with saturated $NH_4Cl$ and the aqueous solution was back extracted with $CH_2Cl_2$. The combined organic phases were dried ($Na_2SO_4$) and concentrated to give a crude solid. It was used to carry through a similar procedure as for preparing cpd J2 and gave compound 03 as a white solid (19%): $^1H$ NMR (300 MHz, $CDCl_3$) δ 7.65 (d, *J* = 8.2 Hz, 2 H), 7.47 (d, *J* = 8.2 Hz, 2 H), 7.16 (s, 1 H), 6.99 (d, *J* = 8.2 Hz, 1 H), 6.69 (d, *J* = 2.5 Hz, 1 H), 6.61 (dd, *J* = 8.1, 2.4 Hz, 1 H), 4.48 (s, 2 H), 3.81 (s, 2 H), 2.90 (m, 4 H), 2.73 (m, 4 H), 2.15 (s, 3 H), 1.48 (s, 9 H); MS (ES) m/z: 532 ($M+H^+$).

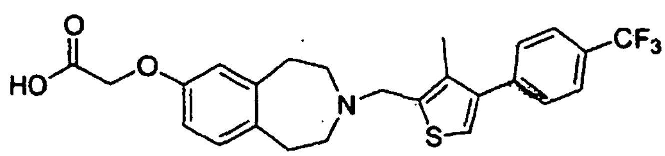

Cpd 15

{3-[3-Methyl-4-(4-trifluoromethyl-phenyl)-thiophen-2-ylmethyl]-2,3,4,5-tetrahydro-1*H*-benzo[*d*]azepin-7-yloxy}-acetic acid

Cpd 15 was prepared following the same procedure as for cpd11. Cpd 15 was obtained as a white solid (50%): $^1H$ NMR (300 MHz, $CD_3OD$) δ 7.71 (d, *J* = 8.3 Hz, 2 H), 7.57 (d, *J* = 8.1 Hz, 2 H), 7.46 (s, 1 H), 6.97 (d, *J* = 8.9 Hz, 1 H), 6.72 - 6.67 (m, 2 H), 4.40 (s, 2 H), 4.22 (s, 2 H), 3.01 (m, 4 H), 2.84 (m, 4 H), 2.21 (s, 3 H); MS (ES) m/z: 476 ($M+H^+$).

Example P

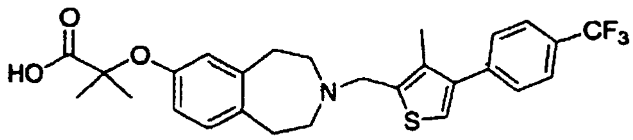

Compound 16: 2-Methyl-2-{3-[3-methyl-4-(4-trifluoromethyl-phenyl)-thiophen-2-ylmethyl]-2,3,4,5-tetrahydro-1*H*-benzo[*d*]azepin-7-yloxy}-propionic acid

The title compound was made according to Scheme P.

**Scheme P**

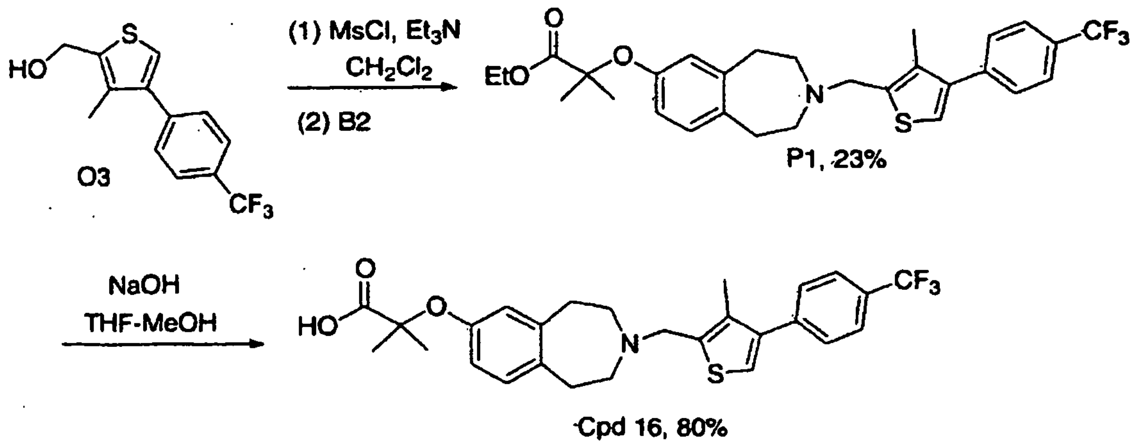


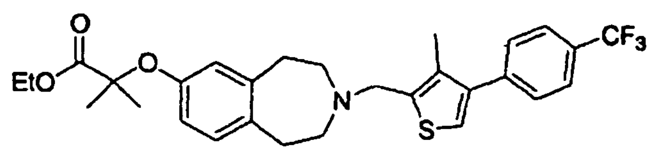

P1

2-Methyl-2-{3-[3-methyl-4-(4-trifluoromethyl-phenyl)-thiophen-2-ylmethyl]-2,3,4,5-tetrahydro-1*H*-benzo[*d*]azepin-7-yloxy}-propionic acid ethyl ester

Cpd P1 was prepared following the same procedure as for cpd J2. Cpd P1 was obtained as a white solid (23%): $^1$H NMR (300 MHz, $CDCl_3$) δ 7.65 (d, *J* = 8.2 Hz, 2 H), 7.47 (d, *J* = 8.2 Hz, 2 H), 7.15 (s, 1 H), 6.93 (d, *J* = 8.2 Hz, 1 H), 6.63 (d, *J* = 2.4 Hz, 1 H), 6.55 (dd, *J* = 8.1, 2.4 Hz, 1 H), 4.24 (q, *J* = 7.1 Hz, 2 H), 3.77 (s, 2 H), 2.86 (m, 4 H), 2.68 (m, 4 H), 2.15 (s, 3 H), 1.58 (s, 6 H), 1.25 (t, *J* = 7.1 Hz, 3 H); MS (ES) m/z: 532 ($M+H^+$).

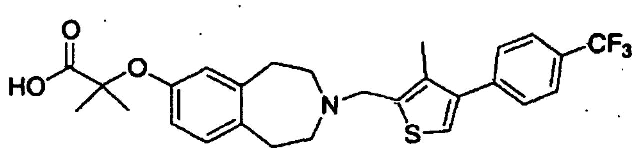

Cpd 16

2-Methyl-2-{3-[3-methyl-4-(4-trifluoromethyl-phenyl)-thiophen-2-ylmethyl]-2,3,4,5-tetrahydro-1*H*-benzo[*d*]azepin-7-yloxy}-propionic acid

Cpd 16 was prepared following the same procedure as for cpd 2. Cpd 16 was obtained as a white solid (80%): $^1$H

NMR (300 MHz, $CD_3OD$) δ 7.73 (d, $J$ = 8.2 Hz, 2 H), 7.72 - 7.56 (m, 3 H), 6.98 (d, $J$ = 8.2 Hz, 1 H), 6.73 - 6.68 (m, 2 H), 4.47 (s, 2 H), 3.30 - 3.12 (m, 4 H), 2.95 - 2.89 (m, 4 H), 2.24 (s, 3 H), 1.57 (s, 6 H); MS (ES) m/z: 504 ($M+H^+$).

Example Q

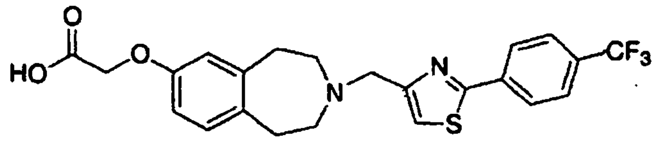

**Compound 17: {3-[2-(4-Trifluoromethyl-phenyl)-thiazol-4-ylmethyl]-2,3,4,5-tetrahydro-1*H*-benzo[*d*]azepin-7-yloxy}-acetic acid**

The title compound was made according to Scheme Q.

**Scheme Q**

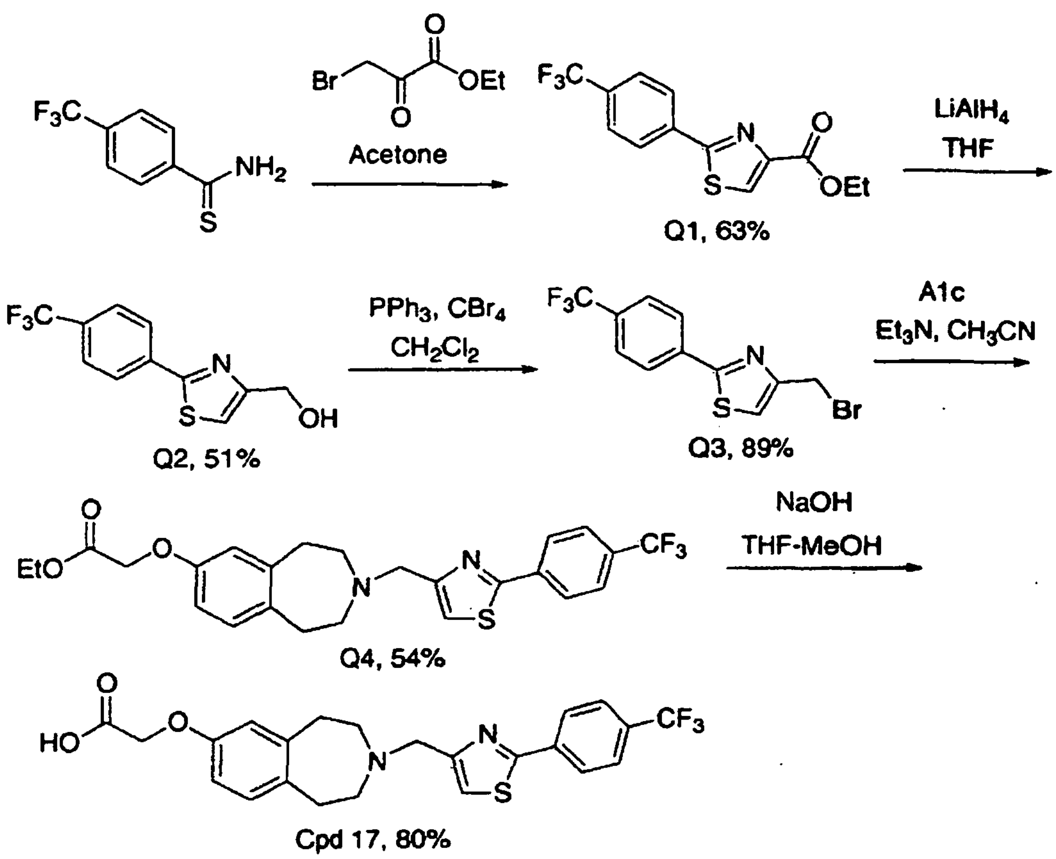


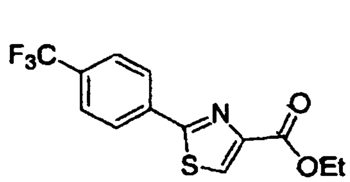

**Q1**

**2-(4-Trifluoromethyl-phenyl)-thiazole-4-carboxylic acid ethyl ester**

To a stirred solution of 4-trifluoromethylthiobenzamkie (2.05 g, 10 mmol) in acetone (10 mL) was added ethyl bromopyruvate (1.95 g, 10 mmol) in acetone (10 mL) dropwise. The mixture was stirred under reflux for 3 h. After cooling to room temperature, the solution was concentrated and purified by column chromatography to give 1.9 g (63%) of cpd Q1 as a white solid: $^1$H NMR (300 MHz, $CDCl_3$) δ 8.23 (s, 1 H), 8.14 (d, *J* = 8.4 Hz, 2 H), 7.72 (d, *J* = 8.4 Hz, 2 H), 4.45 (q, *J* = 7.1 Hz, 2 H), 1.44 (t, *J* = 7.1 Hz, 3 H); MS (ES) m/z: 302 (M+H$^+$).

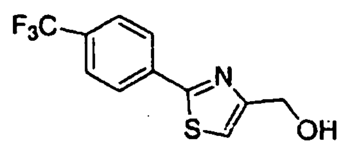

Q2

[2-(4-Trifluoromethyl-phenyl)-thiazol-4-yl]-methanol

Cpd Q2 was prepared following the same procedure as for cpd E1. Cpd Q2 was obtained as a white solid (51%): $^1$H NMR (300 MHz, $CDCl_3$) δ 8.07 (d, *J* = 8.1 Hz, 2H), 7.70 (d, *J* = 8.2 Hz, 2 H), 7.27 (s, 1 H), 4.86 (d, *J* = 6.0 Hz, 2 H), 2.21 (t, *J* = 6.0 Hz, 1 H); MS (ES) m/z: 260 (M+H$^+$).

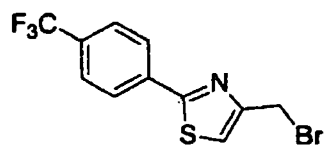

Q3

4-Bromomethyl-2-(4-trifluoromethyl-phenyl)-thiazole

Cpd Q3 was prepared following the same procedure as for cpd A2d. Cpd Q3 was obtained as a white solid (89%): $^1$H NMR (300 MHz, $CDCl_3$) δ 8.07 (d, *J* = 8.1 Hz, 2 H), 7.70 (d, *J* = 8.2 Hz, 2 H), 7.38 (s, 1 H), 4.64 (s, 2 H).

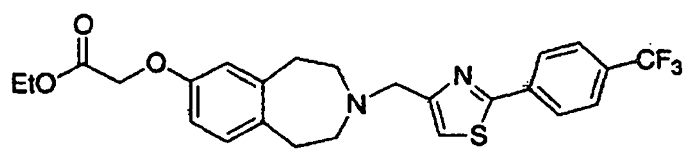

Q4

{3-[2-(4-Trifluoromethyl-phenyl)-thiazol-4-ylmethyl]-2,3,4,5-tetrahydro-1*H*-benzo[*d*]azepin-7-yloxy}-acetic acid ethyl ester

Cpd Q4 was prepared following the same procedure as for cpd A2e. Cpd Q4 was obtained as a white solid (54%): $^1$H NMR (300 MHz, $CDCl_3$) δ 8.05 (d, *J* = 8.2 Hz, 2 H), 7.68 (d, *J* = 8.3 Hz, 2 H), 7.25 (s, 1 H), 6.99 (d, *J* = 8.2 Hz, 1 H), 6.70 (d, *J* = 2.6 Hz, 1 H), 6.61 (dd, *J* = 8.2, 2.6 Hz, 1 H), 4.58 (s, 2 H), 4.26 (q, *J* = 7.1 Hz, 2 H), 3.91 (s, 2 H), 2.94 - 2.88 (m, 4 H), 2.79 - 2.71 (m, 4 H), 1.29 (t, *J* = 7.1 Hz, 3 H); MS (ES) m/z: 491 (M+H$^+$).

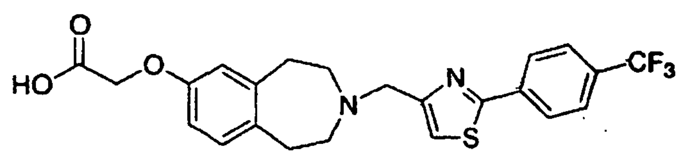

Cmpd 17

{3-[2-(4-Trifluoromethyl-phenyl)-thiazol-4-ylmethyl]-2,3,4,5-tetrahydro-1*H*-benzo[*d*]azepin-7-yloxy}-acetic acid

Cpd 17 was prepared following the same procedure as for cpd A2e. Cpd 17 was obtained as a white solid (80%): $^1$H NMR (300 MHz, $CD_3OD$) δ 8.15 (d, *J* = 8.2 Hz, 2 H), 7.81 (s, 1 H), 7.77 (d, *J* = 8.3 Hz, 2 H), 6.96 (d, *J* = 9.0 Hz, 1 H), 6.71 - 6.68 (m, 2 H), 4.41 (s, 2 H), 4.33 (s, 2 H), 3.14 (m, 4 H), 2.84 (m, 4 H); MS (ES) m/z: 463 (M+H$^+$).

Example R

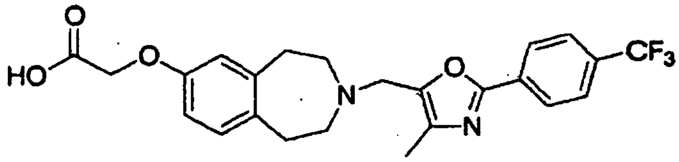

**Compound 18: {3-[4-Methyl-2-(4-trifluoromethyl-phenyl)-oxazol-5-ylmethyl]-2,3,4,5-tetrahydro-1*H*-benzo[*d*]azepin-7-yloxy}-acetic acid**

The title compound was made according to Scheme R.

**Scheme R**

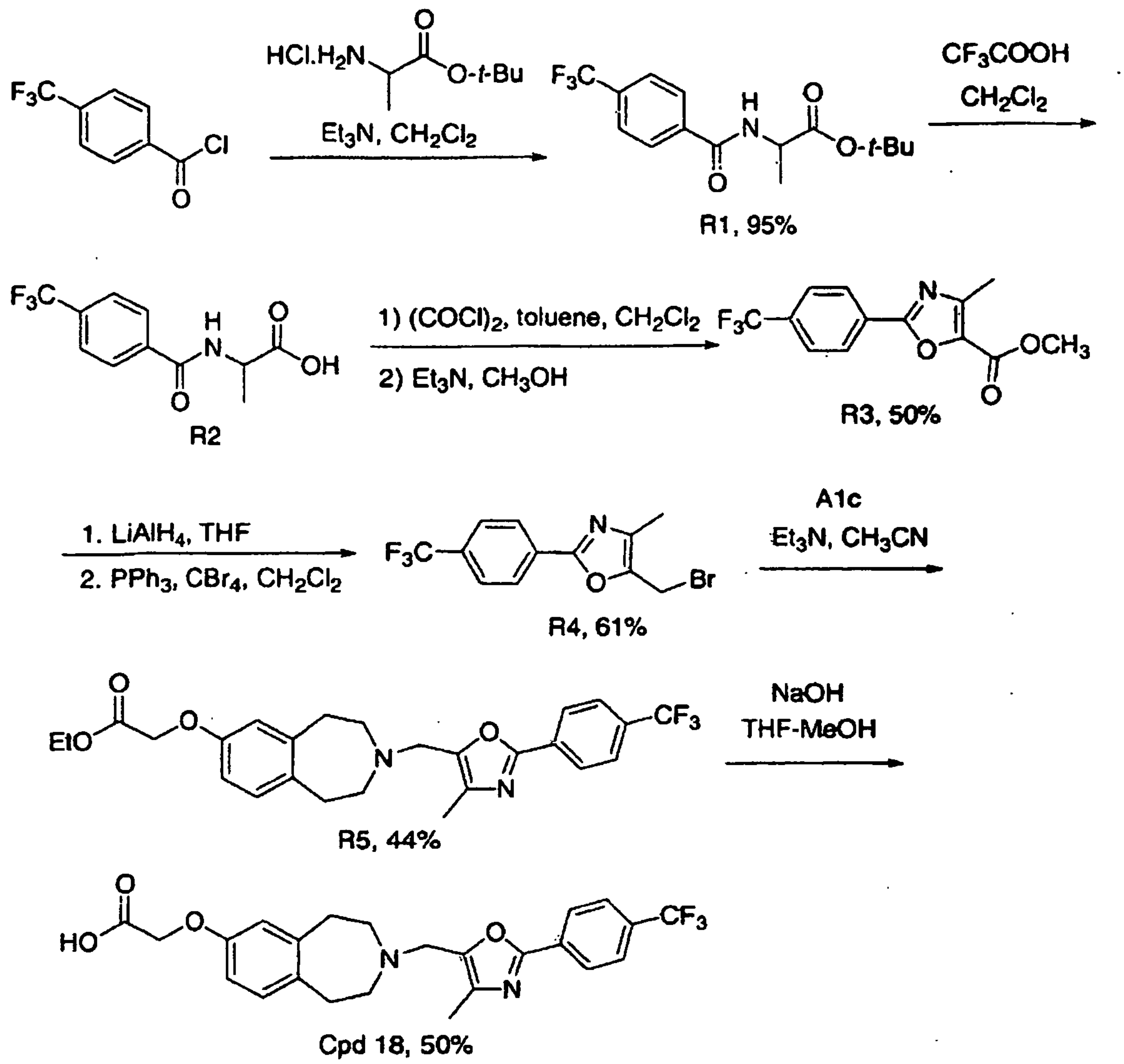


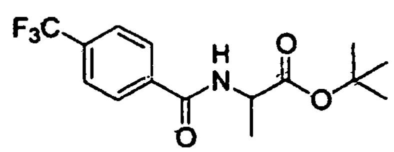

**R1**

**2-(4-Trifluoromethyl-benzoylamino)-propionic acid *tert*-butyl ester**

To a solution of 2-aminopropionic acid ten-butyl ester hydrochloride (2.18 g, 12 mmol) and triethyl amine (3.03 g, 30 mmol) in $CH_2Cl_2$ (60 mL) at 0 °C was added 4-trifluoromethylbenzoyl chloride (1.48 mL, 10 mmol). The mixture was stirred at room temperature for 24 h and then washed with $H_2O$, 1 N HCl and $H_2O$. After drying over $Na_2SO_4$, the solution was concentrated and purified by column chromatography to give 3.0g (95%) of R1 as a white solid: $^1H$ NMR (300 MHz, $CDCl_3$) $\delta$ 7.92 (d, $J$ = 8.2 Hz, 2 H), 7.70 (d, $J$ = 8.5 Hz, 2 H), 6.83 (brs, 1 H), 4.65 (m, 1 H), 1.51 (s, 12 H); MS (ES) m/z: 316 ($M-H^+$).

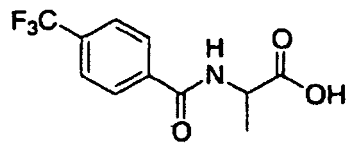

R2

**2-(4-Trifluoromethyl-benzoylamino)-propionic acid**

Cpd R2 was prepared following the same procedure as for cpd 11. Cpd R2 was obtained as a white solid (crude): $^1$H NMR (300 MHz, $CDCl_3$) δ 7.91 (d, $J$ = 8.2 Hz, 2 H), 7.73 (d, $J$ = 8.3 Hz, 2 H), 6.81 (d, $J$ = 6.7 Hz, 1 H), 4.84 (m, 1 H), 1.62 (d, $J$ = 7.2 Hz, 3 H).

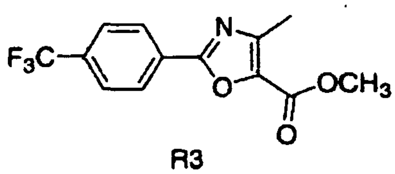

R3

**4-Methyl-2-(4-trifluoromethyl-phenyl)-oxazole-5-carboxyl ic acid methyl ester**

To a solution of R2 (1.95 g, 7.47 mmol) in toluene (30 mmol) and $CH_2Cl_2$ (7.5 mL) was added oxalyl chloride (6.52 mL, 74.7 mmol). The mixture was stirred at room temperature for 24 h and concentrated.

To the above crude intermediate at 0 °C was added $Et_3N$ (1.53 mL, 11.2 mmol) followed by MeOH (56 mL). The mixture was stirred at room temperature for 3 h, concentrated and purified by column chromatography to give 1.06 g (50%) of R3 as a white solid: $^1$H NMR (300 MHz, $CDCl_3$) δ 8.24 (d, $J$ = 8.2 Hz, 2 H), 7.74 (d, $J$ = 8.3 Hz, 2 H), 3.96 (s, 3 H), 2.56 (s, 3 H); MS (ES) m/z: 286 (M+H$^+$).

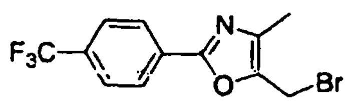

R4

**5-Bromomethyl-4-methyl-2-(4-trifluoromethyl-phenyl)-oxazole**

Cpd R3 was reduced to give a crude alcohol intermediate following the same procedure as in the preparation of compound E1. Cpd R4 was prepared following the same procedure as for cpd A2d. Cpd R4 was obtained as a white solid (61%): $^1$H NMR (300 MHz, $CDCl_3$) δ 8.14 (d, $J$ = 8.6 Hz, 2 H), 7.71 (d, $J$ = 8.4 Hz, 2 H), 4.60 (s, 2 H), 2.26 (s, 3 H).

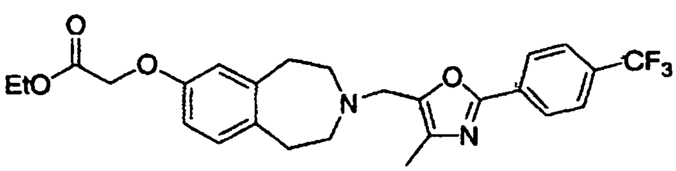

R5

**{3-[4-Methyl-2-(4-trifluoromethyl-phenyl)-oxazol-5-ylmethyl]-2,3,4,5-tetrahydro-1*H*-benz o[*d*]azepin-7-yloxy}-acetic acid ethyl ester**

Cpd R5 was prepared following the same procedure as for cpd A2e. Cpd R5 was obtained as a white solid (44%): $^1$H NMR (300 MHz, $CDCl_3$) δ 8.12 (d, $J$ = 8.4 Hz, 2 H), 7.69 (d, $J$ = 8.5 Hz, 2 H), 6.99 (d, $J$ = 8.2 Hz, 1 H), 6.69 (d, $J$ = 2.6 Hz, 1 H), 6.61 (dd, $J$ = 8.2, 2.6 Hz, 1 H), 4.57 (s, 2 H), 4.26 (q, $J$ = 7.1 Hz, 2 H), 3.77 (s, 2 H), 2.92 - 2.87 (m, 4 H), 2.72 -2.68 (m, 4 H), 2.23 (s, 3 H), 1.29 (t, $J$ = 7.1 Hz, 3 H); MS (ES) m/z: 489 (M+H$^+$).

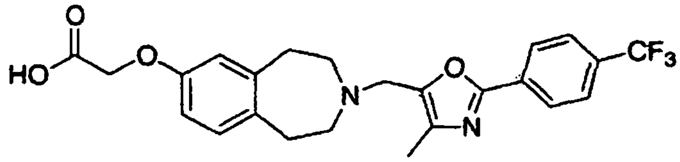

**Cpd 18**

**{3-[4-Methyl-2-(4-trifluoromethyl-phenyl)-oxazol-5-ylmethyl]-2,3,4,5-tetrahydro-1*H*-benzo[*d*]azepin-7-yloxy}-acetic acid**

Cpd 18 was prepared following the same procedure as for cpd A2e. Cpd 18 was obtained as a white solid (50%): $^1$H NMR (300 MHz, $CD_3OD$) δ 8.19 (d, *J* = 8.1 Hz, 2 H), 7.81 (d, *J* = 8.3 Hz, 2 H), 6.98 (d, *J* = 8.0 Hz, 1 H), 6.70 - 6.67 (m, 2 H), 4.42 (s, 2 H), 4.17 (s, 2 H), 3.05 - 2.95 (m, 4 H), 2.90 - 2.80 (m, 4 H), 2.27 (s, 3 H); MS (ES) m/z: 461 (M+H$^+$).

Example S

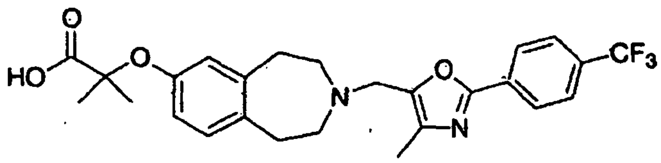

**Compound 19: 2-Methyl-2-{3-[4-methyl-2-(4-trifluoromethyl-phenyl)-oxazol-5-ylmethyl]-2,3,4,5-tetrahydro-1*H*-benzo[*d*]azepin-7-yloxy}-propionic acid**

The title compound was made according to Scheme S.

**Scheme S**

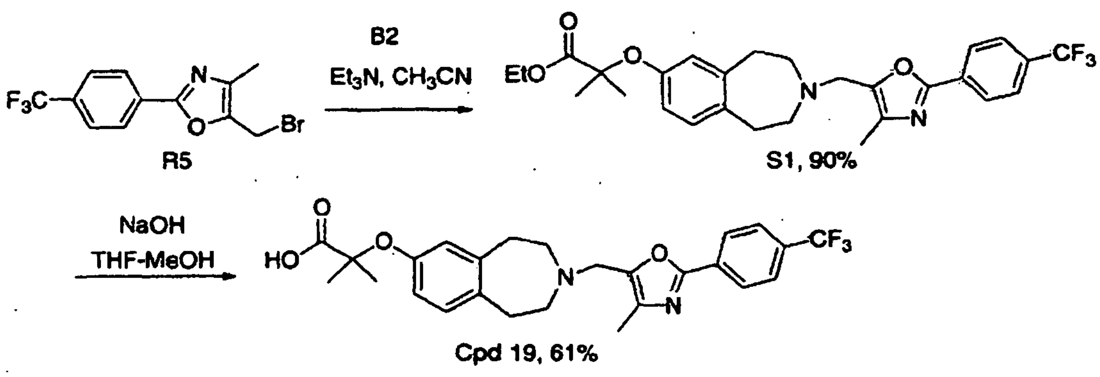


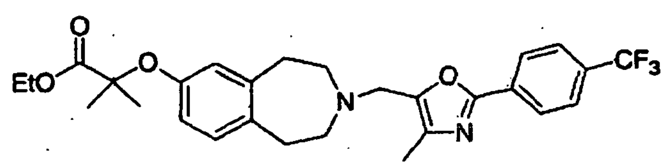

**S1**

**2-Methyl-2-{3-[4-methyl-2-(4-trifluoromethyl-phenyl)-oxazol-5-ylmethyl]-2,3,4,5-tetrahydro-1*H*-benzo[*d*]azepin-7-yloxy}-propionic acid ethyl ester**

Cpd S1 was prepared following the same procedure as for cpd 83. Cpd S1 was obtained as a white solid (90%): $^1$H NMR (300 MHz, $CDCl_3$) δ 8.12 (d, *J* = 8.3 Hz, 2 H), 7.68 (d, *J* = 8.4 Hz, 2 H), 6.92 (d, *J* = 8.2 Hz, 1 H), 6.62

(d, $J$ = 2.5 Hz, 1 H), 6.54 (dd, $J$ = 8.1, 2.5 Hz, 1 H), 4.22 (q, $J$ = 7.1 Hz, 2 H), 3.76 (s, 2 H), 2.90 - 2.82 (m, 4 H), 2.70 - 2.63 (m, 4 H), 2.23 (s, 3 H), 1.56 (s, 6 H), 1.23 (t, $J$ = 7.1 Hz, 3 H); MS (ES) miz: 517 (M+H$^+$).

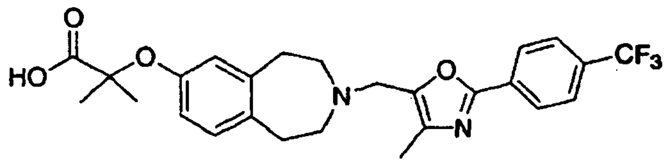

**Cpd 19**

**2-Methyl-2-{3-[4-methyl-2-(4-trifluoromethyl-phenyl)-oxazol-5-ylmethyl]-2,3,4,5-tetrahydro-1*H*-benzo[*d*]azepin-7-yloxy}-propionic acid**

Cpd 19 was prepared following the same procedure as for cpd 2. Cpd 19 was obtained as a white solid (61%): $^1$H NMR (300 MHz, $CD_3OD$) δ 8.24 (d, $J$ = 8.3 Hz, 2 H), 7.85 (d, $J$ = 8.4 Hz, 2 H), 7.12 (d, $J$ = 8.2 Hz, 1 H), 6.80 (d, $J$ = 2.5 Hz, 1 H), 6.73 (dd, $J$ = 8.2, 2.5 Hz, 1 H), 4.64 (s, 2 H), 3.85 - 3.75 (m, 2 H), 3.25 - 3.02 (m, 6 H), 2.34 (s, 3 H), 1.54 (s, 6 H); MS (ES) m/z: 489 (M+H$^+$).

Example 20

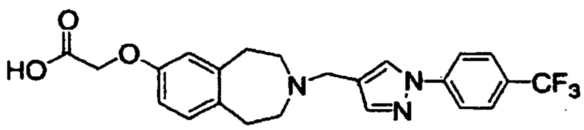

**Compound 20: {3-[1-(4-Trifluoromethyl-phenyl)-1*H*-pyrazol-4-ylmethyl]-2,3,4,5-tetrahydro-1*H*-benzo[*d*]azepin-7-yloxy}-acetic acid**

The title compound was made according to Scheme T.

## Scheme T

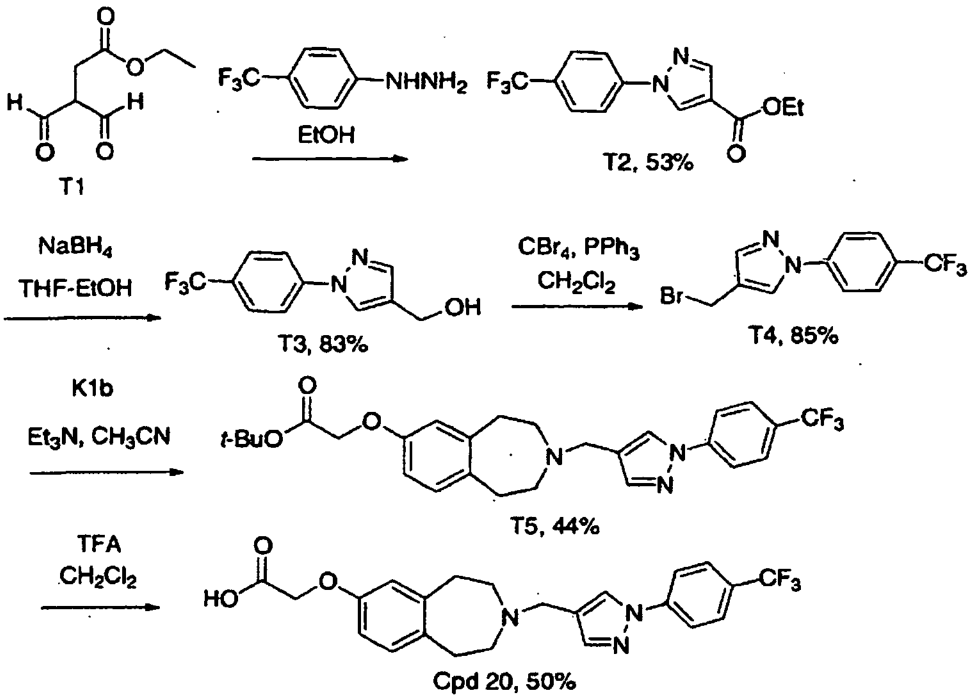


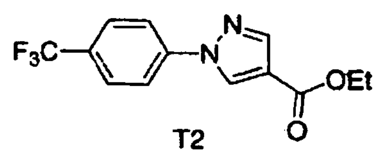

T2

**1-(4-Trifluoromethyl-phenyl)-1*H*-pyrazole-4-carboxylic acid ethyl ester**

To a solution of T1 (864 mg, 6 mmol, J. Org. Chem., 1982, 47, 2217-2218) in EtOH (6 mL) at 0 °C was added (4-trifluoromethylphenyl)hydrazine (1.06 g, 6 mmol) in EtOH (30 mL). The mixture was stirred at room temperature for 2 days and then washed with $H_2O$, 1 N HCl and $H_2O$. After drying over $Na_2SO_4$, the solution was concentrated and purified by column chromatography to give 3.0 g (95%) of R1 as a white solid: $^1H$ NMR (300 MHz, $CDCl_3$) δ 8.47 (s, 1 H), 8.13 (s, 1 H), 7.86 (d, *J* = 8.6 Hz, 2 H), 7.75 (d, *J* = 8.6 Hz, 2 H), 4.36 (q, *J* = 7.1 Hz, 2 H), 1.36 (t, *J* = 7.1 Hz, 3 H).

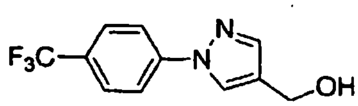

T3

**[1-(4-Trifluoromethyl-phenyl)-1*H*-pyrazol-4-yl]-methanol**

Cpd T3 was prepared following the same procedure as in the preparation of K2c. Cpd T3 was obtained as a white solid (83%): $^1H$ NMR (300 MHz, $CDCl_3$) δ 7.99 (s, 1 H), 7.81 (d, *J* = 8.8 Hz, 2 H), 7.75 (s, 1 H), 7.71 (d, *J* = 8.8 Hz, 2 H), 4.70 (d, *J* = 5.4 Hz, 2 H), 1.66 (t, *J* = 5.5 Hz, 1 H); MS (ES) m/z: 243 (M+H+).

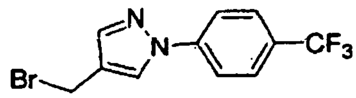

T4

**4-Bromomethyl-1-(4-trifluoromethyl-phenyl)-1*H*-pyrazole**

Cpd T4 was prepared following the same procedure as for cpd A2d. Cpd T4 was obtained as a white solid (85%): $^1$H NMR (300 MHz, $CDCl_3$) δ 7.95 (s, 1 H), 7.75 - 7.60 (m, 5 H), 4.43 (s, 2 H).

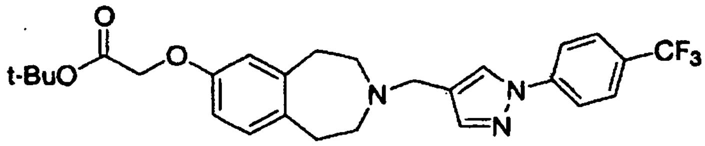

T5

**{3-[1-(4-Trifluoromethyl-phenyl)-1*H*-pyrazol-3-ylmethyl]-2,3,4,5-tetrahydro-1*H*-benzo[*d*]azepin-7-yloxy}-acetic acid *tert*-butyl ester**

Cpd T5 was prepared following the same procedure as for cpd B3. Cpd T5 was obtained as a white solid (44%): $^1$H NMR (300 MHz, $CDCl_3$) δ 8.04 (s, 1 H), 7.82 (d, *J* = 8.6 Hz, 2 H), 7.72 - 7.67 (m, 3 H), 6.99 (d, *J* = 8.2 Hz, 1 H), 6.68 (d, *J* = 2.6 Hz, 1 H), 6.61 (dd, *J* = 8.2, 2.7 Hz, 1 H), 4.47 (s, 2 H), 3.72 (s, 2 H), 2.93 (m, 4 H), 2.73 (m, 4 H), 1.48 (s, 9 H); MS (ES) m/z: 502 (M+H$^+$).

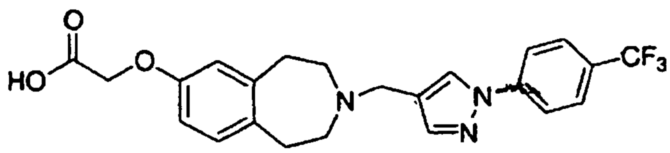

**Cmpd 20**

**{3-[1-(4-Trifluoromethyl-phenyl)-1*H*-pyrazol-3-ylmethyl]-2,3,4,5-tetrahydro-1*H*-benzo[*d*]azepin-7-yloxy}-acetic acid**

Cpd 20 was prepared following the same procedure as in the preparation of cpd 11. Cpd 20 was obtained as a white solid (50%): $^1$H NMR (300 MHz, $CD_3OD$) δ 8.59 (s, 1 H), 8.02 (d, *J* = 8.5 Hz, 2 H), 7.93 (m, 1 H), 7.83 (d, *J* = 8.6 Hz, 2 H), 7.14 (d, *J* = 8.3 Hz, 1 H), 6.83 (d, *J* = 2.6 Hz, 1 H), 6.77 (dd, *J* = 8.3, 2.6 Hz, 1 H), 4.64 (s, 2 H), 4.42 (s, 2 H), 3.84 - 3.78 (m, 2 H), 3.25 - 2.99 (m, 6 H); MS (ES) m/z: 446 (M+H$^+$).

Example U

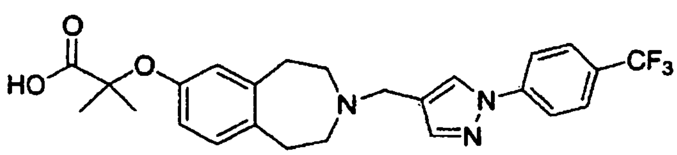

**Compound 21: 2-Methyl-2-{3-[1-(4-trifluoromethyl-phenyl)-1*H*-pyrazol-3-ylmethyl]-2,3,4,5-tetrahydro-1*H*-benzo[*d*]azepin-7-yloxy}-propionic acid**

The title compound was made according to Scheme U.

## Scheme U

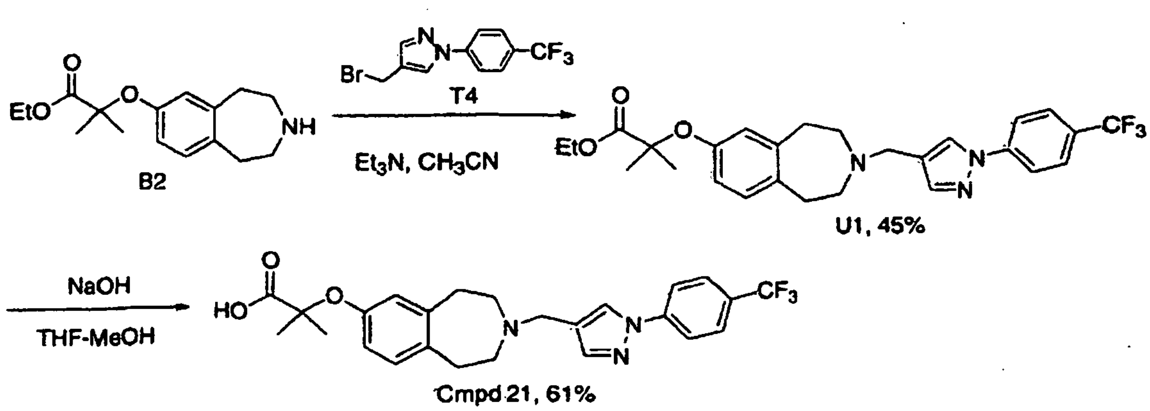


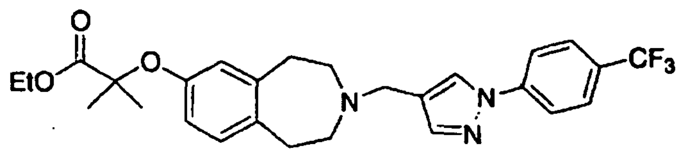

U1

**2-Methyl-2-{3-[1-(4-trifluoromethyl-phenyl)-1*H*-pyrazol-3-ylmethyl]-2,3,4,5-tetrahydro-1*H*-benzo[*d*]azepin-7-yloxy}-propionic acid ethyl ester**

Cpd U1 was prepared following the same procedure as for cpd B3. Cpd U1 was obtained as a white solid (45%): $^1$H NMR (300 MHz, $CDCl_3$) δ 8.44 (s, 1 H), 7.86 (d, *J* = 8.6 Hz, 2 H), 7.75 - 7.68 (m, 3 H), 6.95 (d, *J* = 8.2 Hz, 1 H), 6.65 (d, *J*= 2.6 Hz, 1 H), 6.59 (dd, *J*= 8.2, 2.6 Hz, 1 H), 4.23 (q, *J* = 7.1 Hz, 2 H), 3.91 (s, 2 H), 3.10 (m, 4 H), 2.96 (m, 4 H), 1.58 (s, 6 H), 1.25 (t, *J* = 7.1 Hz, 3 H); MS (ES) m/z: 502 ($M+H^+$).

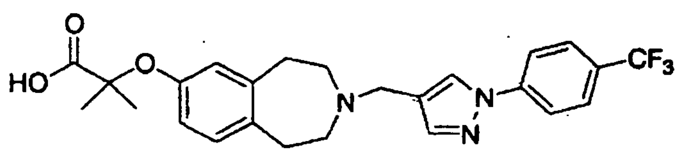

Cmpd 21

**2-Methyl-2-{3-[1-(4-trifluoromethyl-phenyl)-1*H*-pyrazol-3-ylmethyl]-2,3,4,5-tetrahydro-1*H*-benzo[*d*]azepin-7-yloxy}-propionic acid**

Cpd 21 was prepared following the same procedure as for cpd 2. Cpd 21 was obtained as a white solid (61%): $^1$H NMR (300 MHz, $CD_3OD$) δ 8.54 (s, 1 H), 7.99 (d, *J* = 8.6 Hz, 2 H), 7.87 (s, 1 H), 7.81 (d, *J* = 8.7 Hz, 2 H), 6.93 (d, *J* = 7.6 Hz, 1 H), 6.75 - 6.67 (m, 2 H), 4.27 (s, 2 H), 3.30 - 3.10 (m, 4 H), 2.81 (m, 4 H), 1.57 (s, 6 H); MS (ES) m/z: 474 ($M+H^+$).

Example V

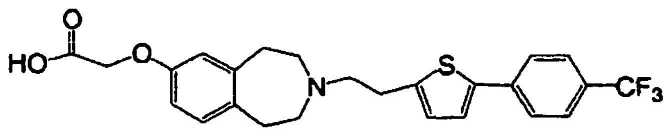

**Compound 22: (3-{2-[5-(4-Trifluoromethyl-phenyl)-thiophen-2-yl]-ethyl}-2,3,4,5-tetrahydro-1*H*-benzo[*d*]azepin-7-yloxy)-acetic acid**

The title compound was made according to Scheme V.

## Scheme V

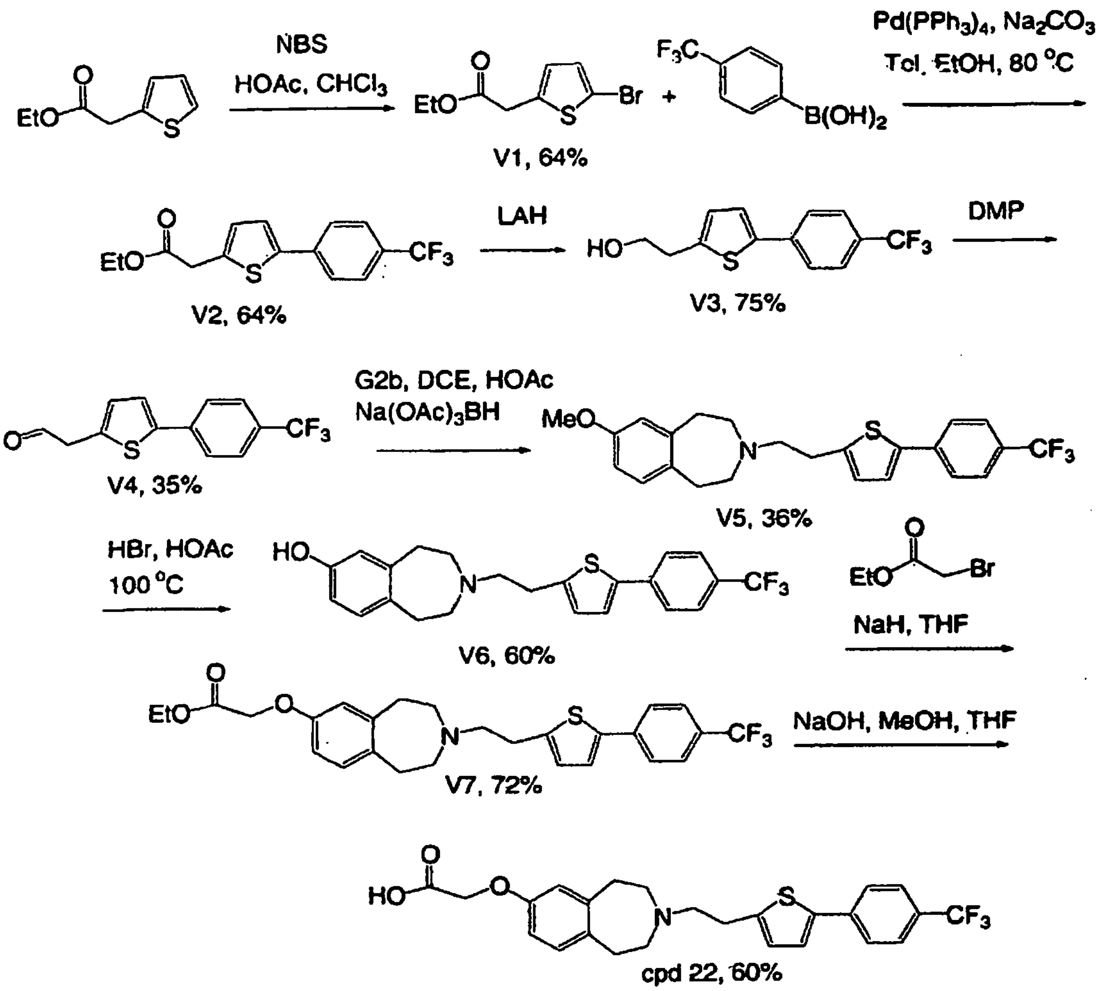


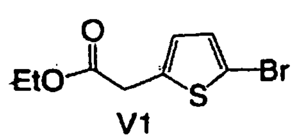

**V1**

**(5-Bromo-thiophen-2-yl)-acetic acid ethyl ester**

A mixture of 2-thiophene acetate (1.380 g, 8.107 mmol), $CHCl_3$ (40 mL) and HOAc (40 mL) was cooled in ice bath. *N*-bromosuccinamide (1.472 g, 8.269 mmol) was added. The resulting mixture was stirred at r.t. for 18 h and then

poured into water (100 mL). The layers were separated and the aqueous phase was extracted with chloroform (2 x 30 mL). The combined organic layers were washed with water, brine, dried ($Na_2SO_4$) and concentrated. The residue was purified by column chromatography eluting with EtOAc/Hexane to give cpd V1 as yellow oil (1.301 g, 64%): $^1H$ NMR (300 MHz, $CDCl_3$) δ 6.89 (d, *J* = 3.7 Hz, 1 H), 6.68-6.69 (m, 1 H), 4.15-4.22 (q, *J* = 7.2 Hz, 2 H), 3.75 (d, J = 0.7 Hz, 2 H), 1.30 (t, *J* = 7.1 Hz, 3 H).

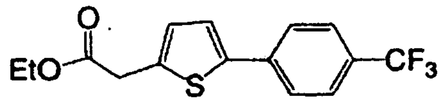

**V2**

**[5-(4-Trifluoromethyl-phenyl)-thiophen-2-yl]-acetic acid ethyl ester**

Cpd V2 (430 mg, 64%) was prepared using a similar procedure as for cpd G1. $^1H$ NMR (300 MHz, $CDCl_3$) δ 7.59-7.68 (m, 4 H), 7.24 (d, *J* = 3.7 Hz, 1 H), 6.94 (d, *J* = 3.6 Hz, 1 H), 4.18-4.25 (q, *J* = 7.1 Hz, 2 H), 3.84 (s, 2 H), 1.28-1.32 (t, *J* = 7.1 Hz, 3 H).

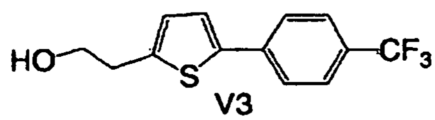

**V3**

**2-[5-(4-Trifluoromethyl-phenyl)-thiophen-2-yl]-ethanol**

To a THF (5 mL) solution of V2 (423 mg, 1.347 mmol) was added a THF solution of LAH (1.0 M, 1.62 mmol) at 0 °C. After stirring for 30 min, the mixture was quenched with aqueous $NH_4Cl$ and then extracted with $Et_2O$. The organic extracts were concentrated and the residue purified by column chromatography eluting with EtOAc/Hexane to give cpd V3 as yellow solid (276 mg, 75%): $^1H$ NMR (300 MHz, $CDCl_3$) δ 7.59-7.67 (m, 4 H), 7.25 (d, *J* = 3.6 Hz, 1 H), 6.88 (d, *J* = 3.6 Hz, 1 H), 3.92 (t, *J* = 6.2 Hz, 2 H), 3.10 (t, *J* = 6.2 Hz, 2 H).

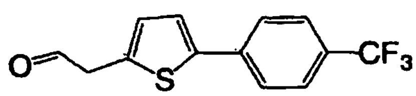

**V4**

**[5-(4-Trifluoromethyl-phenyl)-thiophen-2-yl]-acetaldehyde**

To a mixture of V3 (194 mg, 0.713 mmol) in $CH_2Cl_2$ (7 mL) at 0 °C was added Dess-Martin reagent (333 mg, 0.784 mmol). The mixture was stirred for 30 min and then washed with a mixture of aqueous $NaHCO_3$ and aqueous $Na_2S_2O_3$, followed by brine. After drying over $Na_2SO_4$, the mixture was concentrated and the resulting residue purified by column chromatography eluting with EtOAc/hexane to give cpd V4 as yellow solid (82 mg, 35%): $^1H$ NMR (300 MHz, $CDCl_3$) δ 9.78 (t, *J* = 1.9 Hz, 1 H), 7.60-7.68 (m, 4 H), 7.29 (d, *J* = 3.6 Hz, 1 H), 6.94 (d, *J* = 3.6 Hz, 1 H), 3.92 (d, *J* = 1.2 Hz, 2 H).

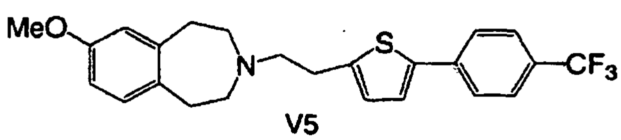

**V5**

**7-Methoxy-3-{2-[5-(4-trifluoromethyl-phenyl)-thiophen-2-yl]-ethyl}-2,3,4,5-tetrahy dro-1*H*-benzo[*d* ]azepine**

Cpd V5 (40 mg, 36%) was prepared according to the same procedure as for cpd G2c. $^1H$ NMR (300 MHz,

DMSO-$d_6$) δ 7.71-7.82 (m, 4 H), 7.49 (d, *J* = 3.6 Hz, 1 H), 7.02 (d, *J* = 8.1 Hz, 1 H), 6.96 (d, *J* = 3.6 Hz, 1 H), 6.71 (d, *J* = 2.5 Hz, 1 H), 6.62-6.66 (dd, *J* = 2.5, 8.1 Hz, 1 H), 3.70 (s, 3 H), 2.98 (m, 2 H), 2.83 (m, 4 H), 2.74 (m, 2 H), 2.64 (m, 4 H); MS (ES) m/z: 432.1 (M+H$^+$).

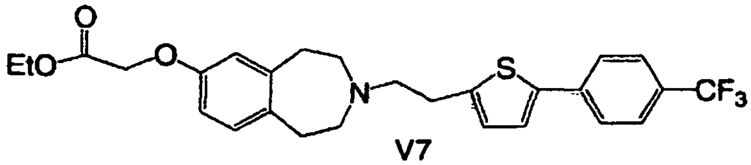

**V7**

**(3-{2-[5-(4-Trifluoromethyl-phenyl)-thiophen-2-yl]-ethyl}-2,3,4,5-tetrahydro-1*H*-benzo[*d*]azepin-7-yloxy)-acetic acid ethyl ester**

Cpd V7 (13 mg, 72%) was prepared according to the same procedure as for cpd G1b. $^1$H NMR (300 MHz, $CDCl_3$) δ 7.58-7.66 (m, 4 H), 7.22 dd, *J* = 3.6 Hz, 1 H), 7.03 (d, *J* = 7.5 Hz, 1 H), 8.88 (d, *J* = 3.7 Hz, 1 H), 6.72 (s, 1 H), 6.67 (br, 1 H), 4.60 (s, 2 H), 4.24-4.31 (q, *J* = 7.1 Hz, 2 H), 2.72-2.91 (m, 12 H), 1.30 (t, *J* = 7.1 Hz, 3 H); MS (ES) m/z: 504.0 (M+H$^+$).

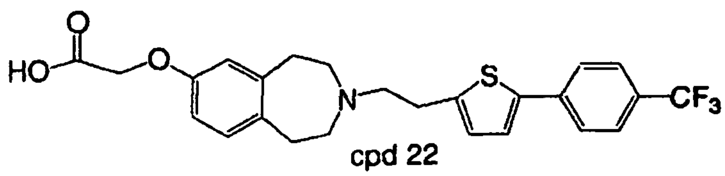

**cpd 22**

**(3-{2-[5-(4-Trifluoromethyl-phenyl)-thiophen-2-yl]-ethyl}-2,3,4,5-tetrahydro-1*H*-benzo[*d*]azepin-7-yloxy)-acetic acid**

Cpd 22 (6mg, 60%) was prepared according to the same procedure as for cpd 7. $^1$H NMR (400 MHz, DMSO-$d_6$) δ 7.83 (d, *J* = 8.3 Hz, 2 H), 7.75 (d, *J* = 8.5 Hz, 2 H), 7.56 (d, *J* = 3.6 Hz, 1 H), 7.11 (d, *J* = 8.3 Hz, 1 H), 7.04 (d, *J* = 3.5 Hz, 1 H), 6.81 (d, *J* = 1.9 Hz, 1 H), 6.68-6.71 (dd, *J* = 2.5, 8.1 Hz, 1 H), 4.64 (s, 2 H), 3.03-3.20 (br, 12 H); MS (ES) m/z: 476 (M+H$^+$), 474 (M-H$^+$).

Example W

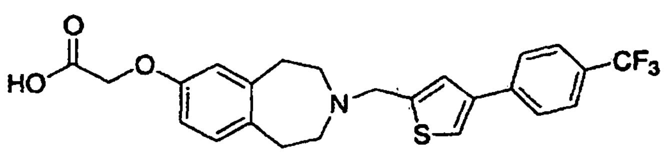

**Compound 23: {3-[4-(4-Trifluoromethyl-phenyl)-thiophen-2-ylmethyl]-2,3,4,5-tetrahydro-1*H*-benzo[*d*]azepin-7-yloxy}-acetic acid**

The title compound was made according to Scheme W.

## Scheme W

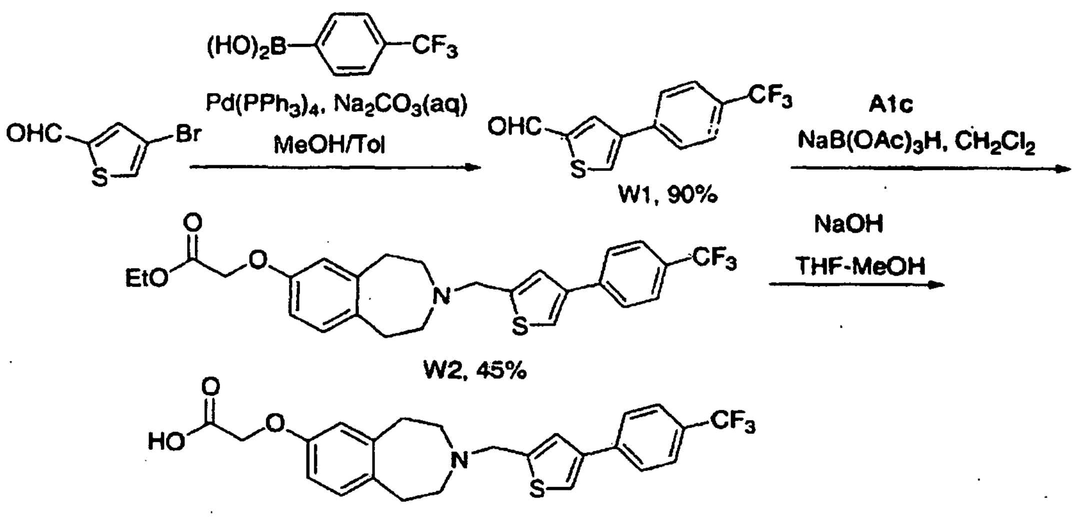


Cpd 23, 91%

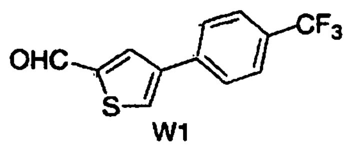

W1

**4-(4-Trifluoromethyl-phenyl)-thiophene-2-carbaldehyde**

Cpd W1 was prepared following the same procedure as for cpd G1

Cpd W1 was obtained as a white solid (90%): $^1$H NMR (300 MHz, $CDCl_3$) δ 10.00 (s, 1 H), 8.07 (s, 1 H), 7.92 (s, 1 H), 7.71 (s, 4 H).

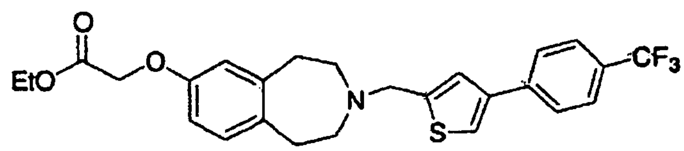

W2

**{3-[4-(4-Trifluoromethyl-phenyl)-thiophen-2-ylmethyl]-2,3,4,5-tetrahydro-1*H* -benzo[*d*]azepin-7-yloxy}-acetic acid ethyl ester**

Cpd W2 was prepared following the same procedure as for cpd G2. Cpd W2 was obtained as a white solid (45%): $^1$H NMR (300 MHz, $CDCl_3$) δ 7.66 (d, $J$ = 8.8 Hz, 2 H), 7.62 (d, $J$ = 8.7 Hz, 2 H), 7.43 (s, 1 H), 7.20 (s, 1 H), 6.99 (d, $J$ = 8.2 Hz, 1 H), 6.69 (d, $J$ = 2.6 Hz, 1 H), 6.62 (dd, $J$ = 8.2, 2.6 Hz, 1 H), 4.58 (s, 2 H), 4.26 (q, $J$ = 7.1 Hz, 2 H), 3.87 (s, 2 H), 2.92 - 2.84 (m, 4 H), 2.73 - 2.65 (m, 4 H), 1.29 (t, $J$ = 7.1 Hz, 3 H); MS (ES) m/z: 490 (M+H$^+$).

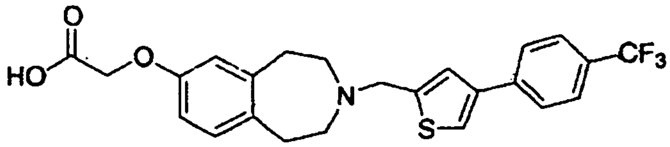

Cpd 23

{3-[4-(4-Trifluoromethyl-phenyl)-thiophen-2-ylmethyl]-2,3,4,5-tetrahydro-1*H*-benzo[*d*]azepin-7-yloxy}-acetic acid

Cpd 23 was prepared following the same procedure as for cpd A2e. Cpd 23 was obtained as a white solid (91%): $^1$H NMR (300 MHz, $CD_3OD$) δ 7.88 (s, 1 H), 7.83 (d, *J* = 8.1 Hz, 2 H), 7.70 - 7.65 (m, 3 H), 6.96 (d, *J* = 8.9 Hz, 1 H), 6.72 - 6.68 (m, 2 H), 4.46 (s, 2 H), 4.42 (s, 2 H), 3.15 - 3.03 (m, 4 H), 2.89 - 2.75 (m, 4 H); MS (ES) m/z: 462 ($M+H^+$).

Example X

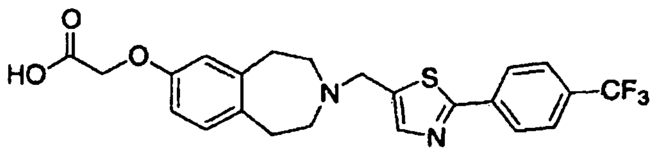

Compound 24: {3-[2-(4-Trifluoromethyl-phenyl)-thiazol-5-ylmethyl]-2,3,4,5-tetrahydro-1*H*-benzo[*d*]azepin-7-ylox y}-acetic acid

The title compound was made according to Scheme X.

## Scheme X

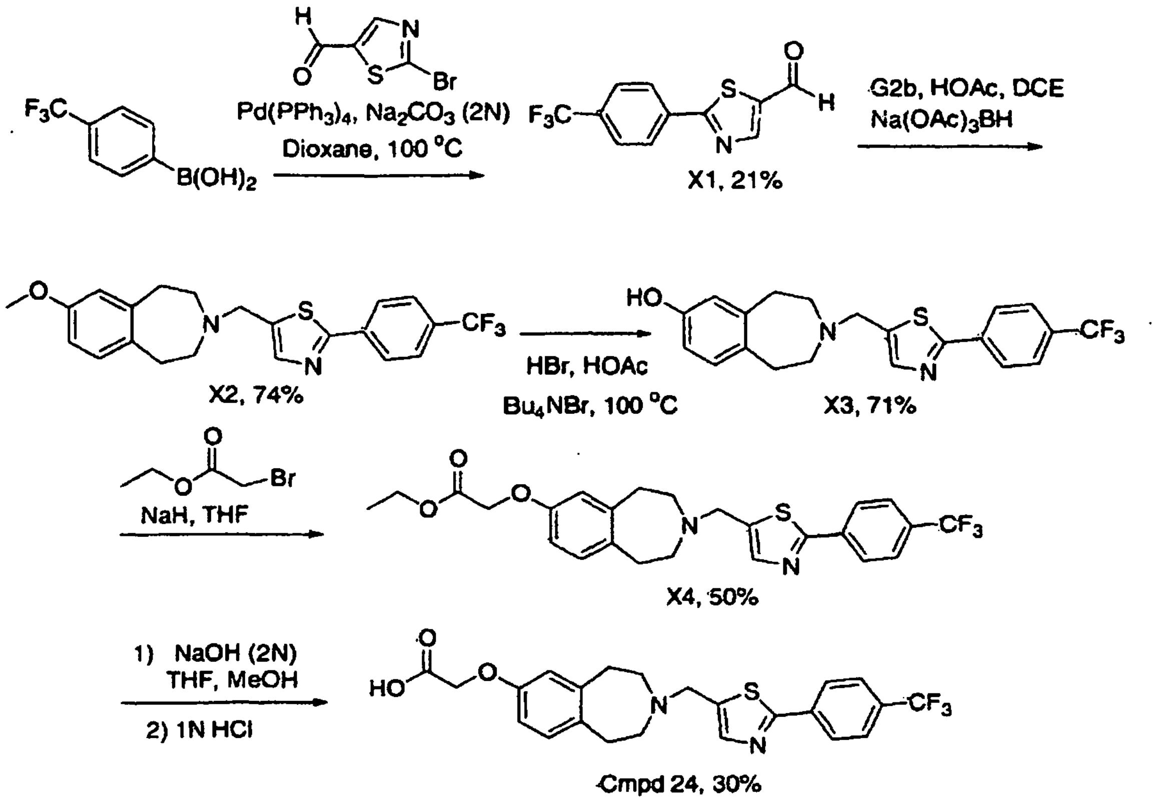


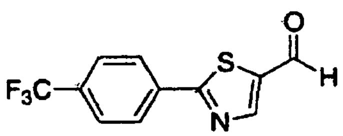

X1

**2-(4-Trifluoromethyl-phenyl)-thiazole-5-carbaldehyde**

A mixture of 2-bromo-5-formylthioazole (525 mg, 2.73 mmol), (4-trifluoromethyl)phenylboronic acid (519 mg, 2.73 mmol), tetrakis(triphenylphosphine)palladium(0) (95 mg, 0.082 mmol), and 2 N aqueous $Na_2CO_3$ (5.5 mL, 10.94 mmol) were refluxed in dioxane (8 mL) for 20 h. It was cooled and partitioned between EtOAc and water. After separating layers, the organic phase was washed with water and brine. It was dried over $Na_2SO_4$ and concentrated under reduced pressure. The crude product was purified by column chromatography Eluting with EtOAc/Hexane. The title compound was obtained in yellow solids (145 mg, 21%).

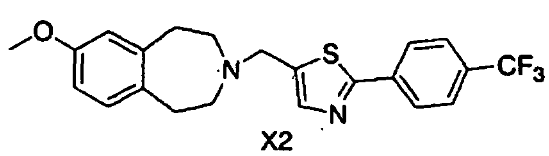

X2

**7-Methoxy-3-[2-(4-trifluoromethyl-phenyl)-thiazol-5-ylmethyl]-2,3,4,5-tetrahydro-1*H*-benzo[*d*]azepine**

Cpd X1 (139 mg, 0.541 mmol) and cpd A1a (96 mg, 0.541 mmol) were mixed with dichloroethane (2 mL) and glacial acetic acid (0.031 mL, 0.541 mmol) was added. After the resulting mixture was stirred at r.t. for 3 h, $Na(OAc)_3BH$ (172 mg, 0.811 mmol) was added and the mixture was stirred for another 21 h. It was then basified with 2N aq. NaOH and extracted with EtOAc. The organic extracts were concentrated under reduced pressure. Purification by column chromatography gave cpd X2 as a white solid (168 mg, 74%). $^1H$ NMR (300 MHz, $CDCl_3$) δ 8.04 (d, $J$ = 8.2 Hz, 2 H),

7.69 (d, *J* = 8.4 Hz, 2 H), 7.67 (s, 1 H), 7.00 (d, *J* = 7.8 Hz, 1 H), 6.66 - 6.63 (m, 2 H), 3.90 (s, 2 H), 3.77 (s, 3 H), 2.88 (m, 4 H), 2.87 (m, 4 H); MS (ES) m/z: 419 (M+H+).

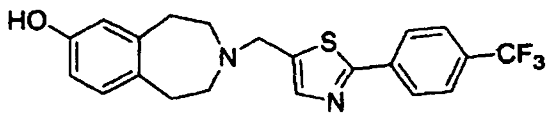

**X3**

**3-[2-(4-Trifluoromethyl-phenyl)-thiazol-5-ylmethyl]-2,3,4,5-tetrahydro-1*H*-benzo[*d*]azepin-7-ol**

A mixture of cpd X2 (145 mg, 0.347 mmol), HBr (48%, 0.8 mL, 6.94 mmol), $^nBu_4NBr$ (12 mg, 0.035 mmol) and HOAc (0.4 mL) were stirred at 100 ˚C for 18 h. After the mixture was cooled, it was diluted with water then basified with saturated aq. $K_2CO_3$ and extracted with EtOAc. The organic extracts were dried with $Na_2SO_4$ and concentrated under reduced pressure. The residue was purified by column chromatography eluting with dichloromethane/acetone (10:1) to give cpd X3 as a white solid (100 mg, 71%). $^1H$ NMR (300 MHz, $CD_3OD$) δ 8.11 (d, *J* = 8.4 Hz, 2 H), 7.77 (d, *J* = 8.4 Hz, 2 H), 7.75 (s, 1 H), 6.88 (d, *J* = 8.0 Hz, 1 H), 6:54 (d, *J* = 2.4 Hz, 1 H), 6.50 (dd, *J* = 8.0, 2.5 Hz, 1 H), 3.95 (s, 2 H), 2.83 (m, 4 H), 2.68 (m, 4 H); MS (ES) m/z: 405 (M+H+).

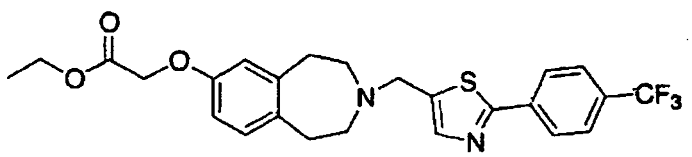

**X4**

**{3-[2-(4-Trifluoromethyl-phenyl)-thiazol-5-ylmethyl]-2,3,4,5-tetrahydro-1*H*-benzo[*d*]azepi n-7-yloxy}-acetic acid ethyl ester**

Cpd X3 (101 mg, 0.250 mmol) was dissolved in THF (3.0 mL). NaH (60%, 30 mg, 0.749 mmol) was added and followed by ethyl bromoacetate (0.033 mL, 0.297 mmol). The mixture was refluxed for 1 h. After cooling, it was treated with saturated aq. $NH_4Cl$ and partitioned between water and $Et_2O$. The organic layers were washed with brine and concentrated under reduced pressure. The resulting residue was purified by column chromatography eluting with EtOAc/ Hexane to give Cpd X4 as colorless oil (60 mg, 50%). $^1H$ NMR (300 MHz, $CDCl_3$) δ 8.04 (d, *J* = 8.2 Hz, 2 H), 7.69 (d, *J* = 8.5 Hz, 2 H), 7.67 (s, 1 H), 7.00 (d, *J* = 8.2 Hz, 1 H), 6.69 (d, *J* = 2.6 Hz, 1 H), 6.62 (dd, *J* = 8.2, 2.6 Hz, 1 H), 4.58 (s, 2 H), 4.26 (q, *J* = 7.1 Hz, 2 H), 3.89 (s, 2 H), 2.87 (m, 4 H), 2.68 (m, 4 H), 1.27 (t, *J* = 7.1 Hz, 3 H); MS (ES) m/z: 491 (M+H+).

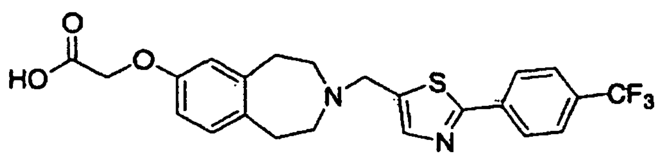

**Cpd 24**

**{3-[2-(4-Trifluoromethyl-phenyl)-thiazol-5-ylmethyl]-2,3,4,5-tetrahydro-1*H*-benzo[*d*]azepin-7-yloxy}-acetic acid**

A mixture of cpd X4 (50 mg, 0.102 mmol) in THF (0.3 mL) and methanol (0.3 mL) was treated with 2N aqueous NaOH (0.080 mL, 0.153 mmol). After stirring for 4 h, the mixture was acidified with 1 N HCl and extracted with dichloromethane. The organic extracts were dried over $Na_2SO_4$ and concentrated under reduced pressure to give cpd 24 (14 mg, 30%). $^1H$ NMR (300 MHz, $CD_3OD$) δ 8.18 (d, *J* = 8.3 Hz, 2 H), 8.04 (s, 1 H), 7.82 (d, *J* = 8.3 Hz, 2 H), 7.12 (d, *J* = 8.1 Hz, 1 H), 6.81 (s, 1 H), 6.76 (dd, *J* = 8.5, 2.6 Hz, 1 H), 4.67 (s, 2 H), 4.61 (s, 2 H), 3.37 (m, 4 H), 3.10 (m, 4 H); MS (ES) m/z: 463 (M+H+).

Example Y

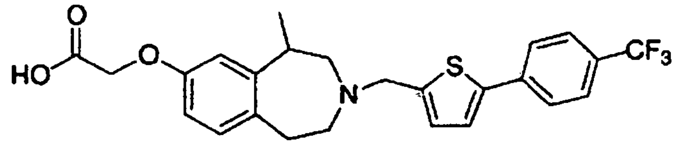

**Compound 25: {5-Methyl-3-[5-(4-trifluoromethyl-phenyl)-thiophen-2-ylmethyl]-2,3,4,5-tetrahydro-1*H*-benzo[*d*]aze pin-7-yloxy}-acetic acid**

The title compound was made according to Schemes Y1 and Y2.

## Scheme Y1

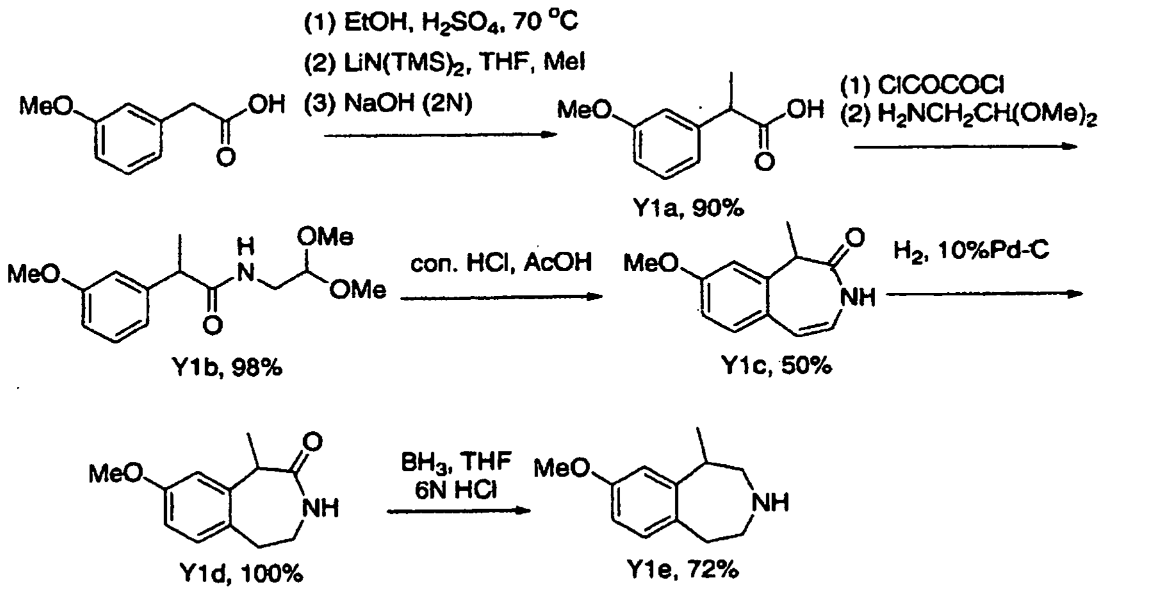


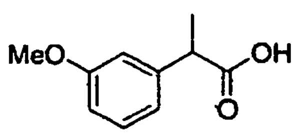

**Y1a**
**2-(3-Methoxy-phenyl)-propionic acid**

(3-methoxy)phenylacetic acid (4.1-64 g, 25.06 mmol) was refluxed in EtOH in presence of catalytic amount of conc. $H_2SO_4$. The mixture was concentrated *in vacuo*, diluted with EtOAc, washed with saturated aqueous $NaHCO_3$ and brine. It was dried over $Na_2SO_4$ and concentrated to give colorless oil (4.69 g, 97%).

The above oil (1.060 g, 5.466 mmol) was dissolved in THF (20 mL) and cooled to -70 °C. It was treated with a THF solution of LiN(TMS)$_2$ (1 M, 6.56 mL). After stirring at -70 °C for 30 min, MeI was added and the resulting mixture was stirred for 3 h and then quenched with saturated aqueous $NH_4Cl$. The mixture was diluted with water and extracted with EtOAc. The organic extracts were dried over $Na_2SO_4$ and concentrated *in vacuo* to give red oil (1.046 g, 92%).

The above oil (1.00 g, 4.807 mmol) was mixed with MeOH (3 mL) and THF (6 mL). 2N aqueous NaOH was added. The mixture was stirred for 4 h and then acidified with 6N HCl till PH =1. Extraction with dichloromethane and subsequent concentration *in vacuo* gave cpd Y1a as yellow oil (869 mg, 100%). $^1H$ NMR (400 MHz, $CDCl_3$) $\delta$ 7.29 - 7.22 (m, 1 H), 6.91 (d, $J$ = 7.7 Hz, 1 H), 6.86 (s, 1 H), 6.81 (d, $J$ = 7.6 Hz, 1 H), 3.80 (s, 3 H), 3.71 (q, $J$ = 7.2 Hz, 1 H), 1.50 (d, $J$ = 7.2 Hz, 3 H); MS (ES) m/z: 181 (M+H$^+$).

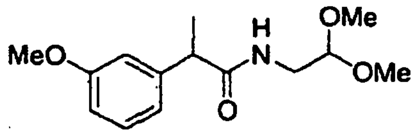

Y1b

*N*-(2,2-Dimethoxy-ethyl)-2-(3-methoxy-phenyl)-propionamide

Cpd Y1 a (822 mg, 4.567 mmol) was mixed with dichloromethane (20 mL). Oxalyl chloride (0.60 mL, 6.85 mmol) and 2 drops of DMF were added. The mixture was stirred at r.t. for 15 h and then concentrated *in vacuo*. The resulting crude residue was added to a mixture of triethylamine (0.96 mL, 6.850 mmol), (2,2-dimethoxy)ethylamine (480 mg, 4.567 mmol) in dichloromethane (20 mL) and the resulting mixture was stirred at r.t. for 18 h. It was partitioned between $CH_2Cl_2$ and water. The organic layer was dried ($Na_2SO_4$) and concentrated *in vacuo*. The crude was purified by column chromatography eluting with EtOAc/Hexane to give cpd Y1b as colorless oil (1.015 g, 83%): $^1$H NMR (300 MHz, $CDCl_3$) δ 7.30 - 7.21 (m, 1 H), 6.90 - 6.79 (m, 3 H), 5.54 (brs, 1 H), 4.28 (t, *J* = 5.4 Hz, 1 H), 3.81 (s, 3 H), 3.53 (q, *J* = 7.1 Hz, 1 H), 3.38 - 3.29 (m, 8 H), 1.51 (d, *J* = 7.2 Hz, 3 H).

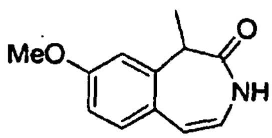

Y1c

8-Methoxy-1-methyl-1,3-dihydro-benzo[*d*]azepin-2-one

A mixture of cpd Y1 b (3.875 g, 14.495 mmol) in conc. HCl (20 mL) and HOAc (20 mL) was stirred at room temperature for 17 h. It was then poured onto ice (66 g). The precipitates were collected by filtration and dried under vacuum at 70 ˚C to give cpd Y1c (1.464 g, 50%) as a yellow solid: $^1$H NMR (300 MHz, DMSO-$d_6$) δ 9.53 (d, *J* = 3.6 Hz, 1 H), 7.23 (d, *J* = 8.5 Hz, 1 H), 6.88 (dd, *J* = 8.5, 2.5 Hz, 1 H), 6.76 (d, *J* = 2.3 Hz, 1 H), 6.32 (d, *J*= 9.0 Hz, 1 H), 6.20 (dd, *J* = 9.0, 4.7 Hz, 1 H), 3.77 (s, 3 H), 3.15 (m, 1 H), 1.43 (d, *J* = 7.0 Hz, 3 H).

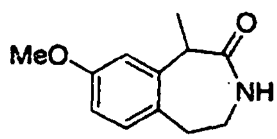

Y1d

8-Methoxy-1-methyl-1,3,4,5-tetrahydro-benzo[*d*]azepin-2-one

Cpd Y1c (1.44 g, 7.085 mmol) was mixed with EtOAc (30 mL) and MeOH (30 mL). Pd/C (10%, 75 mg, 0.071 mmol) was added. The fixture was shaken under $H_2$ atmosphere (45 psi) for 17 h. After filtration through celite, the solution was concentrated to give cpd Y1d (1.44 g, 100%) as a white solid: $^1$H NMR (300 MHz, DMSO-$d_6$) δ 7.33 (brs, 1 H), 7.06 (d, *J* = 8.3 Hz, 1 H), 6.75 (dd, *J* = 8.3, 2.5 Hz, 1 H), 6.68 (d, *J* = 2.4 Hz, 1 H), 4.22 (q, *J* = 13.8, 6.8 Hz, 1 H), 3.73 - 3.64 (m, 4 H), 3.18 - 3.10 (m, 2 H), 2.91 - 2.84 (m, 1 H), 1.33 (d, *J* = 6.9 Hz, 3 H); MS (ES) m/z: 206 (M+H$^+$).

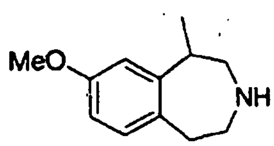

Y1e

8-Methoxy-1-methyl-2,3,4,5-tetrahydro-1*H*-benzo[*d*]azep
ine

To a solution of cpd Y1d (286 mg, 1.393 mmol) in THF (7 mL) was added $BH_3$-THF (1M, 4.18 mmol) at 0 ˚C. The mixture was stirred at reflux for 4 h and then cooled. MeOH (2 mL) was added to quench excessive $BH_3$ at 0 ˚C and the mixture was stirred at room temperature for another 30 min. After concentration, the residue was taken up with

6N HCl and stirred at 100 °C for 1 h. The mixture was extracted with $Et_2O$ and the organic layer was discarded. The remaining aqueous phase was basified with 5N NaOH and then extracted with EtOAc. The organic extracts were dried over $Na_2SO_4$ and concentrated *in vacuo* to give cpd Y1e (191 mg, 72%) as colorless oil: $^1H$ NMR (300 MHz, $CDCl_3$) δ 7.00 (d, *J* = 8.2 Hz, 1 H), 6.74 (d, *J* = 2.6 Hz, 1 H), 6.63 (dd, *J* = 8.2, 2.7 Hz, 1 H), 3.79 (s, 3 H), 3.05 - 2.86 (m, 6 H), 2.74 (dd, *J* = 13.4, 7.6 Hz, 1 H), 2.04 (brs, 1 H), 1.33 (d, *J* = 7.2 Hz, 3 H); MS (ES) m/z: 192 (M+H+).

## Scheme Y2

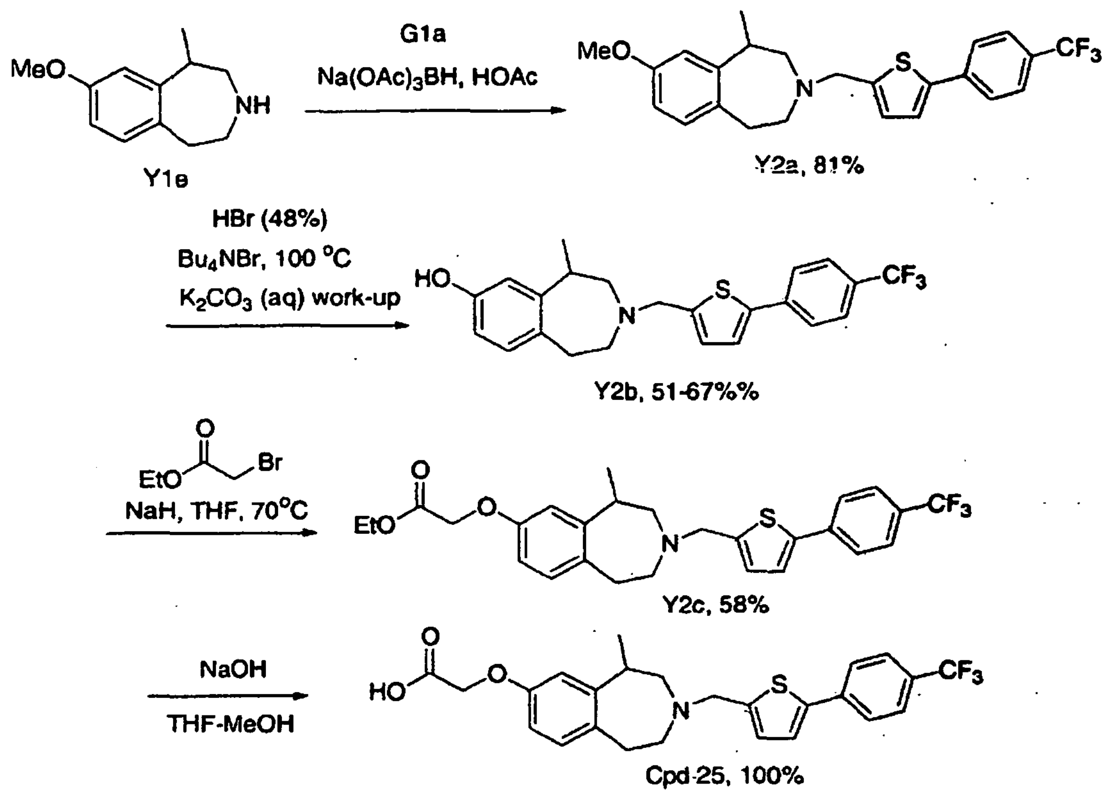


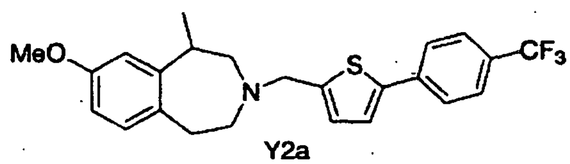

Y2a

**8-Methoxy-1-methyl-3-[5-(4-trifluoromethyl-phenyl)-thiophen-2-ylmethyl]-2,3,4,5-tetrahydro-1*H*-benzo[*d*]azepine**

A mixture of cpd Y1e (84.6 mg, 0.443 mmol), cpd G1a (113 mg, 0.443 mmol), dichloroethane (2 mL) and HOAc (0.025 mL, 0.443 mmol) was stirred at room temperature for 1 h. Na $(OAc)_3BH$ (141 mg, 0.664 mmol) was added. The mixture was continued stirring for another 20 h. After it was basified with 5N NaOH, the mixture was extracted with EtOAc. The organic extracts were dried ($Na_2SO_4$) and concentrated. The residue was purified by column chromatography to give cpd Y2a (154 mg, 81%) as a yellow oil: $^1H$ NMR (300 MHz, $CDCl_3$) δ 7.67 (d, *J* = 8.1 Hz, 2 H), 7.60 (d, *J* = 8.5 Hz, 2 H), 7.23 (d, *J* = 3.6 Hz, 1 H), 6.99 (d, *J* = 8.2 Hz, 1 H), 6.88 (m, 1 H), 6.73 (d, *J* = 2.5 Hz, 1 H), 6.64 (dd, *J* = 8.2, 2.6 Hz, 1 H), 3.83 (d, *J* = 4.7 Hz, 2 H), 3.78 (s, 3 H), 3.19 - 3.06 (m, 1 H), 2.99 - 2.70 (m, 4 H), 2.61 - 2.42 (m, 2 H), 1.36 (d, *J* = 7.2 Hz, 3 H); MS (ES) m/z: 432 (M+H+).

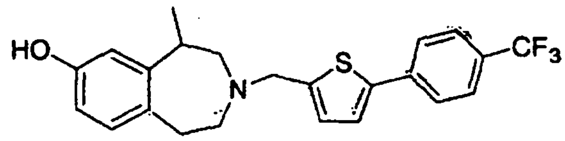

**Y2b**

**5-Methyl-3-[5-(4-trifluoromethyl-phenyl)-thiophen-2-ylmethyl]-2,3,4,5-tetrahydro-1*H*-benzo[*d*]azepin-7-ol**

A mixture of cpd Y2a (150 mg, 0.348 mmol), HBr (48%, 0.5 mL), HOAc (0.5 mL) and $^nBu_4NBr$ (22 mg, 0.070 mmol) was stirred at 100°C for 17 h. After it was cooled to room temperature, the mixture was basified with 5N NaOH and extracted with EtOAc. The organic extracts were washed with water, brine, dried ($Na_2SO_4$) and concentrated. The resulting residue was purified by column chromatography to give cpd Y2b (98 mg, 67%) as brown oil:

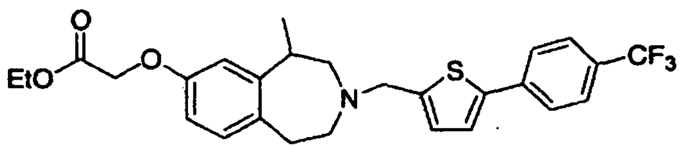

**Y2c**

**{5-Methyl-3-[5-(4-trifluoromethyl-phenyl)-thiophen-2-ylmethyl]-2,3,4,5-tetrahydro-1*H*-benzo[*d*]azepin-7-yloxy}-acetic acid ethyl ester**

To a mixture of cpd Y2b (89 mg, 0.213 mmol) in THF (1.5 mL) was added NaH (60%, 43 mg, 1.07 mmol) and ethyl bromoacetate (0.047 mL, 0.426 mmol) subsequently. After it was stirred at reflux for 50 min, the mixture was cooled to room temperature and quenched with aqueous $NH_4Cl$. The resulting mixture was partitioned between water and $Et_2O$. The layers were separated and the organic phase was dried ($Na_2SO_4$) and concentrated. The resulting residue was purified by column chromatography to give cpd Y2c (62 mg, 58%) as a yellow solid: $^1H$ NMR (300 MHz, $CDCl_3$) δ 7.67 (d, $J$ = 8.2 Hz, 2 H), 7.60 (d, $J$ = 8.6 Hz, 2 H), 7.24 (d, $J$ = 3.6 Hz, 1 H), 6.98 (d, $J$ = 8.3 Hz, 1 H), 6.89 (m, 1 H), 6.78 (d, $J$ = 2.4 Hz, 1 H), 6.59 (dd, $J$ = 8.2, 2.6 Hz, 1 H), 4.58 (s, 2 H), 4.27 (q, $J$ = 7.2 Hz, 2 H), 3.83 (s, 2 H), 3.19 - 3.05 (m, 1 H), 3.01 - 2.67 (m, 4 H), 2.59 - 2.40 (m, 2 H), 1.35 (d, $J$ = 7.2 Hz, 3 H), 1.30 (t, $J$ = 7.1 Hz, 3 H); MS (ES) m/z: 504 ($M+H^+$).

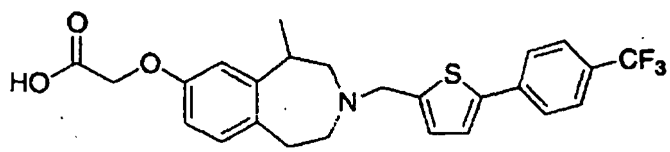

**Cpd 25**

**{5-Methyl-3-[5-(4-trifluoromethyl-phenyl)-thiophen-2-ylmethyl]-2,3,4,5-tetrahydro-1*H*-benzo[*d*]azepin-7-yloxy}-acetic acid**

A mixture of cpd Y2c (56 mg, 0.111 mmol), THF (0.5 mL), MeOH (0.5 mL) and NaOH (2N, 0.11 mL, 0.22 mmol) was stirred at room temperature for 6 h. The solution was acidified with 1 N HCl and extracted with dichloromethane. The organic extracts were dried and concentrated to give cpd 25 (57 mg, 100%) as a pale pink solid: $^1H$ NMR (300 MHz, $CD_3OD$) δ 7.86 (d, $J$ = 8.2 Hz, 2 H), 7.72 (d, $J$ = 8.3 Hz, 2 H), 7.57 (d, $J$ = 3.7 Hz, 1 H), 7.38 (d, $J$ = 3.8 Hz, 1 H), 7.15 (d, $J$ = 8.3 Hz, 1 H), 6.89 (d, $J$ = 2.5 Hz, 1 H), 6.77 (dd, $J$ = 8.3, 2.6 Hz, 1 H), 4.67 - 4.65 (m, 4 H), 3.76 - 3.41 (m, 4 H), 3.25 (m, 1 H), 3.17 - 3.00 (m, 2 H), 1.46 (d, $J$ = 7.2 Hz, 3 H); MS (ES) m/z: 476 ($M+H^+$).

<u>Example Z</u>

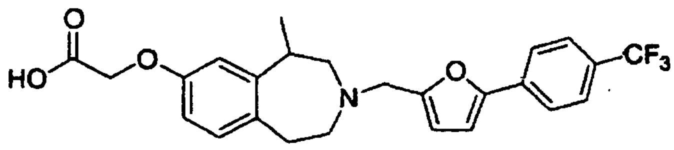

**Compound 26: {5-Methyl-3-[5-(4-trifluoromethyl-phenyl)-furan-2-ylmethyl]-2,3,4,5-tetrahydro-1*H*-benzo[*d*]azepin-7-yloxy}-acetic acid**

The title compound was made according to Scheme Z.

## Scheme Z

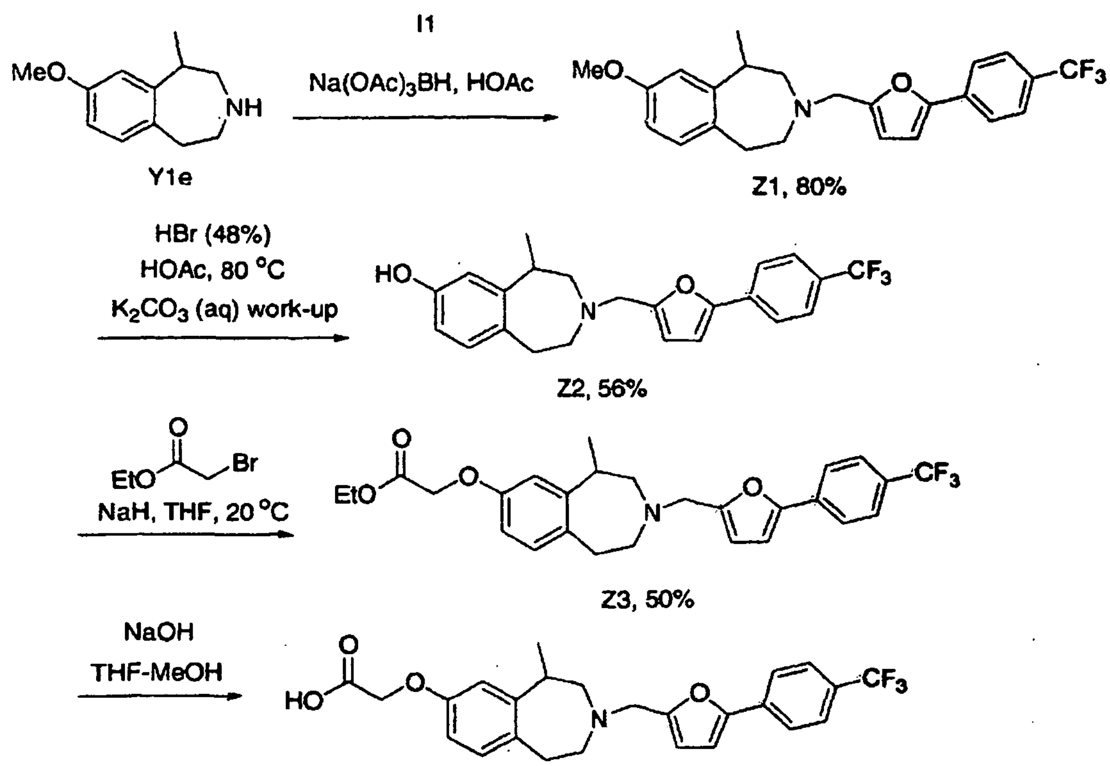


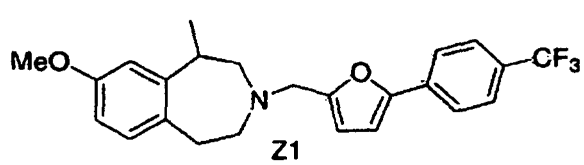

Z1

**8-Methoxy-1-methyl-3-[5-(4-trifluoromethyl-phenyl)-furan-2-ylmethyl]-2,3,4,5-tetrahydro-1*H*-benzo[*d*]azepine**

A mixture of cpd Y1e (89 mg, 0.466 mmol), cpd I1 (112 mg, 0.466 mmol), dichloroethane (2 mL) and HOAc (0.027 mL, 0.466 mmol) was stirred at room temperature for 16 h. $Na(OAc)_3BH$ (148 mg, 0.699 mmol) was added. The resulting mixture was continued stirring for another 24 h, basified with 2N NaOH and extracted with EtOAc. The organic extracts were dried ($Na_2SO_4$) and concentrated. The residue was purified by column chromatography to give cpd Z1 (153 mg, 79%): $^1H$ NMR (300 MHz, $CDCl_3$) δ 7.72 (d, *J* = 8.1 Hz, 2 H), 7.60 (d, *J* = 8.5 Hz, 2 H), 6.98 (d, *J* = 8.1 Hz, 1 H), 6.72(d, *J* = 2.5 Hz, 1 H), 6.68 (d, *J* = 3.3 Hz, 1 H), 6.62(dd, *J* = 8.2, 2.6 Hz, 1 H), 6.29 (d, *J* = 3.1 Hz, 1 H), 3.77 (s, 5 H), 3.11 - 3.16 (m, 1 H), 2.78 - 2.99 (m, 4 H), 2.40 - 2.54 (m, 2 H), 1.35 (d, *J* = 7.2 Hz, 3 H); MS (ES) m/z: 416 ($M+H^+$).

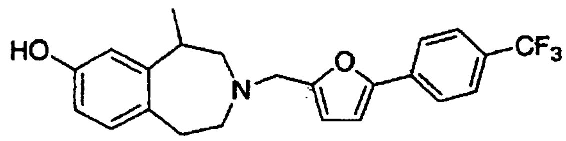

Z2

**5-Methyl-3-[5-(4-trifluoromethyl-phenyl)-furan-2-ylmethyl]-2,3,4,5-tetrahydro-1*H*-benzo[*d*]azepin-7-ol**

A mixture of cpd Z1 (140 mg, 0.337 mmol), HOAc (1 mL) and HBr (48%, 1 mL) was stirred at 80°C for 17 h. Upon cooling, the mixture was diluted with EtOAc and basified with saturated aqueous $K_2CO_3$ till pH 9. It was then extracted with EtOAc. The organic extracts were dried ($Na_2SO_4$) and concentrated to provide cpd Z2 (137 mg, 100%) as brown oil: $^1H$ NMR (300 MHz, $CDCl_3$) δ 7.71 (d, *J*= 8.2 Hz, 2 H), 7.59 (d, *J*= 8.3 Hz, 2 H), 6.90 (d, *J*= 8.0 Hz, 1 H), 6.68 (d, *J*= 3.3 Hz, 1 H), 6.65 (d, *J*=2.5 Hz, 1 H), 6.53 - 6.57 (dd, *J* = 8.0, 2.6 Hz, 1 H), 6.32 (s, 1 H), 3.75 - 3.80 (s, br, 2 H), 2.81 - 3.16 (m, 5 H), 2.37 - 2.51 (m, 2 H), 1.34 (d, *J*= 7.2 Hz, 3 H); MS (ES) m/z: 402 ($M+H^+$), 400 ($M-H^+$).

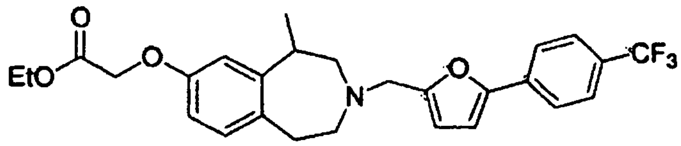

Z3

**{5-Methyl-3-[5-(4-trifluoromethyl-phenyl)-furan-2-ylmethyl]-2,3,4,5-tetrahydro-1*H*-benzo[*d*]azepin-7-yloxy}-acetic acid ethyl ester**

To a solution of cpd Z2 (108 mg, 0.269 mmol) in THF (1.5 mL) was added NaH (95%, 14 mg, 0.539 mmol) and ethyl bromoacetate (0.045 mL, 0.403 mmol). After it was stirred at room temperature for 4 h, it was quenched with aqueous $NH_4Cl$ and extracted with $Et_2O$. The organic extracts were dried, concentrated and the residue was purified by column chromatography to provide cpd Z3 (65 mg, 50%): $^1H$ NMR (300 MHz, $CDCl_3$) δ 7.72 (d, *J* = 8.2 Hz, 2 H), 7.60 (d, *J* = 8.3 Hz, 2 H), 6.98 (d, *J* = 8.2 Hz, 1 H), 6.78 (d, *J* = 2.6 Hz, 1 H), 6.68 (d, J = 3.3 Hz, 1 H), 6.56 - 6.60 (dd, *J* = 2.7, 8.2 Hz, 1 H), 6.30 (s, 1 H), 4.58 (s, 2 H), 4.23 - 4.29 (q, *J* = 7.2 Hz, 2 H), 3.78 (s, 2 H), 2.78 - 3.15 (m, 5 H), 2.38 - 2.51 (m, 2 H), 1.35 (d, *J*= 7.2 Hz, 3 H), 1.26 - 1.31 (t, *J*= 7.1 Hz, 3 H); MS (ES) m/z: 488 ($M+H^+$).

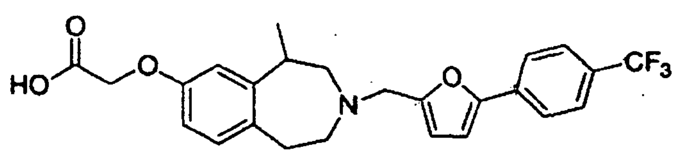

Cpd 26

**{5-Methyl-3-[5-(4-trifluoromethyl-phenyl)-furan-2-ylmethyl]-2,3,4,5-tetrahydro-1*H*-benzo[*d*]azepin-7-yloxy}-acetic acid**

To a solution of cpd Z3 (60 mg, 0.123 mmol) in THF (0.5 mL) and MeOH (0.5 mL) was added NaOH (2N, 0.12 mL). The mixture was stirred at room temperature for 2 h and then acidified with aqueous tartaric acid to pH 3-4. The mixture was extracted with EtOAc and the organic extracts were concentrated after drying over $Na_2SO_4$ to give cpd 26 (60 mg, 100%) as a white powder: $^1H$ NMR (300 MHz, $CDCl_3$) δ 7.61 - 7.71 (m, 4 H), 6.91 (d, *J* = 8.2 Hz, 1 H), 6.75 (s, 3 H), 6.66 - 6.69 (dd, *J*= 2.3, 8.2 Hz, 1 H), 4.62(s, 2 H), 4.27 - 4.44 (m, 2 H), 3.32 - 3.58 (m, 4 H), 2.60 - 2.76 (m, 3 H),

1.32 (d, $J$ = 6.9 Hz, 3 H); MS (ES) m/z: 460 ($M+H^+$).

**Example AA**

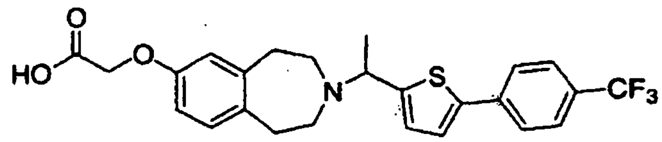

**Compound 27: (3-{1-[5-(4-Trifluoromethyl-phenyl)-thiophen-2-yl]-ethyl}-2,3,4,5-tetrahydro-1*H*-benzo[*d*]azepin-7-yloxy)-acetic acid**

The title compound was made according to Scheme AA.

**Scheme AA**

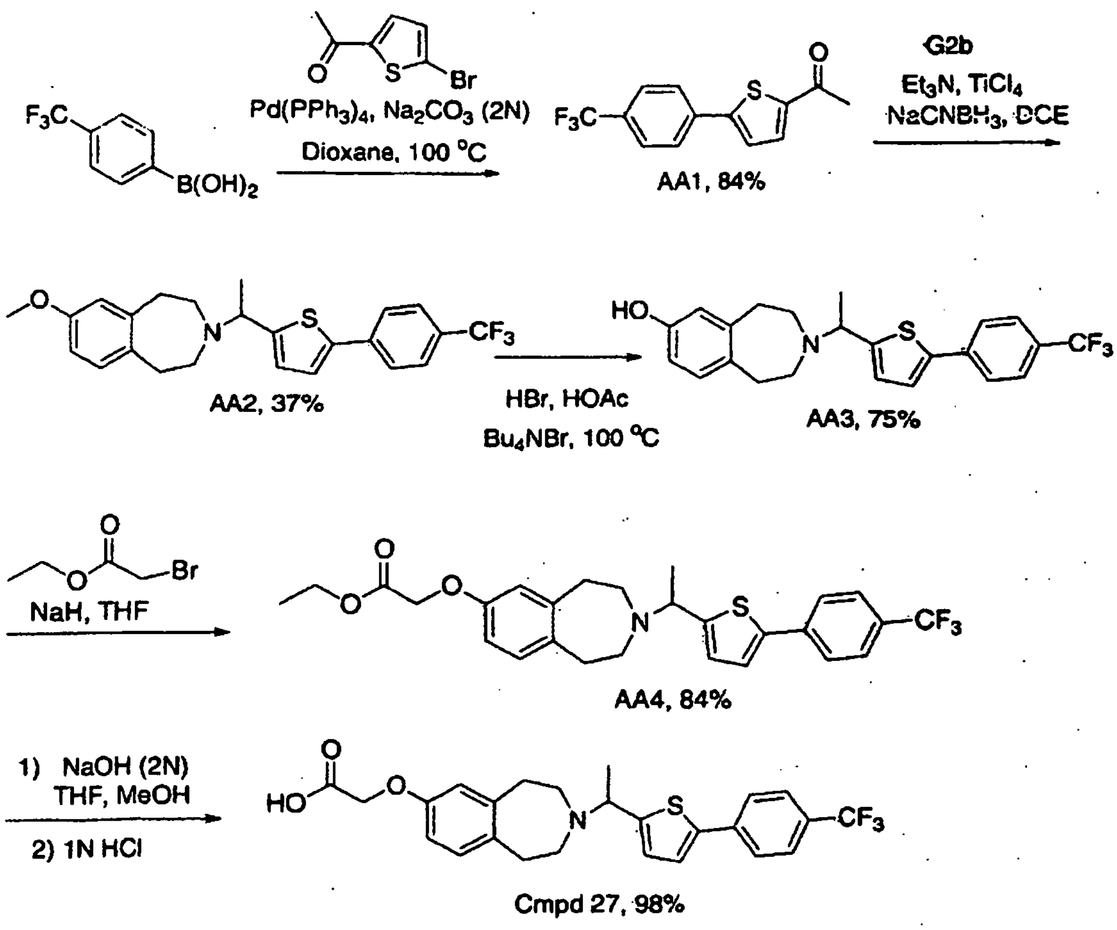


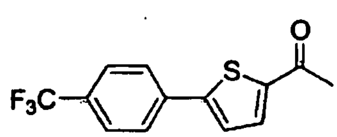

**AA1**

**1-[5-(4-Trifluoromethyl-phenyl)-thiophen-2-yl]-ethanone**

Cpd AA1 was prepared following a similar procedure as for cpd X1. Cpd AA1 was obtained (3.84 g, 84%) as

a pale yellow solid:

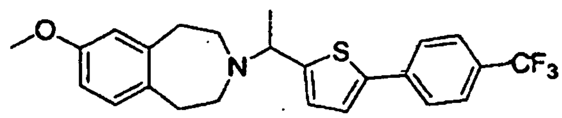

**AA2**

**7-Methoxy-3-{1-[5-(4-trifluoromethyl-phenyl)-thiophen-2-yl]-ethyl}-2,3,4,5-tetrahydro-1*H*-benzo[*d*]azepine**

To a mixture of Cpd G2b (582 mg, 3.288 mmol), cpd AA1 (888 mg, 3.288 mmol) and dichloroethane (30 mL) was added triethylamine (1.37 mL, 9.864 mmol) and $TiCl_4$ (1 M in dichloromethane, 1.60 mL, 1.644 mmol). The resulting mixture was stirred at room temperature for 16 h. A solution of $NaBH_3CN$ (310 mg, 4.932 mmol) in MeOH (2 mL) was added and stirring was continued for another 5.5 h. The mixture was then basified with 2N NaOH and extracted with EtOAc. The organic extracts were dried and concentrated. The residue was purified by column chromatography to give cpd AA2 (487 mg, 34%) as a reddish slurry: $^1H$ NMR (300 MHz, $CDCl_3$) δ 7.67 (d, *J* = 8.9 Hz, 2 H), 7.59 (d, *J* = 8.3 Hz, 2 H), 7.23 (d, *J* = 3.6 Hz, 1 H), 6.99 (d, *J* = 8.0 Hz, 1 H), 6.84 (d, *J* = 3.6 Hz, 1 H), 6.66 - 6.61 (m, 2 H), 4.15 (q, *J* = 7.4 Hz, 1 H), 3.77 (s, 3 H), 2.89 (m, 4 H), 2.70 (m, 4 H), 1.41 (d, *J* = 6.7 Hz, 3 H); MS (ES) m/z: 432 ($M+H^+$).

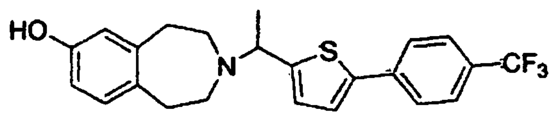

**AA3**

**3-{1-[5-(4-Trifluoromethyl-phenyl)-thiophen-2-yl]-ethyl}-2,3,4,5-tetrahydro-1*H*-benzo[*d*]azepin-7-ol**

Cpd AA3 was prepared according to a similar procedure as for cpd Y2b. Cpd AA3 was obtained (249 mg, 53%) as a foam solid: $^1H$ NMR (300 MHz, $CDCl_3$) δ 7.67 (d, *J* = 8.3 Hz, 2 H), 7.59(d, *J* = 8.5 Hz, 2 H), 7.23 (d, *J* = 3.7 Hz, 1 H), 6.94 (d, *J* = 7.9 Hz, 1 H), 6.84 (d, *J* = 3.5 Hz, 1 H), 6.59 - 6.54 (m, 2 H), 4.49 (brs, 1 H), 2.86 (m, 4 H), 2.71 (m, 4 H), 1.41 (d, *J* = 6.8 Hz, 3 H); MS (ES) m/z: 418 ($M+H^+$).

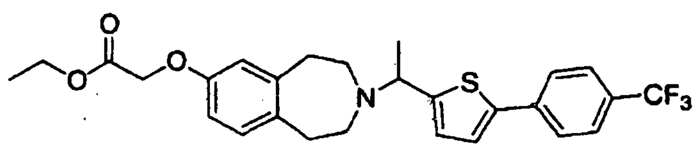

**AA4**

**(3-{1-[5-(4-Trifluoromethyl-phenyl)-thiophen-2-yl]-ethyl}-2,3,4,5-tetrahydro-1*H*-benzo[*d*]azepin-7-yloxy)-acetic acid ethyl ester**

Cpd AA4 was prepared according to a similar procedure as for cpd Y2c. Cpd AA4 was obtained (222 mg, 74%) as brown oil: $^1H$ NMR (300 MHz, $CDCl_3$) δ 7.67 (d, *J* = 8.2 Hz, 2 H), 7.59 (d, *J* = 8.3 Hz, 2 H), 7.23 (d, *J* = 3:6 Hz, 1 H), 6.99 (d, *J* = 8.2 Hz, 1 H), 6.84 (d, *J* = 3.5 Hz, 1 H), 6.69 (d, *J* = 2.6 Hz, 1 H), 6.61 (dd, *J* = 8.2, 2.6 Hz, 1 H), 4.57 (s, 2 H), 4.26 (q, *J* = 7.1 Hz, 2 H), 4.12 (q, *J*=7.1 Hz, 1 H), 2.88 (m, 4 H), 2.69 (m, 4 H), 1.41 (d, *J*= 6.7 Hz, 3 H), 1.29 (t, *J* = 7.2 Hz, 3 H); MS (ES) m/z: 504 ($M+H^+$).

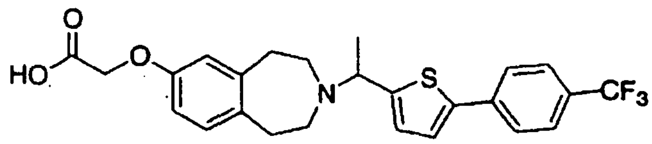

**Cpd 27**

**(3-{1-[5-(4-Trifluoromethyl-phenyl)-thiophen-2-yl]-ethyl}-2,3,4,5-tetrahydro-1*H*-benzo[*d*]azepin-7-yloxy)-acetic acid**

Cpd 27 was prepared according to a similar procedure as for cpd 25. Cpd 27 was obtained (220 mg) in 100% yield: $^1$H NMR (300 MHz, $CDCl_3$) δ 7.62 (s, 4 H), 7.26 (m, 1 H), 7.17 (d, J = 3.5 Hz, 1 H), 6.90 (d, J = 8.2 Hz, 1 H), 6.72 (dd, J = 8.1, 2.4 Hz, 1 H), 6.61 (d, J = 2.2 Hz, 1 H), 4.98 (m, 1 H), 4.60 (s, 2 H), 3.40 - 2.55 (m, 8 H), 1.92 (d, J = 6.9 Hz, 3 H); MS (ES) m/z: 476 ($M+H^+$).

Cpd 27 racemates were separated by chiral HPLC. Condition used: column AD 25 cm, λ 302 nm, flow 1 ml/min; eluents: 70% of (Heptane +0.1 % TFA) and 30% of ((MeOH + EtOH)13/1 + 0.1 % TFA) ; For cpd 27a: α = (-) 45.0 ° (c = 1, MeOH); For cpd 27b: α = (+) 57.7 ° (c = 1, MeOH/CHCl3).

Example BB

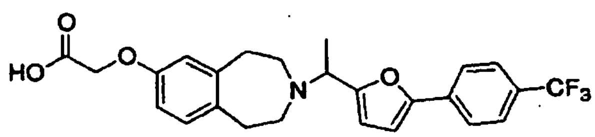

**Cpd 28**

**Compound 28: (3-{1-[5-(4-Trifluoromethyl-phenyl)-furan-2-yl]-ethyl}-2,3,4,5-tetrahydro-1*H*-benzo[*d*]azepin-7-yloxy)-acetic acid; compound with methane**

The title compound was made according to Scheme BB.

## Scheme BB

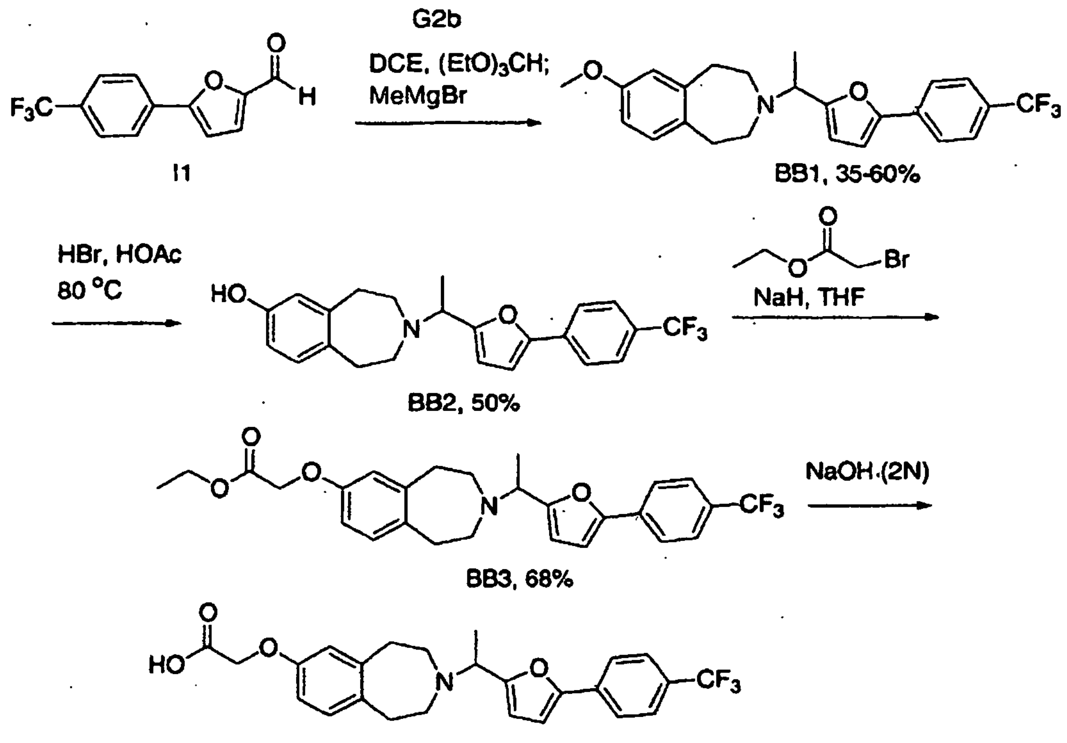

I1
BB1, 35-60%
BB2, 50%
BB3, 68%
Cpd 28, 90%

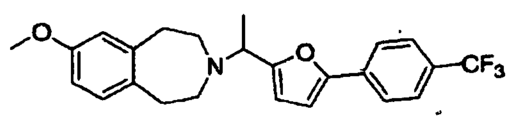

**BB1**

**7-Methoxy-3-{1-[5-(4-trifluoromethyl-phenyl)-furan-2-yl]-ethyl}-2,3,4,5-tetrahydro-1*H*-benzo[*d*]azepine**

To a mixture of cpd I1 (750 mg, 3.124 mmol), cpd G2b (553 mg, 3.124 mmol) and dichloroethane (10 mL) was added triethyl orthoformate (0.52 mL, 3.124 mmol). After stirring at room temperature for 16 h, the resulting mixture was treated with MeMgBr (1.4 M in Toluene and THF, 4.5 mL, 6.25 mmol) with slow addition. The mixture was quenched with aqueous NH4Cl after stirring for another 10 min and then partitioned between EtOAc and water. The organic layers were separated, dried and concentrated. The crude product was purified by column chromatography to provide cpd BB1 (646 mg, 50%) as yellow oil: $^1$H NMR (300 MHz, $CDCl_3$) δ 7.68 (d, *J* = 8.2 Hz, 2 H), 7.58 (d, *J*= 8.4 Hz, 2 H), 6.97 (d, *J* = 8.1 Hz, 1 H), 6.59-6.66 (m, 3 H), 6.22 (s, 1 H), 4.02-4.04 (m, 1 H), 3.74 (s, 3 H), 2.78-2.87 (m, 6 H), 2.56-2.58 (m, 2 H), 1.49 (d, *J* = 6.6 Hz, 3 H); MS (ES) m/z: 416 ($M+H^+$).

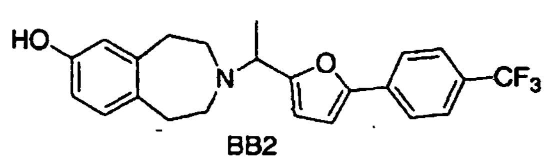

**BB2**

**3-{1-[5-(4-Trifluoromethyl-phenyl)-furan-2-yl]-ethyl}-2,3,4,5-tetrahydro-1*H*-benzo[*d*]azepin-7-ol**

Cpd BB2 was prepared according to a similar procedure as for cpd Z2. Cpd BB2 was obtained (157 mg) in 54% yield: $^1$H NMR (300 MHz, $CDCl_3$) δ 7.68 (d, *J* = 8.4 Hz, 2 H), 7.58 (d, *J* = 8.4 Hz, 2 H), 6.91 (d, *J*= 7.9 Hz, 1 H), 6.66 (d, *J* = 3.3 Hz, 1 H), 6.51-6.57 (m, 2H), 6.23 (d, *J*= 3.2 Hz, 1 H), 4.01-4.09 (m, 1 H), 2.78-2.86 (m, 6 H), 2.57 - 2.68 (m, 2 H), 1.50 (d, *J* = 6.9 Hz, 3 H); MS (ES) m/z: 402 ($M+H^+$).

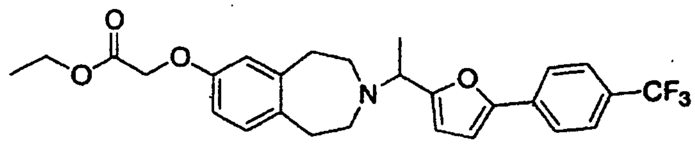

BB3
(3-{1-[5-(4-Trifluoromethyl-phenyl)-furan-2-yl]-ethyl}-2,3,4,5-tetrahydro-1*H*-benzo[*d*]azepin-7-yloxy)-acetic acid ethyl ester

Cpd BB3 was prepared according to a similar procedure as for cpd Z3. Cpd BB3 was obtained (546 mg, 68%) as light brown oil: $^1$H NMR (300 MHz, $CDCl_3$) δ 7.68 (d, *J*= 8.3 Hz, 2 H), 7.59 (d, *J*= 8.3 Hz, 2 H), 6.96 (d, *J* = 8.2 Hz, 1 H), 6.60 - 6.67 (m, 2 H), 6.56 - 6.59 (m, 1 H), 6.21 (s, 1 H), 4.55 (s, 2 H), 4.21 - 4.28 (q, *J* = 7.2 Hz, 2 H), 4.00 - 4.11 (m, 1 H), 2.76 - 2.87 (m, 6 H), 2.55 - 2.58 (m, 2H), 1.47 - 1.50 (d, *J* = 6.8 Hz, 3 H), 1.23 - 1.28 (m, 3 H); MS (ES) m/z: 488 ($M+H^+$). Cpd DD3 racemates were separated by chiral HPLC: column AD 500g, flow rate 80 ml/min, λ 220 nm, eluent: $CH_3CN$. One enantiomer BB3a shows optical rotation α = (+) 7.9° (c = 1, 100 mm, MeOH); The other enantiomer BB3b shows optical rotation α = (-) 3.8 ° (c = 1, 100 mm, MeOH).

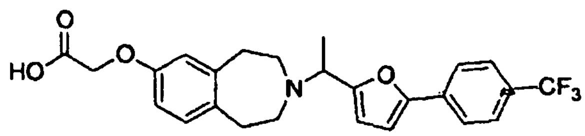

Cpd 28

(3-{1-[5-(4-Trifluoromethyl-phenyl)-furan-2-yl]-ethyl}-2,3,4,5-tetrahydro-1*H*-benzo[*d*]azepin-7-yl oxy)-acetic acid; compound with methane

Cpd 28 was prepared according to a similar procedure as for cpd 26. Cpd 28 was obtained (30 mg) in quantitative yield: $^1$H NMR (300 MHz, DMSO) δ 7.82 (d, *J* = 8.3 Hz, 2 H), 7.74 (d, *J* = 8.5 Hz, 2 H), 7.06 (d, *J* = 3.3 Hz, 1 H), 6.96 (d, *J* = 8.2 Hz, 1 H), 6.65 (d, *J* = 2.6 Hz, 1 H), 6.55-6.57 (m, 1 H), 6.41 (d, *J* = 3.3 Hz, 1H), 4.56 (s, 2 H), 4.07 (m, 1 H), 2.69 - 2.79 (m, 8 H), 1.41 (d, *J* = 7.0 Hz, 3 H); MS (ES) m/z: 460 ($M+H^+$). Cpd 28a: α = (+) 19.2 ° (c = 0.4, MeOH); Cpd 28b: α = (-) 25.4 ° (c = 0.4, MeOH).

Example CC

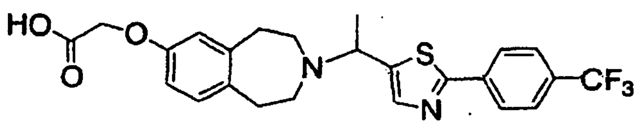

Compound 29: (3-{1-[2-(4-Trifluoromethyl-phenyl)-thiazol-5-yl]-ethyl}-2,3,4,5-tetrahydro-1*H*-benzo[*d*]azepin-7-yloxy)-acetic acid

The title compound was made according to Scheme CC.

## Scheme CC

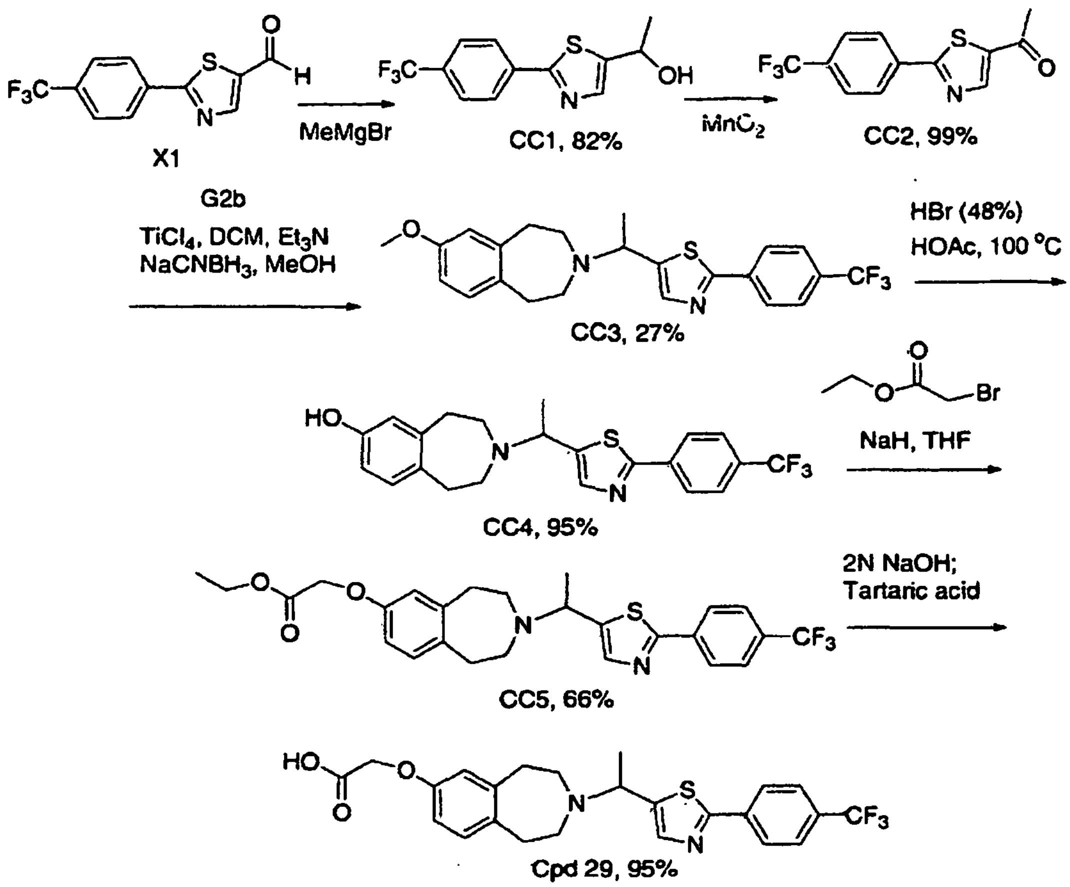


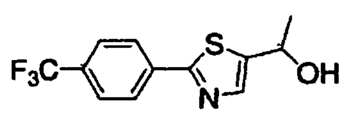

CC1

1-[2-(4-Trifluoromethyl-phenyl)-thiazol-5-yl]-ethanol

To a solution of cpd X1 (445 mg, 1.73 mmol) in THF (5 mL) was added MeMgBr (1.4 M, 1.48 mL, 2.078 mmol) at 0 °C. After stirring at 0 °C for 2 h, the mixture was quenched with aqueous $NH_4Cl$ and extracted with EtOAc. The organic extracts were dried and concentrated. The residue was purified by column chromatography to give cpd CC1 (307 mg) in 65%yield: $^1$H NMR (300 MHz, $CDCl_3$) δ 8.04 (d, *J*= 8.1 Hz, 2 H), 7.16 (s, 1 H), 7.70 (d, *J* = 8.2 Hz, 2H), 5.20-5.27 (q, *J* = 6.3 Hz, 1 H), 1.67 (d, *J* = 6.4 Hz, 3 H); MS (ES) m/z: 274 ($M+H^+$).

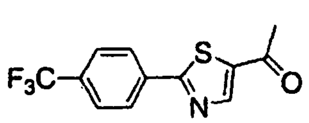

CC2

1-[2-(4-Trifluoromethyl-phenyl)-thiazol-5-yl]-ethanone

A mixture of cpd CC1 (302 mg, 1.106 mmol), $MnO_2$ (1.92 g, 22.12 mmol) in dichloromethane (20 mL) was stirred at room temperature for 7 h. It was filtered through celite and concentrated to give cpd CC2 (295 mg, 99%) as a white solid: $^1$H NMR (300 MHz, $CDCl_3$) δ 8.38 (s, 1 H), 8.12 (d, *J* = 8.2 Hz, 2 H), 7.74 (d, *J* = 8.2 Hz, 2H), 2.64 (s, 3 H); MS (ES) m/z: 272 ($M+H^+$).

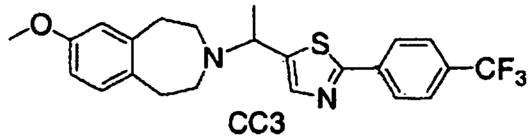

CC3

**7-Methoxy-3-{1-[2-(4-trifluoromethyl-phenyl)-thiazol-5-yl]-ethyl}-2,3,4,5-tetrahydro-1*H*-benzo[*d*] azepine**

Cpd CC3 was prepared according to a similar procedure as for cpd AA2. Cpd CC3 was obtained (130 mg, 27%) as yellow slurry: $^1$H NMR (300 MHz, $CDCl_3$) δ 8.03 (d, *J* = 8.2 Hz, 2 H), 7.68 (d, *J* = 8.2 Hz, 2 H), 7.61 (s, 1 H), 6.99 (d, *J* = 7.9 Hz, 1 H), 6.62-6.65 (m, 2 H), 4.21 (m, 1 H), 3.77 (s, 3 H), 2.85-2.90 (m, 4 H), 2.70-2.76 (m, 4 H), 1.44 (d, *J* = 6.7 Hz, 3H); MS (ES) m/z: 433 ($M+H^+$).

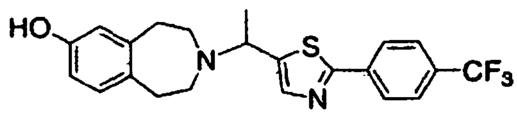

CC4

**3-{1-[2-(4-Trifluoromethyl-phenyl)-thiazol-5-yl]-ethyl}-2,3,4,5-tetrahydr o-1*H*-benzo[*d*]azepin-7-ol**

Cpd CC4 was prepared according to a similar procedure as for cpd Y2b. Cpd CC4 was obtained (38 mg) in 95% yield: $^1$H NMR (300 MHz, $CDCl_3$) δ 8.03 (d, *J* = 8.2 Hz, 2 H), 7.68 (d, *J*=8.3 Hz, 2 H), 7.61 (s, 1 H), 6.94 (d, *J* = 7.9 Hz, 1 H), 6.55 - 6.59 (m, 2 H), 4.16 - 4.22 (m, 1 H), 2.84 (m, 4 H), 2.64- 2.68 (m, 4 H), 1.44 (d, *J*= 6.6 Hz, 3H); MS (ES) m/z: 419 ($M+H^+$), 417 ($M-H^+$).

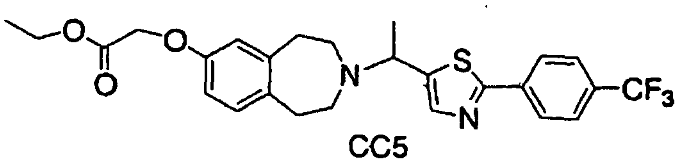

CC5

**(3-{1-[2-(4-Trifluoromethyl-phenyl)-thiazol-5-yl]-ethyl}-2,3,4,5-tetrahydro-1*H*-benzo[*d*]azepin-7-yloxy)-acetic acid ethyl ester**

Cpd CC5 was prepared according to a similar procedure as for cpd Y2c. Cpd CC5 was obtained (23 mg) in 70% yield: $^1$H NMR (300 MHz, $CDCl_3$) δ 8.03 (d, *J*= 8.2 Hz, 2 H), 7.68 (d, *J*= 8.2 Hz, 2 H), 7.61 (s, 1 H), 7.00 (d, *J* = 8.1 Hz, 1 H), 6.69 (d, *J* = 2.5 Hz, 1 H), 6.60 - 6.64 (dd, *J* = 2.6, 8.2 Hz, 1 H), 4.57 (s, 2 H), 4.11 - 4.30 (m, 3 H), 2.86 (m, 4 H), 2.69 (m, 4 H), 1.44 (d, *J* = 6.6 Hz, 3H), 1.23 - 1.31 (m, 3 H); MS (ES) m/z: 505 ($M+H^+$).

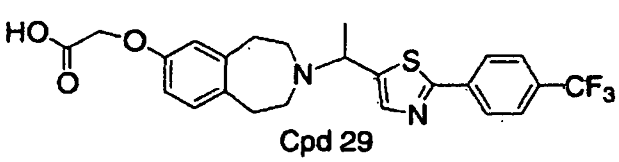

Cpd 29

**(3-{1-[2-(4-Trifluoromethyl-phenyl)-thiazol-5-yl]-ethyl}-2,3,4,5-tetrahydro-1*H*-benzo[*d*]azepin-7-yloxy)-acetic acid**

Cpd 29 was prepared according to a similar procedure as for cpd 27. Cpd 29 was obtained (18 mg, 95%) as a white solid: $^1$H NMR (300 MHz, $CDCl_3$) δ 8.03 (d, *J* = 8.2 Hz, 2 H), 7.75 (s, 1 H), 7.68 (d, *J* = 8.2 Hz, 2 H), 6.90 (d, *J* = 8.1 Hz, 1 H), 6.72 (m, 1 H), 6.60 (s, 1 H), 5.15 (m, 1 H), 4.61 (s, 2 H), 3.00 (m, 8 H), 1.90 (d, *J* = 6.6 Hz, 3H); MS (ES) m/z: 477 ($M+H^+$).

Example DD

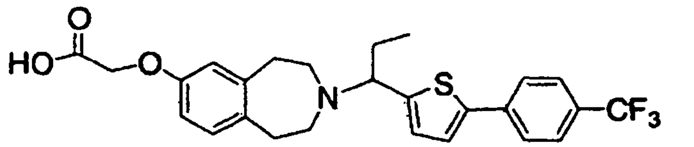

**Compound 30: (3-{1-[5-(4-Trifluoromethyl-phenyl)-thiophen-2-yl]-propyl}-2,3,4,5-tetrahydro-1*H*-benzo[*d*]azepin-7-yloxy)-acetic acid**

The title compound was made according to Scheme DD.

## Scheme DD

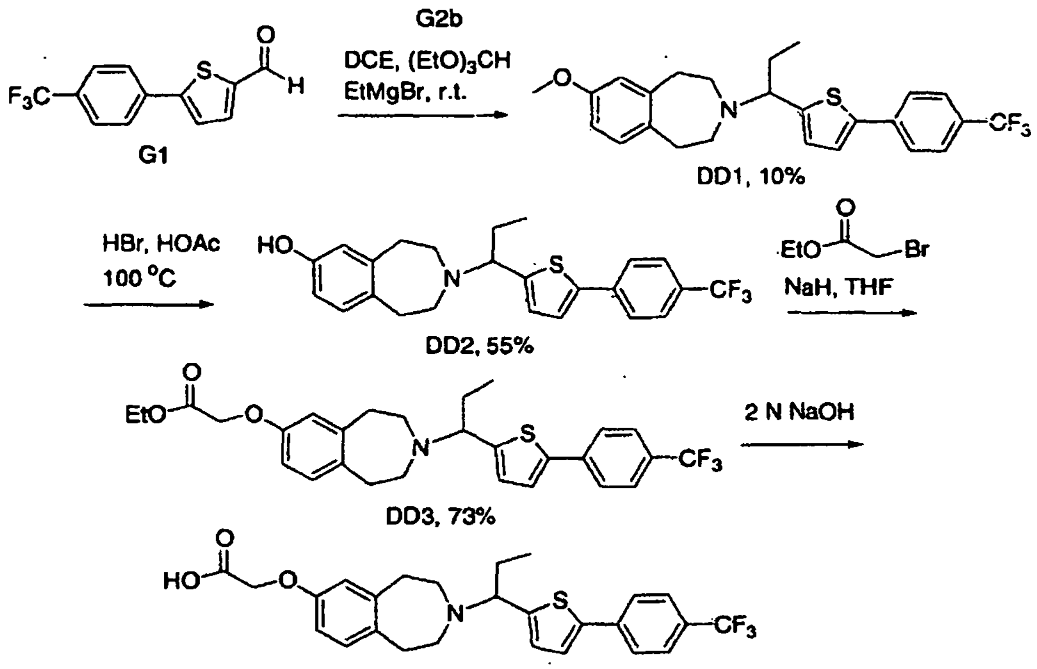


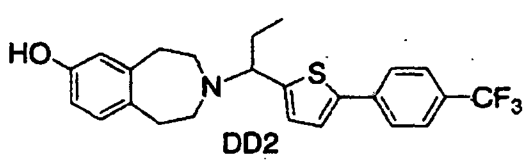

**DD2**

**3-{1-[5-(4-Trifluoromethyl-phenyl)-thiophen-2-yl]-propyl}-2,3,4,5-tetrahydro-1*H*-benzo[*d*]azepin-7-ol**

Cpd DD1 was prepared following a similar procedure as for cpd BB1. Crude cpd DD1 was subjected to the same reaction condition as for preparing cpd Z2 and cpd DD2 was obtained (64 mg, 55%) as brown oil: $^1H$ NMR (300 MHz, $CDCl_3$) δ 7.56-7.64 (m, 4 H), 7.21 (d, *J* = 3.6 Hz, 1 H), 6.91 (d, *J* = 8.0 Hz, 1 H), 6.80 (d, *J* = 3.3 Hz, 1 H), 6.49-6.56 (m, 2 H), 3.83 (t, *J* = 7.7 Hz, 1 H), 2.83-2.92 (m, 4 H), 2.70-2.80 (m, 2 H), 2.54-2.61 (m, 2 H), 1.83-1.99 (m, 2 H), 1.24-1.28

(t, $J$ = 7.2 Hz, 3 H); MS (ES) m/z: 432 ($M+H^+$).

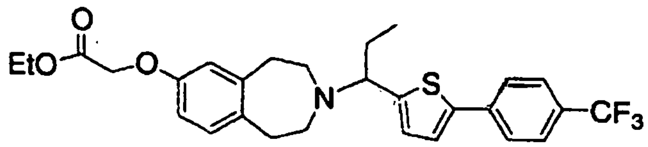

DD3

**(3-{1-[5-(4-Trifluoromethyl-phenyl)-thiophen-2-yl]-propyl}-2,3,4,5-tetrahydro-1*H*-benzo[*d*]azepin-7-yloxy)-acetic acid ethyl ester**

Cpd DD3 was prepared following the same procedure as for cpd Z3. Cpd DD3 was obtained (50 mg) in 73% yield: $^1$H NMR (300 MHz, $CDCl_3$) δ 7.56 - 7.64 (m, 4 H), 7.21 (d, $J$ = 3.6 Hz, 1 H), 6.96 (d, $J$ = 8.2 Hz, 1 H), 6.80 (d, $J$ = 3.6 Hz, 1 H), 6.66 (d, $J$ = 2.5 Hz, 1 H), 6.56 - 6.60 dd, $J$ = 2:6, 8.2 Hz, 1 H), 4.55 (s, 2 H), 4.21-4.28 (q, $J$ = 7.1 Hz, 2 H), 3.83 (m, 1 H), 2.58 - 2.87 (m, 8 H), 1.81-2.11 (m, 2 H), 1.23 - 1.30 (q, $J$ = 7.0 Hz, 3 H), 1.01 (t, $J$ = 7.3 Hz, 3 H); MS (ES) m/z: 518 ($M+H^+$).

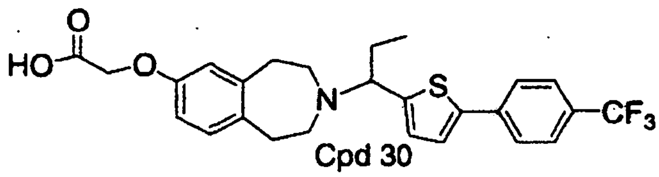

Cpd 30

**(3-{1-[5-(4-Trifluoromethyl-phenyl)-thiophen-2-yl]-propyl}-2,3,4,5-tetrahydro-1*H*-benzo[*d*]azepin-7-yloxy)-acetic acid**

Cpd 30 was prepared according to the same procedure as for cpd 26. Cpd 30 was obtained (17 mg) in 45% yield: $^1$H NMR (300 MHz, $CDCl_3$) δ 7.52-7.62 (m, 5 H), 7.12 - 7.16 (m, 1 H), 6.91 - 6.97 (m, 1 H), 6.67 (d, J = 8.3 Hz, 1 H), 6.62 (s, 1 H), 4.65 - 4.76 (m, 1 H), 4.61 (s, 2 H), 3.56 - 3.85 (m, 4 H), 2.64 - 2.75 (m, 4 H), 1.90 - 2.09 (m, 2 H), 0.95 (t, $J$ = 6.8 Hz, 3 H); MS (ES) m/z: 490 ($M+H^+$).

Example EE

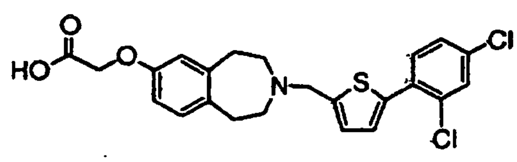

**Compound 31: {3-[5-(2,4-Dichloro-phenyl)-thiophen-2-ylmethyl]-2,3,4,5-tetrahydro-1*H*-benzo[*d*]azepin-7-yloxy}-acetic acid**

The title compound was made according to Scheme EE.

**Scheme EE**

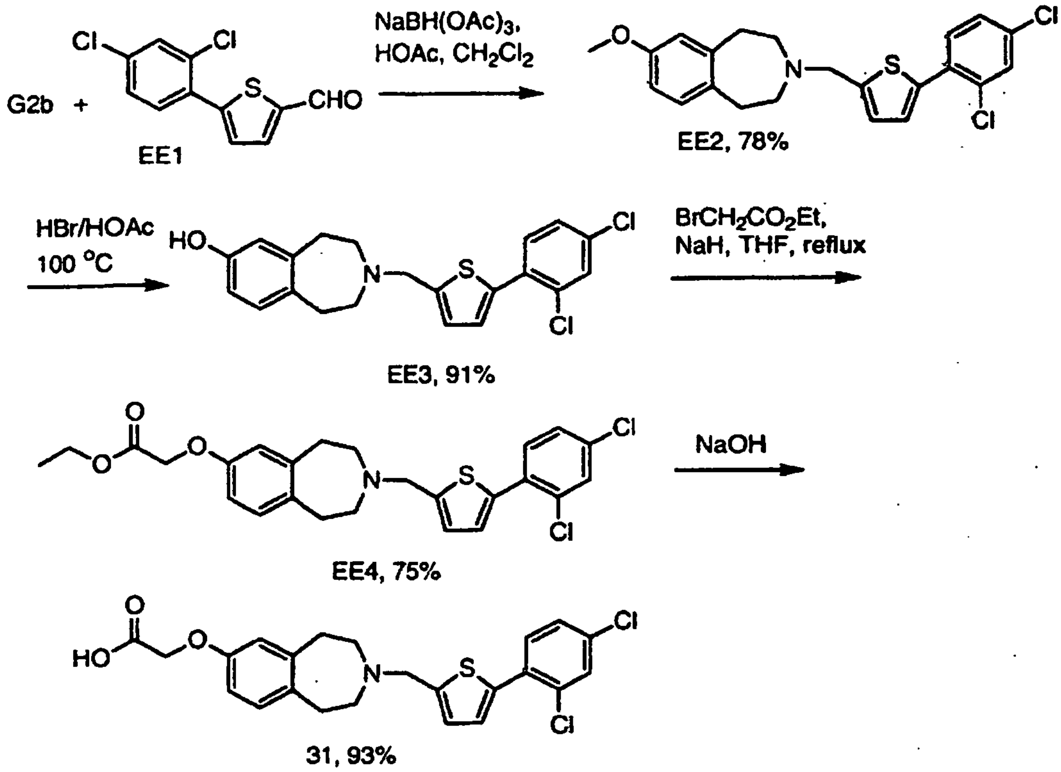


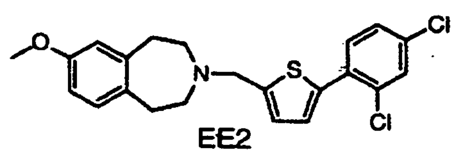

EE2

**3-[5-(2,4-Dichloro-phenyl)-thiophen-2-ylmethyl]-7-methoxy -2,3,4,5-tetrahydro-1*H*-benzo[*d*]azepine**

To a mixture of cpd G2b (0.15 g, 0.85 mmol), 5-(dichlorophenyl)-thiophene-2-carbaldehyde (0.20 g, 0.78 mmol), $CH_2Cl_2$ (15 mL) and HOAc (0.05 mL, 0.87 mmol) at room temperature was added $Na(OAc)_3BH$ (0.26 g, 1.17 mmol). The mixture was stirred for 2 days and additional $Na(OAc)_3BH$ (0.13 g, 0.59 mmol) was added. After the mixture was continued stirring overnight, it was basified with aqueous $NaHCO_3$ and extracted with $CH_2Cl_2$. The organic extracts were dried ($MgSO_4$) and concentrated. The crude product was purified by column chromatography to give cpd EE2 (0.25 g, 78%): $^1H$ NMR (300 MHz, $CDCl_3$) δ 7.39 (m, 2 H), 7.17 (m, 2 H), 6.92 (d, 1 H, *J*= 7.9 Hz), 6.85 (m, 1 H), 6.60 (m, 2 H), 3.84 (s, 2 H), 3.70 (s, 3 H), 2.85 (m, 4 H), 2.67 (m, 4 H); MS (ES) m/z: 418, 420 ($M+H^+$).

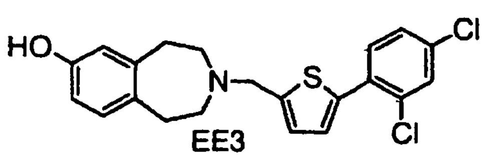

EE3

**3-[5-(2,4-Dichloro-phenyl)-thiophen-2-ylmethyl]-2,3,4,5-tetrahydro -1*H*-benzo[*d*]azepin-7-ol**

Cpd EE3 was prepared according to the same procedure as for cpd X3. Cpd EE3 was obtained (0.22 g, 91%) as a brown solid $^1H$ NMR (300 MHz, CD-$Cl_3$) δ 7.35 (m, 2 H), 7.15 (m, 2 H), 6.83 (m, 2 H), 6.50 (m, 2 H), 3.81 (s, 2 H), 2.80 (m, 4 H), 2.64 (m, 4 H); MS (ES) m/z: 404, 406 ($M+H^+$).

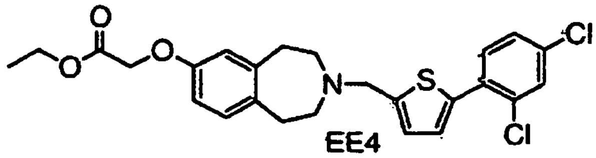

EE4

{3-[5-(2,4-Dichloro-phenyl)-thiophen-2-ylmethyl]-2,3,4,5-tetrahydro -1*H*-benzo[*d*]azepin-7-yloxy}-acetic acid ethyl ester

Cpd EE4 was prepared according to the same procedure as for cpd X4. Cpd EE4 was obtained (0.17 g, 65%) as pale oil: $^1$H NMR (300 MHz, $CDCl_3$) δ 7.39 (s, 1 H), 7.38 (d, *J* = 11 Hz, 1 H), 7.16 (m, 2 H), 6.91 (d, *J* = 8.2 Hz, 1 H), 6.81 (d, *J* = 3.6 Hz, 1 H), 6.61 (d, *J* = 2.7 Hz, 1 H), 6.54 (dd, *J* = 8.2, 2.7 Hz, 1 H), 4.51 (s, 2 H), 4.19 (q, *J* = 7.1 Hz), 3.80 (s, 2H), 2.83 (m, 4 H), 2.63 (m, 4 H); 1.22 (t, *J* = 7.1 Hz, 3 H); MS (ES) m/z: 490, 492 (M+H$^+$).

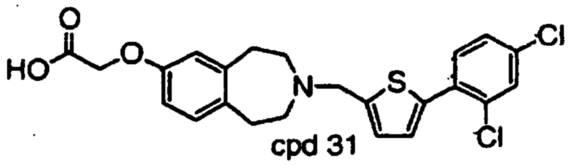

cpd 31

{3-[5-(2,4-Dichloro-phenyl)-thiophen-2-ylmethyl]-2,3,4,5-tetra-hydro-1*H*-benzo[*d*]azepin-7-yloxy}-acetic acid

A solution of cpd EE4 (0.15 g, 0.31 mmol) in THF (2 mL) and methanol (2 mL) was treated with 1 N aqueous NaOH (0.60 mL, 0.60 mmol). After stirring for 1 h, the mixture was concentrated to dryness. The residue was dissolved in $H_2O$, washed with $Et_2O$ twice and then acidified with 1 N HCl. The precipitates were collected and dried to give cpd 31 (0.13 g, 93%). $^1$H NMR (300 MHz, DMSO-$d_6$) δ 7.73 (d, *J*= 2.1 Hz, 1 H), 7.65 (d, *J*= 8.5 Hz, 1 H), 7.47 (dd, *J* = 8.3, 2.2 Hz, 1 H), 7.34 (d, *J* = 3.6 Hz, 1 H), 7.06 (d, *J* = 6.5 Hz, 1 H), 7.00 (d, *J* = 8.3 Hz, 1 H) 6.70 (d, *J* = 2.5 Hz, 1 H), 6.61 (dd, *J* = 8.3, 2.6 Hz, 1 H), 4.59 (s, 2 H), 3.95 (s, 2 H), 2.85 (m, 4 H), 2.68 (m, 4 H); MS (ES) m/z: 462, 464 (M+H$^+$).

<u>Example FF</u>

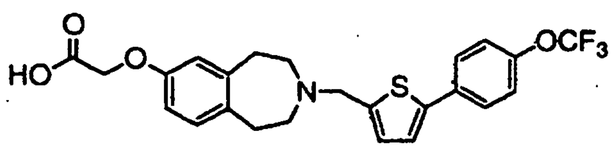

Compound 32: {3-[5-(4-Trifluoromethoxy-phenyl)-thiophen-2-ylmethyl]-2,3,4,5-tetrahydro-1*H*-benzo[*d*]azepin-7-yloxy}-acetic acid

The title compound was made according to Scheme FF.

## Scheme FF

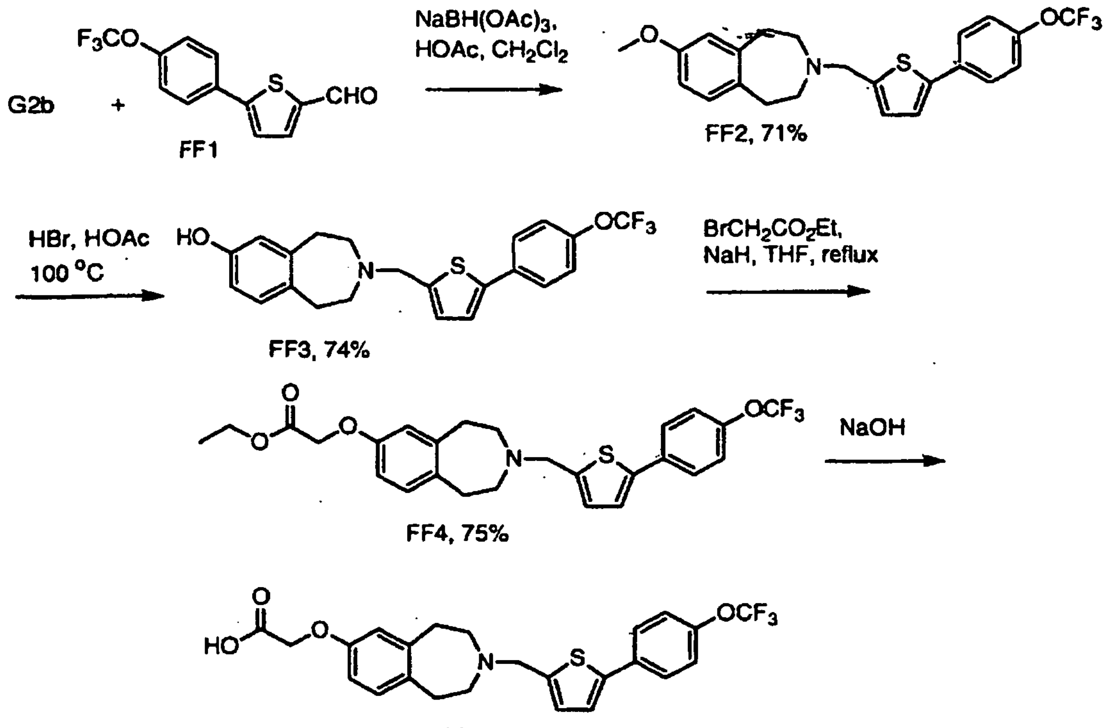


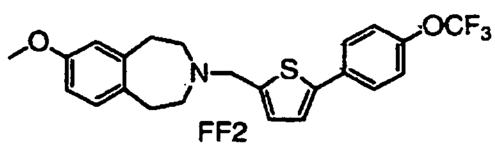

FF2

**7-Methoxy-3-[5-(4-trifluoromethoxy-phenyl)-thiophen-2-ylmethyl]-2,3,4,5-tetrahydro-1*H*-benzo[*d*]azepine**

Cpd FF2 was prepared according to the same procedure as for cpd EE2. Cpd FF2 was obtained (0.24 g, 71%) as a solid: $^1H$ NMR (300 MHz, $CDCl_3$) δ 7.51 (d, *J* = 8.8 Hz, 2 H), 7.16 (m, 2 H), 7.05 (d, *J* = 3.6 Hz, 1 H), 6.92 (d, *J* = 3.6 Hz, 1 H), 6.81 (s, 1 H), 6.56 (m, 2 H), 3.79 (s, 2 H), 3.70 (s, 3 H), 2.83 (m, 4 H), 2.63 (m, 4 H); MS (ES) m/z: 434 ($M+H^+$).

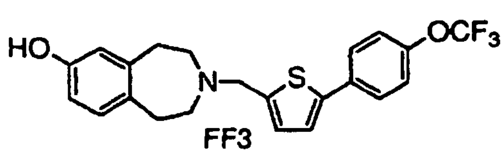

FF3

**3-[5-(4-Trifluoromethoxy-phenyl)-thiophen-2-ylmethyl]-2,3,4,5-tetrahydro-1*H*-benzo[*d*]azepin-7-ol**

Cpd FF3 was prepared according to the same procedure as for cpd EE3. Cpd FF3 was obtained (0.17 g, 74%) as white foam: $^1H$ NMR (300 MHz, $CDCl_3$) δ 7.51 (d, *J* = 8.8 Hz, 2 H), 7.13 (d, *J*= 8.1 Hz, 2H), 7.05 (d, *J* = 3.6 Hz, 1 H), 6.87 (d, *J* = 7.8 Hz, 1 H), 6.80 (d, *J* = 3.3 Hz, 1 H), 6.50 (m, 2 H), 3.79 (s, 2 H), 2.80 (m, 4 H), 2.63 (m, 4 H); MS (ES) m/z: 420 ($M+H^+$).

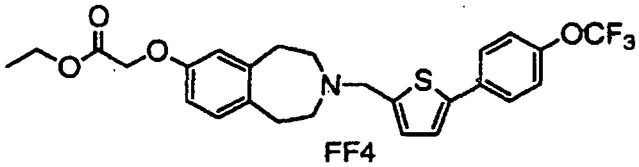

FF4

{3-[5-(4-Trifluoromethoxy-phenyl)-thiophen-2-ylmethyl]-2,3,4,5-tetra -hydro-1*H*-benzo[*d*]azepin-7-yloxy}-acetic acid ethyl ester

Cpd FF4 was prepared according to the same procedure as for cpd EE4. Cpd FF4 was obtained (0.15 g, 75%) as pale oil: $^1$H NMR (300 MHz, $CDCl_3$) δ 7.51 (d, *J* = 8.8 Hz, 2 H), 7.11 (d, *J* = 8.0 Hz, 2 H), 7.04 (d, *J* = 3.6 Hz, 1 H), 6.91 (d, *J* = 8.2 Hz, 1 H), 6.78 (d, *J* = 3.6 Hz, 1 H), 6.61 (d, *J*= 2.7 Hz, 1 H), 6.54 (dd, *J*= 8.2, 2.7 Hz, 1 H), 4.50 (s, 2 H), 4.17 (q, *J*= 7.1 Hz), 3.77 (s, 2 H), 2.81 (m, 4 H), 2.62 (m, 4 H), 1.21 (t, *J* = 7.1 Hz); MS (ES) m/z: 492 ($M+H^+$).

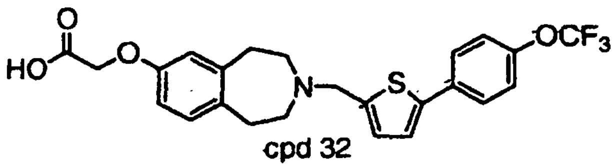

cpd 32

{3-[5-(4-Trifluoromethoxy-phenyl)-thiophen-2-ylmethyl]-2,3,4,5 -tetrahydro-1*H*-benzo[*d*]azepin-7-yloxy}-acetic acid

Cpd 32 was prepared according to the same procedure as for cpd 31. Cpd 32 was obtained (0.08 g, 80%) as a white solid: $^1$H NMR (300 MHz, DMSO-$d_6$) δ 7.74 (d, *J* = 7.7 Hz, 2 H), 7.39 (m, 3 H), 6.98 (d, *J* = 2.7 Hz, 1 H), 6.91 (d, *J* = 8.1 Hz, 1 H), 6.57 (s, 1 H), 6.49 (d, *J*= 8.1 Hz, 1 H), 4.02 (s, 2 H), 3.82 (s, 2 H), 2.77 (m, 4 H), 2.60 (m, 4 H); MS (ES) m/z: 478 ($M+H^+$).

## Example GG

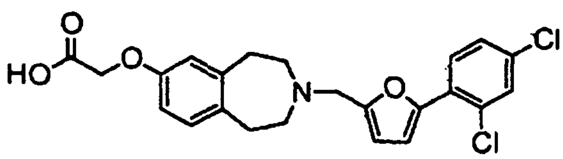

Compound 33: {3-[5-(2,4-Dichloro-phenyl)-furan-2-ylmethyl]-2,3,4,5-tetrahydro-1*H*-benzo[*d*]azepin-7-yloxy}-acetic acid

The title compound was made according to Scheme GG.

## Scheme GG

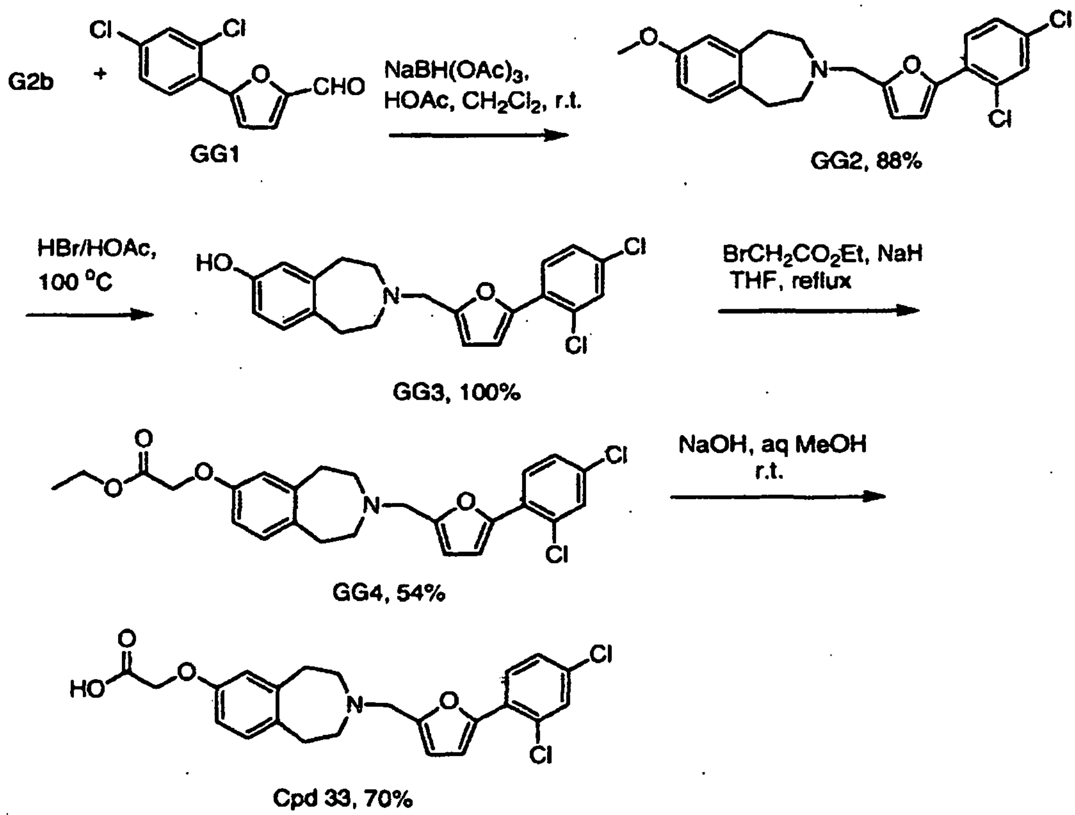


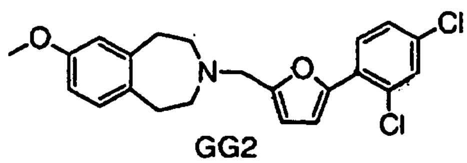

GG2

**3-[5-(2,4-Dichloro-phenyl)-furan-2-ylmethyl]-7-methoxy-2,3,4,5 -tetrahydro-1*H*-benzo[*d*]azepine**

To a mixture of cpd G2b (0.17 g, 0.96 mmol), 5-(2,4-dichlorophenyl)-furan-2-carbaldehyde (0.20 g, 0.83 mmol), $CH_2Cl_2$ (15 mL) and HOAc (0.05 mL, 0.87 mmol) at room temperature was added $Na(OAc)_3BH$ (0.30 g, 1.37 mmol). After the mixture was stirred overnight, it was basified with aqueous $NaHCO_3$ and extracted with $CH_2Cl_2$. The organic extracts were dried ($MgSO_4$) and concentra-ted. The crude product was purified by column chromatography to give cpd GG2 (0.29 g, 88%): $^1H$ NMR (300 MHz, $CDCl_3$) δ 7.69 (d, *J* = 8.6 Hz, 1 H), 7.35 (d, *J* = 2.1 Hz), 7.19n (m, 1H), 6.97 (d, *J* = 3.4 Hz, 1 H), 6.91 (d, *J*= 7.9 Hz, 1 H), 6.56 (m, 2H), 6.26 (d, *J*= 3.4 Hz, 1 H), 3.74 (s, 2H), 3.69 (s, 3H), 2.84 (m, 4H), 2.66 (m, 4H); MS (ES) m/z: 402, 404 ($M+H^+$).

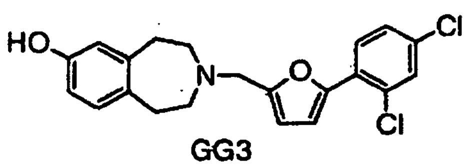

GG3

**3-[5-(2,4-Dichloro-phenyl)-furan-2-ylmethyl]-2,3,4,5-tetrahydro -1*H*-benzo[*d*]azepin-7-ol**

A mixture of GG2 (0.28 g, 0.70 mmol), 48% HBr (0.80 mL, 7.07 mmol) and *n*-$Bu_4NBr$ (30 mg, 0.09 mmol) in

HOAc (0.8 mL) was heated at 100 °C under $N_2$ for 18 h. After cooled to room temperature, the reaction mixture was treated with aqueous $K_2CO_3$ till pH 9. The precipitates were collected, washed with water and dried to give cpd GG3 (0.27 g, 100%) as a beige solid: $^1$H NMR (300 MHz, DMSO-$\delta_6$) δ 9.30 (s, 1H), 7.91 (d, *J* = 8.6 Hz, 1 H), 7.77 (d, *J* = 2.1 Hz, 1 H), 7.60 (dd, *J* = 8.1, 2.2 Hz, 1 H), 7.20 4d, *J* = 3.5 Hz, 1 H), 6.97 (d, *J* = 8.1 Hz, 1 H), 6.88 (d, *J* = 3.1 Hz,1 H), 6.60 (d, *J* = 2.3 Hz, 1 H), 6.56 (dd, *J* = 8.1, 2.3 Hz, 1 H), 4.56 (s, 2 H), 3.10 (m, 4 H), 2.94 (m, 4 H); MS (ES) m/z: 388, 390 (M+H$^+$).

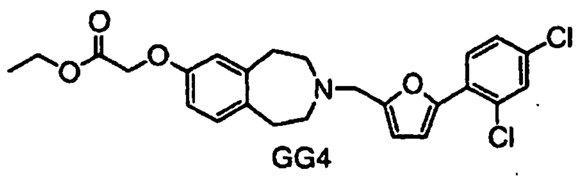

GG4

**{3-[5-(2,4-Dichloro-phenyl)-furan-2-ylmethyl]-2,3,4,5-tetrahydro -1*H*-benzo[*d*]azepin-7-yloxy}-acetic acid ethyl ester**

Cpd GG4 was prepared according to the same procedure as for cpd X4. Cpd GG4 was obtained (65 mg, 54%) as pale oil: $^1$H NMR (300 MHz, $CDCl_3$) δ 7.69 (d, *J* = 8.6 Hz, 1 H), 7.36 (d, *J* = 2.1 Hz, 1 H), 7.24 (m, 1 H), 6.97 (d, *J* = 3.4 Hz, 1 H), 6.91 (d, *J* = 8.2 Hz, 1 H), 6.65 (d, *J* = 5.01 Hz), 6.57 (dd, *J* = 8.2, 2.70 Hz, 1 H), 6.29 (d, *J* = 3.3 Hz, 1 H), 4.50 (s, 2H), 4.19 (q, *J* = 7.1 Hz, 2H), 3.77 (s, 2 H), 2.85 (m, 4 H), 2.68 (m, 4 H); MS (ES) m/z: 474, 476 (M+H$^+$).

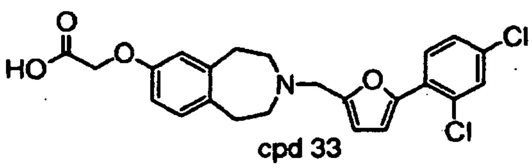

cpd 33

**{3-[5-(2,4-Dichloro-phenyl)-furan-2-ylmethyl]-2,3,4,5-tetrahydro -1*H*-benzo[*d*]azepin-7-yloxy}-acetic acid**

A mixture of cpd GG4 (50 mg, 0.10 mmol) in methanol (2 mL) was treated with 1 N aqueous NaOH (0.30 mL, 0.30 mmol). After stirring for 1 h, the mixture was concentrated to dryness. The residue was dissolved in $H_2O$ and acidified with 1 N HCl. A brown solid was collected and dried to give cpd 33 (32 mg, 70%): $^1$H NMR (300 MHz, DMSO-$d_6$) δ 7.83 (d, *J*= 8.9 Hz, 1 H), 7.72 (m, 1 H), 7.55 (m,1 H), 7.13 (d, *J* = 3.0 Hz, 1 H), 7.03 (d, *J* = 8.9 Hz, 1 H), 6.72 (s, 1H), 6.63 (d, *J* = 6.1 Hz), 4.60 (s, 2 H), 4.61 (s, 2 H), 3.32 (s, 2H), 2.80 (m, 8 H); MS (ES) m/z: 446, 448 (M+H$^+$).

Example HH

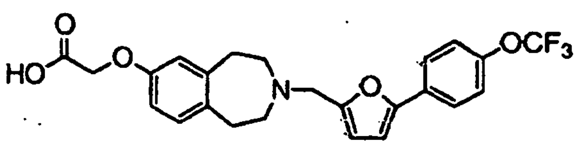

**Compound 34: {3-[5-(4-Trifluoromethoxy-phenyl)-furan-2-ylmethyl]-2,3,4,5-tetrahydro -1*H*-benzo[*d*]azepin-7-yloxy}-acetic acid**

The title compound was made according to Scheme HH.

**Scheme HH**

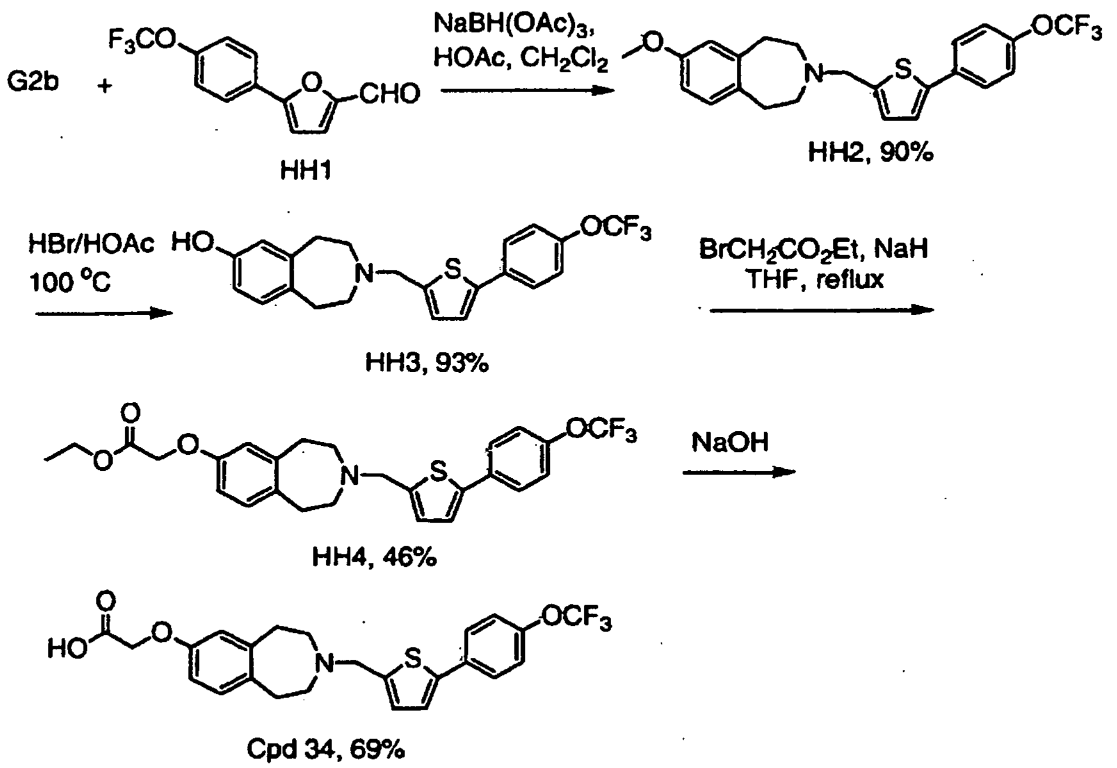


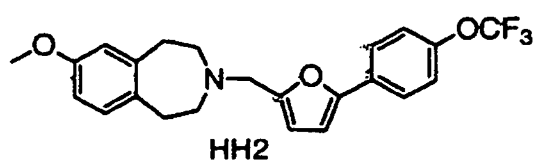

HH2

**7-Methoxy-3-[5-(4-trifluoromethoxy-phenyl)-furan-2-ylmethyl]-2,3,4,5-tetrahydro-1*H*-benzo[*d*]azepine**

Cpd HH2 was prepared according to the same procedure as for cpd GG2. Cpd HH2 was obtained (0.30 g, 90%) as a yellow solid: $^1$H NMR (300 MHz, $CDCl_3$) $\delta$ 7.57 (d, *J* = 8.8 Hz, 2 H), 7.13 (d, *J* = 8.2 Hz, 2 H), 6.91 (d, *J* = 8.2 Hz, 1 H), 6.56 (m, 2 H), 6.49 (d, *J* = 3.39 Hz, 1 H), 3.72 (s, 2 H), 3.69 (s, 3 H), 2.83 (m, 4 H), 2.66 (m, 4 H); MS (ES) m/z: 418 ($M+H^+$).

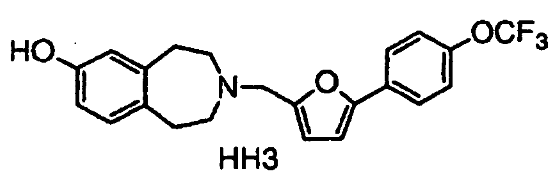

HH3

**3-[5-(4-Trifluoromethoxy-phenyl)-furan-2-ylmethyl]-2,3,4,5-tetrahydro-1*H*-benzo[*d*]azepin-7-ol**

A mixture of cpd HH2 (0.28 g, 0.70 mmol), 48% HBr (0.80 mL, 7.07 mmol), *n*-$Bu_4NBr$ (30 mg, 0.09 mmol) and HOAc (0.8 mL) was heated at 100 °C under $N_2$ for 18 h. After cooled to room temperature, the reaction mixture was

treated with aqueous $K_2CO_3$ till pH 10 and extracted with EtOAc. The organic layer was dried ($MgSO_4$) and concentrated to provide cpd HH3 (0.26 g, 93%) as a brown solid: $^1H$ NMR (300 MHz, DMSO-$\delta_6$) δ 9.04 (s, 1 H), 7.76 (d, *J*= 8.2 Hz, 2 H), 7.39 (d, *J*= 7.6 Hz, 2 H), 6.93 (s, 1 H), 6.85 (d, *J*= 7.6 Hz, 1 H), 6.50 (s,1 H), 6.41 (m, 2 H), 3.71 (s, 2 H), 2.78 (m, 4 H), 2.53 (m, 4 H); MS (ES) m/z: 404 (M+H+).

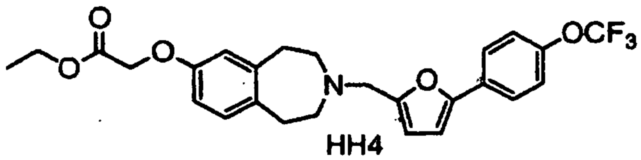

**HH4**

**{3-[5-(4-Trifluoromethoxy-phenyl)-furan-2-ylmethyl]-2,3,4,5-tetrahydro -1*H*-benzo[*d*]azepin-7-yloxy}-acetic acid ethyl ester**

Cpd HH4 was prepared according to the same procedure as for cpd X4. Cpd HH4 was obtained (55 mg, 46%) as light brown oil: $^1H$ NMR (300 MHz, $CDCl_3$) δ 7.57 (d, *J* = 8.7 Hz, 2 H), 7.13 (d, *J* = 8.4 Hz, 2 H), 6.91 (dd, *J* = 8.3, 3.3 Hz, 1 H), 6.62 (m, 1 H), 6.54 (m, 1 H), 6.49 (d, J = 3.0 Hz, 1 H), 6.21 (d, *J* = 3.0 Hz, 1 H), 4.50 (d, J = 5.1 Hz, 2 H), 4.11 (q, *J* = 7.2 Hz, 2 H), 3.72 (s, 2 H), 2.83 (m, 4 H), 2.68 (m, 4 H); MS (ES) m/z: 490 (M+H+).

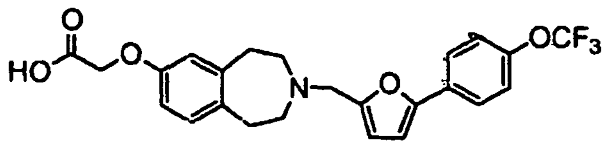

**cpd 34**

**{3-[5-(4-Trifluoromethoxy-phenyl)-furan-2-ylmethyl]-2,3,4,5-tetrahydro -1*H*-benzo[*d*]azepin-7-yloxy}-acetic acid**

Cpd 34 was prepared according to the same procedure as for cpd 33. Cpd 34 was obtained (27 mg, 69%) as a light brown solid: $^1H$ NMR (300 MHz, DMSO-$\delta_6$) δ 7.78 (d, *J*= 6.1 Hz, 2 H), 7.41 (d, *J* = 6.1 Hz, 2 H), 7.00 (d, *J*= 6.0 Hz, 1 H), 6.98 (d, *J*= 3.0 Hz, 1 H), 6.89 (s, 1H), 6.59 (m, 1 H), 6.47 (s, 1 H), 4.62 (s, 2 H), 3.84 (s, 2 H), 3.33 (m, 4 H), 2.68 (m, 4 H); MS (ES) m/z: 462 (M+H+).

Example II

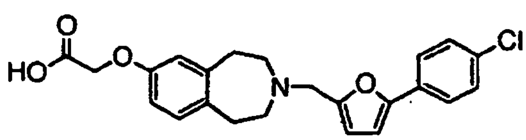

**Compound 35: {3-[5-(4-Chloro-phenyl)-furan-2-ylmethyl]-2,3,4,5-tetrahydro-1*H*-benzo[*d*]azepin-7-yloxy}-acetic acid**

The title compound was made according to Scheme II.

**Scheme II**

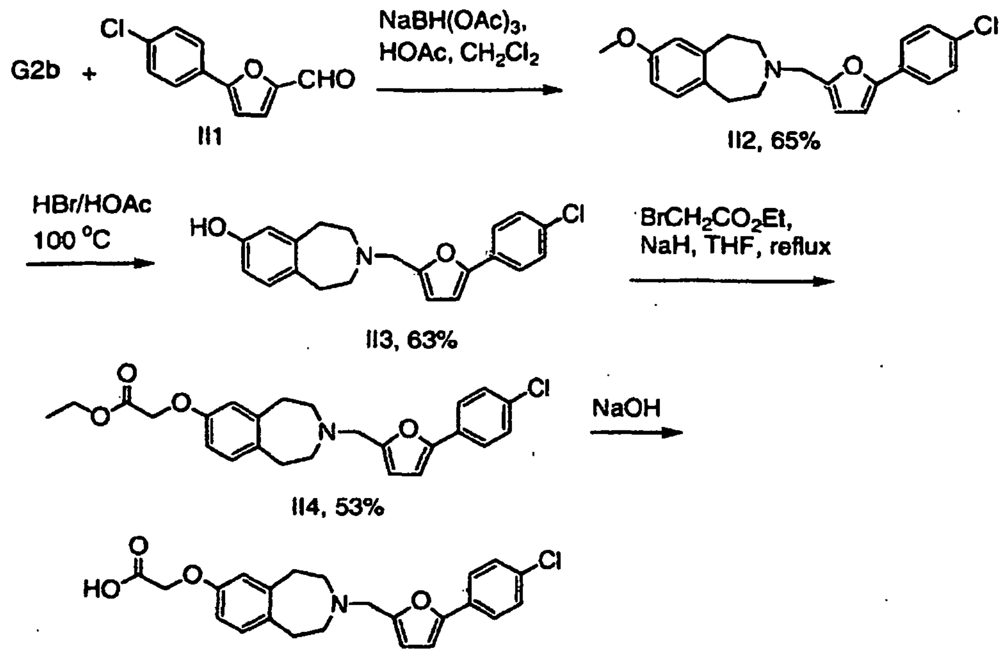


Cpd 35, 95%

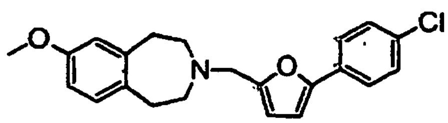

II2

**7-Methoxy-3-[5-(4-chlorophenyl)-furan-2-ylmethyl]-2,3,4,5-tetrahydro -1*H*-benzo[*d*]azepine**

Cpd II2 was prepared according to the same procedure as for cpd X2. Cpd II2 was obtained (0.26 g, 65%) as a brown oil: $^1$H NMR (300 MHz, $CDCl_3$) δ 7.59 (d, *J*= 8.9 Hz, 2 H), 7.35 (d, *J*= 8.9 Hz, 2 H), 7.02 (d, *J*= 9.1 Hz, 1 H), 6.67 (m, 1 H), 6.58 (m, 1 H), 6.29 (d, *J* = 2.1 Hz, 1 H), 3.81 (s, 2 H); 3.78 (s, 3 H), 2.93 (m, 4 H), 2.75 (m, 4 H); MS (ES) m/z: 366, 368 ($M+H^+$).

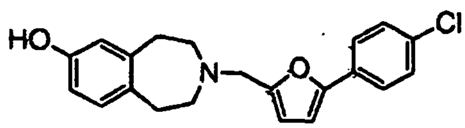

II3

**3-[5-(4-Chlorophenyl)-furan-2-ylmethyl]-2,3,4,5-tetrahydro -1*H*-benzo[*d*]azepin-7-ol**

A mixture of II2 (0.25 g, 0.68 mmol), 48% HBr (0.80 mL, 7.07 mmol) and *n*-$Bu_4NBr$ (26 mg, 0.08 mmol) in HOAc (0.8 mL) was heated at 80°C under $N_2$ overnight. After cooled to room temperature, the reaction mixture was treated with aqueous $K_2CO_3$ and a brown solid was collected and dried (0.11 g, 46%). The aqueous layer was then extracted with $CH_2Cl_2$. The $CH_2Cl_2$ solution was concentrated and purified by column chromatography to give a brown solid (0.04 g) as second batch of the product. Overall, cpd II3 was obtained (0.15 g) in 63% yield: $^1$H NMR (300 MHz, $CD_3OD$) δ 7.58 (d, *J* = 8.8 Hz, 2 H), 7.34 (d, *J* = 9.0 Hz, 1 H), 6.90 (d, *J* = 6.1 Hz, 1 H), 6.57 (m, 3 H), 6.31 (d, *J* = 2.5 Hz, 1 H), 3.78 (s, 2 H), 2.87 (m, 4 H), 2.73 (m, 4 H); MS (ES) m/z: 354, 356 ($M+H^+$).

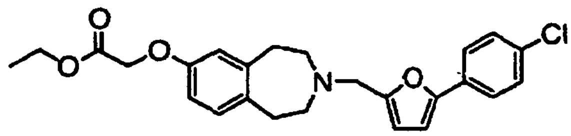

II4

**{3-[5-(4-Chlorophenyl)-furan-2-ylmethyl]-2,3,4,5-tetrahydro-1*H* -benzo[*d*]azepin-7-yloxy}-acetic acid ethyl ester**

Cpd II4 was prepared according to the same procedure as for cpd X4. Cpd II4 was obtained (96 mg, 53 %) as pale oil: $^1H$ NMR (300 MHz, $CDCl_3$) δ 7.49 (d, *J* = 8.4 Hz, 2 H), 7.24 (d, *J* = 8.4 Hz, 2 H), 6.91 (d, *J* = 8.0 Hz, 1 H), 6.61 (d, *J*= 2.4 Hz, 1 H), 6.50 (dd, *J* = 8.0, 2.5 Hz, 1 H), 6.49 (d, *J*= 2.4 Hz, 1 H), 6.21 (s, 1 H), 4.50 (s, 2 H), 4.20 (q, *J* = 7.1 Hz), 3.70 (s, 2 H), 2.82 (m, 4 H), 2.64 (m, 4 H); MS (ES) m/z: 440, 442 ($M+H^+$).

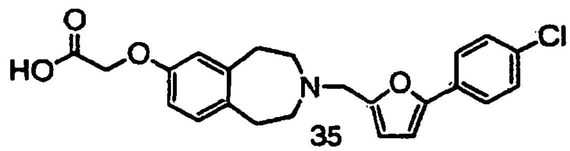

35

**{3-[5-(4-Chloro-phenyl)-furan-2-ylmethyl]-2,3,4,5-tetrahydro-1*H* -benzo[*d*]azepin-7-yloxy}-acetic acid**

Cpd 35 was prepared according to the same procedure as for cpd 33. Cpd 35 was obtained (70 mg, 95 %) as a light yellow solid: $^1H$ NMR (400 MHz, DMSO-$\delta_6$) δ 7.74 (d, *J* = 8.5 Hz, 2 H), 7.51 (d, *J* = 8.5 Hz, 2 H), 7.06 (d, *J* = 8.2 Hz, 1 H), 7.02 (d, *J* = 2.8 Hz, 1 H), 6.75 (s, 1 H), 6.66 (m, 2 H), 4.61 (s, 2 H), 4.21 (bs, 2 H), 3.00 (m, 8 H); MS (ES) m/z: 412, 414 ($M+H^+$).

Example JJ

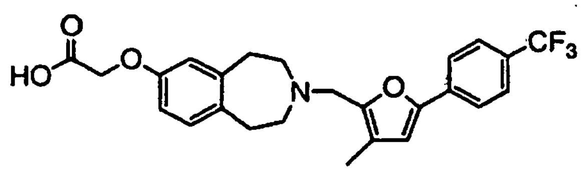

**Compound 36: {3-[3-Methyl-5-(4-trifluoromethyl-phenyl)-furan-2-ylmethyl]-2,3,4,5-tetrahyd-1*H* -benzo[*d*]azepin-7-yloxy}-acetic acid**

The title compound was made according to Schemes JJ1 & JJ2.

**Scheme JJ1**

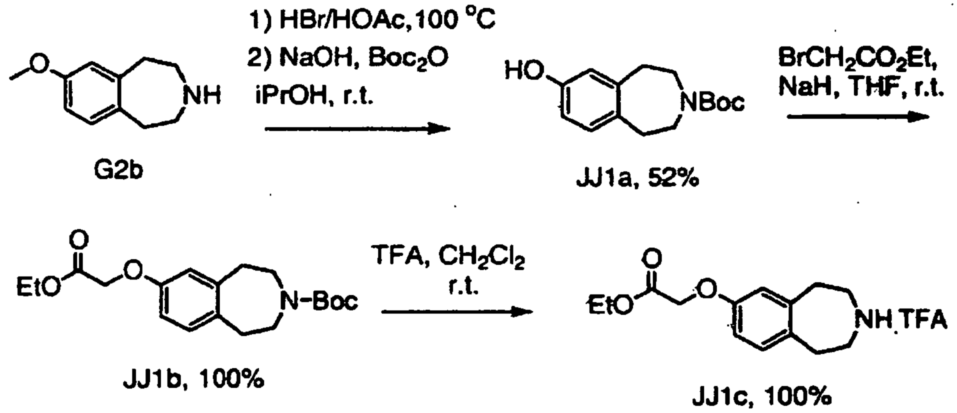


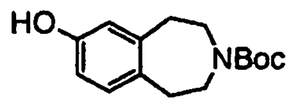

**JJ1a**

**7-Hydroxy-1,2,4,5-tetrahydro-benzo[*d*]azepine-3-carboxylic acid *tert*-butyl ester**

A mixture of G2b (0.26 g, 1.47 mmol), 48% HBr (1.7 mL, 15 mmol) and *n*-$Bu_4NBr$ (50 mg, 0.16 mmol) in HOAc (1.7 mL) was heated at 100 ˚C under $N_2$ overnight. After cooled to room temperature, the reaction mixture was treated with solid NaOH to pH 9-10. To the resulting mixture was added $H_2O$ (5 mL), iso-propanol (5 mL), followed by di-t-butyl dicarbonate (0.6 g, 2.8 mmol). The mixture was stirred at room temperature overnight and then extracted with EtOAc. The organic extracts were concentrated and the residue purified by column chromato-gramphy to give Cpd JJ1a as a white solid (0.20 g, 52%): $^1$H NMR (300 MHz, $CDCl_3$) δ 6.90 (d, *J* = 6.0 Hz, 1 H), 6.54 (m, 2 H), 4.52 (bs, 1 H), 3.46 (m, 4 H), 2.75 (m, 4 H); MS (ES) m/z: 286 (M+Na).

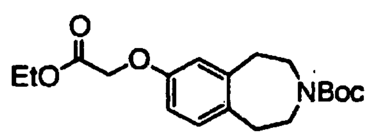

**JJ1b**

**7-Ethoxycarbonylmethoxy-1,2,4,5-tetrahydro-benzo[*d*]azepine-3-c arboxylic acid *tert*-butyl ester**

Cpd JJ1b was prepared according to the same procedure as for cpd EE4. Cpd JJ1b was obtained (0.26 g, 100%) as pale oil: $^1$H NMR (300 MHz, $CDCl_3$) δ 6.95 (d, *J* = 8.9 Hz, 1 H), 6.65 (d, *J* = 2.0 Hz, 1 H), 6.56 (m, 1 H), 6.50 (dd, *J* = 8.0, 2.5 Hz, 1 H), 4.52 (s, 2 H), 4.19 (q, *J* = 7.1 Hz, 2 H), 3.47 (m, 4 H), 2.76 (m, 4 H), 1.23 (t, *J*= 7.1 Hz, 3 H); MS (ES) m/z: 372 (M+Na).

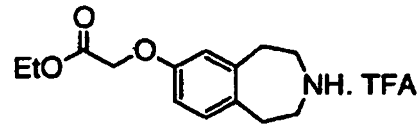

JJ1c

**[3-(2,2,2-Trifluoro-acetyl)-2,3,4,5-tetrahydro-1*H*-benzo[*d*]azepin-7-yloxy]-acetic acid ethyl ester trifluoroacetate**

A mixture of cpd JJ1b (0.26 g, 0.76 mmol) and TFA (1.0 mL, 1.3 mmol) in $CH_2Cl_2$ (1 mL) was stirred at room temperature under $N_2$ for 1 h. The reaction mixture was concentrated and the resulting residue was washed with $Et_2O$, concentrated to give cpd JJ1c (0.17 g, 100% crude yield). $^1H$ NMR (300 MHz, $CDCl_3$) δ 6.99 (m, 1 H), 6.62 (m, 2 H), 6.56 (m, 1 H), 5.56 (bs, 1 H), 4.61 (s, 2 H), 4.19 (m, 2 H), 3.18 (m, 4 H), 3.04 (m, 4 H), 1.19 (m, 3H); MS (ES) m/z: 250 ($M+H^+$).

**Scheme JJ2**

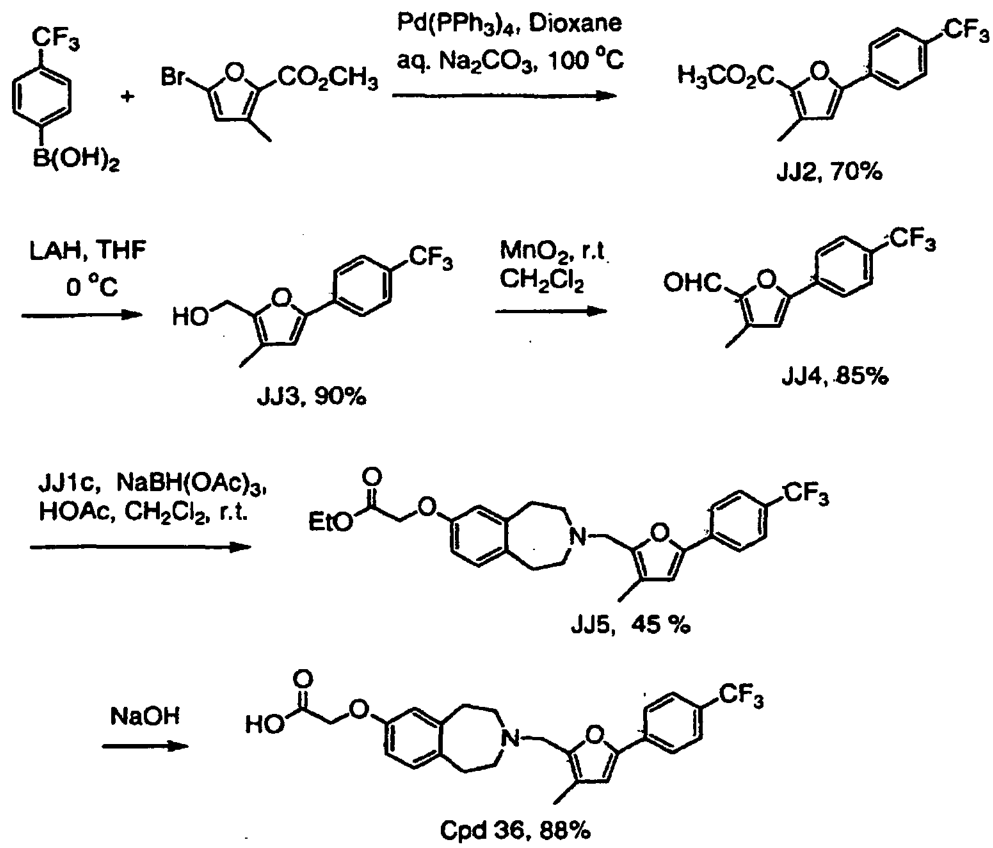


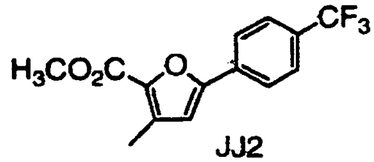

JJ2

**3-Methyl-5-(4-trifluoromethyl-phenyl)-furan-2-carboxylic acid methyl ester**

Cpd JJ2 was prepared using a similar procedure as for cpd G1a. Cpd JJ2 was obtained (1.17 g, 70%) as a white solid: $^1H$ NMR (300 MHz, $CDCl_3$) δ 7.78 (d, *J* = 8.2 Hz, 2 H), 7.58 (d, *J* = 8.2 Hz, 2 H), 6.65 (s, 1 H), 3.86 (s, 3 H), 2.34 (s, 3 H); MS (ES) m/z: 285 (M+H$^+$).

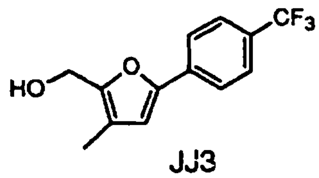

JJ3

[3-Methyl-5-(4-trifluoromethyl-phenyl)-furan-2-yl]-methanol

To a solution of cpd JJ2 (1.17 g, 4.11 mmol) in THF (25 mL) at 0 ˚C under $N_2$ was added $LiAlH_4$ (1.0 M, 2.7 mL). After stirring for 1 h, the mixture was quenched with aqueous $NH_4Cl$ solution and then extracted with EtOAc. The organic phase was dried ($MgSO_4$) and concentrated to provide cpd JJ3 (0.95 g, 91%) as a white solid: $^1H$ NMR (300 MHz, $CDCl_3$) δ 7.65 (d, *J* = 8.3 Hz, 2 H), 7.53 (d, *J*= 8.3 Hz, 2 H), 6.54 (s, 1 H), 4.58 (s, 2 H), 2.04 (s, 3 H); MS (ES) m/z: 239 (M-OH-).

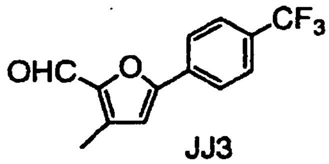

JJ3

3-Methyl-5-(4-trifluoromethyl-phenyl)-furan-2-carbaldehyde

A mixture of cpd JJ3 (0.13 g, 0.51 mmol) and $MnO_2$ (0.87 g, 10.0 mmol) in $CH_2Cl_2$ (16 mL) was stirred at room temperature overnight. $MnO_2$ was removed by filtering the mixture through Celite. The filtrate was concentrated and purified by column chromatography to give cpd JJ4 (0.11g, 85%) as a white solid. $^1H$ NMR (300 MHz, $CDCl_3$) δ 9.76 (s, 1 H), 7.82 (d, *J* = 8.2 Hz, 2 H), 7.62 (d, *J* = 8.2 Hz, 2 H), 6.72 (s, 1 H), 2.38 (s, 3 H).

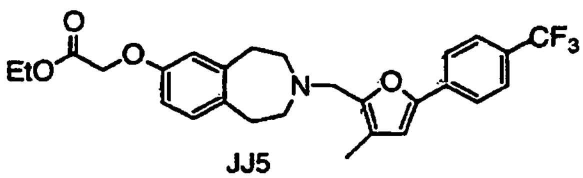

JJ5

{3-[3-Methyl-5-(4-trifluoromethyl-phenyl)-furan-2-ylmethyl]-2,3,4,5-tetrahydro-1*H* -benzo[*d*]azepin-7-yloxy}-acetic acid ethyl ester

Cpd JJ5 was prepared according to the same procedure as for cpd X2. Cpd JJ5 was obtained (40 mg, 45%) as a brown oil: $^1H$ NMR .(300 MHz, $CDCl_3$) δ 7.71 (d, *J*=8.2 Hz, 2 H), 7.61 (d, *J*= 8.2 Hz, 2 H), 7.00 (d, *J*= 8.2 Hz), 6.71 (d, *J*= 2.6 Hz, 1 H), 6.63 (dd, *J* = 8.2, 2.7 Hz, 1 H), 6.60 (s, 1 H), 4.59 (s, 2 H), 4.27 (q, *J* = 7.1 Hz, 2 H), 3.80 (bs, 2 H), 2.94 (m, 4 H), 2.75 (m, 4 H), 2.09 (3 H), 1.30 (t, *J* = 7.1 Hz, 3 H); MS (ES) m/z: 488 (M+H$^+$).

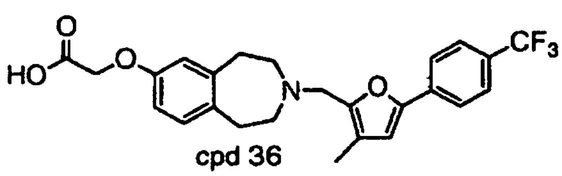

cpd 36

{3-[3-Methyl-5-(4-trifluoromethyl-phenyl)-furan-2-ylmethyl]-2,3,4,5-tetrahyd-1*H* -benzo[*d*]azepin-7-yloxy}-acetic acid

Cpd 36 was prepared according to the same procedure as for cpd 31. Cpd 36 was obtained (25 mg, 68%) as a light brown solid: $^1$H NMR (300 MHz, DMSO-$\delta_6$) $\delta$ 7.83 (d, *J* = 8.2 Hz, 2 H), 7.75 (d, *J*= 8.2 Hz, 2 H), 7.00 (m, 1 H), 6.69 (d, *J* = 2.5 Hz, 1 H), 6.60 (dd, *J* = 8.2, 2.6 Hz, 1 H), 4.58 (s, 2 H), 3.75 (s, 2 H), 2.82 (m, 4 H), 2.64 (m, 4 H), 2.05 (s, 3 H); MS (ES) m/z: 460 (M+H$^+$).

Example KK

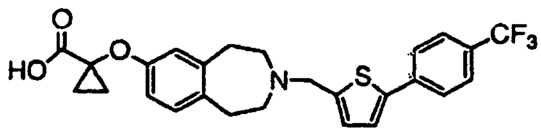

**Compound 37: 1-{3-[5-(4-Trifluoromethyl-phenyl)-thiophen-2-ylmethyl]-2,3,4,5-tetrahydro-1*H*-benzo[*d*]azepin-7-yloxy}-cyclopropanecarboxylic acid**

The title compound was made according to Scheme KK.

## Scheme KK

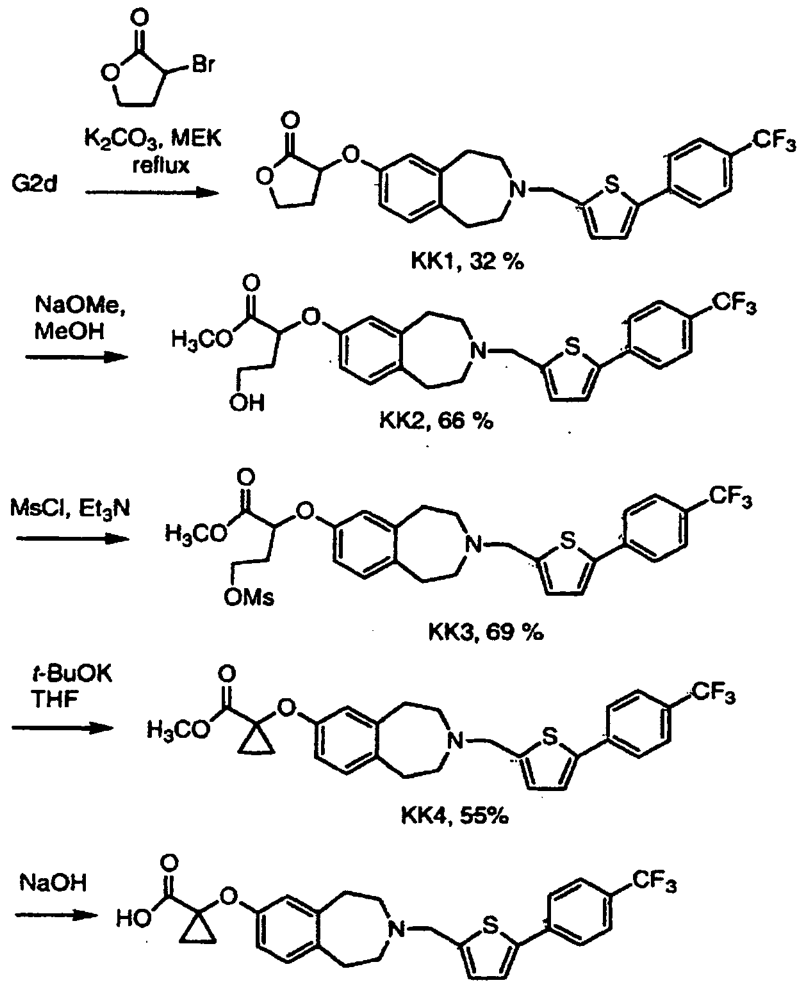


Cpd 37, 33%

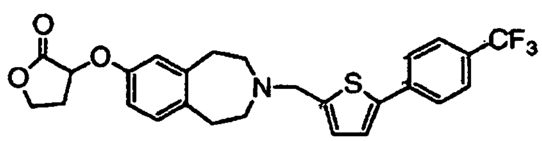

**KK1**

**3-{3-[5-(4-Trifluoromethyl-phenyl)-thiophen-2-ylmethyl]-2,3,4,5-tetrahydro -1*H*-benzo[*d*]azepin-7-yloxy}-dihydro-furan-2-one**

A mixture of 3-bromo-dihydro-furan-2-one (0.62 mL, 6.45 mmol), cpd G2d (1.0 g, 2.48 mmol) and potassium carbonate (1.49 g, 9.33 mmol) in 2-butan-one (25 mL) was heated at reflux for 6 h. After removing solvents, the residue was partitioned between $H_2O$ and EtOAc and the aqueous layer was extracted with EtOAc. The combined organic layers were dried ($MgSO_4$) and purified by column chromato-graphy to give cpd KK1 (0.39 g, 32%) as a brown oil. 0.45 g of cpd G2d was recovered. $^1H$ NMR (300 MHz, $CDCl_3$) δ 7.70 (d, *J* = 8.4 Hz, 2 H), 7.62 (d, *J* = 8.4 Hz, 2 H), 7.27 (m, 2 H), 7.03 (d, *J* = 8.0 Hz, 1 H), 6.92 (m, 1 H), 6.79 (m, 2 H), 4.92 (t, *J*= 7.8 Hz, 1 H), 4.53 (m, 1 H), 4.37 (m, 1 H), 3.89 (s, 2 H), 2.92 (m, 4 H), 2.72 (m, 4 H), 2.66 (m, 1 H), 2.46 (m, 1 H); MS (ES) m/z: 404 ($M+H^+$).

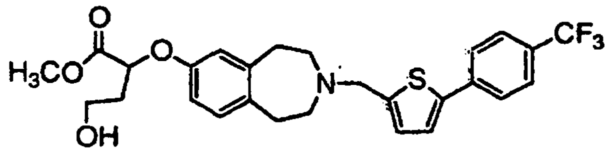

**4-Hydroxy-2-{3-[5-(4-trifluoromethyl-phenyl)-thiophen-2-ylmethyl]-2,3,4,5 -tetrahydro-1*H*-benzo[*d*]azepin-7-yloxy}-butyric acid methyl ester**

To a solution of cpd KK1 (0.35 g, 0.72 mmol) in MeOH (7.2 mL) at room temperature was added NaOMe (0.5 M in MeOH, 1.44 mL, 7.2 mmol). The resulting mixture was stirred at room temperature for 30 min and quenched with aqueous $NH_4Cl$. The mixture was extracted with EtOAc. The organic extracts were dried ($MgSO_4$) and concentrated. The crude product was purified by column chromatography to give cpd KK2 (0.25 g, 66%) as oil: $^1H$ NMR (300 MHz, $CDCl_3$) δ 7.60 (d, *J* = 8.3 Hz, 2 H), 7.53 (d, *J* = 8.3 Hz, 2 H), 7.16 (d, *J*= 3.6 Hz, 1 H), 6.90 (d, *J*= 8.2 Hz, 1 H), 6.83 (m, 1 H), 6.61 (s, *J*= 2.6 Hz, 1 H), 6.52 (dd, *J*= 8.2, 2.7 Hz, 1 H), 4.77 (t, *J*=6.2 Hz, 1 H), 3.79 (m, 4 H), 3.69 (s, 3 H), 2.82 (m, 4 H), 2.63 (m, 4 H), 2.11 (m, 1 H), 1.63 (m, 1 H); MS (ES) m/z: 520 ($M+H^+$).

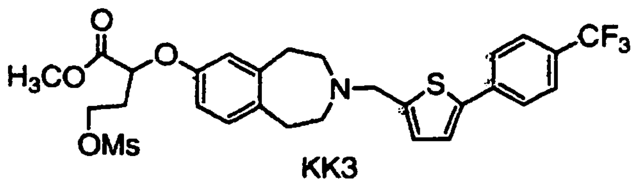

KK3

**4-Methanesulfonyloxy-2-{3-[5-(4-trifluoromethyl-phenyl)-thiophen-2-ylmethyl] -2,3,4,5-tetrahydro-1*H*-benzo[*d*]azepin-7-yloxy}-butyric acid methyl ester**

To a solution of cpd KK2 (0.23 g, 0.44 mmol), methanesulfonyl chloride (38 μL, 0.49 mmol) in $CH_2Cl_2$ (13 mL) at room temperature was added $Et_3N$ (68 μL, 0.49 mmol) and the resulting mixture was stirred for 1 h. It was partitioned between $H_2O$ and $CH_2Cl_2$ and the aqueous layer was extracted with $CH_2Cl_2$. The combined organic layers were dried ($MgSO_4$) and concentrated. The crude product was purified by column chromatography to give cpd KK3 (0.18 g, 69%) as pale oil: $^1H$ NMR (300 MHz, $CDCl_3$) δ 7.60 (d, *J* = 8.3 Hz, 2 H), 7.53 (d, *J* = 8.3 Hz, 2 H), 7.17 (m, 2 H), 6.90 (d, *J* = 8.2 Hz, 1 H), 6.83 (m, 1 H), 6.61 (s, *J* = 2.6 Hz, H), 6.52 (dd, *J* = 8.2, 2.7 Hz, 1 H), 4.77 (t, *J* = 6.2 Hz, 1 H), 3.79 (m, 4 H), 3.69 (s, 3 H), 2.82 (m, 4 H), 2.63 (m, 4 H), 2.11 (m, 1 H), 1.63 (m, 1 H); MS (ES) m/z: 520 ($M+H^+$).

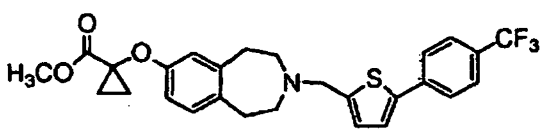

KK4

**1-{3-[5-(4-Trifluoromethyl-phenyl)-thiophen-2-ylmethyl]-2,3,4,5-tetrahydro-1*H* -benzo[*d*]azepin-7-yloxy)-cyclopropanecarboxylic acid methyl ester**

To a solution of cpd KK3 (0.18 g, 0.30 mmol) in THF (10 mL) at 0 ˚C under $N_2$ was added *t*-BuOK (1 M in THF, 0.30 mL) in a dropwise fashion. The resulting mixture was stirred at 0 ˚C for 1 h, acidified with 1 N HCl till pH 4 and extracted with EtOAc. The combined organic layers were dried ($MgSO_4$) and concentrated. The crude product was purified by column chromatography to give cpd KK4 (62 mg, 55%) as pale oil. $^1H$ NMR (300 MHz, $CDCl_3$) δ 7.70 (d, *J* = 8.4 Hz, 2 H), 7.62 (d, *J* = 8.4 Hz, 2 H), 7.27 (m, 2 H), 6.99 (d, *J*= 7.8 Hz, 1 H), 6.65 (m, 2 H), 3.89 (s, 2 H), 3.74 (s, 3 H), 2.91 (m, 4 H), 2.73 (m, 4 H), 1.61 (m, 2 H), 1.32 (m, 2 H); MS (ES) m/z: 502 ($M+H^+$).

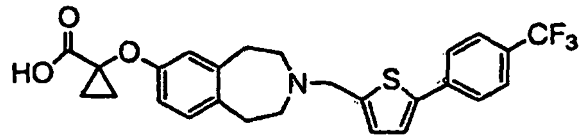

cpd 37

**1-{3-[5-(4-Trifluoromethyl-phenyl)-thiophen-2-ylmethyl]-2,3,4,5-tetrahydro-1*H*-benzo[*d*]azepin-7-yloxy}-cyclopropanecarboxylic acid**

A mixture of cpd KK4 (38 mg, 0.76 mmol) and 1 N NaOH (4.6 mL) in MeOH (1 mL) was stirred at room temperature overnight. The mixture was concentrated, the residue treated with tartaric acid to pH 3 and extracted with EtOAc. The organic layer was dried ($MgSO_4$) and concentrated. The residue was purified by column chromatography to give cpd 37 (12 mg, 33%) as beige solid. $^1H$ NMR (300 MHz, DMSO-$\delta_6$) δ 7.79 (d, *J*= 7.9 Hz, 2 H), 7.68 (d, *J* = 7.9 Hz, 2 H), 7.48 (m, 1 H), 6.96 (m, 2 H), 6.56 (m, 2 H), 3.78 (bs, 2 H), 2.75 (m, 4 H), 2.56 (m, 4 H), 1.41 (m, 2 H), 1.12 (m, 2 H); MS (ES) m/z: 488 ($M+H^+$).

Example LL

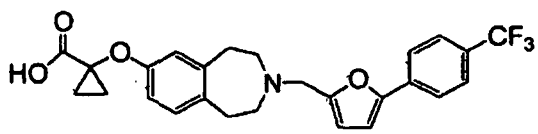

**Compound 38: 1-{3-[5-(4-Trifluoromethyl-phenyl)-furan-2-ylmethyl]-2,3,4,5-tetrahydro-1*H*-benzo[*d*]azepin-7-yloxy}-cyclopropanecarboxylic acid**

The title compound was made according to Schemes LL1 & LL2.

## Scheme LL1

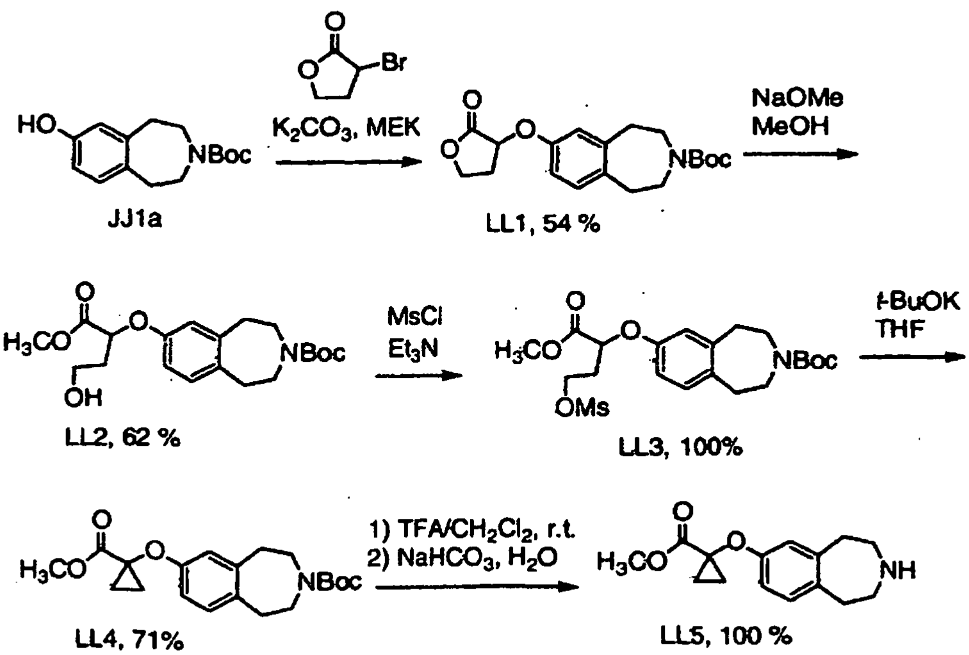

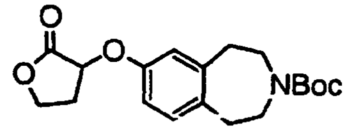

LL1

**7-(2-Oxo-tetrahydro-furan-3-yloxy)-1,2,4,5-tetrahydro -benzo[*d*]azepine-3-carboxylic acid *tert*-butyl ester**

Cpd LL1 was prepared according to the same procedure as for cpd KK1. Cpd LL1 was obtained (0.65 g, 54%) as a white solid: $^1$H NMR (300 MHz, $CDCl_3$) δ 7.06 (d, *J* = 8.0 Hz, 1 H), 6.82 (m, 2 H), 4.93 (t, *J* = 7.7 Hz, 1 H), 4.53 (m, 1 H), 4.36 (m, 1 H), 3.55 (m, 4 H), 2.87 (m, 4 H), 2.73 (m, 1 H), 2.49 (m, 1 H), 1.50 (s, 9 H); MS (ES) m/z: 370 (M+Na).

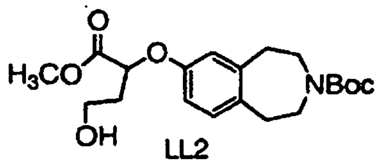

LL2

**7-(3-Hydroxy-1-methoxycarbonyl-propoxy)-1,2,4,5-tetrahydro -benzo[*d*]azepine-3-carboxylic acid *tert*-butyl ester**

Cpd LL2 was prepared according to the same procedure as for cpd KK2. Cpd LL2 was obtained (0.74 g, 62%): $^1$H NMR (300 MHz, $CDCl_3$) δ 7.01 (d, *J* = 8.3 Hz, 1 H), 6.71 (d, *J* = 2.5 Hz, 1 H), 6.63 (dd, *J* = 8.3, 2.5 Hz, 1 H), 4.87 (t, *J* = 6.1 Hz, 1 H), 3.89 (t, *J* = 6.1 Hz, 2 H), 3.78 (s, 3 H), 3.54 (m, 4 H), 2.85 (m, 4 H), 2.21 (q, *J*= 5.9 Hz, 2 H), 1.50 (s, 9 H); MS (ES) m/z: 402 (M+Na).

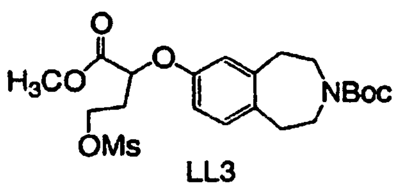

LL3

**7-(3-Methanesulfonyloxy-1-methoxycarbonyl-propoxy)-1,2,4,5 -tetrahydro-benzo[*d*]azepine-3-carboxylicacid *tert*-butyl ester**

Cpd LL3 was prepared according to the same procedure as for cpd KK3. Cpd LL3 was obtained (0.86 g, 100%) as colorless oil: $^1$H NMR (300 MHz, $CDCl_3$) δ δ 7.03 (d, *J* = 8.3 Hz, 1 H), 6.71 (d, *J* = 2.7 Hz, 1 H), 6.63 (dd, *J* = 8.3, 2.7 Hz, 1 H), 4.81 (m, 1 H), 4.47 (t, *J* = 6.0 Hz, 2 H), 3.79 (s, 3 H), 3.53 (m, 4 H), 2.99 (s, 3 H), 2.85 (m, 4 H), 2.39 (m, 2 H), 1.50 (s, 9 H); MS (ES) m/z: 480 (M+Na).

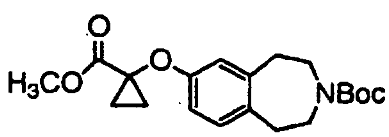

LL4

**7-(1-Methoxycarbonyl-cyclopropoxy)-1,2,4,5-tetrahydro-benzo[*d*]azepine -3-carboxylic acid *tert*-butyl ester**

Cpd LL4 was prepared according to the same procedure as for cpd KK4. Cpd LL4 was obtained (0.49 g, 71%) as a white solid: $^1$H NMR (300 MHz, $CDCl_3$) δ 7.05 (d, *J* = 8.0 Hz, 1 H), 6.80 (m, 2 H), 4.93 (t, *J* = 7.8 Hz, 1 H), 4.53 (m, 1 H), 4.36 (q, *J* = 7.7 Hz, 2 H), 3.54 (m, 4 H), 2.86 (m, 4 H), 2.71 (m, 1 H), 2.46 (m, 1 H), 1.50 (s, 9 H); MS (ES) m/z: 384 (M+Na).

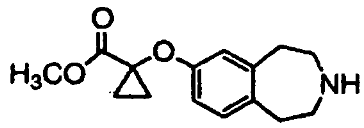

LL5

**1-(2,3,4,5-Tetrahydro-1*H*-benzo[*d*]azepin-7-yloxy)-cyclopropane-carboxylic acid methyl ester**

A mixture of cpd LL4 (0.50 g, 1.38 mmol) and TFA (0.5 mL) in $CH_2Cl_2$ (5 mL) was stirred at room temperature for 30 min. After the mixture was concen-trated, the residue was treated with aqueous $NaHCO_3$ and extracted with EtOAc. The organic layer was dried ($MSO_4$) and concentrated to dryness to give cpd LL5 (0.36 g, 100%) as a pale solid: $^1$H NMR (300 MHz, $CDCl_3$) δ 7.05 (d, *J* = 8.0 Hz, 1 H), 6.80 (m, 2 H), 4.93 (t, *J* = 7.8 Hz, 1 H), 4.53 (m, 1 H), 4.36 (q, *J*= 7.7 Hz, 2 H), 3.54 (m, 4 H), 2.86 (m, 4 H), 2.71 (m, 1 H), 2.46 (m, 1 H); MS (ES) m/z: 262 (M+H$^+$).

**Scheme LL2**

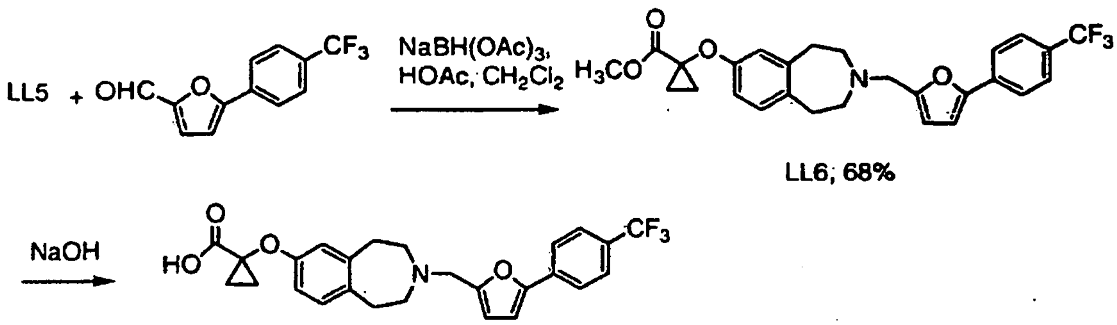


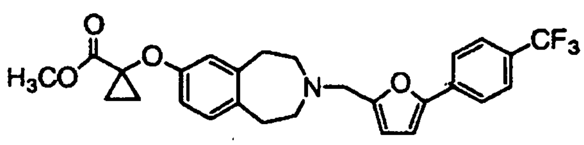

LL6

**1-{3-[5-(4-Trifluoromethyl-phenyl)-furan-2-ylmethyl]-2,3,4,5-tetrahydro-1*H*-benzo[*d*]azepin-7-yloxy}-cyclopropanecarboxylic acid methyl ester**

Cpd LL6 was prepared according to the same procedure as for cpd X2. Cpd MM1 was obtained (25 mg, 68%) as a light brown stolid: $^1$H NMR (300 MHz, $CDCl_3$) δ 7.75 (d, *J* = 8.2 Hz, 2 H), 7.62 (d, *J* = 8.2 Hz, 2 H), 6.98 (d, *J*= 7.9 Hz, 1 H), 6.71 (d, *J*= 3.3 Hz, 1 H), 6.64 (m, 2 H), 6.33 (d, *J*= 3.3 Hz), 3.83 (s, 2 H), 3.73 (s, 3 H), 2.92 (m, 4 H), 2.75 (m, 4 H), 1.60 (m, 2 H), 1.31 (m, 2 H); MS (ES) m/z: 486 (M+H$^+$).

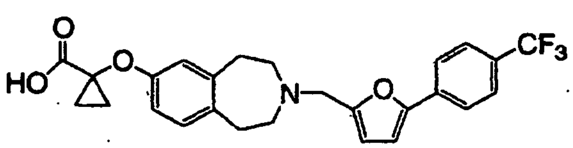

cpd 38

**1-{3-[5-(4-Trifluoromethyl-phenyl)-furan-2-ylmethyl]-2,3,4,5-tetrahydro-1*H*-benzo[*d*]azepin-7-yloxy}-cyclopropanecarboxylic acid**

A mixture of cpd MM1 (20 mg, 0.04 mmol) and 3N NaOH (0.15 mL) in MeOH (0.5 mL) was stirred at room temperature overnight. After the mixture was concentrated, the residue was dissolved in $H_2O$ and washed with EtOAc. The aqueous layer was acidified with dilute tartaric acid till pH 3 and a brown solid was collected to give cpd 38 (15 mg, 79%): $^1H$ NMR (400 MHz, DMSO-$\delta_6$) δ 7.87 (d, *J*= 8.2 Hz, 2 H), 7.77 (d, *J*= 8.2 Hz, 2 H), 7.11 (d, *J*= 3.3 Hz, 1 H), 7.00 (d, *J*= 8.3 Hz, 1 H), 6.65 (d, *J*= 2.6 Hz, 1 H), 6.59 (dd, *J* = 8.2, 2.5 Hz, 1 H), 6.49 (d, *J* = 3.3 Hz, 1 H), 3.79 (s, 2 H), 2.82 (m, 4 H), 2.63 (m, 4 H), 1.47 (m, 2 H), 1.19 (m, 2 H); MS (ES) m/z: 472 (M+H$^+$).

Example NN

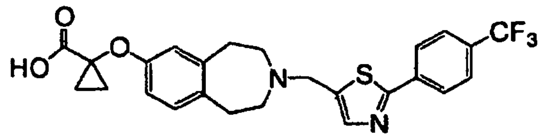

**Compound 39: 1-{3-[2-(4-Trifluoromethyl-phenyl)-thiazol-5-ylmethyl]-2,3,4,5-tetrahydro-1*H*-benzo[*d*]-azepin-7-yloxy}-cyclopropanecarboxylic acid**

The title compound was made according to Scheme NN.

**Scheme MM**

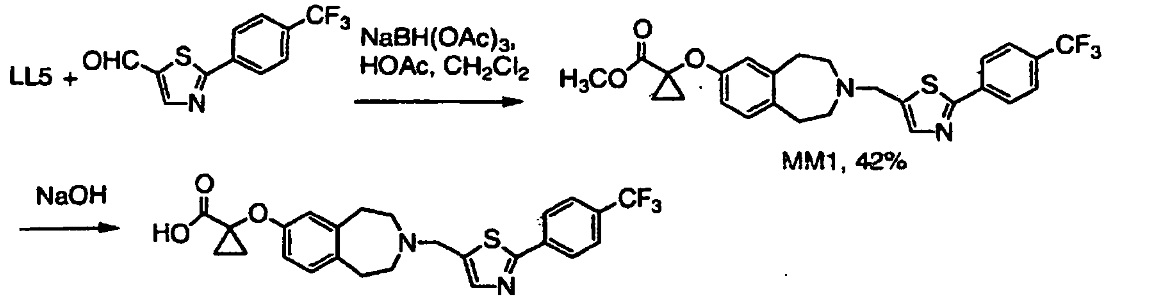


**cpd 39, 80%**

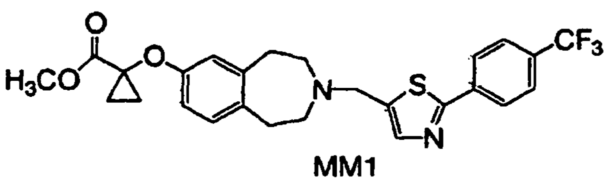

**MM1**

**1-{3-[2-(4-Trifluoromethyl-phenyl)-thiazol-5-ylmethyl]-2,3,4,5-tetrahydro-1*H*-benzo[*d*]-azepin-7-yloxy}-cyclopropanecarboxylic acid methyl ester**

Cpd MM1 was prepared according to the same procedure as for cpd X2. Cpd MM1 was obtained (0.10 g, 53%) as a light brown solid: $^1H$ NMR (300 MHz, $CDCl_3$) δ 8.06 (d, *J*= 7.7 Hz, 2 H), 7.71 4m, 3 H), 8.99 (d, *J*= 8.2 Hz, 1 H), 6.67 (m, 2 H), 3.92 (s, 2 H), 3.74 (s, 3 H), 2.89 (m, 4 H), 2.71 (m, 4 H), 1.63 (m, 2 H), 1.32 (m, 2 H); MS (ES) m/z: 502 (M+H$^+$).

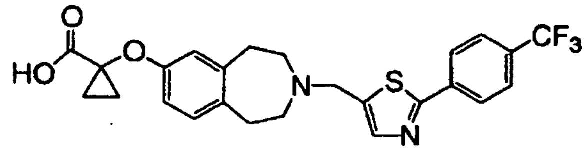

cpd 39

**1-{3-[2-(4-Trifluoromethyl-phenyl)-thiazol-5-ylmethyl]-2,3,4,5-tetrahydro-1*H*-benzo[*d*]-azepin-7-yloxy}-cyclopropanecarboxylic acid**

A mixture of cpd MM1 (86 mg, 0.17 mmol), NaOH (3N, 0.50 mL), MeOH (0.5 mL) and THF (0.5 mL) was stirred at room temperature overnight. The mixture was concentrated, the residue dissolved in $H_2O$ and washed with $Et_2O$. The aqueous layer was treated with tartaric acid to pH 3 and a pale solid was collected by filtration to give cpd 39 (66 mg, 80%): $^1H$ NMR (400 MHz, $CDCl_3$) δ 8.02 (d, *J*= 8.2 Hz, 2 H), 7.74 (d, *J* = 8.2 Hz, 2 H), 6.92 (d, *J* = 8.9 Hz, 1 H), 6.75 (m, 2 H), 4.21 (s, 2 H), 2.91 (m, 4 H), 2.84 (m, 4 H), 1.59 (m, 2 H), 1.25 (m, 2 H); MS (ES) m/z: 489 ($M+H^+$).

Example OO

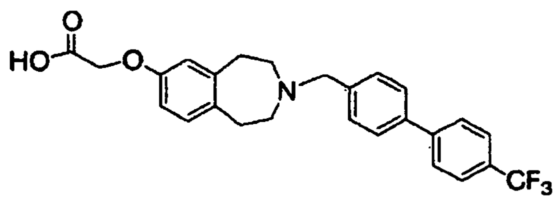

**Compound 40: [3-(4'-Trifluoromethyl-biphenyl-4-ylmethyl)-2,3,4,5-tetrahydro-1*H*-benzo[*d*]azepin-7-yloxy]-acetic acid**

The title compound was made according to Scheme OO.

## Scheme OO

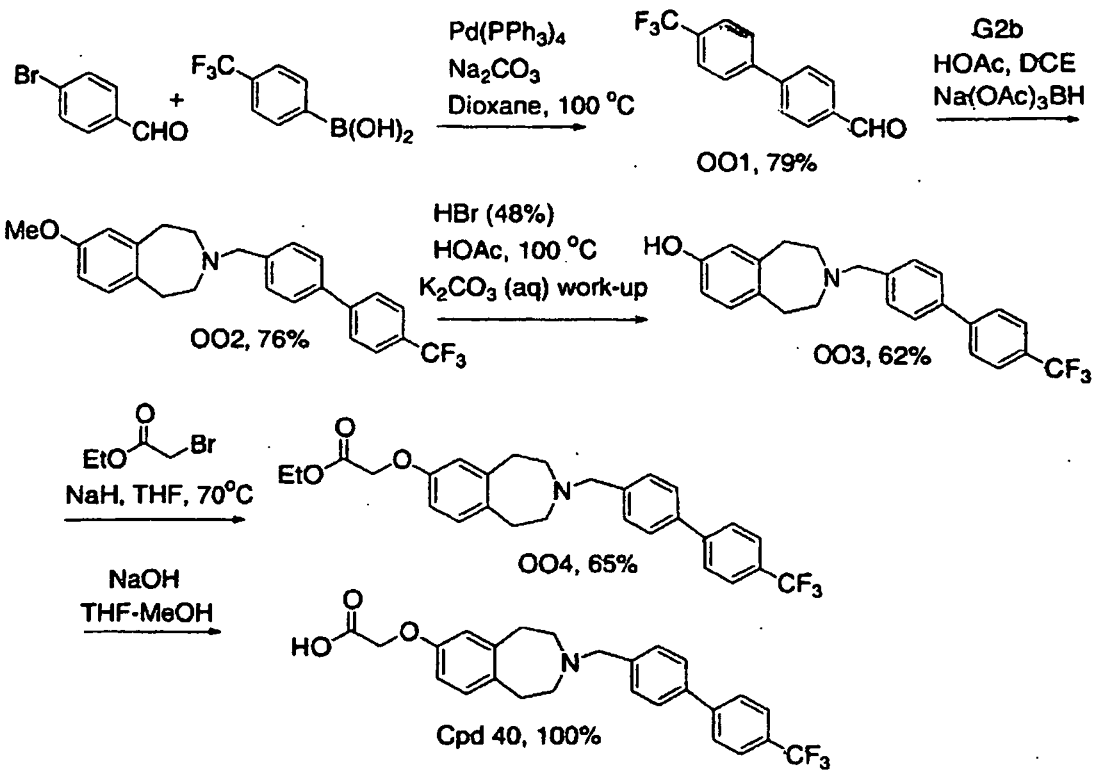


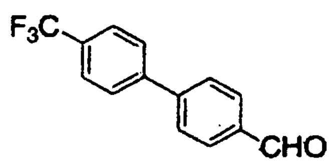

**OO1**

**4'-Trifluoromethyl-biphenyl-4-carbaldehyde**

Cpd OO1 (688 mg, 79%) was prepared according to a similar procedure as for cpd G1a: $^1$H NMR (300 MHz, $CDCl_3$) δ 10.09 (s, 1 H), 7.99 (d, *J*= 8.3 Hz, 2 H), 7.76 (d, *J*= 8.3 Hz, 2 H), 7.74 (s, 4 H).

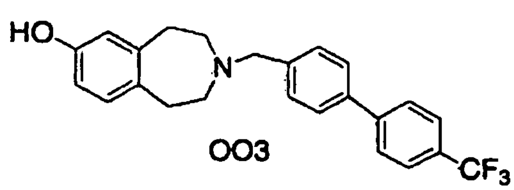

**OO3**

**3-(4'-Trifluoromethyl-biphenyl-4-ylmethyl)-2,3,4,5-tetrahydro-1*H*-benzo[*d*]azepin-7-ol**

Cpd OO3 (62%) was prepared according to a similar procedure as for cpd G2d: $^1$H NMR (300 MHz, DMSO) δ 9.03 (s, 1 H), 7.89 (d, *J*= 8.2 Hz, 2 H), 7.80 (d, *J* = 8.3 Hz, 2 H), 7.70 (d, *J* = 8.1 Hz, 2 H), 7.46 (d, *J* = 8.1 Hz, 2 H), 6.86 (d, *J* = 8.0 Hz, 1 H), 6.50 (d, *J* = 2.3 Hz, 1 H), 6.44-6.47 (dd, *J* =2.4, 8.0 Hz, 1 H), 3.64 (s, 2 H), 2.74 (br,s, 4 H), 2.56 (br, s, 4 H); MS (ES) m/z: 398.1 ($M+H^+$).

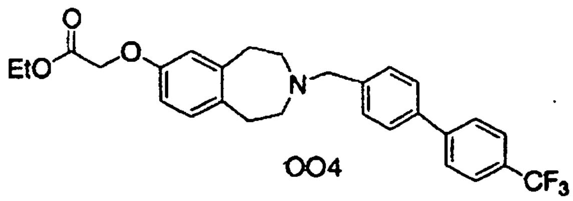

OO4

**[3-(4'-Trifluoromethyl-biphenyl-4-ylmethyl)-2,3,4,5-tetrahydro-1*H*-benzo[*d*]azepin-7-yloxy]-acetic acid ethyl ester**

Cpd OO4 (138 mg, 65%) was prepared according to a similar procedure as for cpd G1b: $^1$H NMR (300 MHz, $CDCl_3$) δ 7.69 (s, 4 H), 7.56 (d, *J* = 8.1 Hz, 2 H), 7.46 (d, *J* = 8.1 Hz, 2 H), 7.00 (d, *J* = 8.2 Hz, 1 H), 6.69 (d, *J* = 2.6 Hz, 1 H), 6.60-6.64 (dd, *J* = 2.6, 8.1 Hz, 1 H), 4.58 (s, 2 H), 4.23-4.30 (q, *J* = 7.1 Hz, 2 H), 3.69 (s, 2 H), 2.89 (s, br, 4 H), 2.65 (s, br, 4 H), 1.27-1.32 (t, *J* = 7.1 Hz, 3 H); MS (ES) m/z: 484.0 ($M+H^+$).

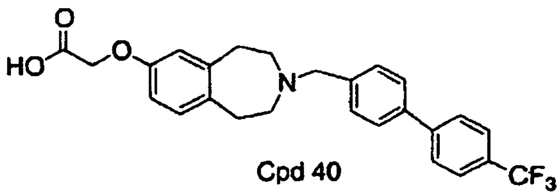

Cpd 40

**[3-(4'-Trifluoromethyl-biphenyl-4-ylmethyl)-2,3,4,5-tetrahydro-1*H*-benzo[*d*]azepin-7-yloxy]-acetic acid**

Cpd 40 (125 mg, 100%) was prepared according to a similar procedure as for cpd 7: $^1$H NMR (400 MHz, DMSO) δ 7.93 (d, *J* = 8.2 Hz, 2 H), 7.81-7.85 (m, 4 H), 7.66 (d, *J* = 7.9 Hz, 2 H), 7.07 (d, *J* = 8.3 Hz, 1 H), 6.76 (d, *J* = 2.4 Hz, 1 H), 6.67-6.70 (dd, *J* = 2.4, 8.2 Hz, 1 H), 4.62 (s, 2 H), 4.25 (s, 2 H), 3.32 (s, br, 4 H), 3.05 (s, br, 4 H); MS (ES) m/z: 456.0 ($M+H^+$), 454.0 ($M-H^+$).

Example PP

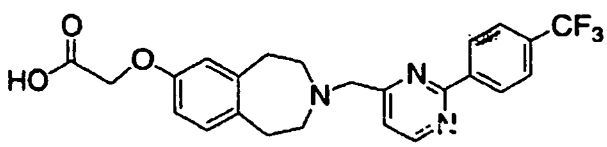

**Compound 41: {3-[2-(4-Trifluoromethyl-phenyl)-pyrimidin-4-ylmethyl]-2,3,4,5-tetrahydro-1*H*-benzo[*d*]azepin-7-yloxy}-acetic acid**

The title compound was made according to Scheme PP.

## Scheme PP

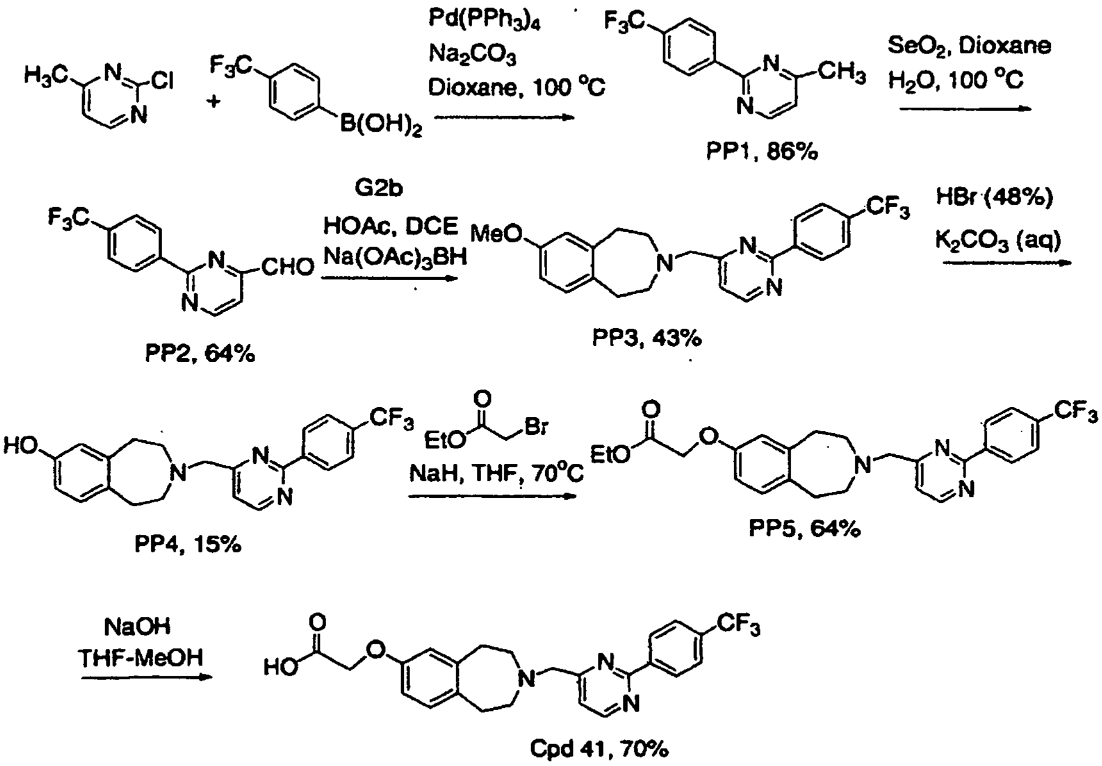


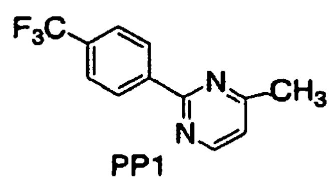

PP1

**4-Methyl-2-(4-trifluoromethyl-phenyl)-pyrimidine**

Cpd PP1 (1.507 g, 86%) was prepared according to similar procedure as for cpd G1a: $^1$H NMR (300 MHz, $CDCl_3$) δ 8.69 (d, *J* = 5.0 Hz, 1 H), 8.58 (d, *J* = 8.2 Hz, 2 H), 7.73 (d, *J* = 8.2 Hz, 2 H), 7.13 (d, *J* = 5.0 Hz, 1 H), 2.62 (s, 3 H); MS (ES) m/z: 239.1 ($M+H^+$).

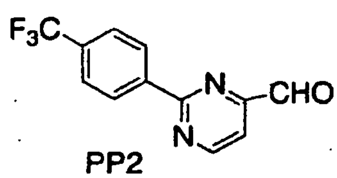

PP2

**2-(4-Trifluoromethyl-phenyl)-pyrimidine-4-carbaldehyde**

A mixture of cpd PP1 (428 mg, 1.798 mmol), $SeO_2$ (998 mg, 8.991 mmol), dioxane (5 mL) and water (0.16 mL) was stirred at 100 °C for 16 h. After the mixture was cooled to room temperature, it was filtered to remove solids and concentrated under *vacuo.* The residue was purified by column chromatography eluting with EtOAc/Hexane to give cpd PP2 (291 mg, 64%) as a white solid: $^1$H NMR (300 MHz, $CDCl_3$) δ 10.15 (s, 1 H), 9.11 (d, J = 4.8 Hz, 1 H), 8.68 (d, J = 8.1 Hz, 2 H), 7.75-7.81 (m, 3 H).

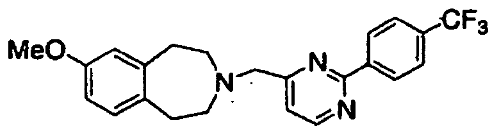

PP3

**7-Methoxy-3-[2-(4-trifluoromethyl-phenyl)-pyrimidin-4-ylmethyl]-2,3,4,5-tetrahydro-1*H*-benzo[*d*]azepine**

Cpd PP3 (179 mg, 43%) was prepared according to a similar procedure as for cpd G2c: $^1H$ NMR (300 MHz, $CDCl_3$) δ 8.80 (d, *J* = 5.0 Hz, 1 H), 8.56 (d, *J* = 8.2 Hz, 2 H), 7.73 (d, *J* = 8.3 Hz, 2 H), 7.57 (s, br, 1 H), 7.02 (d, *J* = 7.8 Hz, 1 H), 6.64-6.68 (m, 2 H), 3.86 (s, 2 H), 3.78 (s, 3 H), 2.93 (s, br, 4 H), 2.75 (s, br, 4H); MS (ES) m/z: 414.1 ($M+H^+$).

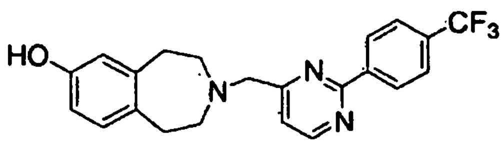

PP4

**3-[2-(4-Trifluoromethyl-phenyl)-pyrimidin-4-ylmethyl]-2,3,4,5-tetrahydro-1*H*-benzo[*d*]azepin-7-ol**

Cpd PP4 (26 mg, 20%) was prepared according to a similar procedure as for cpd G2d: $^1H$ NMR (300 MHz, $CDCl_3$) δ 8.82 (d, *J* = 5.0 Hz, 1 H), 8.56 (d, *J* = 8.2 Hz, 2 H), 7.73 (d, *J* = 8.3 Hz, 2 H), 7.62 (s, br, 1 H), 6.95 (d, *J* = 7.8 Hz, 1 H), 6.58-6.61 (m, 2 H), 3.93 (s, br, 2 H), 2.94 (s, br, 4 H), 2.81 (s, br, 4 H); MS (ES) m/z: 400.0 ($M+H^+$).

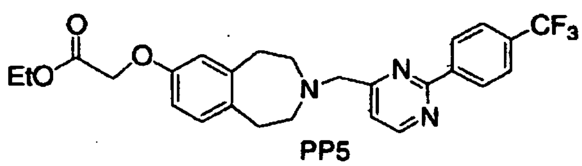

PP5

**{3-[2-(4-Trifluoromethyl-phenyl)-pyrimidin-4-ylmethyl]-2,3,4,5-tetrahydro-1*H*-benzo[*d*]azepin-7-yloxy}-acetic acid ethyl ester**

Cpd PP5 (16 mg, 64%) was prepared according to a similar procedure as for cpd G1b: $^1H$ NMR (300 MHz, $CDCl_3$) δ 8.85 (s, br, 1 H), 8.54 (d, *J* = 8.1 Hz, 2 H), 7.73 (d, *J* = 8.3 Hz, 2 H), 7.61 (s, br, 1 H), 7.03 (d, *J* = 8.2 Hz, 1 H), 6.72 (d, *J* = 2.5 Hz, 1 H), 6.64-6.67 (m, 1 H), 4.59 (s, 2 H), 4.23- 4.30 (q, *J* = 7.1 Hz, 2 H), 3.92 (s, br, 2 H), 2.80-2.93 (br, 8 H), 1.29 (t, *J* = 7.1 Hz, 3 H); MS (ES) m/z: 486.1 ($M+H^+$).

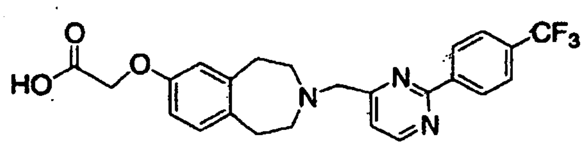

Cpd 41

**{3-[2-(4-Trifluoromethyl-phenyl)-pyrimidin-4-ylmethyl]-2,3,4,5-tetrahydro-1*H*-benzo[*d*]azepin-7-yloxy}-acetic acid**

Cpd 41 (10 mg, 70%) was prepared according to a similar procedure as for cpd 7: $^1H$ NMR (400 MHz, DMSO) δ 8.94 (d, *J* = 5.0 Hz, 1 H), 8.58 (d, *J* = 8.3 Hz, 2 H), 7.90 (d, *J*= 8.5 Hz, 2 H), 7.65 (d, *J* = 5.1 Hz, 1 H), 7.01 (d, *J* = 8.3 Hz, 1 H), 6.70 (d, *J* = 2.6 Hz, 1 H), 6.60-6.62 (dd, *J* = 2.6, 8.2 Hz, 1 H), 4.60 (s, 2 H), 3.88 (s, 2 H), 2.86 (s, br, 4 H, 2.67 (s, br, 4 H); MS (ES) m/z: 458.1 ($M+H^+$), 456.0 ($M-H^+$).

Example NN

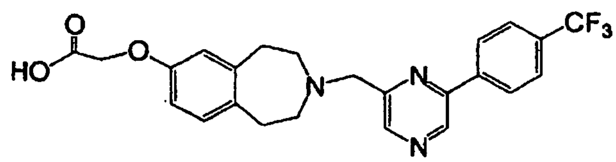

**Compound 42: {3-[6-(4-Trifluoromethyl-phenyl)-pyrazin-2-ylmethyl]-2,3,4,5-tetrahydro-1*H*-benzo[*d*]azepin-7-yloxy}-acetic acid**

The title compound was made according to Scheme NN.

**Scheme NN**

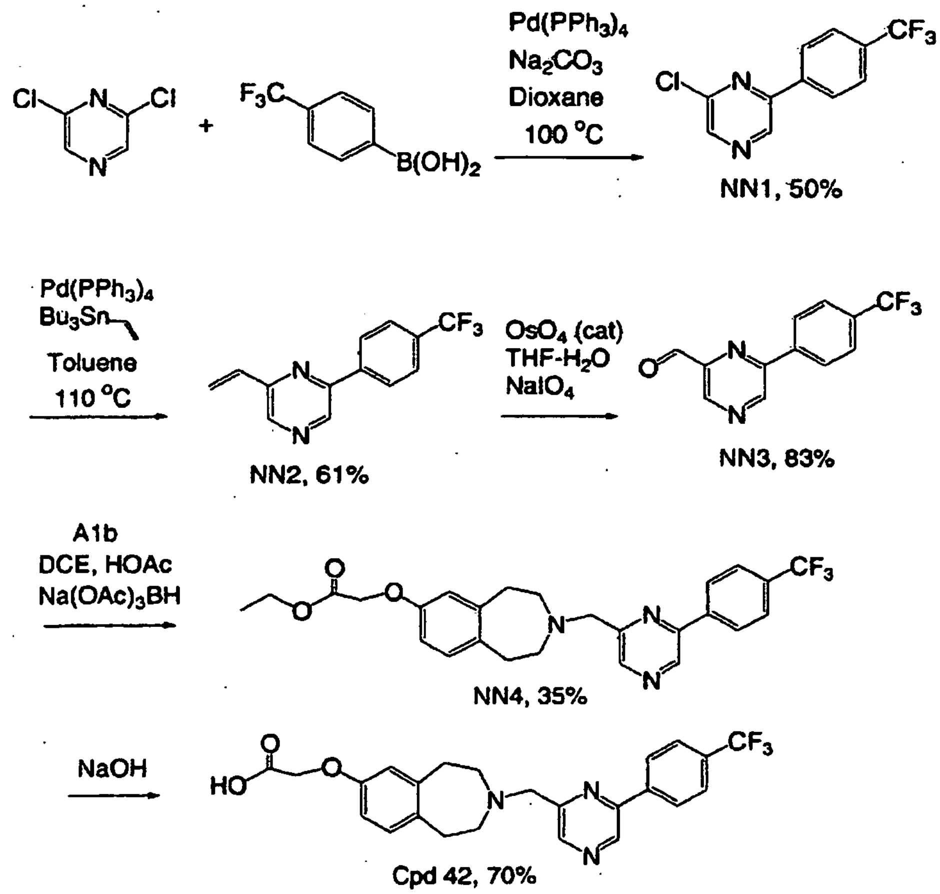


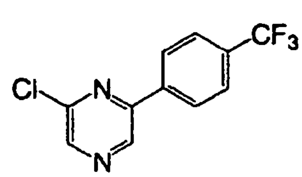

**2-Chloro-6-(4-trifluoromethyl-phenyl)-pyrazine**

Cpd NN1 (522 mg, 50%) was prepared according to a similar procedure as for cpd G1a. $^1$H NMR (300 MHz, $CDCl_3$) δ 8.97 (s, 1 H), 8.60 (s, 1 H), 8.15 (d, *J* = 8.2 Hz, 2 H), 7.78 (d, *J* = 8.3 Hz, 2 H).

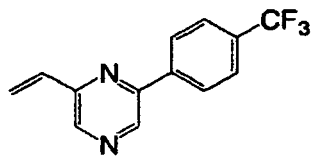

**2-(4-Trifluoromethyl-phenyl)-6-vinyl-pyrazine**

A mixture of cpd NN1 (106 mg, 0.411 mmol), $Pd(PPh_3)_4$ (47 mg, 0.041 mmol), tributylvinyltin (0.18 mL, 0.616 mmol) and toluene (2.0 mL) was refluxed under $N_2$ for 18 h. The mixture was cooled and concentrated. Cpd NN2 was obtained after column chromatography purification as clear oil (98 mg, 95%): $^1H$ NMR (300 MHz, $CDCl_3$) δ 8.92 (s, 1 H), 8.58 (s, 1 H), 8:20 (d, *J* = 8.1 Hz, 2 H), 7.78 (d, *J* = 8.2 Hz, 2 H), 6.87-6.97 (m, 1 H), 6.49-6:55 (m, 1 H), 5.70-5.74 (m, 1 H).

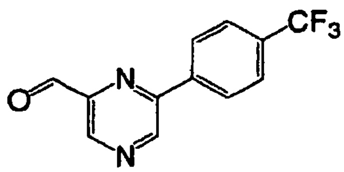

**6-(4-Trifluoromethyl-phenyl)-pyrazine-2-carbaldehyde**

A mixture of NN2 (72 mg, 0.288 mmol), THF (2.5 mL), water (2.5 mL), $OsO_4$ (2.5 wt%, 2 drops) and $NaIO_4$ (123 mg, 0.576 mmol) was stirred at r.t. for 18 h. The mixture was poured into aqueous $NaHCO_3$, extracted with $CH_2Cl_2$. The organic extracts were washed with water, brine, dried ($Na_2SO_4$) and concentrated. The residue was purified by column chromatography to give NN3 (60 mg, 83%) as beige solid: $^1H$ NMR (300 MHz, $CDCl_3$) δ 10.25 (s, 1 H), 9.28 (s, 1 H), 9.16 (s, 1 H), 8.25 (d, *J* = 8.1 Hz, 2 H), 7.83 (d, *J* = 8.2 Hz, 2 H).

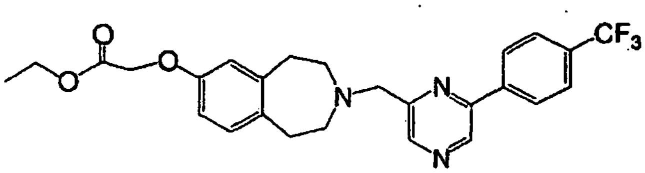

**{3-[6-(4-Trifluoromethyl-phenyl)-pyrazin-2-ylmethyl]-2,3,4,5-tetrahydro-1*H*-benzo[*d*]azepin-7-yloxy}-acetic acid ethyl ester**

Cpd NN4 (38 mg, 35%) was prepared according to a similar procedure as for cpd G1b. $^1H$ NMR (300 MHz, $CDCl_3$) δ 8.94 (s, 1 H), 8.81 (s, 1 H), 8.14 (d, J = 8.1 Hz, 2 H), 7.76 (d, J = 8.3 Hz, 2 H), 7.00 (d, J =.8.2 Hz, 1 H), 6.70 (d, J = 2.6 Hz, 1 H), 6.61-6.65 (dd, J = 2.7, 8.2 Hz, 1 H), 4.58 (s, 2 H), 4.23-4.30 (q, J = 7.1 hz, 2 H), 3.94 (s, 2 H), 2.91 (s, br, 4 H), 2.75 (s, br, 4 H), 1.27-1.32 (t, J = 7.1 Hz, 3 H); MS (ES) m/z: 486.1 ($M+H^+$).

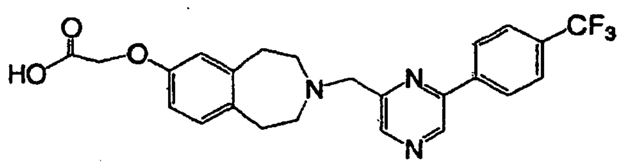

**{3-[6-(4-Trifluoromethyl-phenyl)-pyrazin-2-ylmethyl]-2,3,4,5-tetrahydro-1*H*-benzo[*d*]azepin-7-yloxy}-acetic acid**

Cpd 42 (21 mg, 70%) was prepared according to a similar procedure as for cpd 7. $^1H$ NMR (400 MHz, DMSO-$d_6$) δ 9.40 (s, 1 H), 8.89 (s, 1 H), 8.43 (d, *J* = 7.6 Hz, 2 H), 7.95 (d, *J* = 8.4 Hz, 2 H), 7.09 (d, *J* = 5.9 Hz, 1 H), 6.78 (s, 1 H), 6.68 (s, br, 1 H), 5.76 (s, 2 H), 4.62 (s, 2 H), 3.00 (br, 8 H); MS (ES) m/z: 458.1 ($M+H^+$)

Example QQ

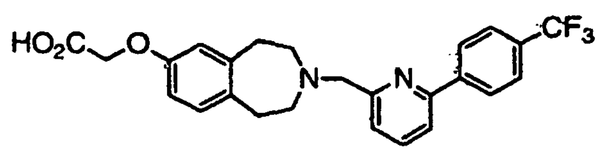

**Compound 43: {3-[6-(4-Trifluoromethyl-phenyl)-pyridin-2-ylmethyl]-2,3,4,5-tetra-hydro-1*H*-benzo[*d*]azepin-7-yloxy}-acetic acid**

The title compound was made according to Scheme QQ.

## Scheme QQ

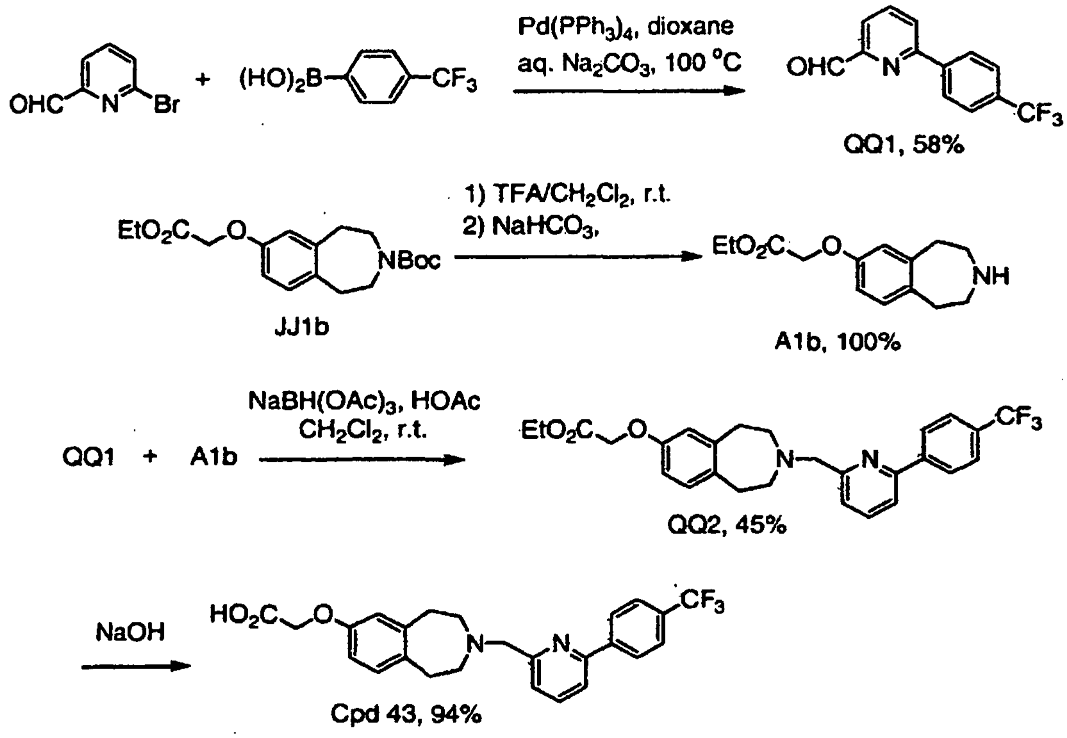


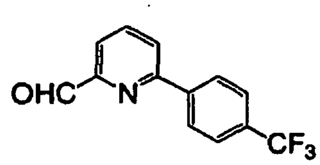

**6-(4-Trifluoromethyl-phenyl)-pyridine-2-carbaldehyde**

Cpd QQ1 (0.78 g, 58%) was prepared according to the same procedure as for cpd G1a: $^1H$ NMR (300 MHz, $CDCl_3$) δ 10.2 (s, 1H), 8.25 (d, *J*= 8.2 Hz, 2 H), 8.02 (m, 3 H), 7.80 (d, *J*= 8.2 Hz, 2 H); MS (ES) m/z: 284 (M+Na).

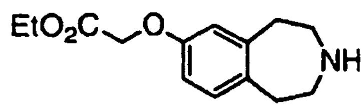

(2,3,4,5-Tetrahydro-1*H*-benzo[*d*]azepin-7-yloxy)-acetic acid ethyl ester

A mixture of cpd JJ1b (0.93 g, 2.66 mmol) and TFA (1.10 mL, 14.8 mmol) in $CH_2Cl_2$ (1.5 mL) was stirred at room temperature under $N_2$ for 21 h. After the mixture was concentrated, the residue was treated with aqueous $NaHCO_3$ and extracted with EtOAc. The organic layer was dried ($MgSO_4$) and concentrated to give cpd A1b (0.66 g, 100%) as oil: $^1H$ NMR (300 MHz, $CDCl_3$) δ 7.09 (d, *J* = 8.2 Hz, 1 H), 6.77 (d, *J* = 2.5 Hz, 1 H), 6.71 (dd, *J* = 8.2, 2.6 Hz, 1 H), 4.62 (s, 2 H), 4.30 (q, *J* = 7.1 Hz, 2 H), 3.27 (m, 4 H), 3.16 (m, 4 H), 1.30 (t, *J* = 7.1 Hz, 3 H); MS (ES) m/z: 250 ($M+H^+$).

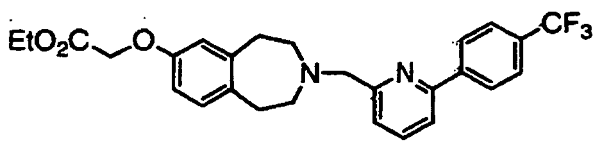

{3-[6-(4-Trifluoromethyl-phenyl)-pyridin-2-ylmethyl]-2,3,4,5-tetrahydro-1*H*-benzo[*d*]azepin-7-yloxy}-acetic acid ethyl ester

Cpd QQ2 was prepared according to the same procedure as for cpd X2. Cpd QQ2 was obtained (43 mg, 45%) as an oil: $^1H$ NMR (300 MHz, $CDCl_3$) δ 8.13 (d, *J* = 8.1 Hz, 2 H), 7.81 (t, *J* = 7.70 Hz, 1 H), 7.73 (d, *J* = 8.1 Hz, 2 H), 7.65 (d, *J* = 7.6 Hz, 1 H), 7.58 (d, *J* = 7.6 Hz, 1 H), 7.02 (d, *J* = 8.2 Hz, 1 H), 6.72 (d, *J*= 2.5 Hz, 1 H), 6.64 (dd, *J*= 8.1, 2.6 Hz, 1 H), 4.60 (s, 2 H), 4.28 (q, *J*= 7.2 Hz, 2 H), 3.93 (s, 2 H), 2.94 (m, 4 H), 2.78 (m, 4 H), 2.09 (3 H), 1.30 (t, *J* = 7.2 Hz, 3 H); MS (ES) m/z: 485 ($M+H^+$).

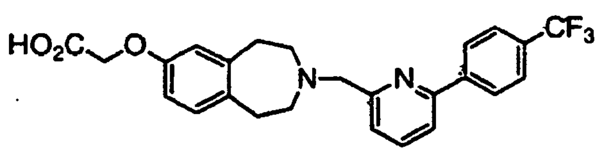

{3-[6-(4-Trifluoromethyl-phenyl)-pyridin-2-ylmethyl]-2,3,4,5-tetrahydro-1*H*-benzo[*d*]azepin-7-yloxy}-acetic acid

Cpd 43 was prepared according to the same procedure as for cpd 7. Cpd 43 was obtained (31 mg, 94%) as an off-white solid: $^1H$ NMR (400 MHz, DMSO-$d_6$) δ 8.30 (d, *J* = 8.1 Hz, 2 H), 7.95 (m, 2 H), 7.86 (d, *J* = 8.2 Hz, 1 H), 7.58 (m, 1 H), 7.00 (d, *J* = 8.2 Hz, 1 H), 6.69 (d, *J* = 2.3 Hz, 1 H), 6.61 (dd, *J* = 8.2, 2.5 Hz, 1 H), 4.60 (s, 2 H), 3.87 (s, 2 H), 2.84 (m, 4 H), 2.67 (m, 4 H); MS (ES) m/z: 457 ($M+H^+$).

Example RR

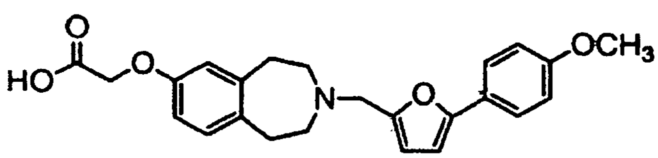

Compound 44: {3-[5-(4-Methoxy-phenyl)-furan-2-ylmethyl]-2,3,4,5-tetrahydro-1*H*-benzo[*d*]azepin-7-yloxy}-acetic acid

The title compound was made according to Scheme RR.

**Scheme RR**

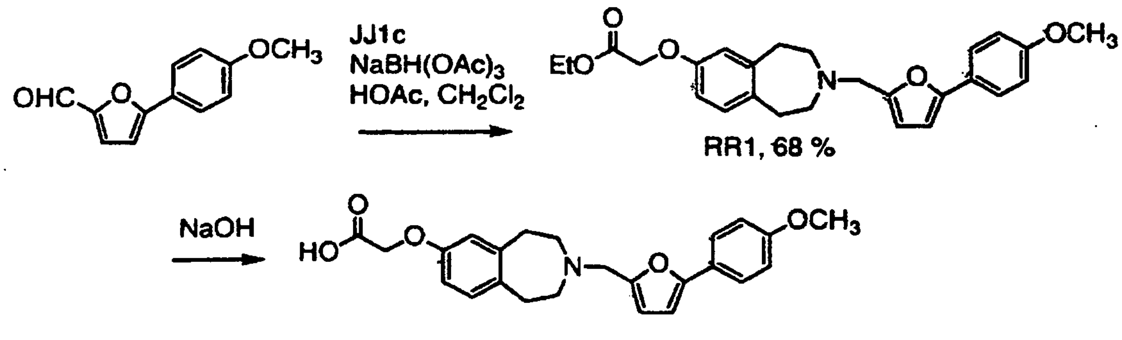


Cpd 44, 82 %

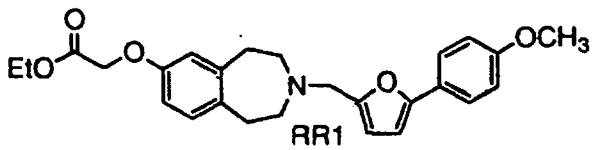

RR1

**{3-[5-(4-Methoxyphenyl)-furan-2-ylmethyl]-2,3,4,5-tetrahydro-1*H* -benzo[*d*]azepin-7-yloxy}-acetic acid ethyl ester**

Cpd RR1 was prepared according to the same procedure as for cpd X4. Cpd RR1 was obtained (65 mg, 68 %) as yellow oil: $^1H$ NMR (400 MHz, $CDCl_3$) δ 7.49 (d, *J* = 8.9 Hz, 2 H), 6.90 (d, *J* = 8.2 Hz, 1 H), 6.82 (d, *J* = 8.9 Hz, 2 H), 6.60 (d, *J* = 2.7 Hz, 1 H), 6.57 (dd, *J* = 8.2, 2.7 Hz, 1 H), 6.35 (d, *J* = 3.2 Hz, 1 H), 6.17 (d, *J*= 3.2 Hz, 1 H), 4.49 (s, 2 H), 4.18 (q, *J*= 7.2 Hz, 2 H), 3.75 (s, 3 H), 3.71 (s, 2 H), 2.83 (m, 4 H), 2.65 (m, 4 H), 1.22 (t, *J* = 7.2 Hz, 3 H); MS (ES) m/z: 436 ($M+H^+$).

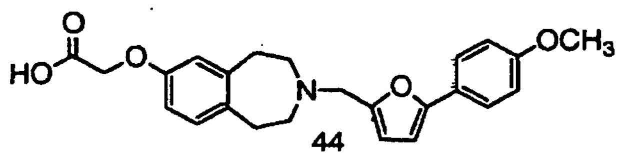

44

**{3-[5-(4-Methoxy-phenyl)-furan-2-ylmethyl]-2,3,4,5-tetrahydro-1*H* -benzo[*d*]azepin-7-yloxy}-acetic acid**

A solution of cpd RR1 (55 mg, 0.13 mmol) in methanol (2 mL) was treated with 1 N aqueous NaOH (0.26 mL, 0.26 mmol). After stirring overnight, the mixture was concentrated to dryness. The residue was dissolved in $H_2O$, washed with $Et_2O$ twice and then acidified with 1 N HCl. The acidic solution was extracted with EtOAc. The organic extracts were dried and concentrated to give cpd 44 (42 mg, 82 %) as a yellow solid: $^1H$ NMR (300 MHz, $CDCl_3$) δ 7.46 (d, *J* = 8.8 Hz, 2 H), 6.92 (d, *J* = 7.3 Hz, 1 H), 6.85 (d, *J* = 8.8 Hz, 2 H), 6.62 (m, 2 H), 6.57 (d, *J* = 3.3 Hz, 1 H), 6.43 (d, *J* = 3.3 Hz, 1 H), 4.56 (s, 2 H), 4.34 (s, 2 H), 3.76 (s, 3 H), 3.63 (m, 4 H), 2.70 (m, 4 H); MS (ES) m/z: 408 ($M+H^+$).

<u>Example SS</u>

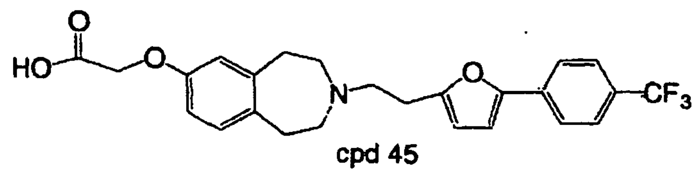

cpd 45

**(3-{2-[5-(4-Trifluoromethyl-phenyl)-furan-2-yl]-ethyl}-2,3,4,5-tetrahydro-1*H*-benzo[*d*]azepin-7-yloxy)-acetic acid**

The title compound was made according to Scheme SS.

## Scheme SS

Scheme SS

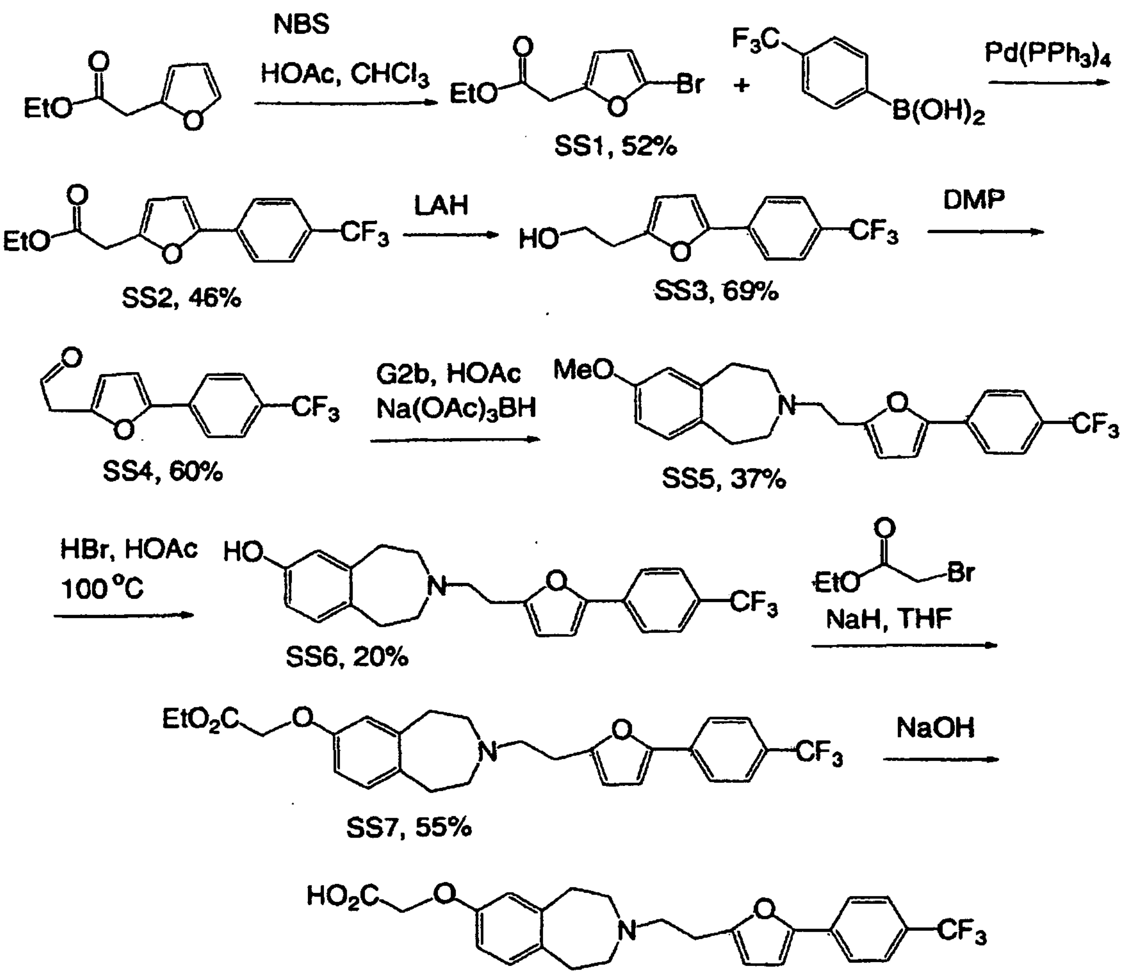


cpd 45, 60%

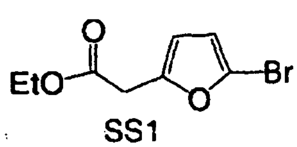

SS1

**(5-Bromo-furan-2-yl)-acetic acid ethyl ester**

Cpd SS1 (765 mg, 52%) was prepared using the same procedure as for cpd V1. $^1$H NMR (400 MHz, $CDCl_3$)

δ 6.25 (d, $J$ = 3.2 Hz, 1 H), 6.22 (d, $J$ = 3.2 Hz, 1 H), 4.16-4.22 (q, $J$ = 7.1 Hz, 2 H), 3.65 (s, 2 H), 1.24-1.29 (t, $J$ = 7.2 Hz, 3 H).

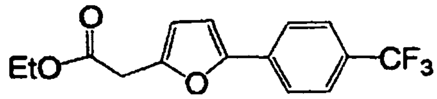

SS2

**[5-(4-Trifluoromethyl-phenyl)-furan-2-yl]-acetic acid ethyl ester**

Cpd SS2 (448 mg, 46%) was prepared using the same procedure as for cpd V2. $^1$H NMR (300 MHz, $CDCl_3$) δ 7.72 (d, $J$= 8.2 Hz, 2 H), 7.60 (d, $J$ = 8.3 Hz, 2 H), 6.71 (d, $J$ = 3.3 Hz, 1 H), 6.35 (d, $J$ = 3.3 Hz, 1 H), 4.18-4.25 (q, $J$ = 7.2 Hz, 2 H), 3.76 (s, 2 H), 1.27 - 1.31 (t, $J$ = 7.1 Hz, 3 H); MS (ES) m/z: 299.1 ($M+H^+$).

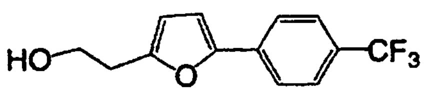

SS3

**2-[5-(4-Trifluoromethyl-phenyl)-furan-2-yl]-ethanol**

Cpd SS3 (249 mg, 69%) was prepared using the same procedure as for cpd V3. $^1$H NMR (400 MHz, $CDCl_3$) δ 7.71 (d, $J$= 8.1 Hz, 2 H), 7.61 (d, $J$ = 8.1 Hz, 2 H), 6.70 (d, $J$ = 3.3 Hz, 1 H), 6.24 - 6.25 (m, 1 H), 3.96 (t, $J$ = 6.2 Hz, 2 H), 2.99 (t, $J$ = 6.2 Hz, 2 H).

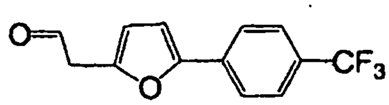

SS4

**[5-(4-Trifluoromethyl-phenyl)-furan-2-yl]-acetaldehyde**

Cpd SS4 (144 mg, 60%) was prepared using the same procedure as for cpd V4. $^1$H NMR (300 MHz, $CDCl_3$) δ 9.79 (t, $J$ = 2.0 Hz, 1 H), 7.73 (d, $J$ = 8.2 Hz, 2 H), 7.62 (d, $J$ = 8.4 Hz, 2 H), 6.75 (d, $J$ = 3.3 Hz, 1 H), 6.38 (d, $J$ = 3.3 Hz, 1 H), 3.81 (d, $J$ = 1.9 Hz, 2 H).

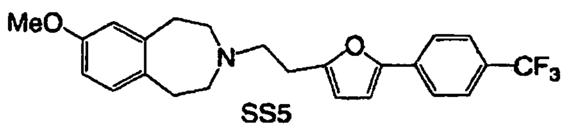

SS5

**7-Methoxy-3-{2-[5-(4-trifluoromethyl-phenyl)-furan-2-yl]-ethyl}-2,3,4,5-tetrahydro-1*H*-benzo[*d*]azepine**

Cpd SS5 (81 mg, 37%) was prepared using the same procedure as for cpd G2c. $^1$H NMR (300 MHz, DMSO-$d_6$) δ 7.84 (d, $J$ = 8.2 Hz, 2 H), 7.74 (d, $J$ = 8.4 Hz, 2 H), 7.05 (d, $J$ = 3.3 Hz, 1 H), 7.01 (d, $J$ = 8.2 Hz, 1 H), 6.70 (d, $J$ = 2.6 Hz, 1 H), 6.62 - 6.65 (d, $J$ = 8.1 Hz, 1 H), 6.34 (d, $J$ = 3.2 Hz, 1 H), 3.70 (s, 3 H), 2.76 - 2.88 (m, 8 H), 2.51 - 2.65 (m, 4H); MS (ES) m/z: 416.2 ($M+H^+$).

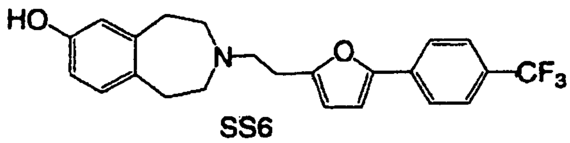

SS6

**3-{2-[5-(4-Trifluoromethyl-phenyl)-furan-2-yl]-ethyl}-2,3,4,5-tetrahydro-1*H*-benzo[*d*]azepin-7-ol**

Cpd SS6 (15 mg, 20%) was prepared using the same procedure as for cpd G2d. $^1$H NMR (400 MHz, $CDCl_3$) δ 7.60 - 7.68 (m, 4 H), 6.97 - 7.00 (m, 1 H), 6.63 - 6.69 (m, 3 H), 6.32 (d, *J* = 3.1 Hz, 1 H), 3.70 - 3.91 (m, 4 H), 3.41 - 3.49 (m, 4 H), 2.78 - 2.86 (m, 4 H); MS (ES) m/z: 402.1 ($M+H^+$).

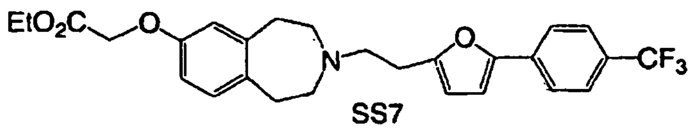

SS7

**(3-{2-[5-(4-Trifluoromethyl-phenyl)-furan-2-yl]-ethyl}-2,3,4,5-tetrahydro-1*H*-benzo[*d*]azepin-7-yloxy)-acetic acid ethyl ester**

Cpd SS7 (10 mg, 55%) was prepared using the same procedure as for cpd G1b. $^1$H NMR (300 MHz, $CDCl_3$) δ 7.68 (d, *J* = 8.6 Hz, 2 H), 7.59 - 7.62 (m, 2 H), 7.04 (d, *J* = 7.9 Hz, 1 H), 6.72 (s, 1 H), 6.68 (d, *J* = 3.3 Hz, 2 H), 6.28 (br, 1 H), 4.59 (s, 2 H), 4.23 - 4.31 (q, *J* = 7.1 Hz, 2 H), 2.87 - 3.41 (m, 12 H), 1.30 (t, *J* = 7.1 Hz, 3 H); MS (ES) m/z: 488.1 ($M+H^+$).

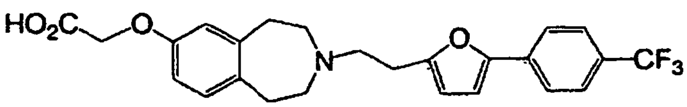

cpd 45

**(3-{2-[5-(4-Trifluoromethyl-phenyl)-furan-2-yl]-ethyl}-2,3,4,5-tetrahydro-1*H*-benzo[*d*]azepin-7-yloxy)-acetic acid**

Cpd 45 (6 mg, 60%) was prepared using the same procedure as for cpd 7. $^1$H NMR (400 MHz, MeOD) δ 7.83 (d, *J*= 8.3 Hz, 2 H), 7:67 (d, *J*= 8.2 Hz, 2 H), 7.11 (d, *J* = 8.2 Hz, 1 H), 6.91 (d, *J* = 3.2 Hz, 1 H), 6.76 - 6.81 (m, 2 H), 6.42 (d, *J* = 3.0 Hz, 1 H), 4.57 (s, 2 H), 3.53 - 3.59 (m, 2 H), 3.43 (m, 2 H), 3.09 - 3.15 (m, 8 H); MS (ES) m/z: 460.0 ($M+H^+$), 458.1 ($M-H^+$).

<u>Example TT</u>

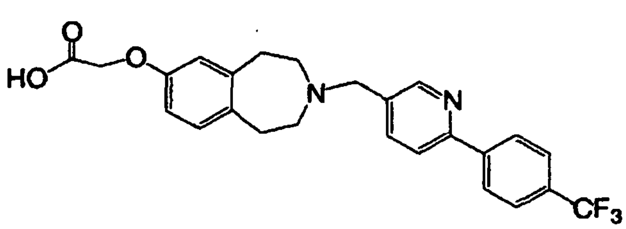

**Compound 46: {3-[6-(4-Trifluoromethyl-phenyl)-pyridin-3-ylmethyl]-2,3,4,5-tetrahydro-1*H*-benzo[*d*]azepin-7-yloxy}-acetic acid**

The title compound was made according to Scheme TT.

## Scheme TT

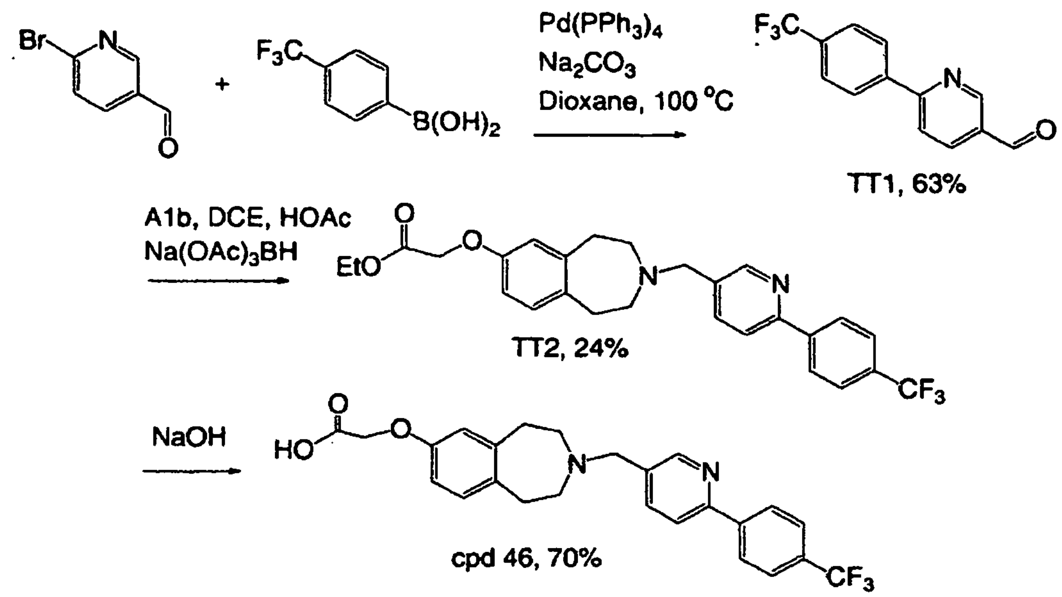


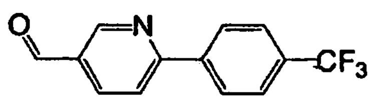

TT1

**6-(4-Trifluoromethyl-phenyl)-pyridine-3-carbaldehyde**

Cpd TT1 (498 mg, 63%) was prepared using the same procedure as for cpd G1a. $^1$H NMR (300 MHz, $CDCl_3$) δ 10.18 (s, 1 H), 9.18 (d, $J$= 1.5 Hz, 1 H), 8.29 - 8.33 (dd, $J$ = 2.1, 8.2 Hz, 1 H), 8.22 (d, $J$ = 8.1 Hz, 2 H), 7.97 (d, $J$ = 8.2 Hz, 1 H), 7.78 (d, $J$= 8.2 Hz, 2 H).

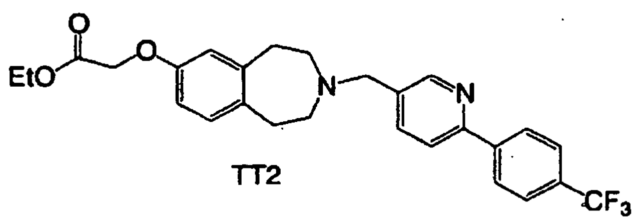

TT2

**{3-[6-(4-Trifluoromethyl-phenyl)-pyridin-3-ylmethyl]-2,3,4,5-tetrahydro-1*H*-benzo[*d*]azepin-7-yloxy}-acetic acid ethyl ester**

Cpd TT2 (47 mg, 24%) was prepared using the same procedure as for cpd G1b. $^1$H NMR (300 MHz, $CDCl_3$) δ 8.66 (s, 1 H), 8.12 (d, $J$= 8.2 Hz, 2 H), 7.71 - 7.82 (m, 4 H), 6.99 (d, $J$ = 8.1 Hz, 1 H), 6.69 (d, $J$ = 2.5 Hz, 1 H), 6.61 - 6.65 (dd, $J$ = 2.5, 8.2 Hz, 1 H), 4.58 (s, 2 H), 4.23 - 4.30 (q, $J$ = 7.1 Hz, 2 H), 3.70 (s, br, 2 H), 2.88 (s, br, 4 H), 2.65 (s, br, 4 H), 1.29 (t, $J$ = 7.1 Hz, 3 H); MS (ES) m/z: 485.1 ($M+H^+$).

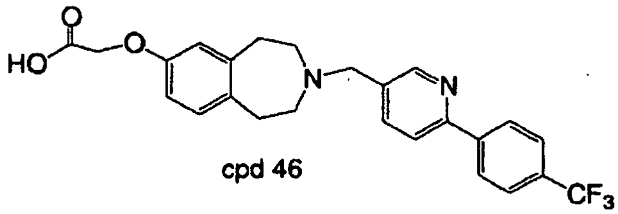

cpd 46

{3-[6-(4-Trifluoromethyl-phenyl)-pyridin-3-ylmethyl]-2,3,4,5-tetrahydro-1*H*-benzo[*d*]azepin-7-yloxy}-acetic acid

Cpd 46 (28 mg, 70%) was prepared using the same procedure as for cpd 7. $^1$H NMR (400 MHz, DMSO-$d_6$) δ 8.87 (s, 1 H), 8.35 (d, *J* = 8.2 Hz, 2 H), 8.20 (s, 2 H), 7.89 (d, *J* = 8.3 Hz, 2 H), 7.09 (d, *J* = 8.3 Hz, 1 H), 6.79 (d, *J* = 2.4 Hz, 1 H), 6.69 - 6.72 (dd, *J* = 2.5, 8.3 Hz, 1 H), 4.63 (s, 2 H), 4.45 (s, br, 2 H), 2.98 (br, 8 H); MS (ES) m/z: 457.0 (M+H$^+$), 455.1 (M-H$^+$).

Example UU

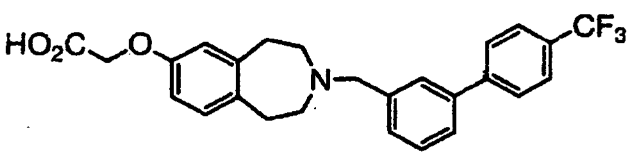

**Compound 47: [3-(4'-Trifluoromethyl-biphenyl-3-ylmethyl)-2,3,4,5-tetrahydro-1*H*-benzo[*d*]azepin-7-yloxy]-acetic acid**

The title compound was made according to Scheme UU.

## Scheme UU

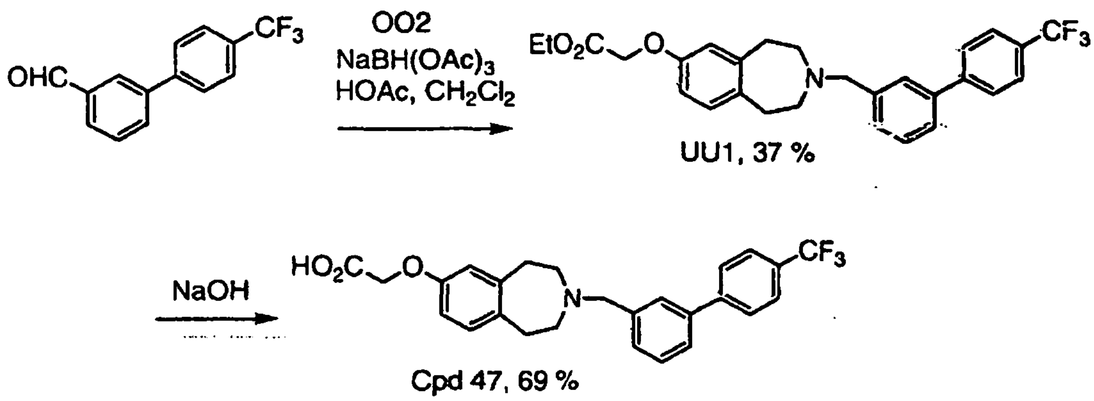


Cpd 47, 69 %

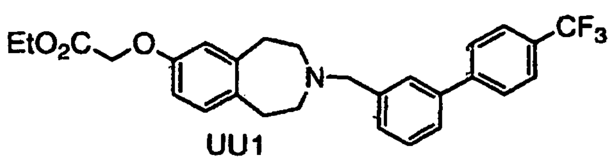

UU1

**[3-(4'-Trifluoromethyl-biphenyl-3-ylmethyl)-2,3,4,5-tetrahydro -1*H*-benzo[*d*]azepin-7-yloxy]-acetic acid ethyl ester**

To a mixture of cpd 002 (0.10 g, 0.40 mmol), 3-(4-trifluoromethylphenyl)-benzaldehyde (0.15 g, 0.59 mmol), $CH_2Cl_2$ (10 mL) and HOAc (0.02 mL, 0.35 mmol) at room temperature was added Na(-OAc)$_3$BH (0.13 g, 0.58 mmol). After the mixture was stirred overnight, additional Na(OAc)$_3$BH (0.06 g, 0.27 mmol) was added. The mixture was stirred at r.t. overnight and heated at reflux for 30 min. After cooled to room temperature, it was basified with aqueous $NaHCO_3$ and extracted with EtOAc. The organic extracts where dried ($MgSO_4$) and concentrated. The crude product was purified by column chromatography to give cpd UU1 (70 mg, 37%): $^1$H NMR (300 MHz, $CDCl_3$) δ 7.72 (m, 4 H), 7.61 (s, 1 H), 7.51 (m, 1 H), 7.43 (m, 2 H), 7.01 (d, *J*= 8.2 Hz, 1 H), 6.71 (d, *J* = 2.7 Hz, 1 H), 6.64 (dd, *J* = 8.2, 2.7 Hz, 1 H), 4.60 (s, 2 H), 4.28 (q, *J* = 7.2 Hz, 2 H), 3.72 (s, 2 H), 2.90 (m, 4 H), 2.67 (m, 4 H), 1.32 (t, *J* = 7.2 Hz, 3 H); MS (ES) m/z: 484 (M+H$^+$).

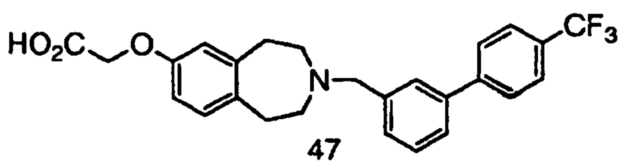

47

**[3-(4'-Trifluoromethyl-biphenyl-3-ylmethyl)-2,3,4,5-tetrahydro -1*H*-benzo[*d*]azepin-7-yloxy]-acetic acid**

Cpd 47 was prepared according to the same procedure as for cpd 31. Cpd 47 was obtained (41 mg, 69 %) as a beige solid: $^1$H NMR (300 MHz, DMSO-$d_6$) δ 7.92 (d, *J*= 8.2 Hz, 2 H), 7.84 (m, 3 H), 7.72 (d, *J*= 7.3 Hz, 1 H), 7.55 (m, 2 H), 7.04 (d, *J*= 8.3 Hz, 1 H), 6.72 (d, *J* = 2.1 Hz, 1 H), 6.65 (d, *J* = 8.3 Hz, 1 H), 4.61 (s, 2 H), 3.99 (bs, 2 H), 2.81 (m, 8 H); MS (ES) m/z: 456 (M+H$^+$).

Compounds 1 through 47 of Formula (I) in Table 1 were prepared according to the methods described by the Schemes and Examples described herein.

Table 1. Representative Compounds

| Cpd No. | Structure |
|---|---|
| 1 | 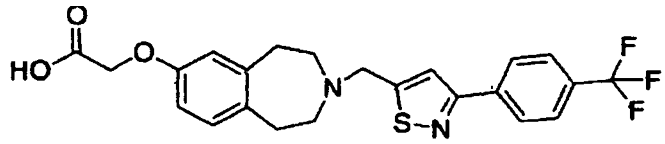 |
| 2 | 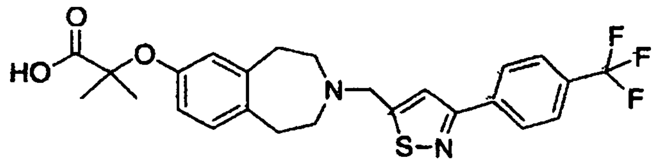 |
| 3 | 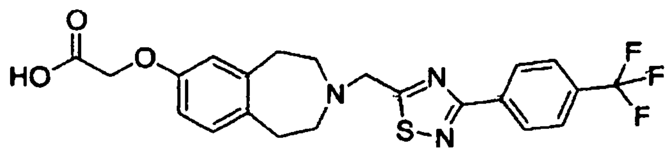 |
| 4 | 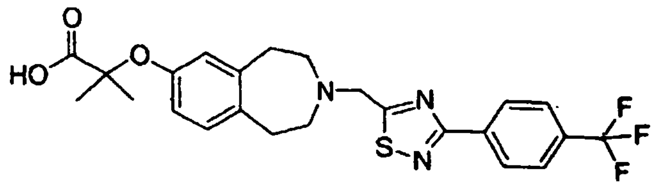 |
| 5 | 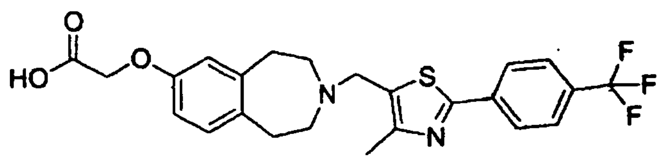 |
| 6 | 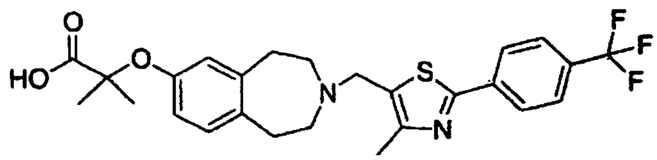 |
| 7 | 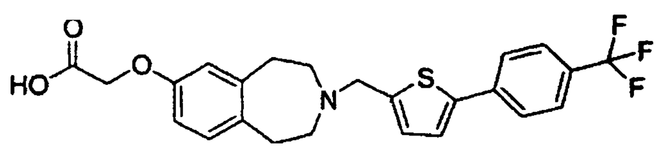 |
| 8 | 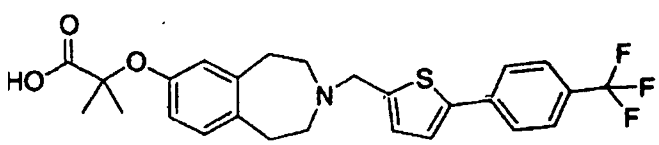 |
| 9 | 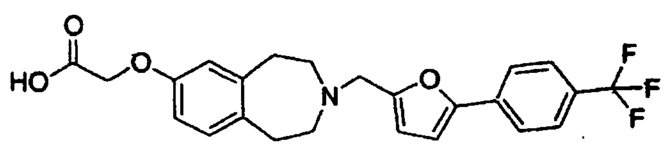 |
| 10 | 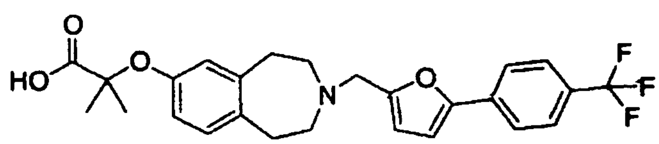 |

(continued)

| Cpd No. | Structure |
|---|---|
| 11 | 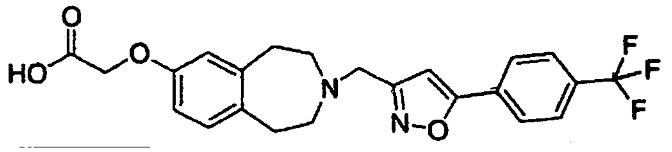 |
| 12 | 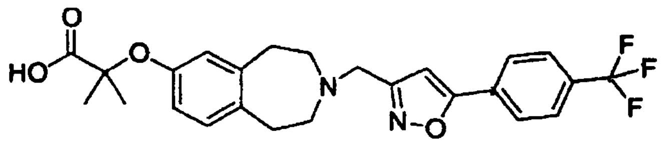 |
| 13 | 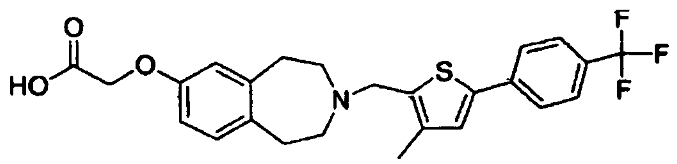 |
| 14 | 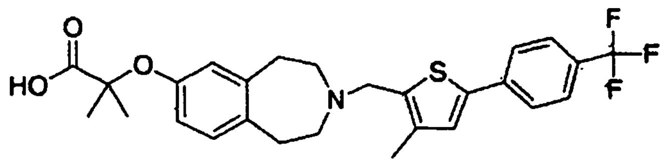 |
| 15 | 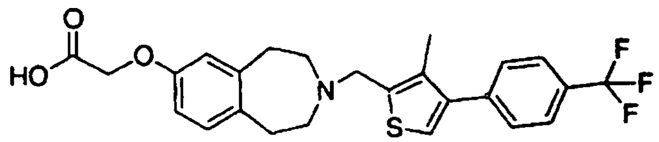 |
| 16 | 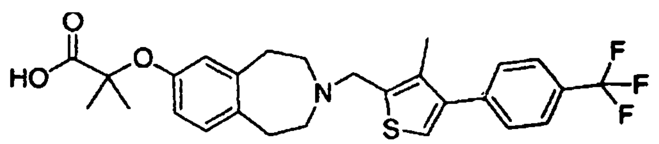 |
| 17 | 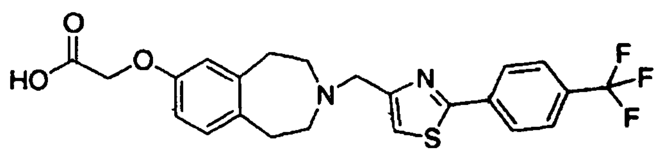 |
| 18 | 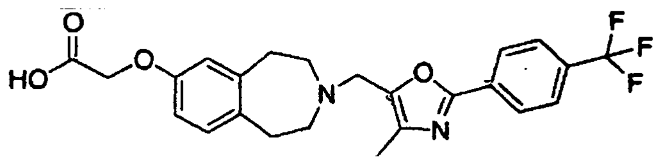 |
| 19 | 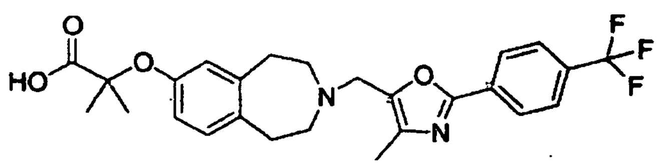 |
| 20 | 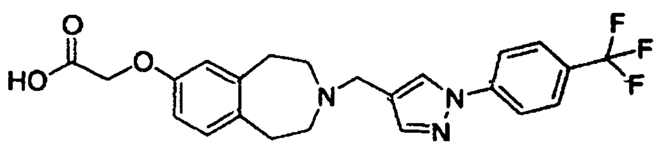 |

(continued)

| Cpd No. | Structure |
|---|---|
| 21 | 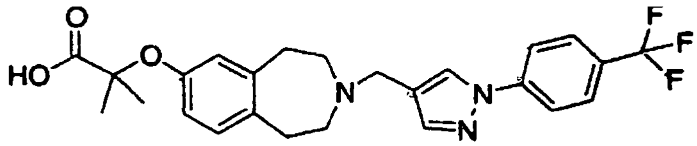 |
| 22 | 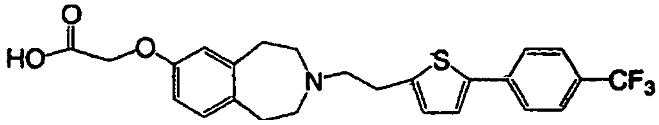 |
| 23 | 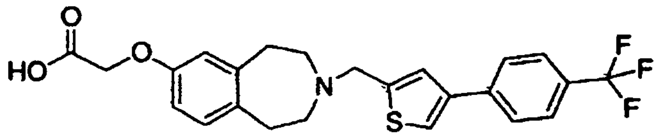 |
| 24 | 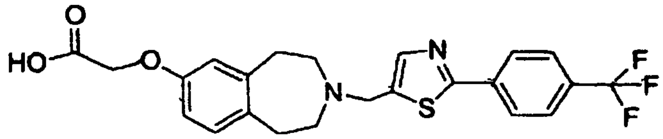 |
| 25 | 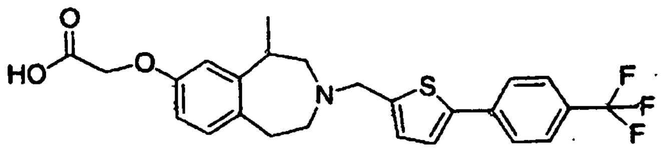 |
| 26 | 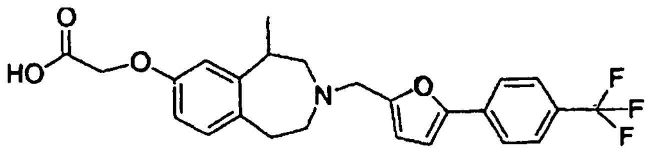 |
| 27 | 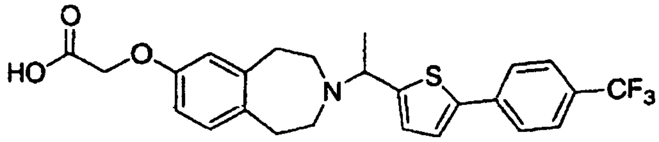 |
| 27a | 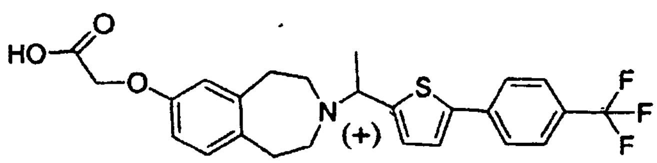 |
| 27b | 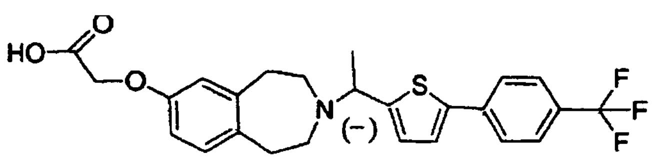 |
| 28 | 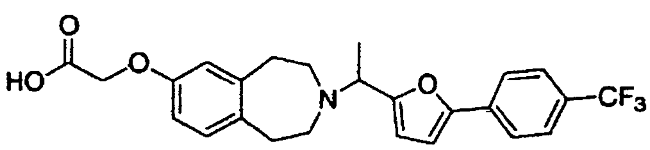 |

(continued)

| Cpd No. | Structure |
|---|---|
| 28a | 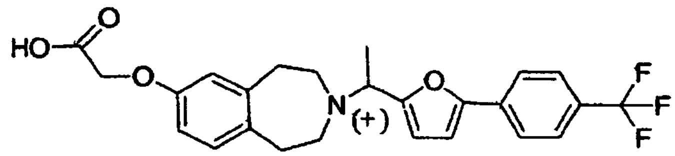 |
| 28b | 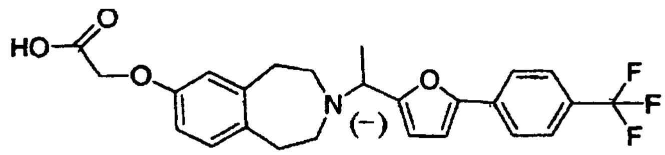 |
| 29 | 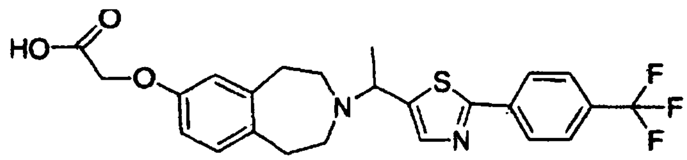 |
| 30 | 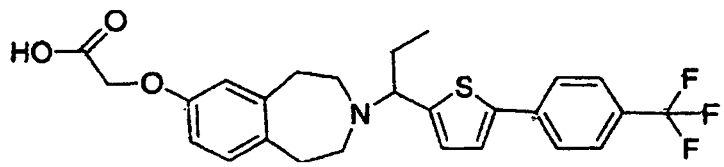 |
| 31 | 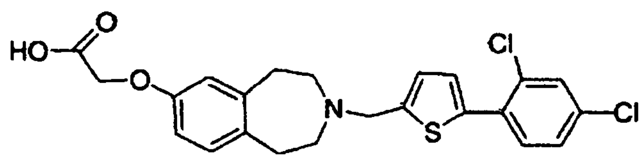 |
| 32 | 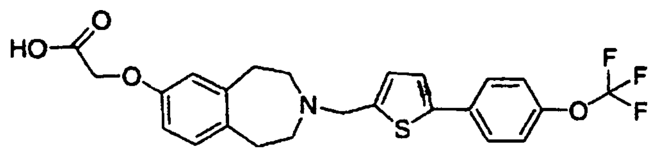 |
| 33 | 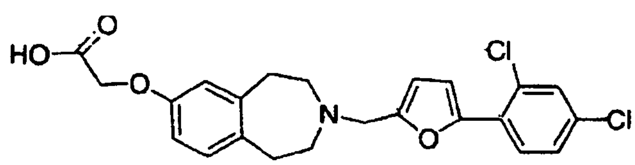 |
| 34 | 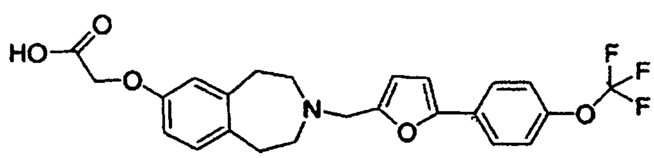 |
| 35 | 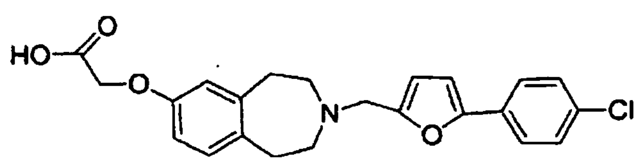 |

(continued)

| Cpd No. | Structure |
|---|---|
| 36 | 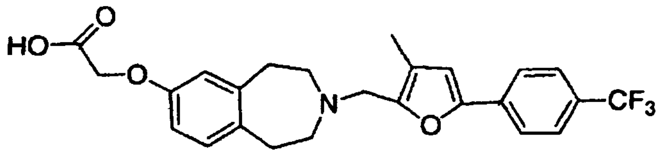 |
| 37 | 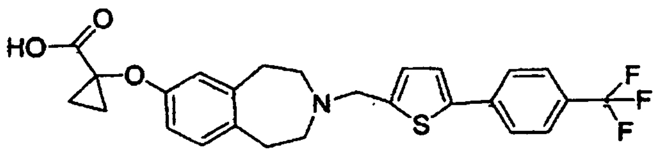 |
| 38 | 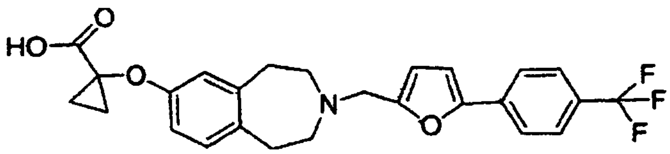 |
| 39 | 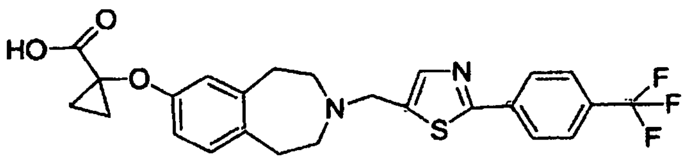 |
| 40 | 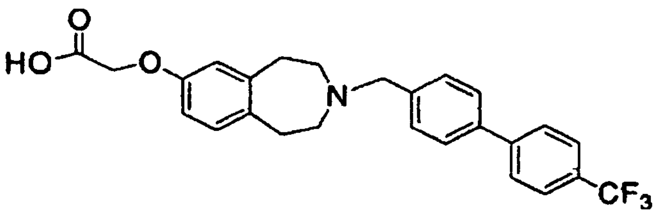 |
| 41 | 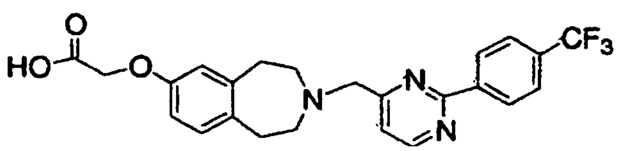 |
| 42 | 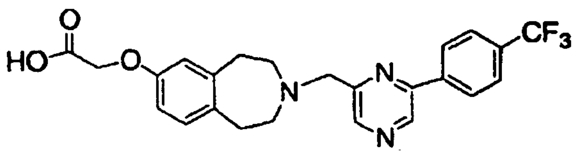 |
| 43 | 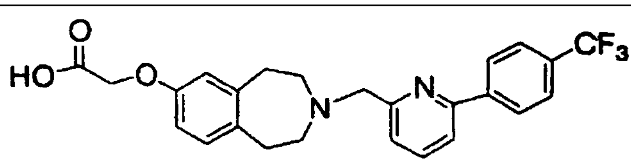 |
| 44 | 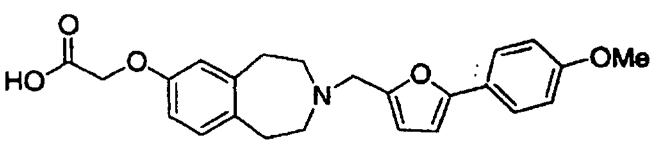 |

(continued)

| Cpd No. | Structure |
|---|---|
| 45 | 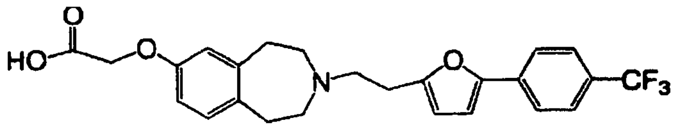 |
| 46 | 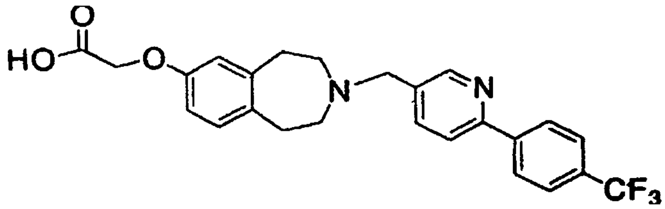 |
| 47 | 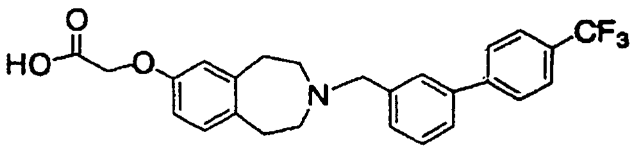 |

Biological Examples

Example 1

***Transfection assay method for PPAR* α, γ or δ *receptors***

HEK293 cells were grown in DMEM/F12 medium supplemented with 10% FBS and glutamine (Invitrogen) and incubated in a 5% $CO_2$ incubator at 37°C. The cells were co-transfected using DMRIE-C reagent (Invitrogen) in serum free medium (Opti-MEM, Invitrogen) with two mammalian expression plasmids, one containing the DNA sequence coding for the ligand binding domains of either PPARα, γ or δ fused to the yeast GAL4 DNA binding domain and the other containing the promoter sequence of the yeast GAL4 (UAS) fused to the firefly luciferase cDNA reporter. The next day, the medium was changed to DMEM/F12 medium supplemented with 5% charcoal treated serum (Hyclone) and glutamine. After 6 hrs the cells were trypsinized and seeded at a density of 50,000 cells/well into 96 well plates and incubated overnight as above. The cells were then treated with test compounds or vehicle and incubated for 18-24 hrs as above. Luciferase reporter activity was measured using the Steady-Glo Luciferase Assay Kit from Promega. DMRIE-C Reagent was purchased from GIBCO Cat. No. 10459-014. OPTI-MEM I Reduced Serum Medium was purchased from GIBCO (Cat. No. 31985). Steady-Glo Luciferase Assay Kit was purchased from Promega (Part# E254B).

A variety of example compounds have been made and tested, with a range of in vitro results. Below, in Table 2, are representative compounds and data; in some cases, where multiple $EC_{50}$'s are shown, multiple measurements were taken. Naturally, different compounds in Formula (I) may have not activities identical to any one compound below.

Table 2. In vitro data of PPARdelta agonists

| Cpd No. | PPARδ $EC_{50}$ (nM) | PPARγ $EC_{50}$ (nM) | PPARα $EC_{50}$ (nM) |
|---|---|---|---|
| 1 | 34.1<br>66.6 | >3000<br>>3000 | 546 758 |
| 2 | 161.8 | >3000 | >3000 |
| 3 | 142.9 | >3000 | >3000 |
| 4 | 95.2 | >3000 | >3000 |
| 5 | 51.6 | >3000 | >3000 |
| 6 | 243.1 | >3000 | >1000 |
| 7 | 14.5<br>18.9 | >3000<br>>3000 | >3000 |

(continued)

| Cpd No. | PPARδ $EC_{50}$ (nM) | PPARγ $EC_{50}$ (nM) | PPARα $EC_{50}$ (nM) |
|---|---|---|---|
| 8 | 85.9<br>122.7 | >3000 | >1000 |
| 9 | 74.2<br>70.9 | >3000<br>>3000 | >3000 |
| 10 | 53.0<br>87.7 | >3000<br>>3000 | >3000 |
| 11 | 135.8 | >3000 | >1000 |
| 12 | 121.7 | >3000 | >1000 |
| 13 | 174.3<br>193 | >3000<br>>3000 | >3000 |
| 14 | 397.8 | >3000 | >3000 |
| 15 | 103.6<br>104 | >3000 | >3000 |
| 16 | 375.4 | >3000 | >3000 |
| 17 | 439.2 | >3000 | >3000 |
| 18 | 174.4 | >3000 | >3000 |
| 19 | 230.6 | >3000 | >3000 |
| 20 | >1000 | >3000 | >3000 |
| 21 | 421.7 | >3000 | >3000 |
| 22 | >3000 | >3000 | >3000 |
| 23 | 109.3<br>70.2 | >3000<br>>3000 | >3000 |
| 24 | 27.1<br>22.0 | >3000<br>>3000 | >1000 |
| 25 | 11.0<br>10.8 | >3000<br>>3000 | >1000 |
| 26 | 15.8 | >3000 | >3000 |
| 27 | <3<br>1.4 | >3000<br>>3000 | >3000 |
| 27a | 0.92 | >1000 | >3000 |
| 27b | 12.6<br>13.9 | >3000<br>>3000 | >3000<br>>3000 |
| 28 | <3 | >3000 | >3000 |
| 28a | 1.8 | >3000 | >3000 |
| 28b | 5.4<br>9.8 | >3000<br>>3000 | >3000<br>>3000 |
| 29 | <3 | 622 | >3000 |
| 30 | 0.9 | >3000 | >3000 |
| 31 | 237 | >3000 | >3000 |
| 32 | 157.0 | >3000 | >3000 |

(continued)

| Cpd No. | PPARδ $EC_{50}$ (nM) | PPARγ $EC_{50}$ (nM) | PPARα $EC_{50}$ (nM) |
|---|---|---|---|
| 33 | 147.9<br>167 | >3000<br>>3000 | >3000<br>>3000 |
| 34 | 372.8 | >3000<br>>3000 | >3000<br>>3000 |
| 35 | 169.2 | >3000 | >3000 |
| 36 | 29.2 | >3000 | >3000 |
| 37 | 5.3 | >3000 | >3000 |
| 38 | 18 | >1000 | 31 |
| 39 | 12 | >1000 | >1000 |
| 40 | >1000 | >3000 | >3000 |
| 41 | >1000 | >3000 | >3000 |
| 42 | >1000 | >3000 | >3000 |
| 43 | >3000 | >3000 | >3000 |
| 44 | >1000 | >3000 | >3000 |
| 45 | >1000 | >3000 | >3000 |
| 46 | 312.3 | >3000 | >1000 |
| 47 | >3000 | >3000 | >3000 |

Example 2

***Rat Study of Compound 7***

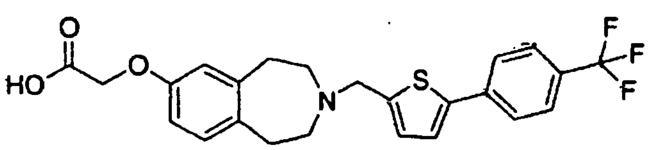

Compound 7

Rats carry the majority of serum cholesterol in the HDL lipoprotein fraction while humans carry the majority of serum cholesterol in LDL and VLDL lipoproteins. To mimic a human hypercholesterolemic state in a rodent model, Sprague Dawley rats were fed a diet high in cholesterol (Research Diets, C13002) for 6 days before treatment with the compounds for another 8 days while remaining on the diet. Under this dietary regimen, the vehicle controls (HC: High Cholesterol) typically have serum total cholesterol and LDL-C levels that are increased by 3-8 fold and serum HDL-C is decreased by approximately 30-50% compared with chow diet (lean) controls.

Treatment of Sprague Dawley rats fed the high cholesterol diet with Compound 7 for 8 days significantly increased HDL-C levels, achieving the levels of the chow fed controls (Chow). There were also significant decreases in serum total cholesterol and LDL-C levels. Compound 7 had no effect on serum triglycerides or liver weights. There were dose-related increases in drug plasma levels (Cmax and AUC) and there may be accumulation after multiple doses as evidenced by the differences in Cmax and AUC between the single and multiple doses at 3mg/kg. Data from this study are shown in Tables 3 and 4 below.

Table 3. Compound 7 *in vivo* data (I)

| Dose (mg/kg) | HDL-C (mg/dL) | LDL-C (mg/dL) | Total Cholesterol (mg/dL) | Triglycerides (mg/dL) | Liver Weights |
|---|---|---|---|---|---|
| Veh (Chow) | 38.0±1.1 | 10.4±0.8 | 59.0±2.3 | 168.9±13.0 | |
| Veh (HC) | 23.7±3.3 | 35.8±7.3 | 260.0±32.1 | 263.7±21.3 | 25.1±0.4 |
| 0.1 | 24.2±2.9 | 32.7±4.7 | 239.0±22.4 | 269.8±27.8 | 24.9±20.8 |
| 0.3 | 29.6±1.4 | 31.2±1.5 | 233.2±9.5 | 264.7±29.8 | 25.0±0.8 |
| 1 | 31.8±0.9* | 24.4±1.3 | 173.3±7.9* | 237.7±25.4 | 23.9±0.5 |
| 3 | 39.6±1.6** | 20.6±1.5* | 152.2±8.5** | 299.5±35.9 | 27.2±0.9 |
| *p<0.05<br>**p<0.001 | | | | | |

Table 4. Compound 7 *in vivo* data (II)

| Dose | Dose Interval | t½ (hr) | $T_{max}$ (ng/mL) | $C_{max}$ (ng/mL) | AUC $_{0-24}$ (ng-hr/mL) |
|---|---|---|---|---|---|
| 0.1[b] | Multiple | ~13 | 2.5 | 57.0 | 772 |
| 0.3 | Multiple | ~22 | 3.0 | 124 | 1640 |
| 1[b] | Multiple | ND | 4.0 | 595 | 10875 |
| 3 | Multiple | ~36 | 5.0 | 2030 | 37100 |
| 3[b] | Single | ~29 | 5.5 | 733 | 14200 |
| ND - t½ could not be determined<br>b: n=2 | | | | | |

References:

Auboeuf et al., 1997, Diabetes 46(8):1319-1327
Braissant et al., 1996, Endocrinology 137(1): 354-366
Barak et al, 2002, Proc. Natl. Acad. Sci. USA 99(1):303-308
Lawn et al., 1999, J. Clin. Investigation 104(8): R25-R31
Leibowitz et al., 2000, FEBS Lett. 473(3):333-336
Oliver et al., 2001, Proc. Natl. Acad. Sci. URSA 98(9):5306-5311
Tanaka et al, 2003, Proc. Natl. Acad. Sci. USA 100(26):15924-15929
Wang et al., 2003, Cell 113:159-170

## Claims

**1.** A compound of Formula (I)

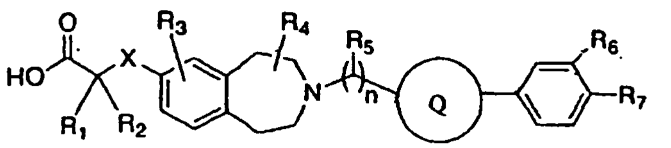

Formula (I)

wherein:

X is a covalent bond, O, or S;

$R_1$ and $R_2$ are independently selected from the group consisting of H, $C_{1-8}$alkyl, and substituted $C_{1-8}$alkyl, or $R_1$, $R_2$ and the carbon atom to which they are attached together may form $C_{3-7}$cycloalkyl;

$R_3$ is H;

$R_4$ and $R_5$ are independently selected from the group consisting of H, halo, $C_{1-8}$alkyl, $C_{3-7}$cycloalkyl, $C_{3-7}$cycloalkyl-$C_{1-4}$alkyl, $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{6-10}$aryl substituted $C_{1-4}$alkyl, cyano substituted $C_{1-4}$alkyl, and hydroxy substituted $C_{1-4}$alkyl;

$R_6$ and $R_7$ are independently selected from the group consisting of H, halo, $C_{1-3}$alkyl, halo substituted $C_{1-3}$alkyl, $C_{1-3}$alkoxy, and halo substituted $C_{1-3}$alkoxy;

n is 1; and

Q is selected from the group consisting of

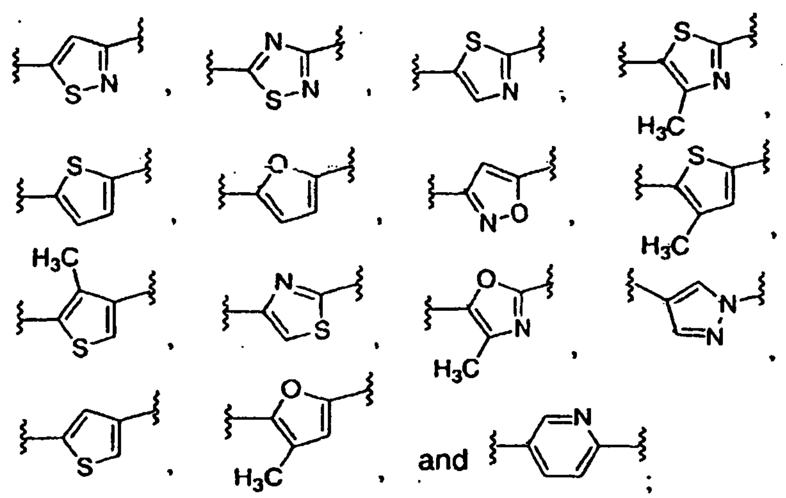

, , , , , , , , , , , , , , and ;

and enantiomers, diastereomers, tautomers, solvates, or pharmaceutically acceptable salts thereof.

**2.** The compound according to claim 1 wherein $R_1$ and $R_2$ are independently selected from the group consisting of H and $C_{1-8}$alkyl, or $R_1$, $R_2$ and the carbon atom to which they are attached together may form $C_{3-5}$cycloalkyl.

**3.** The compound according to claim 1 wherein $R_1$ and $R_2$ are independently selected from the group consisting of H and $CH_3$, or $R_1$, $R_2$ and the carbon atom to which they are attached together may form

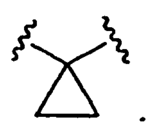

.

**4.** The compound according to claim 1 wherein $R_6$ and $R_7$ are independently selected from the group consisting of H, halo, halo substituted $C_{1-3}$alkyl, $C_{1-3}$alkoxy, and halo substituted $C_{1-3}$alkoxy.

**5.** The compound according to claim 1 wherein $R_6$ is H and $R_7$ is selected from the group consisting of halo, halo substituted $C_{1-3}$alkyl, and halo substituted $C_{1-3}$alkoxy.

**6.** The compound according to claim 5 wherein $R_7$ is selected from the group consisting of F, $CF_3$, and -O-$CF_3$.

**7.** The compound according to claim 1 wherein $R_4$ and $R_5$ are independently selected from the group consisting of H and $C_{1-8}$alkyl.

**8.** The compound according to claim 1 wherein $R_5$ is H, $CH_3$, or - $CH_2CH_3$.

**9.** The compound according to claim 1 wherein Q is selected from the group consisting of

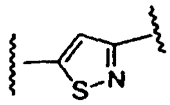, 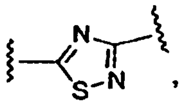, 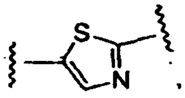, 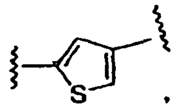,

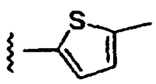, 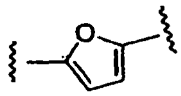, 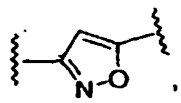, 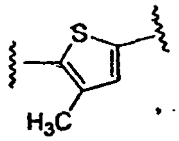,

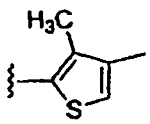, 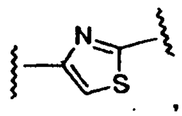, 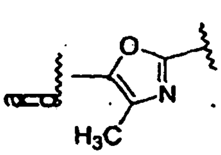.

**10.** The compound according to claim 1 wherein Q is selected from the group consisting of

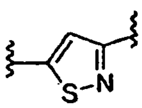, 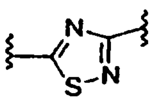, 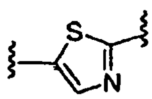, 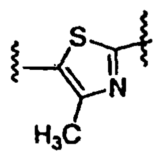,

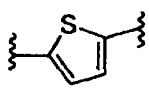, 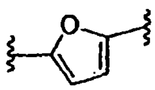, 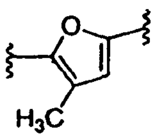 and 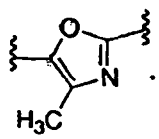.

**11.** The compound according to claim 1 wherein X is O.

**12.** A compound according to claim 1 wherein

X is O;

$R_1$, $R_2$, $R_4$, and $R_5$ are independently selected from the group consisting of H and $C_{1-3}$alkyl, or $R_1$, $R_2$ and the carbon atom to which they are attached together may form

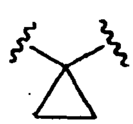;

and

$R_6$ and $R_7$ are independently selected from the group consisting of H, halo, $C_{1-3}$alkoxy, and halo substituted $C_{1-3}$alkyl, and halo substituted $C_{1-3}$alkoxy;

and enantiomers, diastereomers, tautomers, solvates, or pharmaceutically acceptable salts thereof.

**13.** A compound of Formula (Ia) according to claim 1

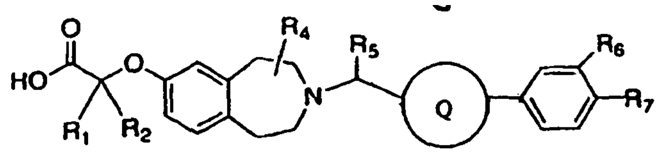

**Formula (Ia)**

wherein

$R_1$ and $R_2$ are independently selected from the group consisting of H and $C_{1-8}$alkyl, or $R_1$, $R_2$ and the carbon atom to which they are attached together may form $C_{3-5}$cycloalkyl;
$R_6$ and $R_7$ are independently selected from the group consisting of H, $C_{1-3}$alkyl, halo, and halo substituted $C_{1-3}$alkyl;
$R_4$ and $R_5$ are independently selected from the group consisting of H and $C_{1-8}$alkyl; and
Q is selected from the group consisting of

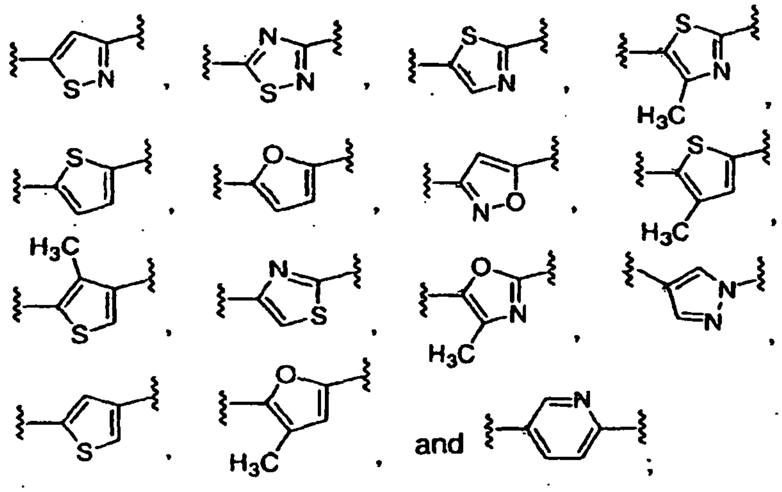

, , , , , , , , , , , , , , and ;

and enantiomers, diastereomers, tautomers, solvates, or pharmaceutically acceptable salts thereof.

**14.** A compound of Formula (Ib) according to claim 1

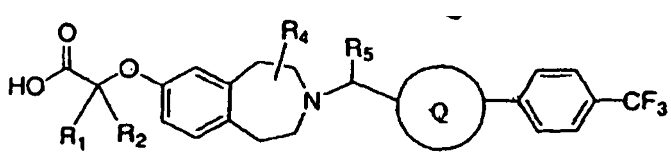

**Formula (Ib)**

wherein

$R_1$ and $R_2$ are independently selected from the group consisting of H and $CH_3$, or $R_1$, $R_2$ and the carbon atom to which they are attached together may form

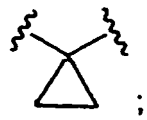

$R_4$ and $R_5$ are independently selected from the group consisting of H, $CH_3$, and $-CH_2CH_3$; and Q is selected from the group consisting of

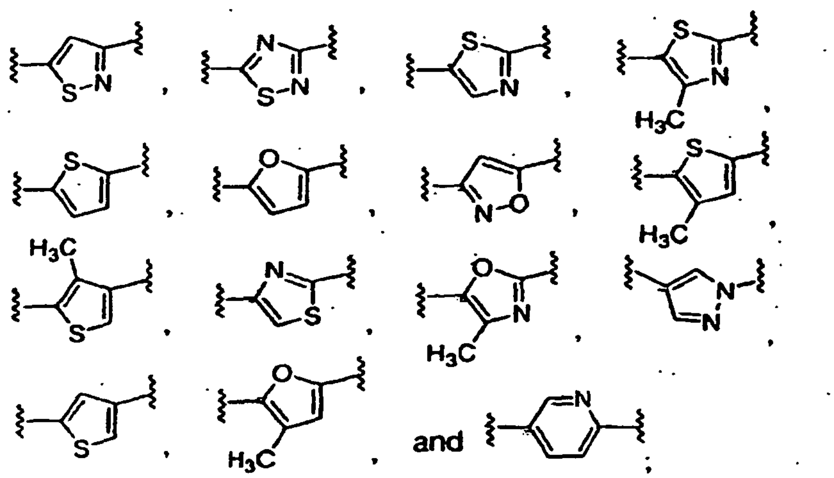

and enantiomers, diastereomers, tautomers, solvates, or pharmaceutically acceptable salts thereof.

**15.** A compound of Formula (Ic) according to claim 1

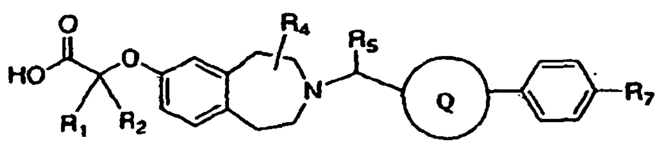

**Formula (Ic)**

wherein $R_1$, $R_2$, and $R_4$, are independently selected from the group consisting of H and $CH_3$, or $R_1$, $R_2$ and the carbon atom to which they are attached together may form

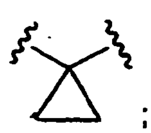

$R_5$ is selected from the group consisting of H, $CH_3$, and $-CH_2CH_3$;
$R_7$ is halo or halo substituted $C_{1-3}$alkyl; and
Q is selected from the group consisting of

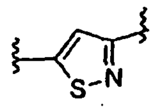, 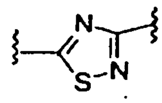, 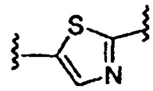, 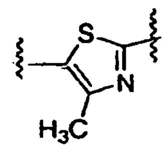,

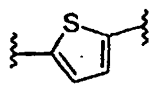, 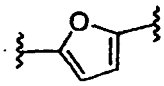, 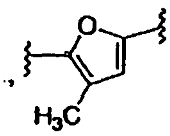 and 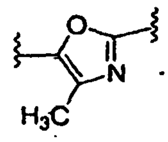.

and enantiomers, diastereomers, tautomers, solvates, or pharmaceutically acceptable salts thereof.

**16.** A compound according to claim 15 wherein:

(a) $R_1$ or $R_2$ is H;
(b) $R_1$ and $R_2$ are both H;
(c) $R_1$ or $R_2$ is $CH_3$;
(d) $R_1$ and $R_2$ are both $CH_3$;
(e) $R_1$, $R_2$ and the carbon atom to which they are attached together form

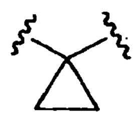

(f) $R_4$ is H or $CH_3$;
(g) $R_5$ is H, $CH_3$, or-$CH_2CH_3$;
(h) $R_1$ or $R_2$ is H and $R_4$ is H or $CH_3$;
(i) $R_1$ and $R_2$ are both H and $R_4$ is H or $CH_3$;
(j) $R_1$ or $R_2$ is H and $R_5$ is H, $CH_3$, or-$CH_2CH_3$;
(k) $R_1$ and $R_2$ are both H and $R_5$ is H, $CH_3$, or $-CH_2CH_3$;
(l) $R_1$ or $R_2$ is $CH_3$ and $R_5$ is H, $CH_3$, or $-CH_2CH_3$;
(m) $R_1$ and $R_2$ are both $CH_3$ and $R_5$ is H, $CH_3$, or $-CH_2CH_3$;
(n) $R_1$ or $R_2$ is H, $R_4$ is H, and $R_5$ is H, $CH_3$, or $-CH_2CH_3$;
(o) $R_1$ and $R_2$ are both H, $R_4$ is H, and $R_5$ is H, $CH_3$, or $CH_2CH_3$;
(p) $R_1$ or $R_2$ is $CH_3$, $R_4$ is H, and $R_5$ is H, $CH_3$, or $-CH_2H_3$;
(q) $R_1$ and $R_2$ are both $CH_3$, $R_4$ is H, and $R_5$ is H, $CH_3$, or $-CH_2CH_3$;
(r) $R_1$, $R_2$ and the carbon atom to which they are attached together form

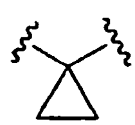,

$R_4$ is H, and $R_5$ is H, $CH_3$, or $-CH_2CH_3$;
(s) $R_7$ is $CF_3$;
(t) $R_7$ is Cl;
(u) $R_1$ or $R_2$ is H, $R_7$ is $CF_3$, and $R_4$ is H or $CH_3$;
(v) $R_1$ and $R_2$ are both H, $R_7$ is $CF_3$, and $R_4$ is H or $CH_3$;
(w) $R_1$ or $R_2$ is H, $R_7$ is $CF_3$, and $R_5$ is H, $CH_3$, or $-CH_2CH_3$;
(x) $R_1$ and $R_2$ are both H, $R_7$ is $CF_3$, and $R_5$ is H, $CH_3$, or $-CH_2CH_3$;
(y) $R_1$ or $R_2$ is $CH_3$, $R_7$ is $CF_3$, and $R_5$ is H, $CH_3$, or $-CH_2CH_3$;

(z) $R_1$ and $R_2$ are both $CH_3$, $R_7$ is $CF_3$, and $R_5$ is H, $CH_3$, or $-CH_2CH_3$;
(aa) $R_1$ or $R_2$ is H, $R_4$ is H, $R_7$ is $CF_3$, and $R_5$ is H, $CH_3$, or $-CH_2CH_3$;
(bb) $R_1$ and $R_2$ are both H, $R_4$ is H, $R_7$ is $CF_3$, and $R_5$ is H, $CH_3$, or $-CH_2CH_3$;
(cc) $R_1$ or $R_2$ is $CH_3$, $R_4$ is H, $R_7$ is $CF_3$, and $R_5$ is H, $CH_3$, or $-CH_2CH_3$;
(dd) $R_1$ and $R_2$ are both $CH_3$, $R_4$ is H, $R_7$ is $CF_3$, and $R_5$ is H, $CH_3$, or $-CH_2CH_3$;
(ee) $R_1$, $R_2$ and the carbon atom to which they are attached together form

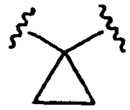,

$R_4$ is H, $R_7$ is $CF_3$, and $R_5$ is H, $CH_3$, or $-CH_2CH_3$;
(ff) Q is selected from the group consisting of

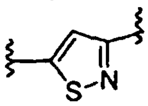, 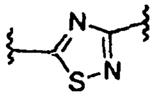, 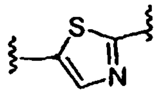, 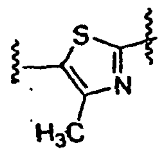,

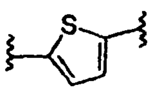, 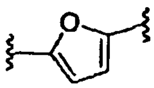, 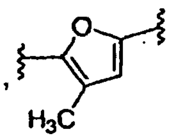 and 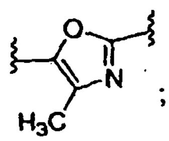;

(gg) $R_1$ or $R_2$ is H and Q is selected from the group consisting of

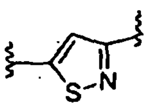, 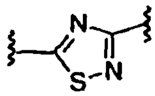, 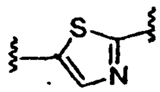, 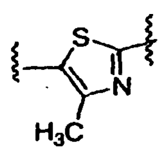,

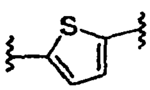, 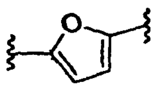, 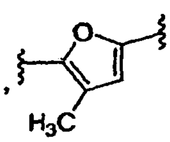 and 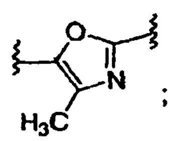;

(hh) $R_1$ and $R_2$ are both H and Q is selected from the group consisting of

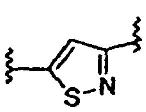, 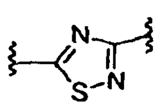, 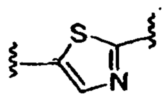, 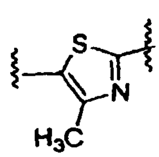,

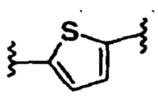, 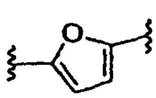, 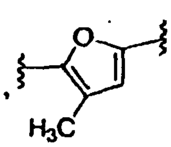 and 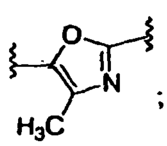;

(ii) $R_1$ or $R_2$ is $CH_3$ and Q is selected from the group consisting of

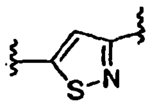, 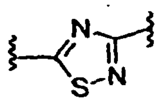, 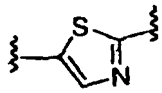, 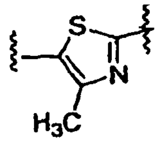,

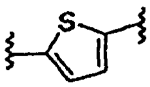, 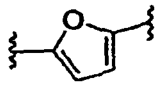, 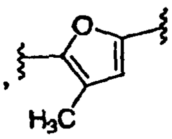 and 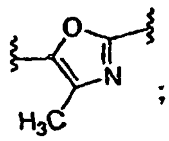;

(ii) $R_1$ and $R_2$ are both $CH_3$ and Q is selected from the group consisting of

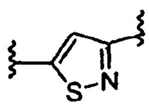, 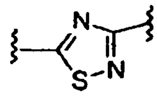, 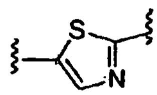, 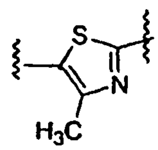,

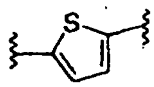, 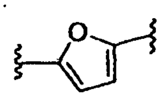, 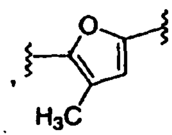 and 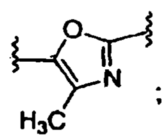;

(kk) $R_4$ is H or $CH_3$ and Q is selected from the group consisting of

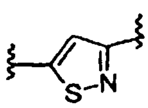, 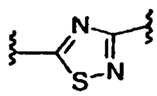, 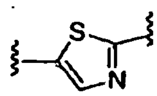, 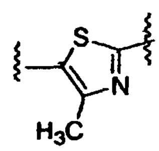,

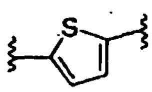, 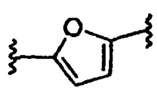, 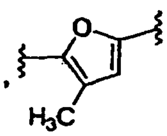 and 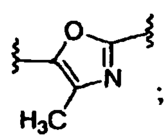;

(ll) $R_5$ is H, $CH_3$, or $-CH_2CH_3$ and Q is selected from the group consisting of

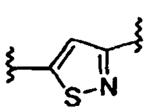, 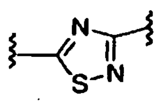, 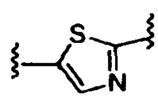, 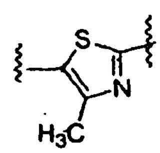,

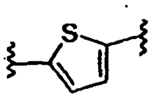, 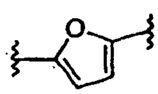, 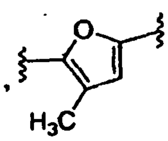 and 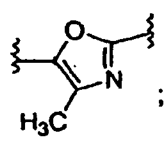;

(<u>mm</u>) $R_1$ or $R_2$ is H and $R_4$ is H or $CH_3$ and Q is selected from the group consisting of

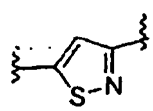, 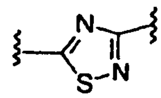, 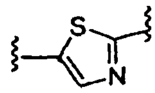, 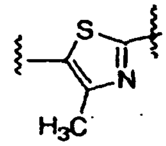,

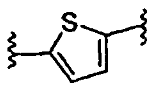, 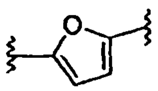, 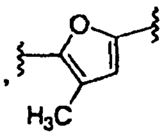 and 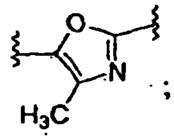;

(<u>nn</u>) $R_1$ and $R_2$ are both H and $R_4$ is H or $CH_3$ and Q is selected from the group consisting of

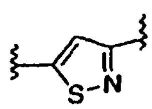, 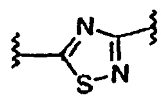, 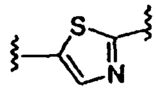, 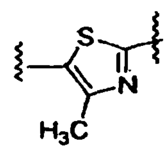,

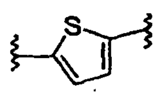, 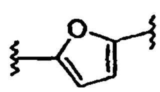, 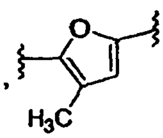 and 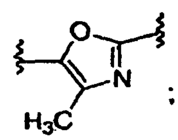;

(<u>oo</u>) $R_1$ or $R_2$ is H and $R_5$ is H, $CH_3$, or $-CH_2CH_3$ and Q is selected from the group consisting of

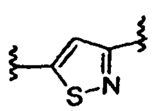, 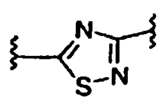, 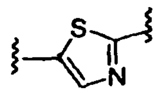, 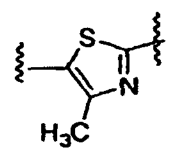,

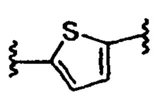, 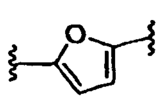, 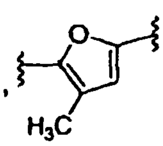 and 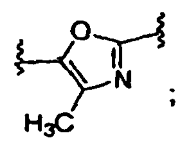;

(<u>pp</u>) R, and $R_2$ are both H and $R_5$ is H, $CH_3$, or $-CH_2CH_3$ and Q is selected from the group consisting of

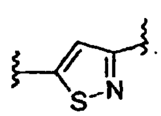, 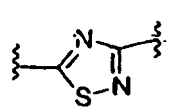, 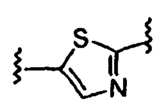, 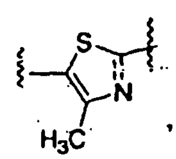,

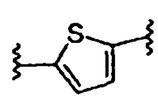, 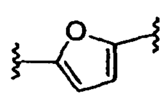, 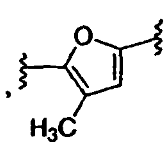 and 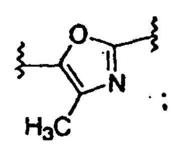;

(<u>qq</u>) $R_1$ or $R_2$ is $CH_3$ and $R_5$ is H, $CH_3$, or $-CH_2CH_3$ and Q **is** selected from the group consisting of

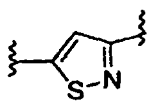, 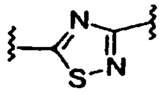, 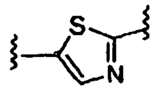, 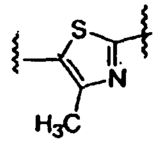,

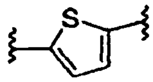, 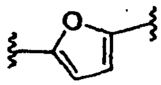, 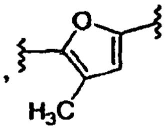 and 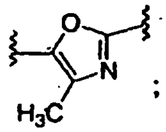;

(rr) $R_1$ and $R_2$ are both $CH_3$, $R_5$ is H, $CH_3$, or $-CH_2CH_3$, and Q is selected from the group consisting of

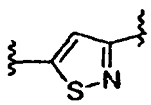, 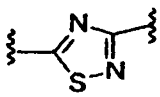, 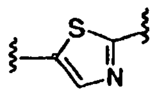, 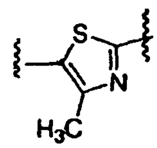,

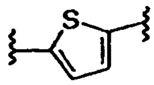, 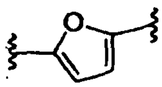, 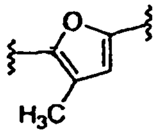 and 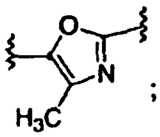;

(ss) $R_1$ or $R_2$ is H, $R_4$ is H, $R_5$ is H, $CH_3$, or $-CH_2CH_3$, and Q is selected from the group consisting of

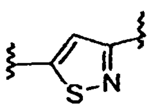, 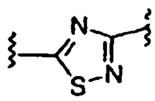, 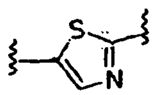, 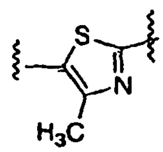,

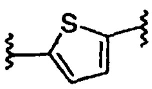, 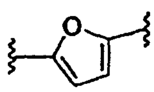, 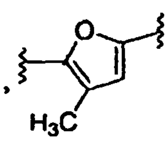 and 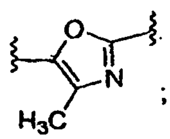;

(tt) $R_1$ and $R_2$ are both H, $R_4$ is H, $R_5$ is H, $CH_3$, or $-CH_2CH_3$, and Q is selected from the group consisting of

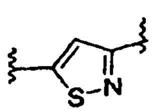, 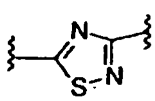, 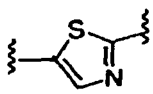, 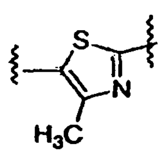,

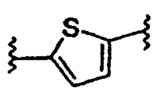, 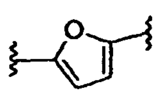, 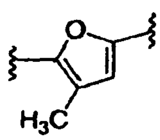 and 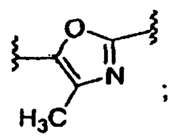;

(uu) $R_1$ or $R_2$ is $CH_3$, $R_4$ is H, $R_5$ is H, $CH_3$, or $-CH_2CH_3$, and Q is selected from the group consisting of

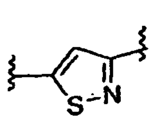 , 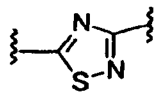 , 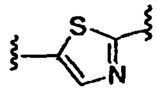 , 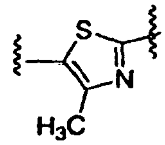 ,

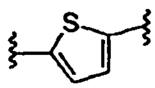 , 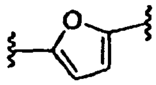 , 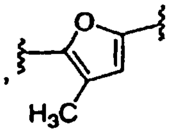 and 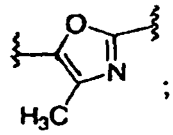 ;

(vv) $R_1$ and $R_2$ are both $CH_3$, $R_4$ is H, $R_5$ is H, $CH_3$, or $-CH_2CH_3$, and Q is selected from the group consisting of

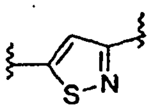 , 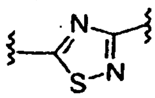 , 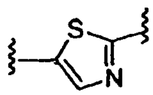 , 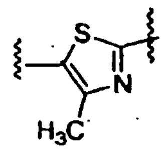 ,

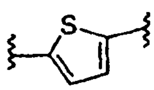 , 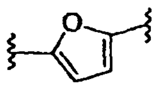 , 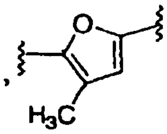 and 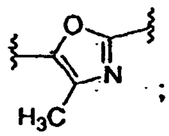 ;

(ww) $R_1$, $R_2$ and the carbon atom to which they are attached together form

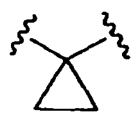 ,

$R_4$ is H, $R_5$ is H, $CH_3$, or $-CH_2CH_3$, and Q is selected from the group consisting of

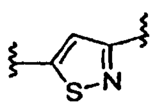 , 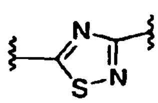 , 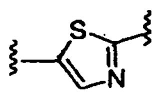 , 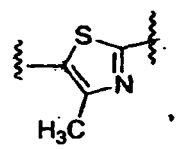 ,

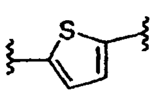 , 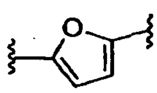 , 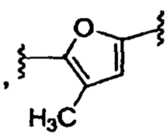 and 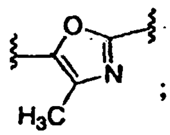 ;

(xx) $R_7$ is $CF_3$ and Q is selected from the group consisting of

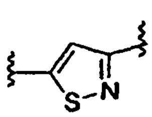 , 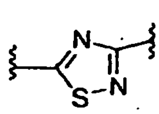 , 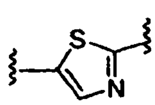 , 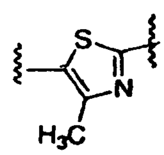 ,

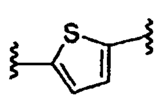, 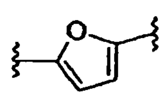, 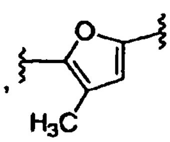 and 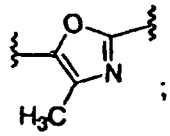;

(yy) $R_7$ is Cl and Q is selected from the group consisting of

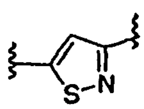, 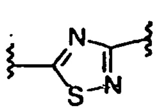, 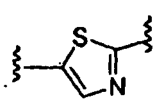, 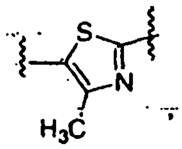,

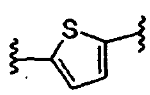, 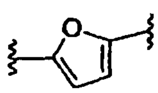, 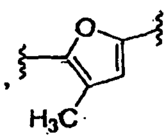 and 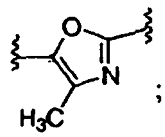;

(zz) $R_1$ or $R_2$ is H, $R_7$ is $CF_3$, $R_4$ is H or $CH_3$, and Q is selected from the group consisting of

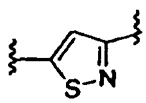, 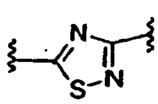, 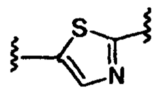, 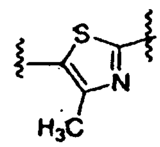,

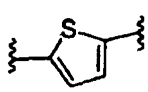, 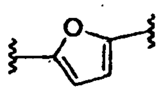, 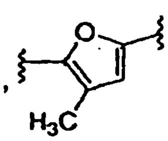 and 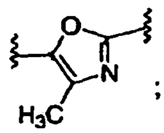;

(aaa) $R_1$ and $R_2$ are both H, $R_7$ is $CF_3$, $R_4$ is H or $CH_3$, and Q is selected from the group consisting of

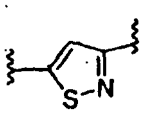, 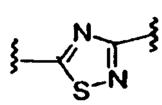, 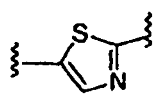, 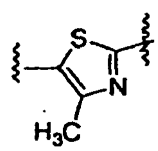,

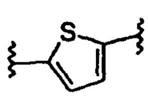, 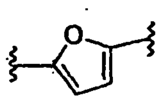, 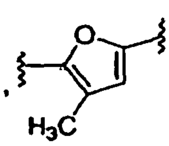 and 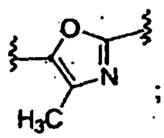;

(bbb) $R_1$ or $R_2$ is H, $R_7$ is $CF_3$, $R_5$ is H, $CH_3$, or $-CH_2CH_3$, and Q is selected from the group consisting of

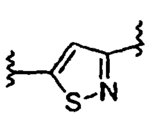, 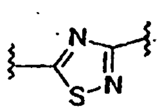, 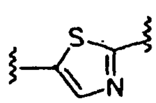, 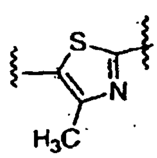,

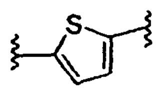, 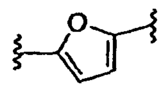, 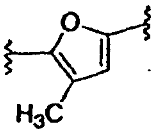 and 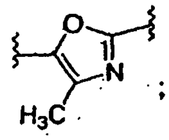;

(ccc) R, and $R_2$ are both H, $R_7$ is $CF_3$, $R_5$ is H, $CH_3$, or $-CH_2CH_3$, and Q is selected from the group consisting of

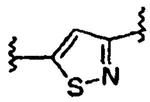, 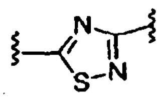, 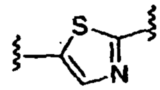, 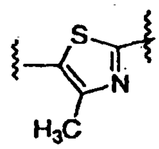,

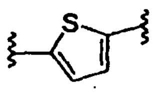, 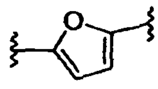, 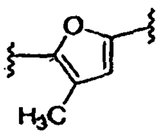 and 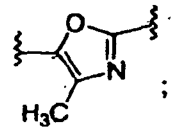;

(ddd) $R_1$ or $R_2$ is $CH_3$, $R_7$ is $CF_3$, $R_5$ is H, $CH_3$, or $-CH_2CH_3$, and Q is selected from the group consisting of

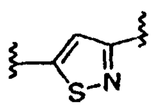, 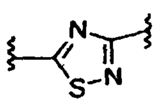, 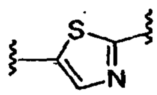, 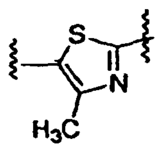,

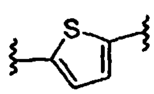, 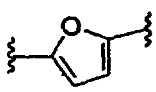, 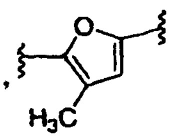 and 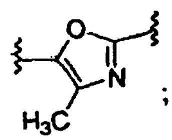;

(eee) $R_1$ and $R_2$ are both $CH_3$, $R_7$ is $CF_3$, $R_5$ is H, $CH_3$, or $-CH_2CH_3$, and Q is selected from the group consisting of

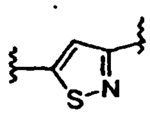, 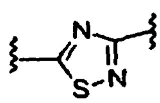, 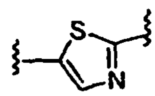, 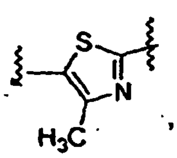,

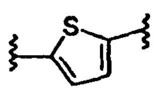, 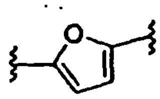, 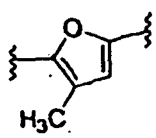 and 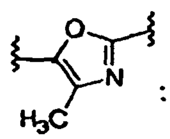;

(fff) $R_1$ or $R_2$ is H, $R_4$ is H, $R_7$ is $CF_3$, $R_5$ is H, $CH_3$, or $-CH_2CH_3$, and Q is selected from the group consisting of

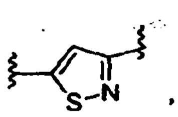, 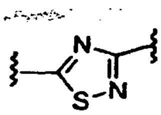, 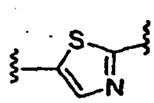, 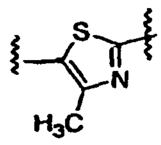,

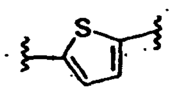, 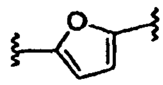, 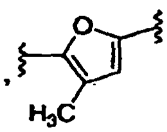 and 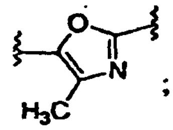;

(ggg) $R_1$ and $R_2$ are both H, $R_4$ is H, $R_7$ is $CF_3$, $R_5$ is H, $CH_3$, or - $CH_2CH_3$, and Q is selected from the group consisting of

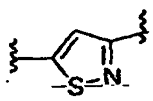, 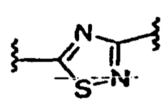, 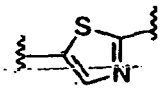, 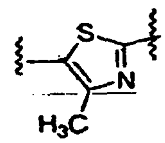,

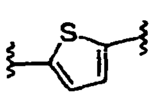, 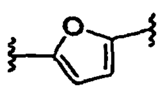, 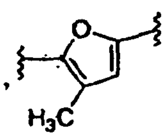 and 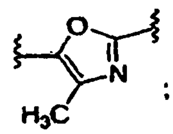;

(hhh) $R_1$ or $R_2$ is $CH_3$, $R_4$ is H, $R_7$ is $CF_3$, $R_5$ is H, $CH_3$, or -$CH_2CH_3$, and Q is selected from the group consisting of

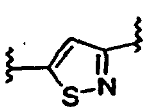, 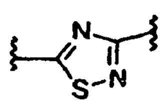, 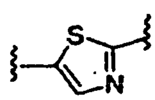, 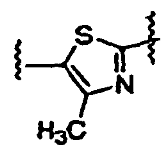,

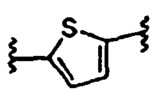, 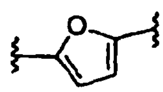, 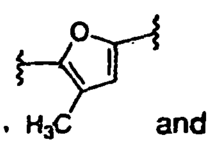 and 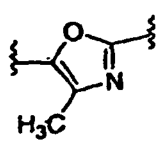;

or

(iii) $R_1$ and $R_2$ are both $CH_3$, $R_4$ is H, $R_7$ is $CF_3$, $R_5$ is H, $CH_3$, or-$CH_2CH_3$, and Q is selected from the group consisting of

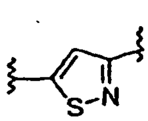, 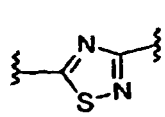, 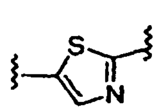, 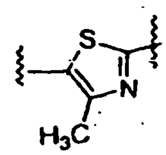,

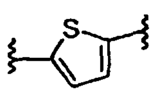, 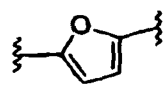, 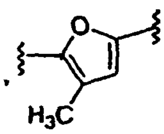 and 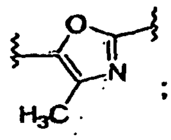;

and enantiomers, diastereomers, tautomers, solvates, or pharmaceutically acceptable salts thereof.

**17.** A compound according to claim 1 selected from the group consisting of

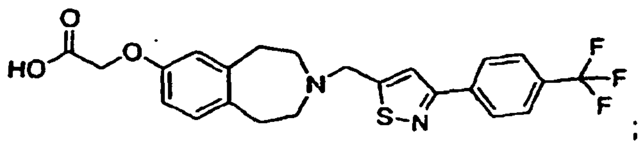;

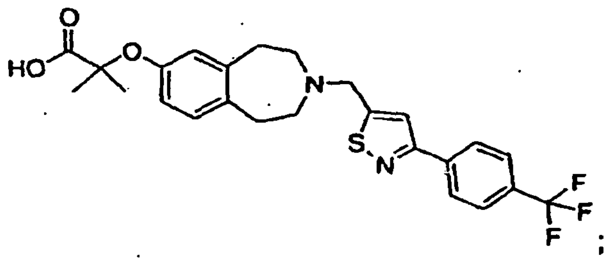;

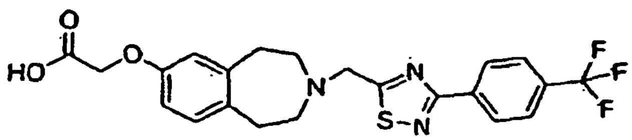;

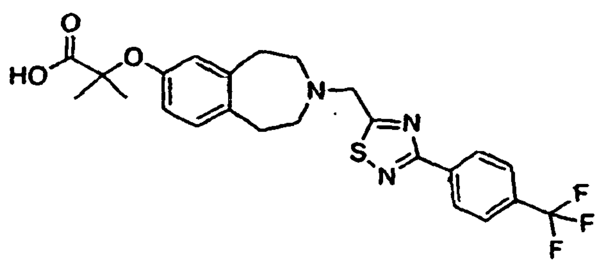;

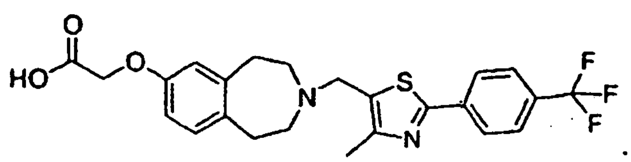;

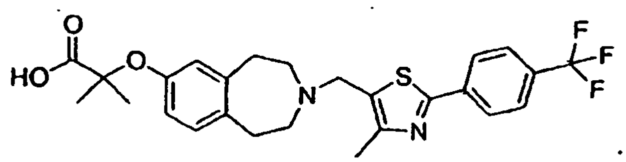;

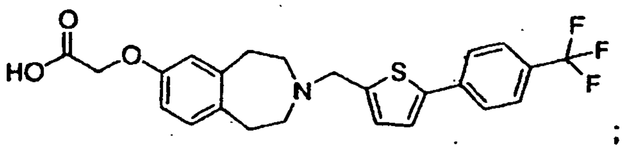
;

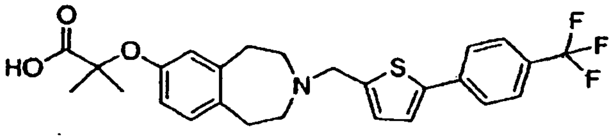
;

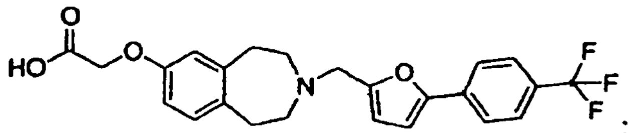
;

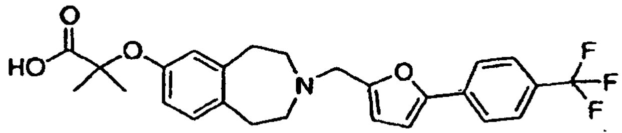
;

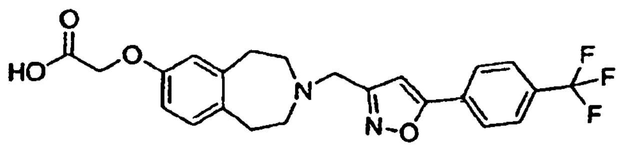
;

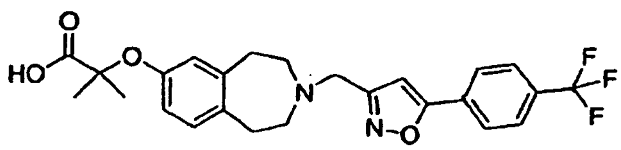
;

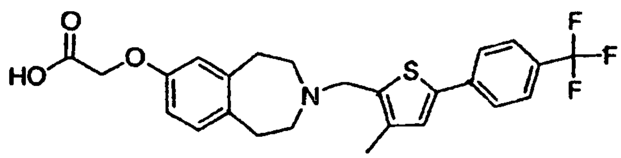
;

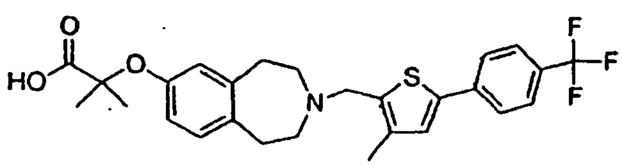
;

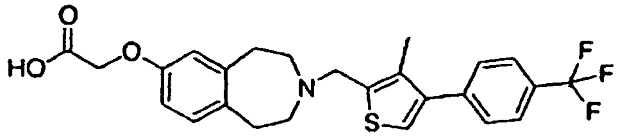
;

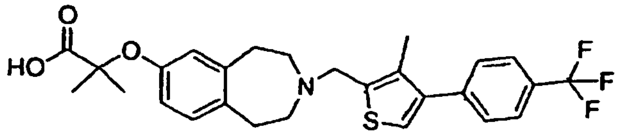
;

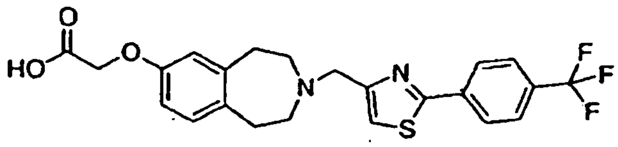
;

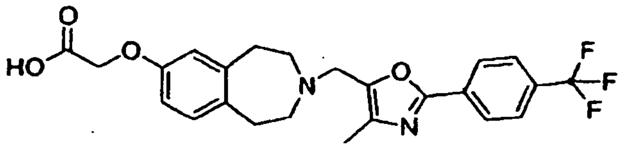
;

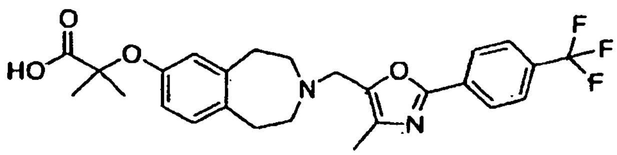
;

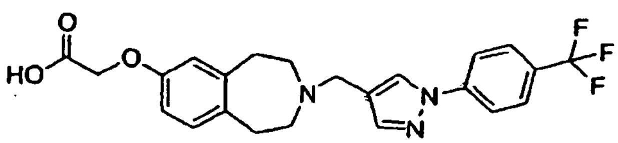
;

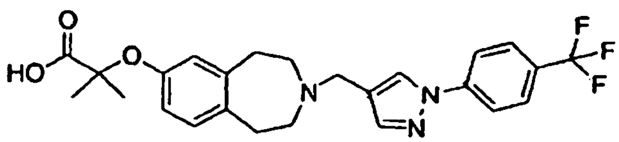
;

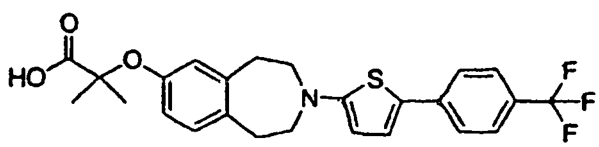
;

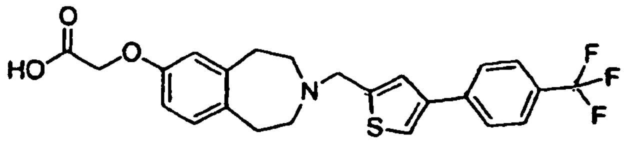
;

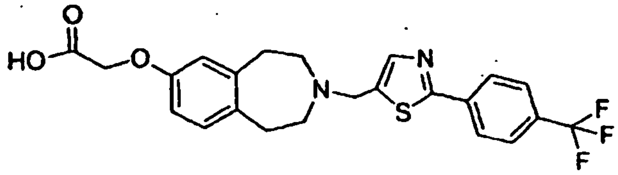
;

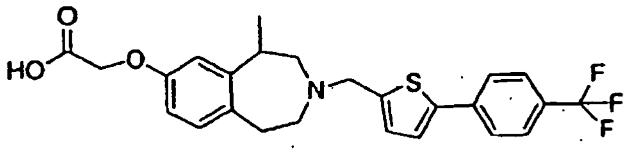
;

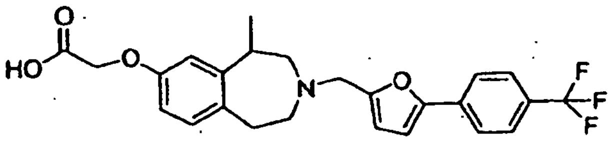
;

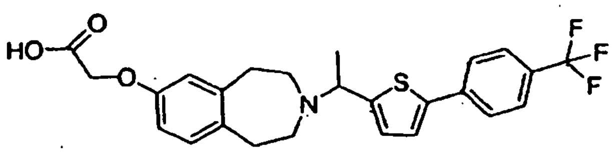
;

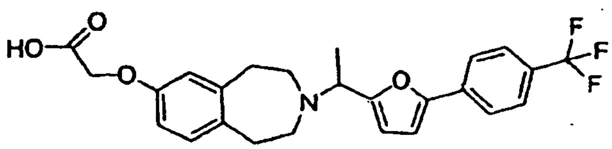
;

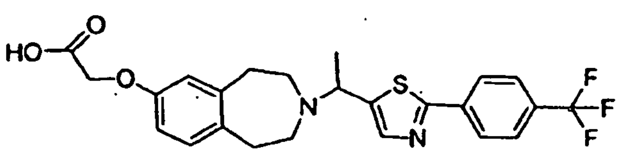
;

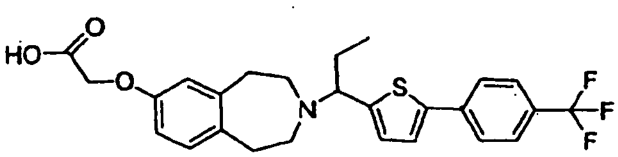
;

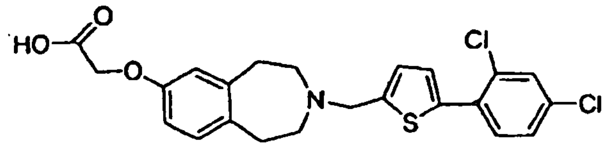
;

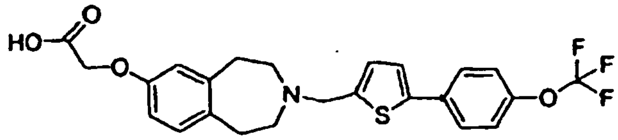
;

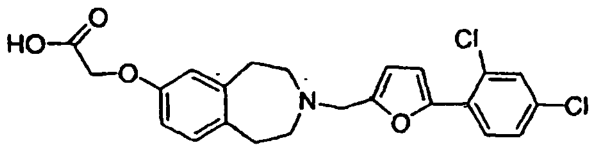
;

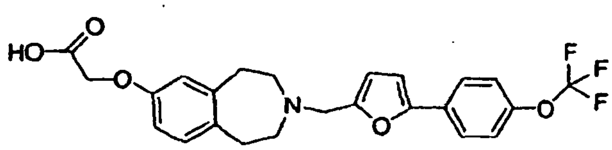
;

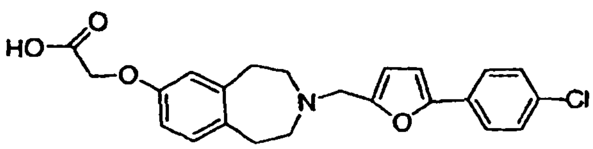
;

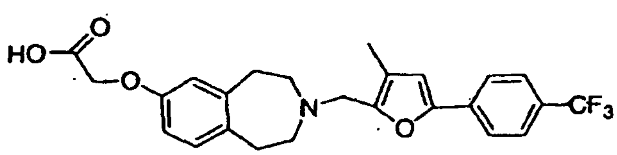
;

and

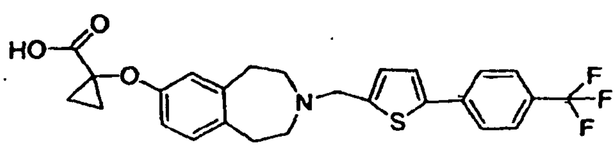
;

and enantiomers, diastereomers, tautomers, solvates, or pharmaceutically acceptable salts thereof.

**18.** A compound according to claim 1 selected from the group consisting of

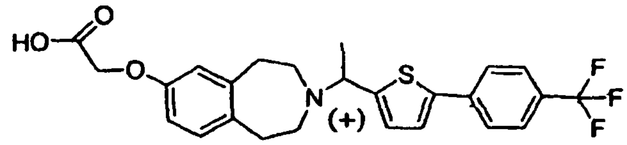,

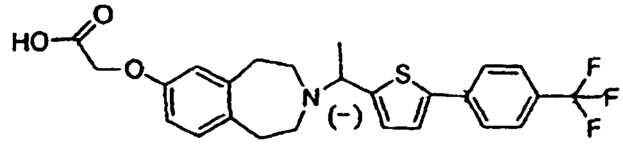,

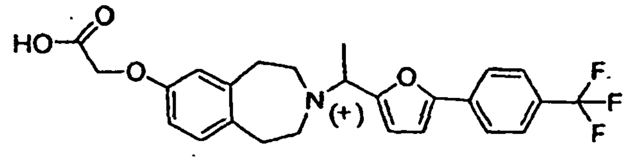,

and

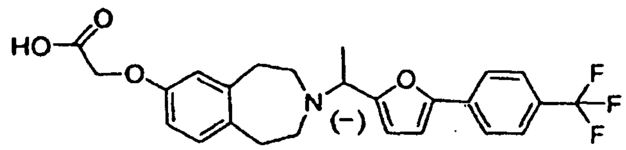;

wherin the compound is substantially free from the corresponding other enantiomer.

**19.** A compound according to claim 1 of the formula

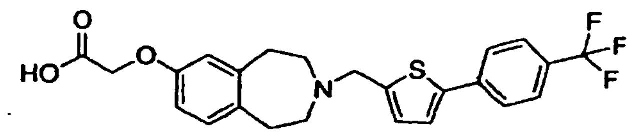.

**20.** A pharmaceutical composition comprising a compound, salt or solvate according to any of claims 1 - 19 admixed with a pharmaceutically acceptable carrier, excipient or diluent.

**21.** A compound, salt or solvate of claim 1, for use in a method of treating or preventing a disease or condition in a mammal which disease or condition is affected by the modulation of PPAR receptors.

**22.** A compound, salt or solvate of claim 1, for use in a method of treating or preventing a disease or condition in a mammal which disease or condition is affected by the modulation of PPAR delta.

**23.** The claim 21 or 22 wherein said of compound, salt or solvate thereof is administered in a dose range of from about 0.1 mg to about 15,000 mg.

**24.** The claim 21 or 22 wherein said of compound, salt or solvate thereof is administered in a dose range of from about 50 mg to about 1000 mg.

**25.** The claim 21 or 22wherein said of compound, salt or solvate thereof is administered in a dose range of from about 100 mg to about 1000 mg.

**26.** A compound, salt or solvate of claim 1, for use in a method for treating or preventing a disease or condition selected from the group consisting of diabetes, nephropathy, neuropathy, retinopathy, polycystic ovary syndrome, hypertension, ischemia, stroke, irritable bowel disorder, inflammation, cataract, cardiovascular diseases, Metabolic X Syndrome, hyper-LDL-cholesterolemia, dyslipidemia (including hypertriglyceridemia, hypercholesterolemia, mixed hyperlipidemia, and hypo-HDL-cholesterotemia), atherosclerosis, and obesity.

**27.** The claim 26 wherein said of compound, salt or solvate thereof is administered in a dose range of from about 0.1 mg to about 15,000 mg.

**28.** The claim 26 wherein said of compound, salt or solvate thereof is administered in a dose range of from about 50 mg to about 1000 mg.

**29.** The claim 26 wherein said of compound, salt or solvate thereof is administered in a dose range of from about 100 mg to about 1000 mg.

**30.** A kit comprising in one or more containers an amount of the composition of claim 1 effective to treat or prevent a disease or condition selected from the group consisting of diabetes, nephropathy, neuropathy, retinopathy, polycystic ovary syndrome; hypertension, ischemia, stroke, irritable bowel disorder, inflammation, cataract, cardiovascular diseases, Metabolic X Syndrome, hyper-LDL-cholesterolemia, dyslipidemia (including hypertriglyceridemia, hypercholesterolemia, mixed hyperlipidemia, and hypo-HDL-cholesterolemia), atherosclerosis, and obesity.

**31.** A compound of Formula (II)

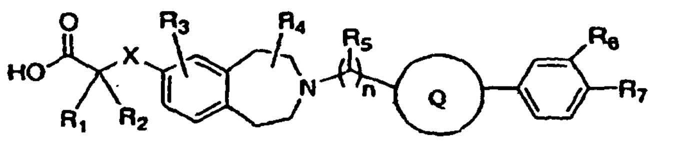

Formula (II)

wherein:

X is a covalent bond, O, or S;
$R_1$ and $R_2$ are independently selected from the group consisting of H; $C_{1-8}$alkyl, and substituted $C_{1-8}$alkyl, or $R_1$, $R_2$ and the carbon atom to which they are attached together may form $C_{3-7}$cycloalkyl;
$R_3$ is H;
$R_4$ and $R_5$ are independently selected from the group consisting of H, halo, $C_{1-8}$alkyl, $C_{3-7}$cycloalkyl, $C_{3-7}$cycloalkyl-$C_{1-4}$alkyl, $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{6-10}$aryl substituted $C_{1-4}$alkyl, heteroaryl substituted $C_{1-4}$alkyl, cyano substituted $C_{1-4}$alkyl, and hydroxy substituted $C_{1-4}$alkyl;
$R_6$ and $R_7$ are independently selected from the group consisting of H, halo, $C_{1-3}$alkyl, halo substituted $C_{1-3}$alkyl, $C_{1-3}$alkoxy, and halo substituted $C_{1-3}$alkoxy;
n is 1; and
Q is $C_{1-10}$aryl;

and enantiomers, diastereomers, tautomers, solvates, or pharmaceutically acceptable salts thereof.

**32.** A compound of Formula (III)

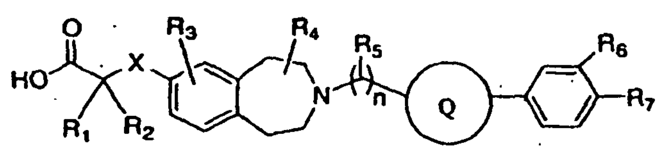

Formula (III)

wherein:

X is a covalent bond, O, or S;
$R_1$ and $R_2$ are independently selected from the group consisting of H, $C_{1-8}$alkyl, and substituted $C_{1-8}$alkyl, or $R_1$, $R_2$ and the carbon atom to which they are attached together may form $C_{3-7}$cycloalkyl;
$R_3$ is H;
$R_4$ and $R_5$ are independently selected from the group consisting of H, halo, $C_{1-8}$alkyl, $C_{3-7}$cycloalkyl, $C_{3-7}$cycloalkyl-$C_{1-4}$alkyl, $C_{3-7}$cycloalkyloxy-$C_{1-4}$alkyl, $C_{1-6}$alkoxy-$C_{1-4}$ alkyl, $C_{8-10}$aryl, heteroaryl, halo substituted $C_{1-4}$alkyl, amino substituted $C_{1-4}$alkyl, $C_{6-10}$aryl substituted $C_{1-4}$alkyl, heteroaryl substituted $C_{1-4}$alkyl, cyano substituted $C_{1-4}$alkyl, and hydroxy substituted $C_{1-4}$alkyl;
$R_6$ and $R_7$ are independently selected from the group consisting of H, halo, $C_{1-3}$alkyl, halo substituted $C_{1-3}$alkyl, $C_{1-3}$alkoxy, and halo substituted $C_{1-3}$alkoxy;
n is 1 or 2; and
Q is selected from the group consisting of

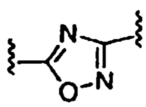 , 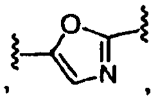 , 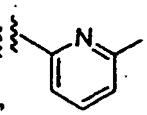 , 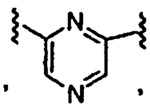 , and 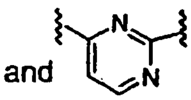 ;

and enantiomers, diastereomers, tautomers, solvates, or pharmaceutically acceptable salts thereof.

**33.** A compound of Formula (IV)

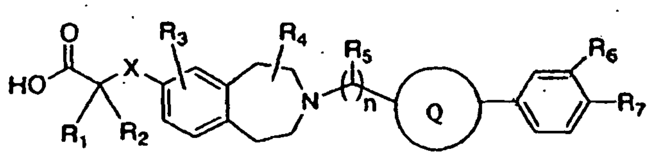

Formula (IV)

wherein:

X is a covalent bond, O, or S;
$R_1$ and $R_2$ are independently selected from the group consisting of H, $C_{1-8}$alkyl, and substituted $C_{1-8}$alkyl, or $R_1$, $R_2$ and the carbon atom to which they are attached together may form $C_{3-7}$cycloalkyl;
$R_3$ is H;
$R_4$ and $R_5$ are independently selected from the group consisting of H, halo, $C_{1-8}$alkyl, $C_{3-7}$cycloalkyl, $C_{3-7}$cycloalkyl-$C_{1-4}$alkyl, $C_{3-7}$cycloalkyloxy-$C_{1-4}$alkyl, $C_{1-6}$alkoxy-$C_{1-4}$ alkyl, $C_{6-10}$aryl, heteroaryl, halo substituted $C_{1-4}$alkyl, amino substituted $C_{1-4}$alkyl, $C_{6-10}$aryl substituted $C_{1-4}$alkyl, cyano substituted $C_{1-4}$alkyl, and hydroxy substituted $C_{1-4}$alkyl;
$R_6$ and $R_7$ are independently selected from the group consisting of H, halo, $C_{1-3}$alkyl, halo substituted $C_{1-3}$alkyl, $C_{1-3}$alkoxy, and halo substituted $C_{1-3}$alkoxy;
n is 2; and
Q is selected from the group consisting of

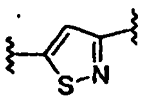, 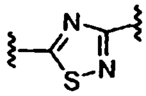, 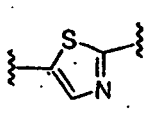, 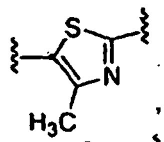,

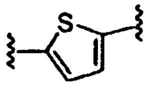, 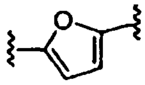, 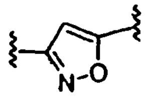, 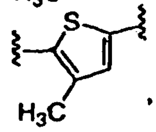,

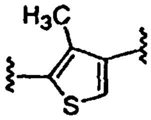, 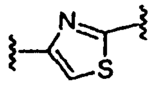, 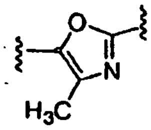, 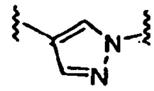,

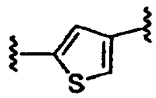, 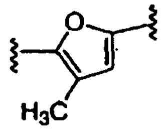, and 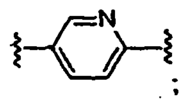;

and enantiomers, diastereomers, tautomers, solvates, or pharmaceutically acceptable salts thereof.

**34.** A compound according to claim 31, 32 or 33 selected from the group consisting of

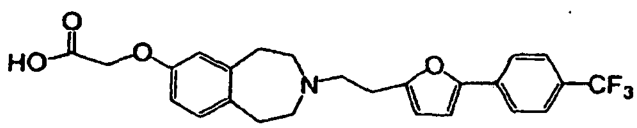,

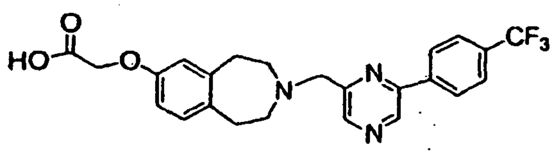,

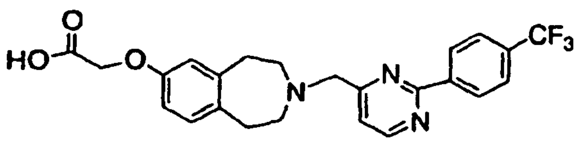,

and

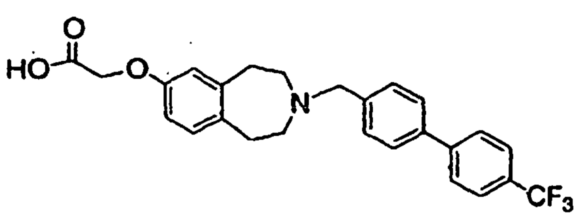.

**Patentansprüche**

**1.** Verbindungen der Formel (I)

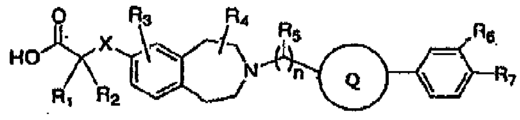

Formel (I)

wobei:

X für eine kovalente Bindung, O oder S steht;
$R_1$ und $R_2$ unabhängig voneinander aus der aus H, $C_{1-8}$-Alkyl und substituiertem $C_{1-8}$-Alkyl bestehenden Gruppe ausgewählt sind oder $R_1$, $R_2$ und das Kohlenstoffatom, an das sie gebunden sind, zusammen $C_{3-7}$-Cycloalkyl bilden können;
$R_3$ für H steht;
$R_4$ und $R_5$ unabhängig voneinander aus der aus H, Halogen, $C_{1-8}$-Alkyl, $C_{3-7}$-Cycloalkyl, $C_{3-7}$-Cycloalkyl-$C_{1-4}$-alkyl, $C_{3-7}$-Cycloalkyloxy-$C_{1-4}$-alkyl, $C_{1-6}$-Alkoxy-$C_{1-4}$-alkyl, $C_{6-10}$-Aryl, Heteroaryl, halogensubstituiertem $C_{1-4}$-Alkyl, aminosubstituiertem $C_{1-4}$-Alkyl, $C_{6-10}$-arylsubstituiertem $C_{1-4}$-Alkyl, cyanosubstituiertem $C_{1-4}$-Alkyl und hydroxysubstituiertem $C_{1-4}$-Alkyl bestehenden Gruppe ausgewählt sind;
$R_6$ und $R_7$ unabhängig voneinander aus der aus H, Halogen, $C_{1-3}$-Alkyl, halogensubstituiertem $C_{1-3}$-Alkyl, $C_{1-3}$-Alkoxy und halogensubstituiertem $C_{1-3}$-Alkoxy bestehenden Gruppe ausgewählt sind;
n für 1 steht und
Q aus der aus

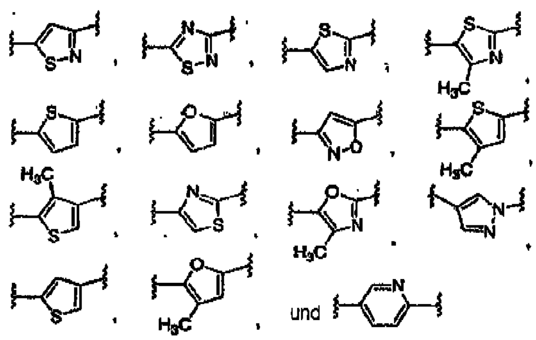

, , ; , , , , , , , , , , und

bestehenden Gruppe ausgewählt ist;
und deren Enantiomere, Diastereomere, Tautomere, Solvate und pharmazeutisch unbedenkliche Salze.

2. Verbindungen nach Anspruch 1, wobei $R_1$ und $R_2$ unabhängig voneinander aus der aus H und $C_{1-8}$-Alkyl bestehenden Gruppe ausgewählt sind oder
$R_1$, $R_2$ und das Kohlenstoffatom, an das sie gebunden sind, zusammen $C_{3-5}$-Cycloalkyl bilden können.

3. Verbindungen nach Anspruch 1, wobei $R_1$ und $R_2$ unabhängig voneinander aus der aus H und $CH_3$ bestehenden Gruppe ausgewählt sind oder $R_1$, $R_2$ und das Kohlenstoffatom, an das sie gebunden sind, zusammen

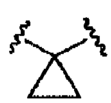

bilden können.

4. Verbindungen nach Anspruch 1, wobei $R_6$ und $R_7$ unabhängig voneinander aus der aus H, Halogen, halogensubstituiertem $C_{1-3}$-Alkyl, $C_{1-3}$-Alkoxy und halogensubstituiertem $C_{1-3}$-Alkoxy bestehenden Gruppe ausgewählt sind.

5. Verbindungen nach Anspruch 1, wobei $R_6$ für H steht und $R_7$ aus der aus Halogen, halogensubstituiertem $C_{1-3}$-Alkyl und halogensubstituiertem $C_{1-3}$-Alkoxy bestehenden Gruppe ausgewählt ist.

6. Verbindungen nach Anspruch 5, wobei $R_7$ aus der aus F, $CF_3$ und $-O-CF_3$ bestehenden Gruppe ausgewählt ist.

7. Verbindungen nach Anspruch 1, wobei $R_4$ und $R_5$ unabhängig voneinander aus der aus H und $C_{1-6}$-Alkyl bestehenden Gruppe ausgewählt sind.

8. Verbindungen nach Anspruch 1, wobei $R_5$ für H, $CH_3$ oder $-CH_2CH_3$ steht.

9. Verbindungen nach Anspruch 1, wobei Q aus der aus

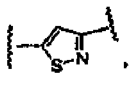, 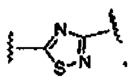, 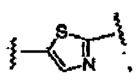, 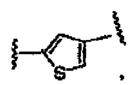,

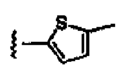, 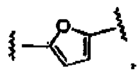, 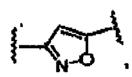, 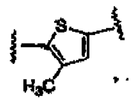,

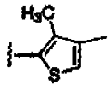, 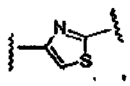, und 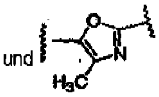

bestehenden Gruppe ausgewählt ist.

10. Verbindungen nach Anspruch 1, wobei Q aus der aus

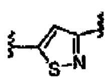, 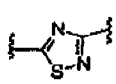, 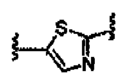, 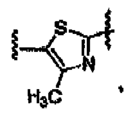,

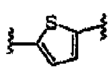, 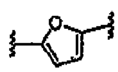, 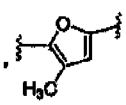 und 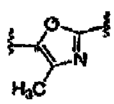

bestehenden Gruppe ausgewählt ist.

11. Verbindungen nach Anspruch 1, wobei X für O steht.

12. Verbindungen nach Anspruch 1, wobei
X für O steht;
$R_1$, $R_2$, $R_4$ und $R_5$ unabhängig voneinander aus der aus H und $C_{1-3}$-Alkyl bestehenden Gruppe ausgewählt sind oder $R_1$, $R_2$ und das Kohlenstoffatom, an das sie gebunden sind, zusammen

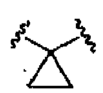

bilden können; und $R_6$ und $R_7$ unabhängig voneinander aus der aus H, Halogen, $C_{1-3}$-Alkoxy und halogensubstituiertem $C_{1-3}$-Alkyl und halogensubstituiertem $C_{1-3}$-Alkoxy bestehenden Gruppe ausgewählt sind;
und deren Enantiomere, Diastereomere, Tautomere, Solvate und pharmazeutisch unbedenkliche Salze.

13. Verbindungen der Formel (Ia) nach Anspruch 1

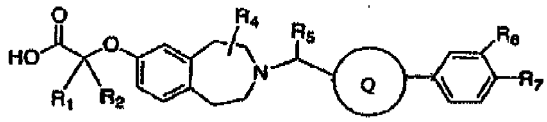

Formel (Ia),

wobei
$R_1$ und $R_2$ unabhängig voneinander aus der aus H und $C_{1-6}$-Alkyl bestehenden Gruppe ausgewählt sind oder $R_1$, $R_2$ und das Kohlenstoffatom, an das sie gebunden sind, zusammen $C_{3-5}$-Cycloalky bilden können;
$R_6$ und $R_7$ unabhängig voneinander aus der aus H, $C_{1-3}$-Alkyl, Halogen und halogensubstituiertem $C_{1-3}$-Alkyl bestehenden Gruppe ausgewählt sind;
$R_4$ und $R_5$ unabhängig voneinander aus der aus H und $C_{1-8}$-Alkyl bestehenden Gruppe ausgewählt sind und Q aus der aus

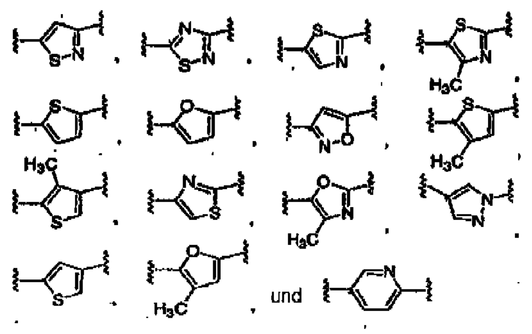

und

bestehenden Gruppe ausgewählt ist;
und deren Enantiomere, Diastereomere, Tautomere, Solvate und pharmazeutisch unbedenkliche Salze.

**14.** Verbindungen der Formel (Ib) nach Anspruch 1

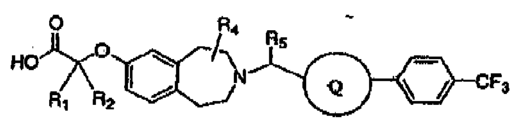

Formel (Ib),

wobei
$R_1$ und $R_2$ unabhängig voneinander aus der aus H und $CH_3$ bestehenden Gruppe ausgewählt sind oder $R_1$, $R_2$ und das Kohlenstoffatom, an das sie gebunden sind,
zusammen

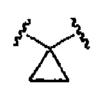

bilden können;
$R_4$ und $R_5$ unabhängig voneinander aus der aus H, $CH_3$ und $-CH_2CH_3$ bestehenden Gruppe ausgewählt sind und Q aus der aus

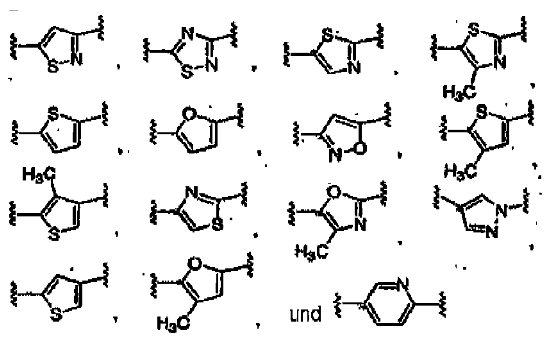

und

bestehenden Gruppe ausgewählt ist;
und deren Enantiomere, Diastereomere, Tautomere, Solvate und pharmazeutisch unbedenkliche Salze.

**15.** Verbindungen der Formel (Ic) nach Anspruch 1

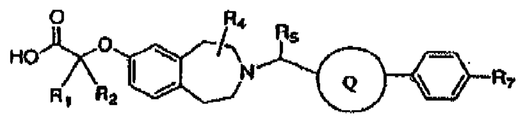

Formel (Ic),

wobei
$R_1$, $R_2$ und $R_4$ unabhängig voneinander aus der aus H und $CH_3$ bestehenden Gruppe ausgewählt sind oder $R_1$, $R_2$ und das Kohlenstoffatom, an das sie gebunden sind, zusammen

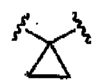

bilden können;
$R_5$ aus der aus H, $CH_3$ und $-CH_2CH_3$ bestehenden Gruppe ausgewählt ist;
$R_7$ für Halogen oder halogensubstituiertes $C_{1-3}$-Alkyl steht und
Q aus der aus

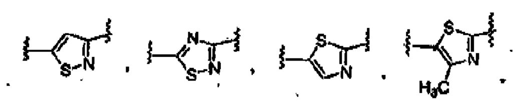

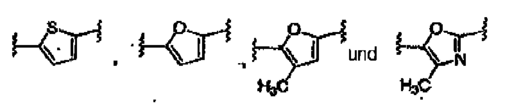

und

bestehenden Gruppe ausgewählt ist;
und deren Enantiomere, Diastereomere, Tautomere, Solvate und pharmazeutisch unbedenkliche Salze.

**16.** Verbindungen nach Anspruch 15, wobei:

(a) $R_1$ oder $R_2$ für H steht;
(b) $R_1$ und $R_2$ beide für H stehen;
(c) $R_1$ oder $R_2$ für $CH_3$ steht;
(d) $R_1$ und $R_2$ beide für $CH_3$ stehen;
(e) $R_1$, $R_2$ und das Kohlenstoffatom, an das sie gebunden sind, zusammen

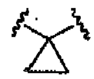

bilden;
(f) $R_4$ für H oder $CH_3$ steht;
(g) $R_5$ für H, $CH_3$ oder $-CH_2CH_3$ steht;
(h) $R_1$ oder $R_2$ für H steht und $R_4$ für H oder $CH_3$ steht;
(i) $R_1$ und $R_2$ beide für H stehen und $R_4$ für H oder $CH_3$ steht;
(j) $R_1$ oder $R_2$ für H steht und $R_5$ für H, $CH_3$ oder - $CH_2CH_3$ steht;
(k) $R_1$ und $R_2$ beide für H stehen und $R_5$ für H, $CH_3$ oder $-CH_2CH_3$ steht;
(l) $R_1$ oder $R_2$ für $CH_3$ steht und $R_5$ für H, $CH_3$ oder $-CH_2CH_3$ steht;
(m) $R_1$ und $R_2$ beide für $CH_3$ stehen und $R_5$ für H, $CH_3$ oder $-CH_2CH_3$ steht:
(n) $R_1$ oder $R_2$ für H steht, $R_4$ für H steht und $R_5$ für H, $CH_3$ oder $-CH_2CH_3$ steht;
(o) $R_1$ oder $R_2$ beide für H stehen, $R_4$ für H steht und $R_5$ für H, $CH_3$ oder $-CH_2CH_3$ steht;
(p) $R_1$ oder $R_2$ für $CH_3$ steht, $R_4$ für H steht und $R_5$ für H, $CH_3$ oder $-CH_2CH_3$ steht;
(q) $R_1$ und $R_2$ beide für $CH_3$ stehen, $R_4$ für H steht und $R_5$ für H, $CH_3$ oder $-CH_2CH_3$ steht;
(r) $R_1$, $R_2$ und das Kohlenstoffatom, an das sie gebunden sind, zusammen

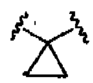

bilden, $R_4$ für H steht und $R_5$ für H, $CH_3$ oder $-CH_2CH_3$ steht;
(s) $R_7$ für $CF_3$ steht;
(t) $R_7$ für Cl steht:
(u) $R_1$ oder $R_2$ für H steht, $R_7$ für $CF_3$ steht und $R_4$ für H oder $CH_3$ steht;
(v) $R_1$ und $R_2$ beide für H stehen, $R_7$ für $CF_3$ steht und $R_4$ für H oder $CH_3$ steht;
(w) $R_1$ oder $R_2$ für H steht, $R_7$ für $CF_3$ steht und $R_5$ für H, $CH_3$ oder $-CH_2CH_3$ steht;
(x) $R_1$ und $R_2$ beide für H stehen, $R_7$ für $CF_3$ steht und $R_5$ für H, $CH_3$ oder $-CH_2CH_3$ steht;
(y) $R_1$ oder $R_2$ für $CH_3$ steht, $R_7$ für $CF_3$ steht und $R_5$ für H, $CH_3$ oder $-CH_2CH_3$ steht;
(z) $R_1$ und $R_2$ beide für $CH_3$ stehen, $R_7$ für $CF_3$ steht und $R_5$ für H, $CH_3$ oder $-CH_2CH_3$ steht;
(aa) $R_1$ oder $R_2$ für H steht, $R_4$ für H steht, $R_7$ für $CF_3$ steht und $R_5$ für H, $CH_3$ oder $-CH_2CH_3$ steht;
(bb) $R_1$ und $R_2$ beide für H stehen, $R_4$ für H steht, $R_7$ für $CF_3$ steht und $R_5$ für H, $CH_3$ oder $-CH_2CH_3$ steht;
(cc) $R_1$ oder $R_2$ für $CH_3$ steht, $R_4$ für H steht, $R_7$ für $CF_3$ steht und $R_5$ für H, $CH_3$ oder $-CH_2CH_3$ steht;
(dd) $R_1$ und $R_2$ beide für $CH_3$ stehen, $R_4$ für H steht, $R_7$ für $CF_3$ steht und $R_5$ für H, $CH_3$ oder $-CH_2CH_3$ steht;
(ee) $R_1$, $R_2$ und das Kohlenstoffatom, an das sie gebunden sind, zusammen

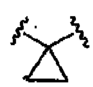

bilden, $R_4$ für H steht, $R_7$ für $CF_3$ steht und $R_5$ für H, $CH_3$ oder $-CH_2CH_3$ steht;
(ff) Q aus der aus

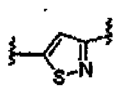, 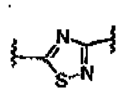, 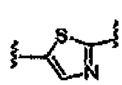, 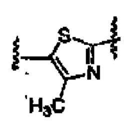,

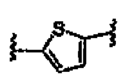, 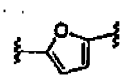, 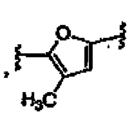 und 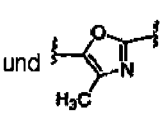

bestehenden Gruppe ausgewählt ist;
(gg) $R_1$ oder $R_2$ für H steht und Q aus der aus

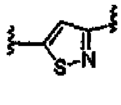, 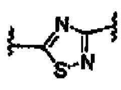, 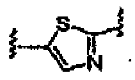, 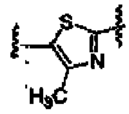,

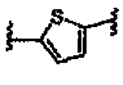, 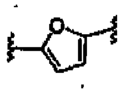, 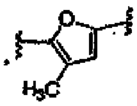 und 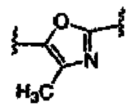

bestehenden Gruppe ausgewählt ist;
(hh) $R_1$ und $R_2$ beide für H stehen und Q aus der aus

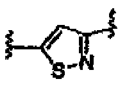, 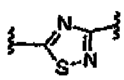, 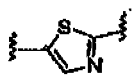, 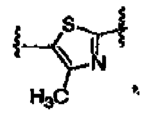,

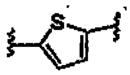, 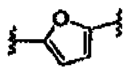, 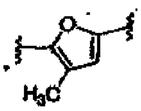 und 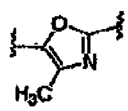

bestehenden Gruppe ausgewählt ist;
(ii) $R_1$ oder $R_2$ für $CH_3$ steht und Q aus der aus

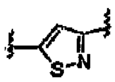, 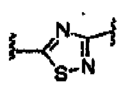, 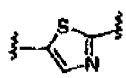, 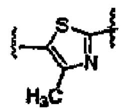,

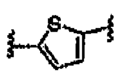, 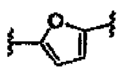, 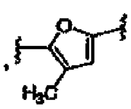 und 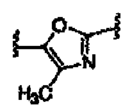

bestehenden Gruppe ausgewählt ist;
(jj) $R_1$ und $R_2$ beide für $CH_3$ stehen und Q aus der aus

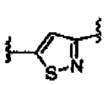, 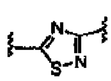, 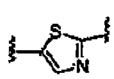, 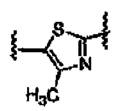,

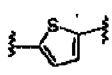, 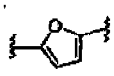, 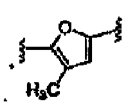 und 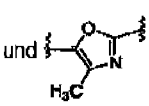

bestehenden Gruppe ausgewählt ist;
(kk) $R_4$ für H oder $CH_3$ steht und Q aus der aus

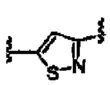, 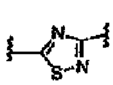, 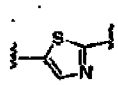, 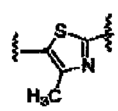,

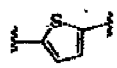, 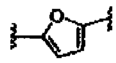, 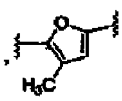 und 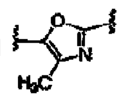

bestehenden Gruppe ausgewählt ist;
(ll) $R_5$ für H, $CH_3$ oder $-CH_2CH_3$ steht und Q aus der aus

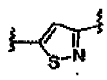, 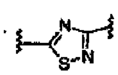, 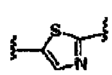, 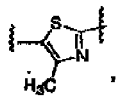,

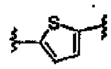, 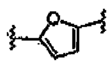, 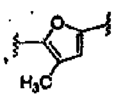 und 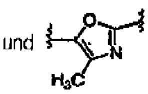

bestehenden Gruppe ausgewählt ist;
(mm) $R_1$ oder $R_2$ für H steht und $R_4$ für H oder $CH_3$ steht und Q aus der aus

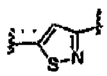, 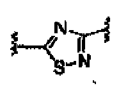, 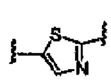, 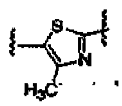,

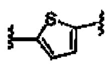, 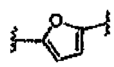, 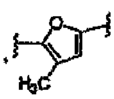 und 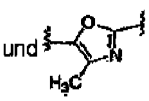

bestehenden Gruppe ausgewählt ist;
(nn) $R_1$ und $R_2$ beide für H stehen und $R_4$ für H oder $CH_3$ steht und Q aus der aus

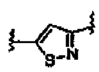, 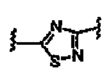, 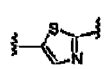, 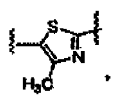,

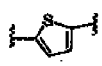, 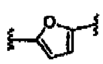, 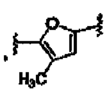 und 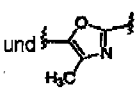

bestehenden Gruppe ausgewählt ist;
(oo) $R_1$ oder $R_2$ für H steht und $R_5$ für H, $CH_3$ oder $- CH_2CH_3$ steht und Q aus der aus

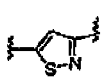, 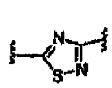, 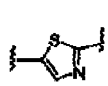, 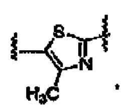,

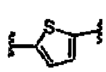, 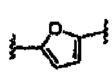, 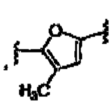 und 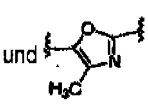

bestehenden Gruppe ausgewählt ist;

(pp) $R_1$ und $R_2$ beide für H stehen und $R_5$ für H, $CH_3$ oder $-CH_2CH_3$ steht und Q aus der aus

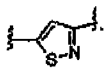, 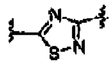, 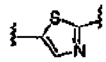, 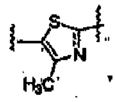,

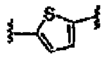, 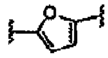, 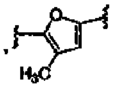 und 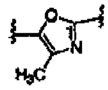

bestehenden Gruppe ausgewählt ist;

(qq) $R_1$ oder $R_2$ für $CH_3$ steht und $R_5$ für H, $CH_3$ oder $-CH_2CH_3$ steht und Q aus der aus

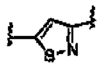, 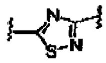, 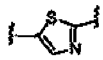, 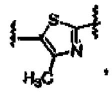,

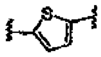, 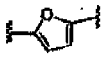, 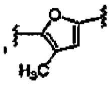 und 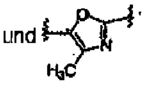

bestehenden Gruppe ausgewählt ist;

(rr) $R_1$ und $R_2$ beide für $CH_3$ stehen, $R_5$ für H, $CH_3$ oder $-CH_2CH_3$ steht und Q aus der aus

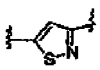, 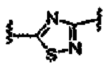, 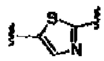, 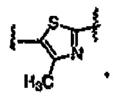,

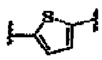, 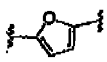, 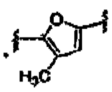 und 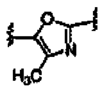

bestehenden Gruppe ausgewählt ist;

(ss) $R_1$ oder $R_2$ für H steht, $R_4$ für H steht, $R_5$ für H, $CH_3$ oder $-CH_2CH_3$ steht und Q aus der aus

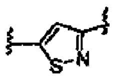, 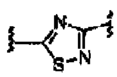, 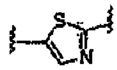, 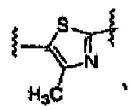,

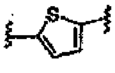, 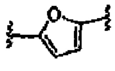, 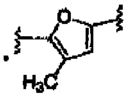 und 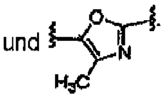.

bestehenden Gruppe ausgewählt ist;

(tt) $R_1$ und $R_2$ beide für H stehen, $R_4$ für H steht, $R_5$ für H, $CH_3$ oder $-CH_2CH_3$ steht und Q aus der aus

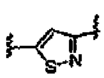, 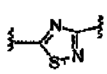, 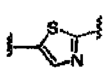, 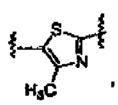,

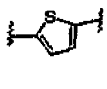, 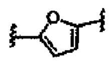, 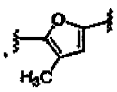 und 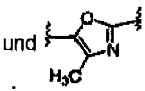

bestehenden Gruppe ausgewählt ist;

(uu) $R_1$ oder $R_2$ für $CH_3$ steht, $R_4$ für H steht, $R_5$ für H, $CH_3$ oder $-CH_2CH_3$ steht und Q aus der aus

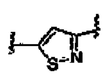, 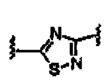, 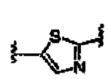, 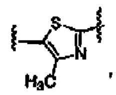,

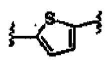, 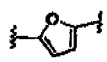, 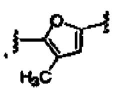 und 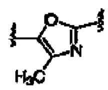

bestehenden Gruppe ausgewählt ist;

(vv) $R_1$ und $R_2$ beide für $CH_3$ stehen, $R_4$ für H steht, $R_5$ für H, $CH_3$ oder $-CH_2CH_3$ steht und Q aus der aus

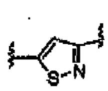, 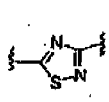, 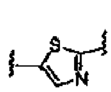, 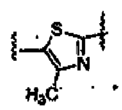,

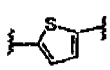, 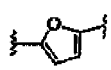, 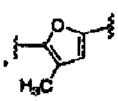 und 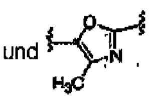

bestehenden Gruppe ausgewählt ist;

(ww) $R_1$, $R_2$ und das Kohlenstoffatom, an das sie gebunden sind, zusammen

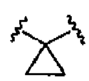

bilden, $R_4$ für H steht, $R_5$ für H, $CH_3$ oder $-CH_2CH_3$ steht und Q aus der aus

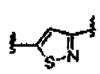, 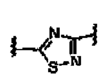, 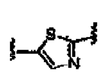, 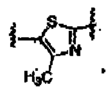,

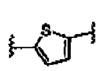, 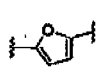, 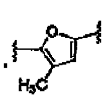 und 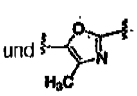

bestehenden Gruppe ausgewählt ist;

(xx) $R_7$ für $CH_3$ steht und Q aus der aus

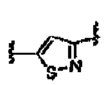, 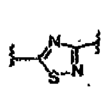, 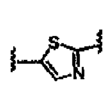, 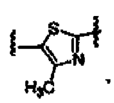,

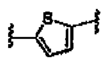, 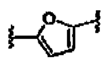, 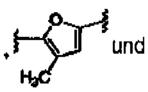 und 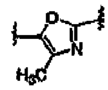

bestehenden Gruppe ausgewählt ist;

(yy) $R_7$ für Cl steht und Q aus der aus

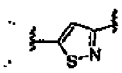, 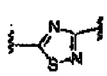, 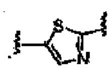, 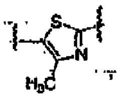

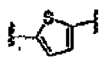, 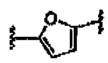, 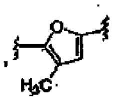 und 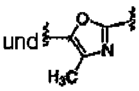

bestehenden Gruppe ausgewählt ist;

(zz) $R_1$ oder $R_2$ für H steht, $R_7$ für $CF_3$ steht, $R_4$ für H oder $CH_3$ steht und Q aus der aus

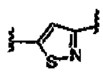, 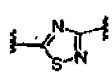, 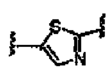, 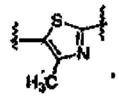,

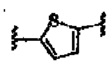, 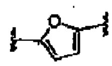, 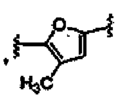 und 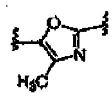

bestehenden Gruppe ausgewählt ist;

(aaa) $R_1$ und $R_2$ beide für H stehen, $R_7$ für $CF_3$ steht, $R_4$ für H oder $CH_3$ steht und Q aus der aus

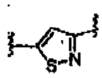, 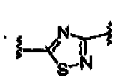, 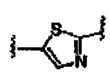, 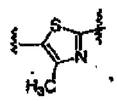,

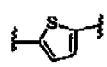, 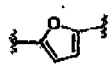, 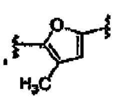 und 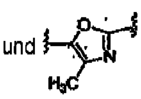

bestehenden Gruppe ausgewählt ist;

(bbb) $R_1$ oder $R_2$ für H steht, $R_7$ für $CF_3$ steht, $R_5$ für H, $CH_3$ oder $-CH_2CH_3$ steht und Q aus der aus

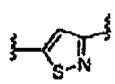, 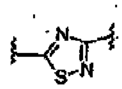, 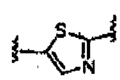, 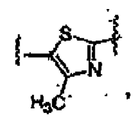,

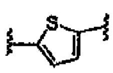, 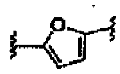, 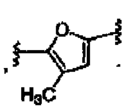 und 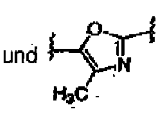

bestehenden Gruppe ausgewählt ist;

(ccc) $R_1$ und $R_2$ beide für H stehen, $R_7$ für $CF_3$ steht, $R_5$ für H, $CH_3$ oder $-CH_2CH_3$ steht und Q aus der aus

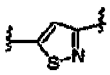 , 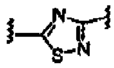 , 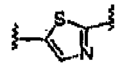 , 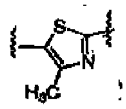 ,

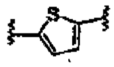 , 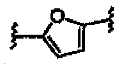 , 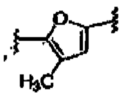 und 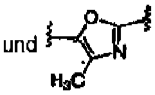

bestehenden Gruppe ausgewählt ist;

(ddd) $R_1$ oder $R_2$ für $CH_3$ steht, $R_7$ für $CF_3$ steht, $R_5$ für H, $CH_3$ oder $-CH_2CH_3$ steht und Q aus der aus

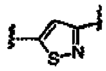 , 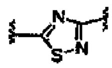 , 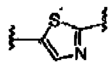 , 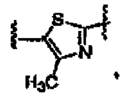 ,

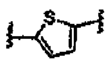 , 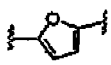 , 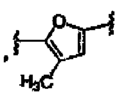 und 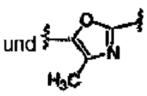

bestehenden Gruppe ausgewählt ist;

(eee) $R_1$ und $R_2$ beide für $CH_3$ stehen, $R_7$ für $CF_3$ steht, $R_5$ für H, $CH_3$ oder $-CH_2CH_3$ steht und Q aus der aus

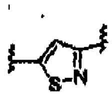 , 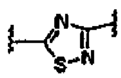 , 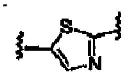 , 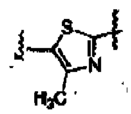

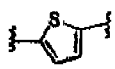 , 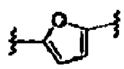 , 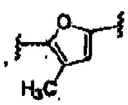 und 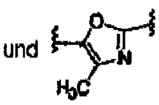

bestehenden Gruppe ausgewählt ist;

(fff) $R_1$ oder $R_2$ für H steht, $R_4$ für H steht, $R_7$ für $CF_3$ steht, $R_5$ für H, $CH_3$ oder $-CH_2CH_3$ steht und Q aus der aus

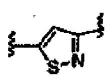 , 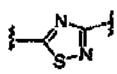 , 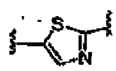 , 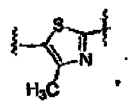 ,

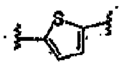 , 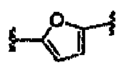 , 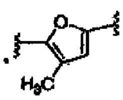 und 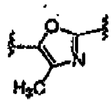

bestehenden Gruppe ausgewählt ist;

(ggg) $R_1$ und $R_2$ beide für H stehen, $R_4$ für H steht, $R_7$ für $CF_3$ steht, $R_5$ für H, $CH_3$ oder $-CH_2CH_3$ steht und Q aus der aus

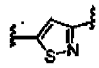, 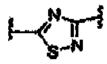, 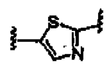, 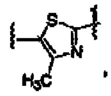,

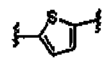, 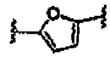, 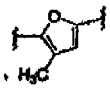 und 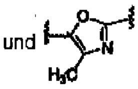

bestehenden Gruppe ausgewählt ist;

(hhh) $R_1$ oder $R_2$ für $CH_3$ steht, $R_4$ für H steht, $R_7$ für $CF_3$ steht, $R_5$ für H, $CH_3$ oder $-CH_2CH_3$ steht und Q aus der aus

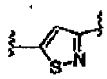, 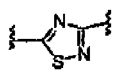, 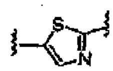, 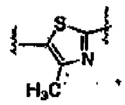,

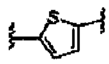, 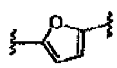, 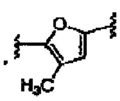 und 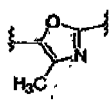

bestehenden Gruppe ausgewählt ist; oder

(iii) $R_1$ und $R_2$ beide für $CH_3$ stehen, $R_4$ für H steht, $R_7$ für $CF_3$ steht, $R_5$ für H, $CH_3$ oder $-CH_2CH_3$ steht und Q aus der aus

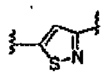, 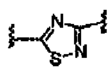, 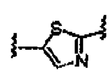, 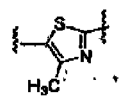,

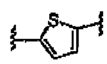, 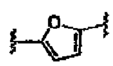, 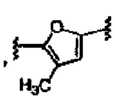 und 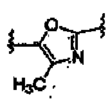

bestehenden Gruppe ausgewählt ist;

und deren Enantiomere, Diastereomere, Tautomere, Solvate und pharmazeutisch unbedenkliche Salze.

**17.** Verbindungen nach Anspruch 1, ausgewählt aus der Gruppe bestehend aus

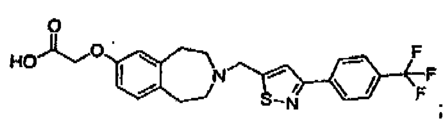;

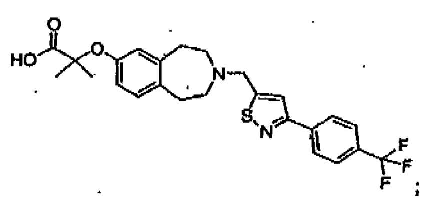;

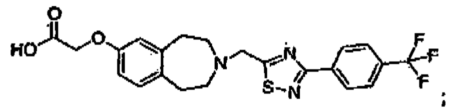
;

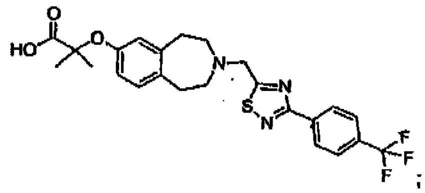
;

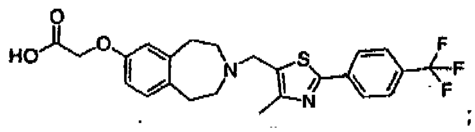
;

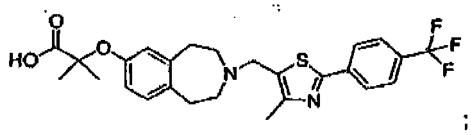
;

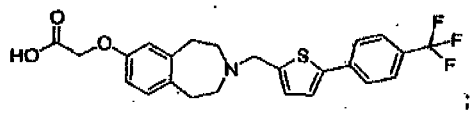
;

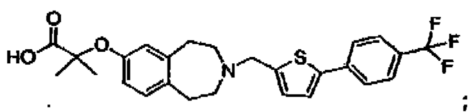
;

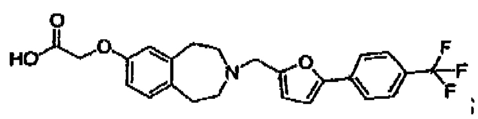
;

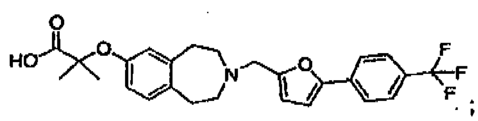
;

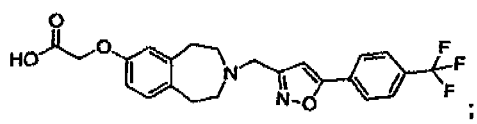
;

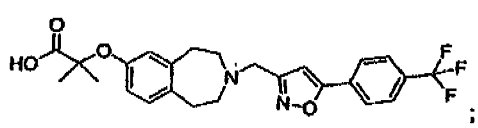
;

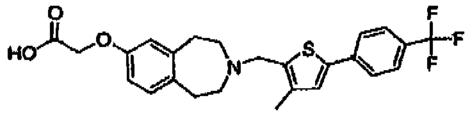
;

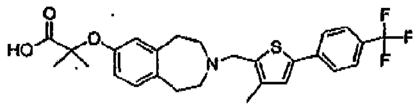
;

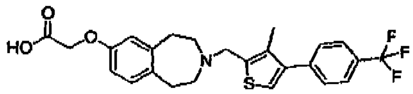
;

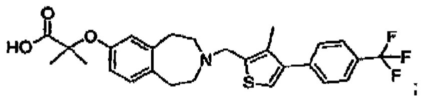
;

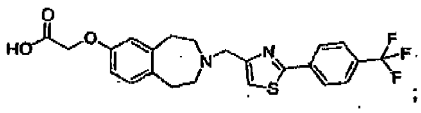
;

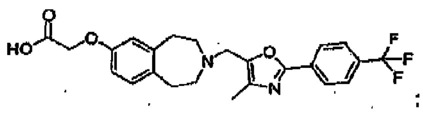
;

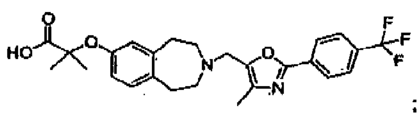
;

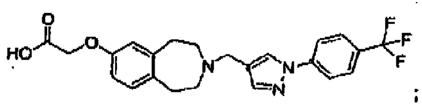
;

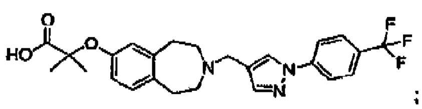
;

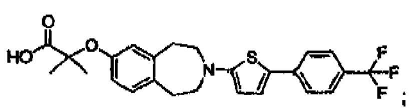
;

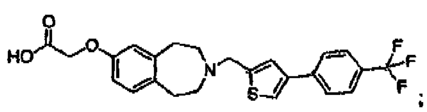
;

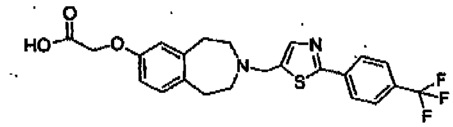
;

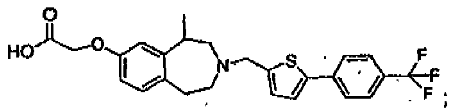
;

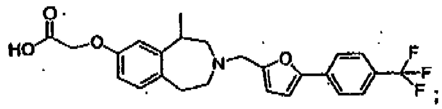
;

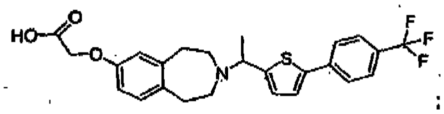
;

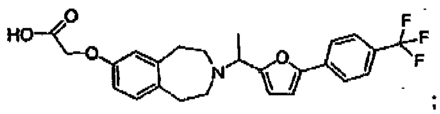
;

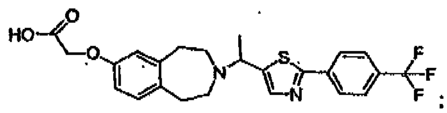
;

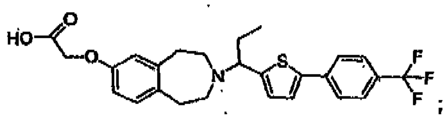
;

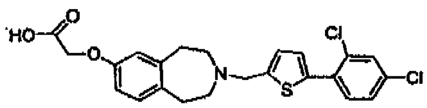
;

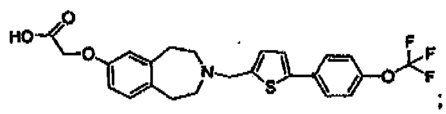
;

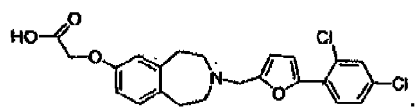
;

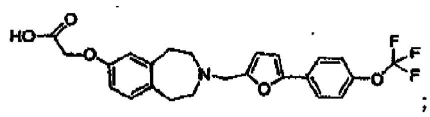
;

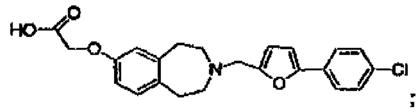
;

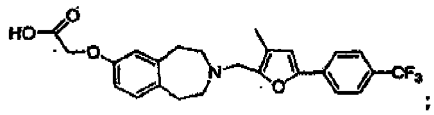
;

und

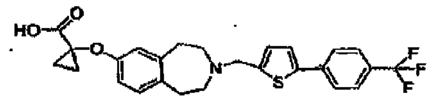

und deren Enantiomere, Diastereomere, Tautomere, Solvate und pharmazeutisch unbedenkliche Salze.

**18.** Verbindungen nach Anspruch 1, ausgewählt aus der Gruppe bestehend aus

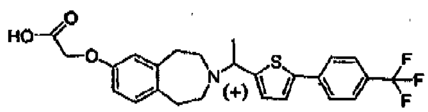
,

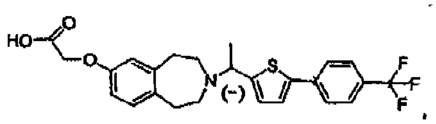
,

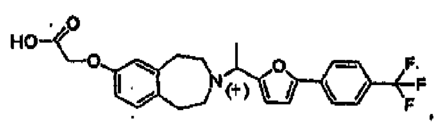
,

und

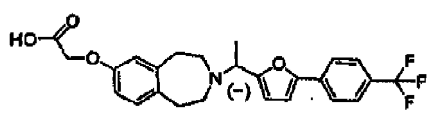

wobei die Verbindung im Wesentlichen frei vom entsprechenden anderen Enantiomer ist.

**19.** Verbindung nach Anspruch 1 mit der Formel

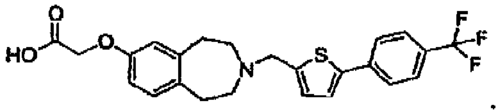
.

**20.** Pharmazeutische Zusammensetzung, enthaltend eine Verbindung, ein Salz oder ein Solvat nach einem der Ansprüche 1-19 in einer Mischung mit einem pharmazeutisch unbedenklichen Träger, Hilfsstoff oder Verdünnungsmittel.

**21.** Verbindung, Salz oder Solvat nach Anspruch 1 zur Verwendung in einem Verfahren zur Behandlung oder Prävention einer Krankheit bzw. eines Leidens bei einem Säugetier, wobei die Krankheit bzw. das Leiden durch die Modulation von PPAR-Rezeptoren beeinflusst wird.

**22.** Verbindung, Salz oder Solvat nach Anspruch 1 zur Verwendung in einem Verfahren zur Behandlung oder Prävention einer Krankheit oder eines Leidens in einem Säugetier, wobei die Krankheit bzw. das Leiden durch die Modulation von PPAR-delta beeinflusst wird.

**23.** Vebindung, Salz oder Solvat nach Anspruch 21 oder 22, wobei die Verbindung, das Salz bzw. das Solvat in einem Dosisbereich von etwa 0,1 mg bis etwa 15000 mg verabreicht wird.

**24.** Verbindung, Salz oder Solvat nach Anspruch 21 oder 22, wobei die Verbindung, das Salz bzw. das Solvat in einem Dosisbereich von etwa 50 mg bis etwa 1000 mg verabreicht wird.

**25.** Verbindung, Salz oder Solvat nach Anspruch 21 oder 22, wobei die Verbindung, das Salz bzw. das Solvat in einem Dosisbereich von etwa 100 mg bis etwa 1000 mg verabreicht wird.

**26.** Verbindung, Salz oder Solvat nach Anspruch 1 zur Verwendung bei einem Verfahren zur Behandlung oder Prävention einer Krankheit oder eines Leidens ausgewählt aus der Gruppe bestehend aus Diabetes, Nephropathie, Neuropathie, Retinopathie, Syndrom der polyzystischer Ovarien, Bluthochdruck, Ischämie, Schlaganfall, Reizkolon, Entzündungen, Katarakt, Herz-Kreislauf-Erkrankungen, metabolischem X-Syndrom, Hyper-LDL-Cholesterinämie, Dyslipidämie (einschließlich Hypertriglyzeridämie, Hypercholesterinämie, gemischter Hyperlipidämie und Hypo-HDL-Cholesterinämie), Atheriosklerose und Obesitas.

**27.** Verbindung, Salz oder Solvat nach Anspruch 26, wobei die Verbindung, das Salz bzw. das Solvat in einem Dosisbereich von etwa 0,1 mg bis etwa 15000 mg verabreicht wird.

**28.** Verbindung, Salz oder Solvat nach Anspruch 26, wobei die Verbindung, das Salz bzw. das Solvat in einem Dosisbereich von etwa 50 mg bis etwa 1000 mg verabreicht wird.

**29.** Verbindung, Salz oder Solvat nach Anspruch 26, wobei die Verbindung, das Salz bzw. das Solvat in einem Dosisbereich von etwa 100 mg bis etwa 1000 mg verabreicht wird.

**30.** Kit, enthaltend einen oder mehrere Behälter mit einer zur Behandlung oder Prävention einer Krankheit oder eines Leiden ausgewählt aus der Gruppe bestehend aus Diabetes, Nephropathie, Neuropathie, Retinopathie, Syndrom der polyzystischen Ovarien, Bluthochdruck, Ischämie, Schlaganfall, Reizkolon, Entzündungen, Katarakt, Herz-Kreislauf-Erkrankungen, metabolischem X-Syndrom, Hyper-LDL-Cholesterinämie, Dyslipidämie (einschließlich Hypertriglyzeridämie, Hyperoholesterinämie, gemischter Hyperlipidämie und Hypo-HDL-Cholesterinämie), Atheriosklerose und Obesitas wirksamen Menge der Zusammensetzung nach Anspruch 1.

**31.** Verbindungen der Formel (II)

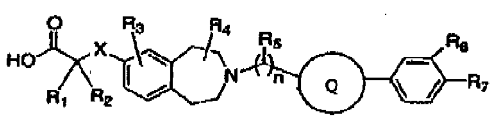

Formel (II)

wobei:

X für eine kovalente Bindung, O oder S steht;
$R_1$ und $R_2$ unabhängig voneinander aus der aus H, $C_{1-6}$-Alkyl und substituiertem $C_{1-8}$-Alkyl bestehenden Gruppe ausgewählt sind oder $R_1$, $R_2$ und das Kohlenstoffatom, an das sie gebunden sind, zusammen $C_{3-7}$-Cycloalkyl bilden können;
$R_3$ für H steht;
$R_4$ und $R_5$ unabhängig voneinander aus der aus H, Halogen, $C_{1-8}$-Alkyl, $C_{3-7}$-Cycloalkyl, $C_{3-7}$-Cycloalkyl-$C_{1-4}$-alkyl, $C_{3-7}$-Cycloalkyloxy-$C_{1-4}$-alkyl, $C_{1-6}$-Alkoxy-$C_{1-4}$-alkyl, $C_{6-10}$-Aryl, Heteroaryl, halogensubstituiertem $C_{1-4}$-Alkyl, aminosubstituiertem $C_{1-4}$-Alkyl, $C_{6-10}$-arylsubstituiertem $C_{1-4}$-Alkyl, heteroarylsubstituiertem $C_{1-4}$-Alkyl, cy-

anosubstituiertem $C_{1-4}$-Alkyl und hydroxysubstituiertem $C_{1-4}$-Alkyl bestehenden Gruppe ausgewählt sind;
$R_6$ und $R_7$ unabhängig voneinander aus der aus H, Halogen, $C_{1-3}$-Alkyl, halogensubstituiertem $C_{1-3}$-Alkyl, $C_{1-3}$-Alkoxy und halogensubstituiertem $C_{1-3}$-Alkoxy bestehenden Gruppe ausgewählt sind;
n für 1 steht und
Q für $C_{6-10}$-Aryl steht;

und deren Enantiomere, Diastereomere, Tautomere, Solvate und pharmazeutisch unbedenkliche Salze.

**32.** Verbindungen der Formel (III)

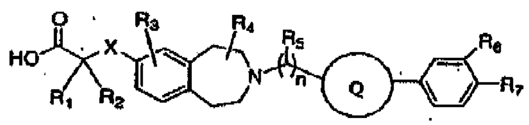

Formel (III)

wobei:

X für eine kovalente Bindung, O oder S steht;
$R_1$ und $R_2$ unabhängig voneinander aus der aus H, $C_{1-8}$-alkyl und substituiertem $C_{1-8}$-Alkyl bestehenden Gruppe ausgewählt sind oder $R_1$, $R_2$ und das Kohlenstoffatom, an das sie gebunden sind, zusammen $C_{3-7}$-Cycloalkyl bilden können;
$R_3$ für H steht;
$R_4$ und $R_5$ unabhängig voneinander aus der aus H, Halogen, $C_{1-6}$-Alkyl, $C_{3-7}$-Cycloalkyl, $C_{3-7}$-Cycloalkyl-$C_{1-4}$-alkyl, $C_{3-7}$-Cycloalkyloxy-$C_{1-4}$-alkyl, $C_{1-6}$-Alkoxy-$C_{1-4}$-alkyl, $C_{6-10}$-Aryl, Heteroaryl, halogensubstituiertem $C_{1-4}$-Alkyl, aminosubstituiertem $C_{1-4}$-Alkyl, $C_{6-10}$-arylsubstituiertem $C_{1-4}$-Alkyl, heteroarylsubstituiertem $C_{1-4}$-Alkyl, cyanosubstituiertem $C_{1-4}$-Alkyl und hydroxysubstituiertem $C_{1-4}$-Alkyl bestehenden Gruppe ausgewählt sind;
$R_6$ und $R_7$ unabhängig voneinander aus der aus H, Halogen, $C_{1-3}$-Alkyl, halogensubstituiertem $C_{1-3}$-Alkyl, $C_{1-3}$-Alkoxy und halogensubstituiertem $C_{1-3}$-Alkoxy bestehenden Gruppe ausgewählt sind;
n für 1 oder 2 steht und
Q ausgewählt ist aus der Gruppe bestehend aus

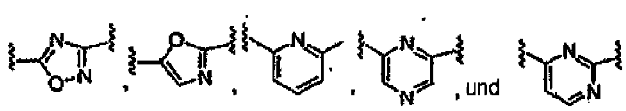

, , , , und

und deren Enantiomere, Diastereomere, Tautomere, Solvate und pharmazeutisch unbedenkliche Salze.

**33.** Verbindungen der Formel (IV)

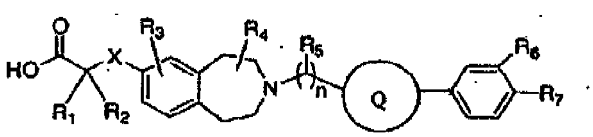

Formel (IV)

wobei:

X für eine kovalente Bindung, O oder S steht;
$R_1$ und $R_2$ unabhängig voneinander aus der aus H, $C_{1-6}$-Alkyl und substituiertem $C_{1-8}$-Alkyl bestehenden Gruppe ausgewählt sind oder $R_1$, $R_2$ und das Kohlenstoffatom, an das sie gebunden sind, zusammen $C_{3-7}$-Cycloalkyl bilden können;
$R_3$ für H steht;
$R_4$ und $R_5$ unabhängig voneinander aus der aus H, Halogen, $C_{1-8}$-Alkyl, $C_{3-7}$-Cycloalkyl, $C_{3-7}$-Cycloalkyl-$C_{1-4}$-alkyl, $C_{3-7}$-Cycloalkyloxy-$C_{1-4}$-alkyl, $C_{1-6}$-Alkoxy-$C_{1-4}$-alkyl, $C_{6-10}$-Aryl, Heteroaryl,

halogensubstituiertem $C_{1-4}$-Alkyl, aminosubstituiertem $C_{1-4}$-Alkyl, $C_{6-10}$-arylsubstituiertem $C_{1-4}$-Alkyl, cyanosubstituiertem $C_{1-4}$-Alkyl und
hydroxysubstituiertem $C_{1-4}$-Alkyl bestehenden Gruppe ausgewählt sind;
$R_6$ und $R_7$ unabhängig voneinander aus der aus H, Halogen, $C_{1-3}$-Alkyl, halogensubstituiertem $C_{1-3}$-Alkyl, $C_{1-3}$-Alkoxy und halogensubstituiertem $C_{1-3}$-Alkoxy bestehenden Gruppe ausgewählt sind;
n für 2 steht und
Q ausgewählt ist aus der Gruppe bestehend aus

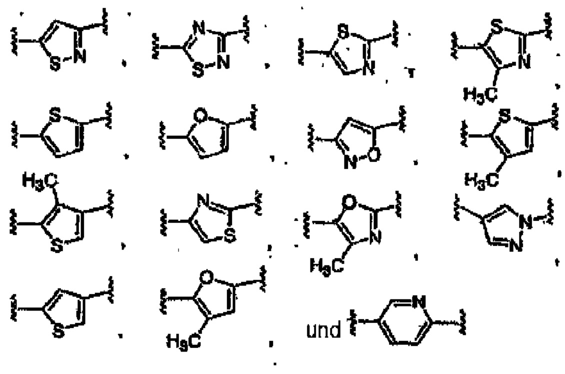

, und

und deren Enantiomere, Diastereomere, Tautomere, Solvate und pharmazeutisch unbedenkliche Salze.

**34.** Verbindungen nach einem der Ansprüche 31, 32 und 33, ausgewählt aus der Gruppe bestehend aus

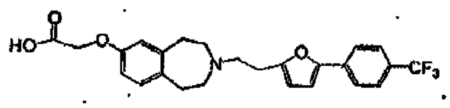

,

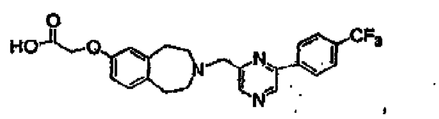

,

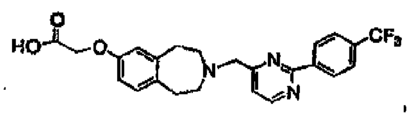

,

und

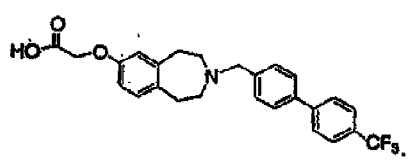

.

**Revendications**

**1.** Composé de formule (I)

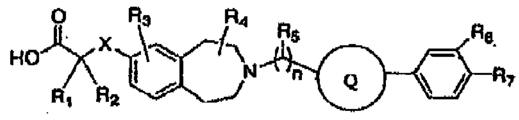

formule (I)

dans laquelle :

X est une liaison covalente, O ou S $R_1$ et $R_2$ sont choisis indépendamment dans le groupe constitué de H, $C_{1-8}$-alkyle et $C_{1-8}$ alkyle substitué, ou $R_1$, $R_2$ et l'atome de carbone auquel ils sont liés peuvent former ensemble $C_{3-7}$-cycloalkyle ;
$R_3$ est H ;
$R_4$ et $R_5$ sont choisis indépendamment dans le groupe constitué de H, halogéno, $C_{1-8}$-alkyle, $C_{3-7}$-cycloalkyle, $C_{3-7}$-cycloalkyl-$C_{1-4}$-alkyle, $C_{3-7}$-cycloalkyloxy-$C_{1-1}$-alkyle, $C_{1-6}$-alcoxy-$C_{1-4}$-alkyle, $C_{6-10}$-aryle, hétéroaryle, $C_{1-4}$-alkyle substitué par halogéno, $C_{1-4}$-alkyle substitué par amino, $C_{1-4}$-alkyle substitué par $C_{6-10}$-aryle, $C_{1-4}$-alkyle substitué par cyano et $C_{1-4}$-alkyle substitué par hydroxy ;
$R_6$ et $R_7$ sont choisis indépendamment dans le groupe constitué de H, halogéno, $C_{1-3}$-alkyle, $C_{1-3}$-alkyle substitué par halogéno, $C_{1-3}$-alcoxy et $C_{1-3}$-alcoxy substitué par halogéno ;
n est 1 ; et
Q est choisi dans le groupe constitué de

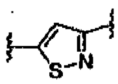 , 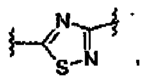 , 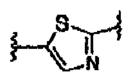 ; 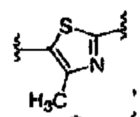 ,
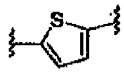 , 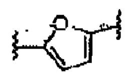 , 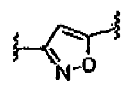 , 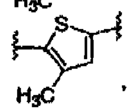 ,
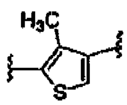 , 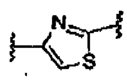 , 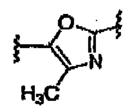 , 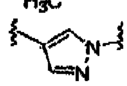 ,
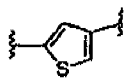 , 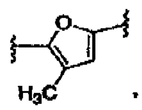 , et 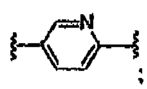 ;

et les énantiomères, les diastéréoisoméres, les tautomères, les solvates ou les sels pharmaceutiquement acceptables de celui-ci.

2. Composé selon la revendication 1, **caractérisé en ce que** $R_1$ et $R_2$ sont choisis indépendamment dans le groupe constitué de H et $C_{1-8}$-alkyle, ou $R_1$, $R_2$ et l'atome de carbone auquel ils sont liés peuvent former ensemble $C_{3-5}$-cycloalkyle.

3. Composé selon la revendication 1, **caractérisé en ce que** $R_1$ et $R_2$ sont choisis indépendamment dans le groupe constitué de H et $CH_3$, ou $R_1$, $R_2$ et l'atome de carbone auquel ils sont liés peuvent former ensemble

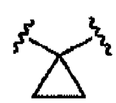 .

4. Composé selon la revendication 1, **caractérisé en ce que** $R_6$ et $R_7$ sont choisis indépendamment dans le groupe constitué de H, halogéno, $C_{1-3}$-alkyle substitué par halogéno, $C_{1-3}$-alcoxy et $C_{1-3}$-alcoxy substitué par halogéno.

5. Composé selon la revendication 1, **caractérisé en ce que** $R_6$ est H et $R_7$ est choisi dans le groupe constitué d'halogéno, $C_{1-3}$-alkyle substitué par halogéno et $C_{1-3}$-alcoxy substitué par halogéno.

6. Composé selon la revendication 5, **caractérisé en ce que** $R_7$ est choisi dans le groupe constitué de F, $CF_3$ et -O-$CF_3$.

7. Composé selon la revendication 1, **caractérisé en ce que** $R_4$ et $R_5$ sont choisis indépendamment dans le groupe constitué de H et $C_{1-6}$-alkyle.

8. Composé selon la revendication 1, **caractérisé en ce que** $R_5$ est H, $CH_3$ ou $-CH_2CH_3$.

9. Composé selon la revendication 1, **caractérisé en ce que** Q est choisi dans le groupe constitué de

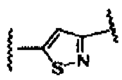, 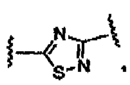, 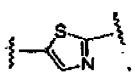, 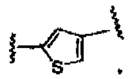, 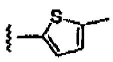, 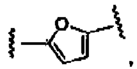, 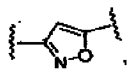, 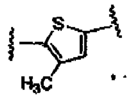, 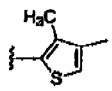, 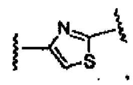, et 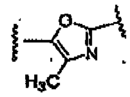.

10. Composé selon la revendication 1, **caractérisé en ce que** Q est choisi dans le groupe constitué de

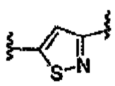, 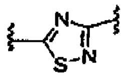, 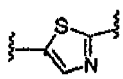, 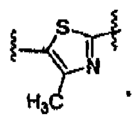, 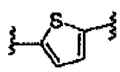, 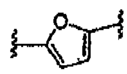, 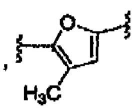 et 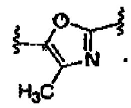.

11. Composé selon la revendication 1, **caractérisé en ce que** X est O.

12. Composé selon la revendication 1, **caractérisé en ce que**
X est O ;
$R_1$, $R_2$, $R_4$ et $R_5$ sont choisis indépendamment dans le groupe constitué de H et $C_{1-3}$-alkyle, ou $R_1$, $R_2$ et l'atome de carbone auquel ils sont liés peuvent former ensemble

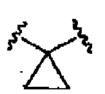 ;

et
$R_6$ et $R_7$ sont choisis indépendamment dans le groupe constitué de H, halogéno, $C_{1-3}$-alcoxy et $C_{1-3}$-alkyle substitué par halogéno et $C_{1-3}$-alcoxy substitué par halogéno ;
et les énantiomères, les diastéréoisomères, les tautomères, les solvates ou les sels pharmaceutiquement acceptables de celui-ci.

13. Composé de formule (Ia) selon la revendication 1

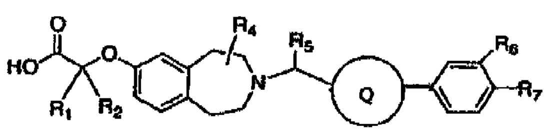

formula (Ia)

dans laquelle

> $R_1$ et $R_2$ sont choisis indépendamment dans le groupe constitué de H et $C_{1-8}$-alkyle, ou $R_1$, $R_2$ et l'atome de carbone auquel ils sont liés peuvent former ensemble $C_{3-5}$-cycloalkyle ;
> $R_6$ et $R_7$ sont choisis indépendamment dans le groupe constitué de H, $C_{1-3}$-alkyle, halogéno et $C_{1-3}$-alkyle substitué par halogéno ;
> $R_4$ et $R_5$ sont choisis indépendamment dans le groupe constitué de H et $C_{1-8}$-alkyle ; et
> Q est choisi dans le groupe constitué de

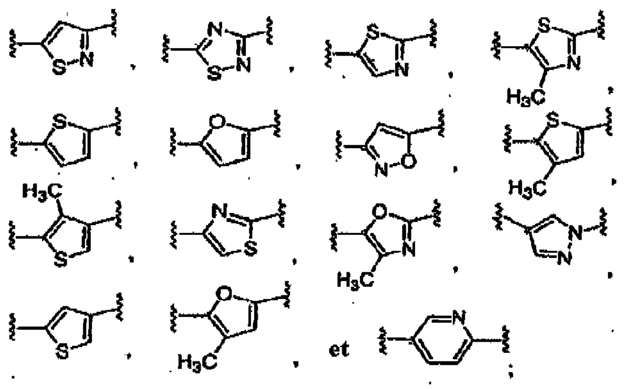

et ;

et les énantiomères, les diastéréoisomères, les tautomères, les solvates ou les sels pharmaceutiquement acceptables de celui-ci.

**14.** Composé de formule (Ib) selon la revendication 1

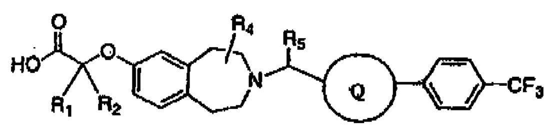

formule (Ib)

dans laquelle

> $R_1$ et $R_2$ sont choisis indépendamment dans le groupe constitué de H et $CH_3$, ou $R_1$, $R_2$ et l'atome de carbone auquel ils sont liés peuvent former ensemble

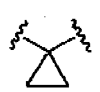

;

> $R_4$ et $R_5$ sont choisis indépendamment dans le groupe constitué de H, $CH_3$ et $-CH_2CH_3$ ; et
> Q est choisi dans le groupe constitué de

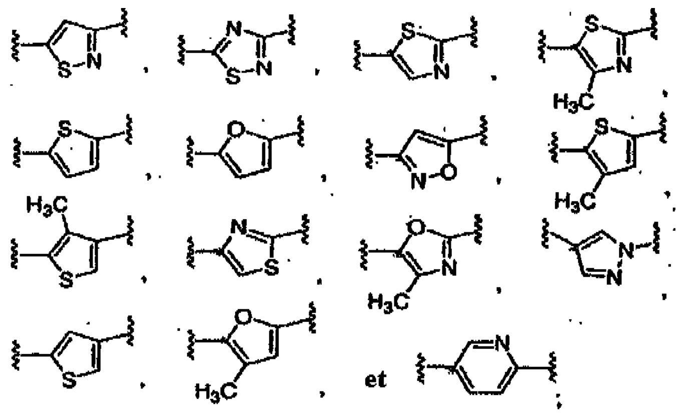

, , , , , , , , , , , , , , et ;

et les énantiomères, les diastéréoisomères, les tautomères, les solvates ou les sels pharmaceutiquement acceptables de celui-ci.

**15.** Composé de formule (Ic) selon la revendication 1

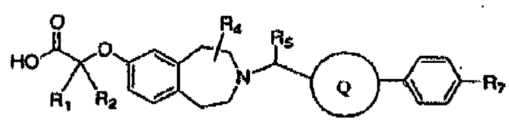

formule (Ic)

dans laquelle

$R_1$, $R_2$ et $R_4$ sont choisis indépendamment dans le groupe constitué de H et $CH_3$, ou $R_1$, $R_2$ et l'atome de carbone auquel ils sont liés peuvent former ensemble

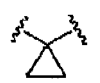 ;

$R_5$ est choisi dans le groupe constitué de H, $CH_3$ et $-CH_2CH_3$ ;
$R_7$ est halogéno ou $C_{1-3}$-alkyle substitué par halogéno ; et
Q est choisi dans le groupe constitué de

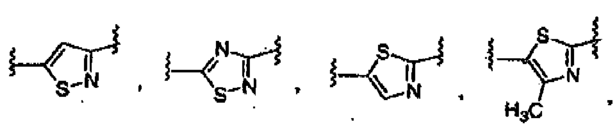

, , , ,

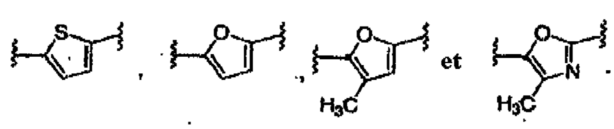

, , et .

et les énantiomères, les diastéréoisomères, les tautomères, les solvates ou les sels pharmaceutiquement acceptables de celui-ci.

**16.** Composé selon la revendication 15, **caractérisé en ce que** :

(a) $R_1$ ou $R_2$ est H ;
(b) $R_1$ et $R_2$ sont tous deux H ;

(c) $R_1$ ou $R_2$ est $CH_3$ ;
(d) $R_1$ et $R_2$ sont tous deux $CH_3$ ;
(e) $R_1$, $R_2$ et l'atome de carbone auquel ils sont liés forment ensemble

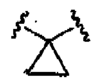 ;

(f) $R_4$ est H ou $CH_3$ ;
(g) $R_5$ est H, $CH_3$ ou $-CH_2CH_3$ ;
(h) $R_1$ ou $R_2$ est H et $R_4$ est H ou $CH_3$ ;
(i) $R_1$ et $R_2$ sont tous deux H et $R_4$ est H ou $CH_3$ ;
(j) $R_1$ ou $R_2$ est H et $R_5$ est H, $CH_3$ ou $-CH_2CH_3$ ;
(k) $R_1$ et $R_2$ sont tous deux H et $R_5$ est H, $CH_3$ ou $-CH_2CH_3$ ;
(l) $R_1$ ou $R_2$ est $CH_3$ et $R_5$ est H, $CH_3$ ou $-CH_2CH_3$ ;
(m) $R_1$ et $R_2$ sont tous deux $CH_3$ et $R_5$ est H, $CH_3$ ou $-CH_2CH_3$ ;
(n) $R_1$ ou $R_2$ est H, $R_4$ est H et $R_5$ est H, $CH_3$ ou $-CH_2CH_3$ ;
(o) $R_1$ ou $R_2$ sont tous deux H, $R_4$ est H et $R_5$ est H, $CH_3$ ou $-CH_2CH_3$ ;
(p) $R_1$ ou $R_2$ est $CH_3$, $R_4$ est H et $R_5$ est H, $CH_3$ ou $-CH_2CH_3$ ;
(g) $R_1$ et $R_2$ sont tous deux $CH_3$, $R_4$ est H et $R_5$ est H, $CH_3$ ou $-CH_2CH_3$ ,
(r) $R_1$, $R_2$ et l'atome de carbone auquel ils sont liés forment ensemble

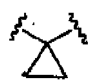

$R_4$ est H et $R_5$ est H, $CH_3$ ou $-CH_2CH_3$ ;
(s) $R_7$ est $CF_3$ ;
(t) $R_7$ est Cl ;
(u) $R_1$ ou $R_2$ est H, $R_7$ est $CF_3$ et $R_4$ est H ou $CH_3$ ;
(v) $R_1$ et $R_2$ sont tous deux H, $R_7$ est $CF_3$ et $R_4$ est H, $CH_3$ ;
(w) $R_1$ ou $R_2$ est H, $R_7$ est $CF_3$ et $R_5$ est H, $CH_3$ ou - $CH_2CH_3$ ;
(x) $R_1$ et $R_2$ sont tous deux H, $R_7$ est $CF_3$ et $R_5$ est H ou $CH_3$ ou $-CH_2CH_3$ ,
(y) $R_1$ ou $R_2$ est $CH_3$, $R_7$ est $CF_3$ et $R_5$ est H, $CH_3$ ou $-CH_2CH_3$ ;
(z) $R_1$ et $R_2$ sont tous deux $CH_3$, $R_7$ est $CF_3$ et $R_5$ est H, $CH_3$ ou $-CH_2CH_3$ ;
(aa) $R_1$ ou $R_2$ est H, $R_4$ est H, $R_7$ est $CF_3$ et $R_5$ est H, $CH_3$ ou $-CH_2CH_3$ ;
(bb) $R_1$ et $R_2$ sont tous deux H, $R_4$ est H, $R_7$ est $CF_3$ et $R_5$ est H, $CH_3$ ou $-CH_2CH_3$
(cc) $R_1$ ou $R_2$ est $CH_3$, $R_4$ est H, $R_7$ est $CF_3$ et $R_5$ est H, $CH_3$ ou $-CH_2CH_3$ ;
(dd) $R_1$ et $R_2$ sont tous deux $CH_3$, $R_4$ est H, $R_7$ est $CF_3$ et $R_5$ est H, $CH_3$ ou $-CH_2CH_3$ ;
(ee) $R_1$, $R_2$ et l'atome de carbone auquel ils sont liés forment ensemble

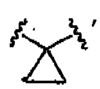

$R_4$ est H, $R_7$ est $CF_3$
et $R_5$ est H, $CH_3$ ou $-CH_2CH_3$ ;
(ff) Q est choisi dans le groupe constitué de

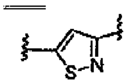 , 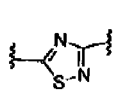 , 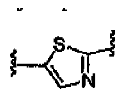 , 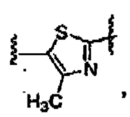 ,

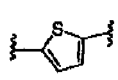 , 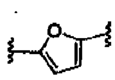 , 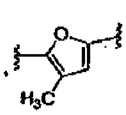 et 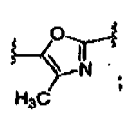 ;

(gg) $R_1$ ou $R_2$ est H et Q est choisi dans le groupe constitué de

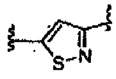, 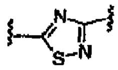, 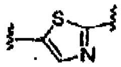, 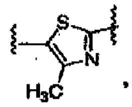,

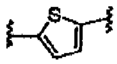, 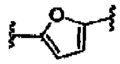, 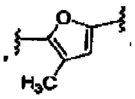 et 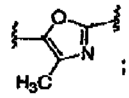;

(hh) $R_1$ et $R_2$ sont tous deux H et Q est choisi dans le groupe constitué de

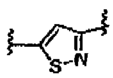, 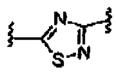, 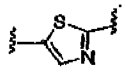, 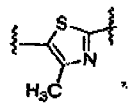,

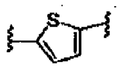, 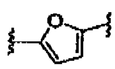, 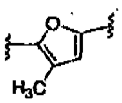 et 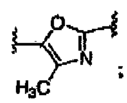;

(ii) $R_1$ ou $R_2$ est $CH_3$ et Q est choisi dans le groupe constitué de

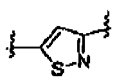, 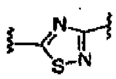, 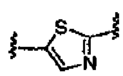, 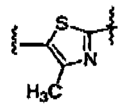,

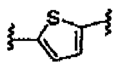, 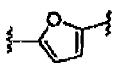, 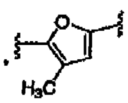 et 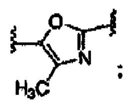;

(jj) $R_1$ et $R_2$ sont tous deux $CH_3$ et Q est choisi dans le groupe constitué de

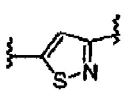, 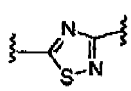, 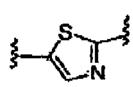, 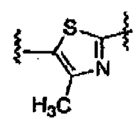,

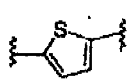, 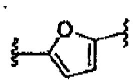, 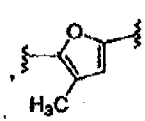 et 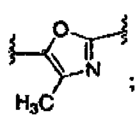;

(kk) $R_4$ est H ou $CH_3$ et Q est choisi dans le groupe constitué de

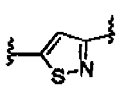, 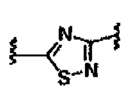, 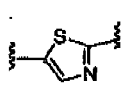, 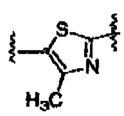,

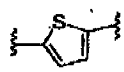, 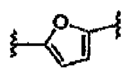, 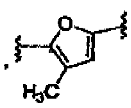 et 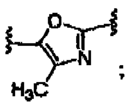;

(ll) $R_5$ est H, $CH_3$ ou $-CH_2CH_3$ et Q est choisi dans le groupe constitué de

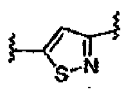, 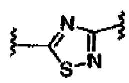, 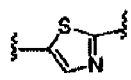, 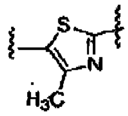,

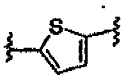, 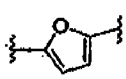, 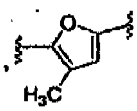 et 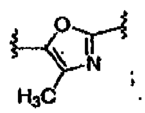;

(mm) $R_1$ ou $R_2$ est H et $R_4$ est H ou $CH_3$ et Q est choisi dans le groupe constitué de

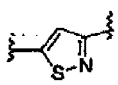, 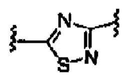, 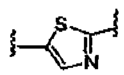, 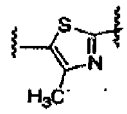,

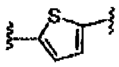, 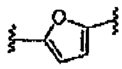, 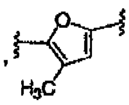 et 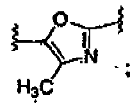;

(nn) $R_1$ et $R_2$ sont tous deux H et $R_4$ est H ou $CH_3$ et Q est choisi dans le groupe constitué de

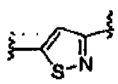, 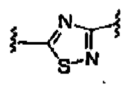, 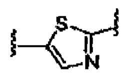, 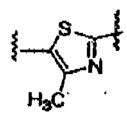,

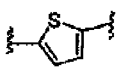, 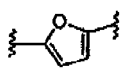, 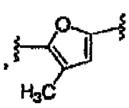 et 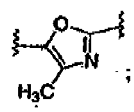;

(oo) $R_1$ ou $R_2$ est H et $R_5$ est H, $CH_3$ ou $-CH_2CH_3$ et Q est choisi dans le groupe constitué de

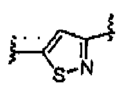, 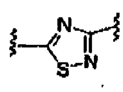, 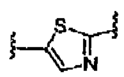, 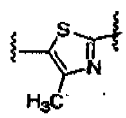,

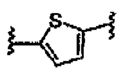, 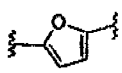, 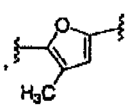 et 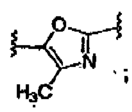;

(pp) $R_1$ et $R_2$ sont tous deux H et $R_5$ est H, $CH_3$ ou $-CH_2CH_3$ et Q est choisi dans le groupe constitué de

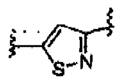, 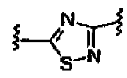, 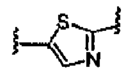, 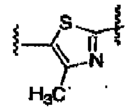,

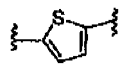, 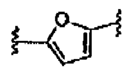, 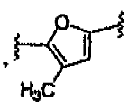 et 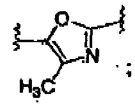;

(qq) $R_1$ ou $R_2$ est $CH_3$ et $R_5$ est H, $CH_3$ ou $-CH_2CH_3$, et Q est choisi dans le groupe constitué de

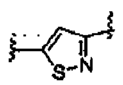, 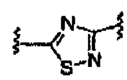, 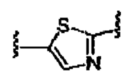, 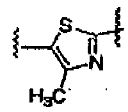,

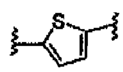, 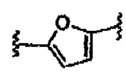, 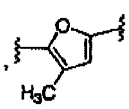 et 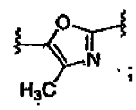;

(rr) $R_1$ et $R_2$ sont tous deux $CH_3$ et $R_5$ est H, $CH_3$ ou $-CH_2CH_3$, et Q est choisi dans le groupe constitué de

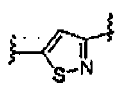, 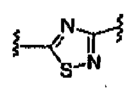, 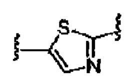, 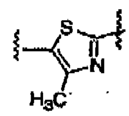,

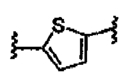, 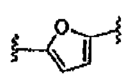, 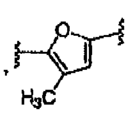 et 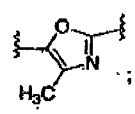;

(ss) $R_1$ ou $R_2$ est H, $R_4$ est H, $R_5$ est H, $CH_3$ ou $-CH_2CH_3$, et Q est choisi dans le groupe constitué de

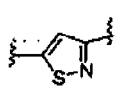, 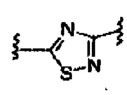, 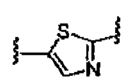, 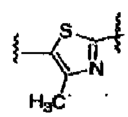,

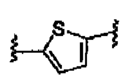, 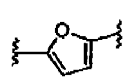, 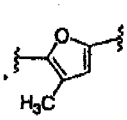 et 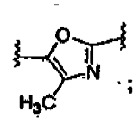;

(tt) $R_1$ et $R_2$ sont tous deux est H, $R_4$ est H, $R_5$ est H, $CH_3$ ou $-CH_2CH_3$, et Q est choisi dans le groupe constitué de

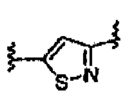, 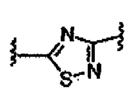, 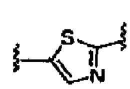, 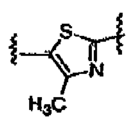,

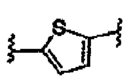, 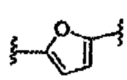, 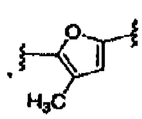 et 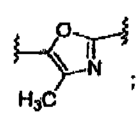;

(uu) $R_1$ ou $R_2$ est $CH_3$, $R_4$ est H, $R_5$ est H, $CH_3$ ou $-CH_2CH_3$, et Q est choisi dans le groupe constitué de

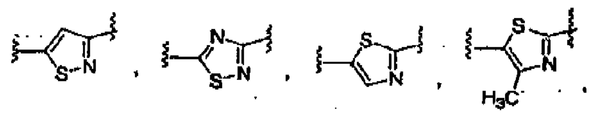

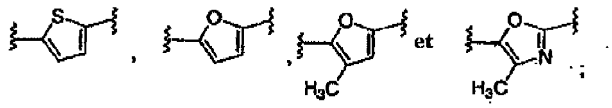 et ;

(vv) $R_1$ et $R_2$ sont tous deux $CH_3$, $R_4$ est H, $R_5$ est H, $CH_3$ ou $-CH_2CH_3$, et Q est choisi dans le groupe constitué de

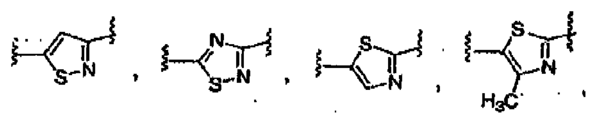

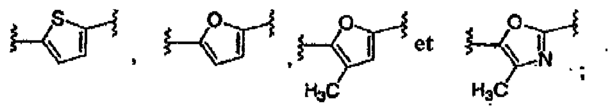 et ;

(ww) $R_1$, $R_2$ et l'atome de carbone auquel ils sont liés forment ensemble

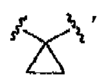

$R_4$ est H, $R_5$ est H, $CH_3$ ou $-CH_2CH_3$, et Q est choisi dans le groupe constitué de

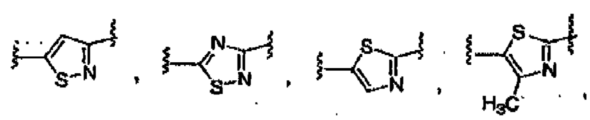

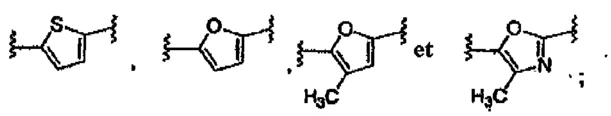 et ;

(xx) $R_7$ est $CF_3$ et Q est choisi dans le groupe constitué de

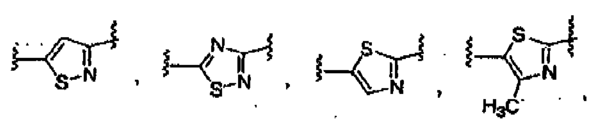

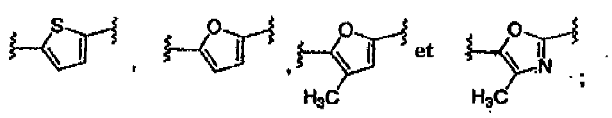 et ;

(yy) $R_7$ est Cl et Q est choisi dans le groupe constitué de

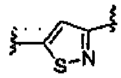, 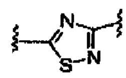, 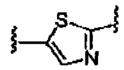, 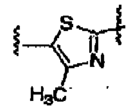,

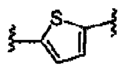, 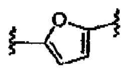, 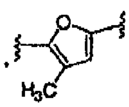 et 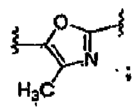;

(zz) $R_1$ ou $R_2$ est H, $R_7$ est $CF_3$, $R_4$ est H ou $CH_3$, et Q est choisi dans le groupe constitué de

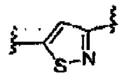, 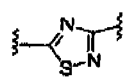, 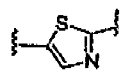, 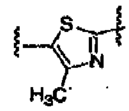,

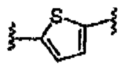, 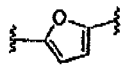, 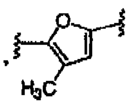 et 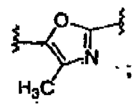;

(aaa) $R_1$ et $R_2$ sont tous deux H, $R_7$ est $CF_3$, $R_4$ est H ou $CH_3$, et Q est choisi dans le groupe constitué de

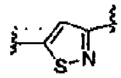, 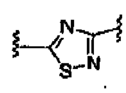, 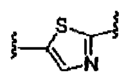, 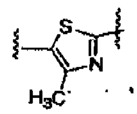,

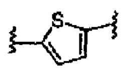, 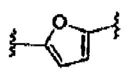, 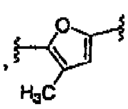 et 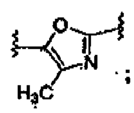;

(bbb) $R_1$ ou $R_2$ est H, $R_7$ est $CF_3$, $R_5$ est H, $CH_3$ ou $-CH_2CH_3$, et Q est choisi dans le groupe constitué de

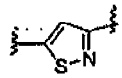, 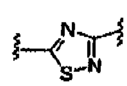, 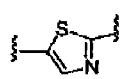, 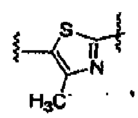,

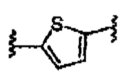, 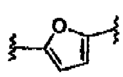, 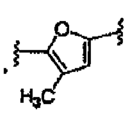 et 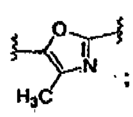;

(ccc) $R_1$ et $R_2$ sont tous deux H, $R_7$ est $CF_3$, $R_5$ est H, $CH_3$ ou $-CH_2CH_3$, et Q est choisi dans le groupe constitué de

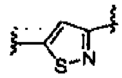, 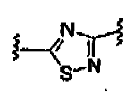, 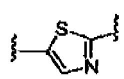, 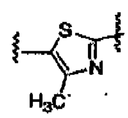,

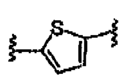, 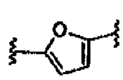, 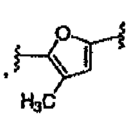 et 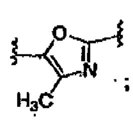;

(ddd) $R_1$ ou $R_2$ est $CH_3$, $R_7$ est $CF_3$, $R_5$ est H, $CH_3$ ou $-CH_2CH_3$, et Q est choisi dans le groupe constitué de

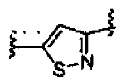, 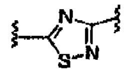, 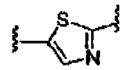, 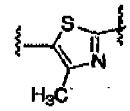,

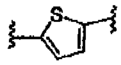, 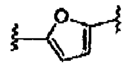, 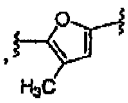 et 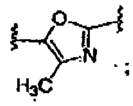;

(eee) $R_1$ et $R_2$ sont tous deux $CH_3$, $R_7$ est $CF_3$, $R_5$ est H, $CH_3$ ou $-CH_2CH_3$, et Q est choisi dans le groupe constitué de

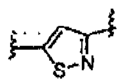, 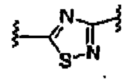, 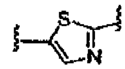, 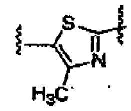,

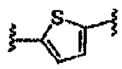, 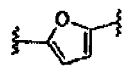, 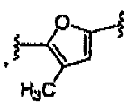 et 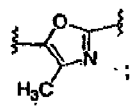;

(fff) $R_1$ ou $R_2$ est H, $R_4$ est H, $R_7$ est $CF_3$, $R_5$ est H, $CH_3$ ou $-CH_2CH_3$ et Q est choisi dans le groupe constitué de

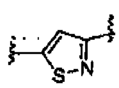, 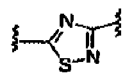, 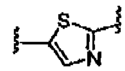, 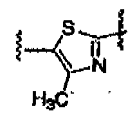,

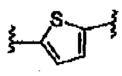, 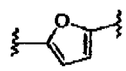, 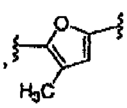 et 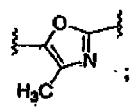;

(ggg) $R_1$ et $R_2$ sont tous deux H, $R_4$ est H, $R_7$ est $CF_3$, $R_5$ est H, $CH_3$ ou $-CH_2CH_3$, et Q est choisi dans le groupe constitué de

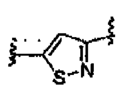, 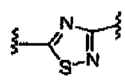, 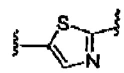, 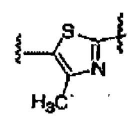,

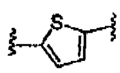, 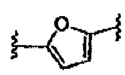, 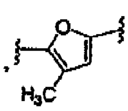 et 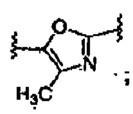;

(hhh) $R_1$ ou $R_2$ est $CH_3$, $R_4$ est H, $R_7$ est $CF_3$, $R_5$ est H, $CH_3$ ou $-CH_2CH_3$, et Q est choisi dans le groupe constitué de

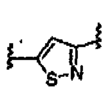, 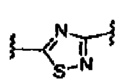, 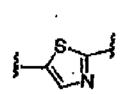, 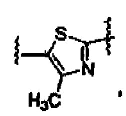,

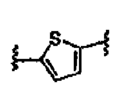, 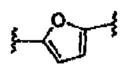, 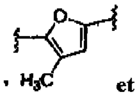 et 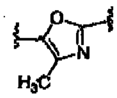 ;

ou

(iii) $R_1$ et $R_2$ sont tous deux $CH_3$, $R_4$ est H, $R_7$ est $CF_3$, $R_5$ est H, $CH_3$ ou $-CH_2CH_3$, et Q est choisi dans le groupe constitué de

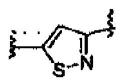, 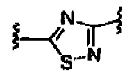, 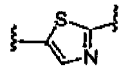, 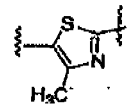,

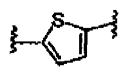, 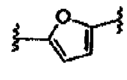, 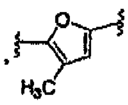 et 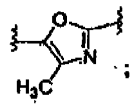 ;

et les énantiomères, les diastéréosiomères, les tautomères, les solvates ou les sels pharmaceutiquement acceptables de celui-ci.

**17.** Composé selon la revendication 1, choisi dans le groupe constitué de

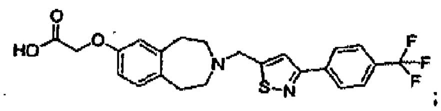 ;

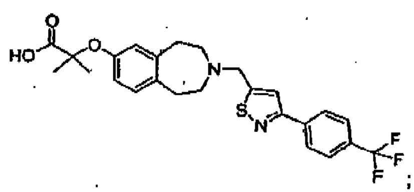 ;

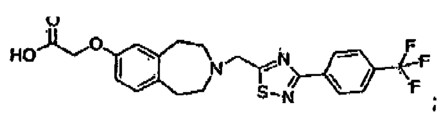 ;

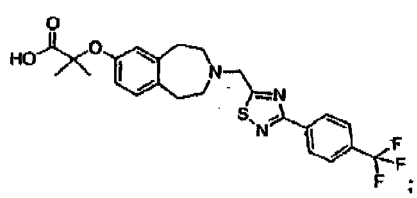 ;

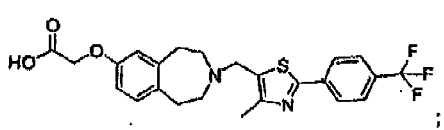 ;

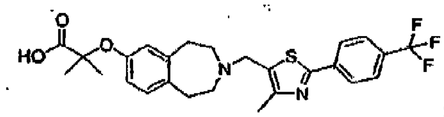
;

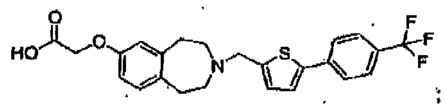
;

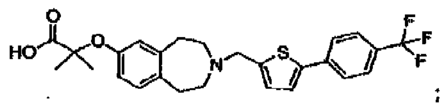
;

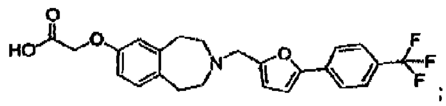
;

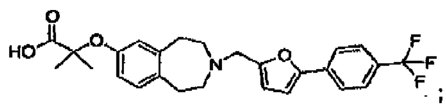
;

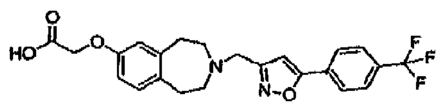
;

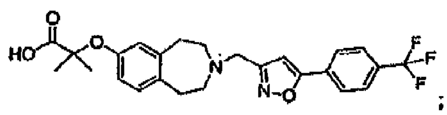
;

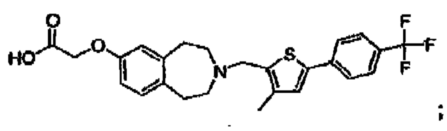
;

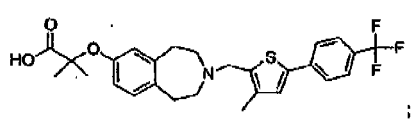
;

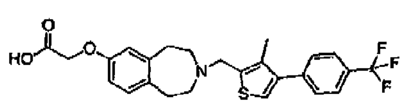
;

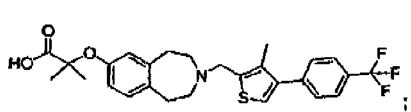
;

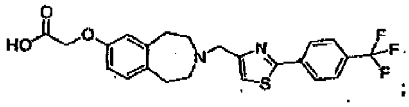
;

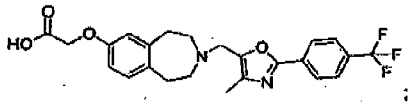
;

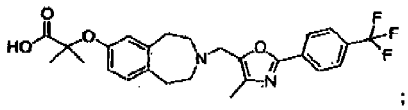
;

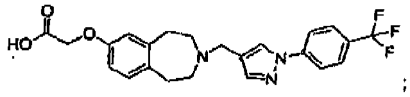
;

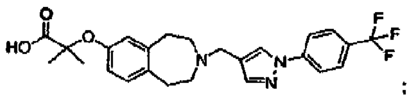
;

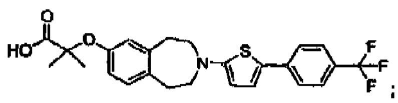
;

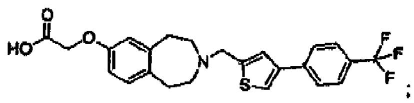
;

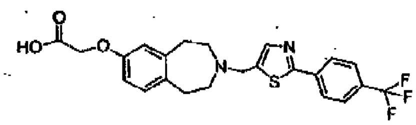
;

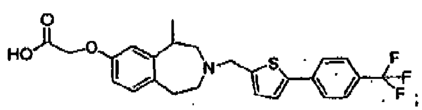
;

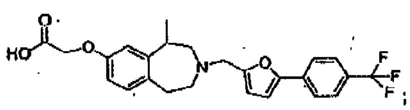
;

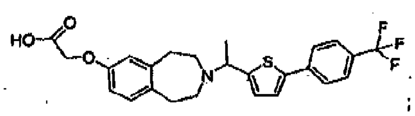
;

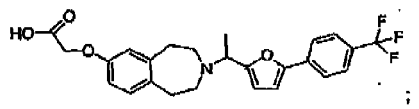

;

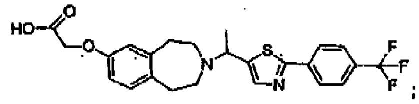

;

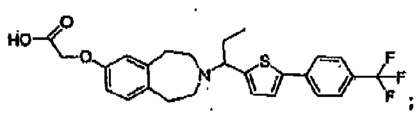

;

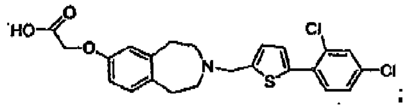

;

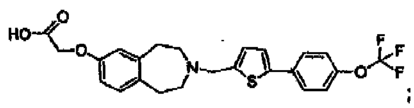

;

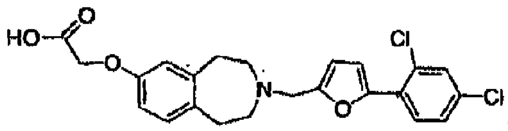

;

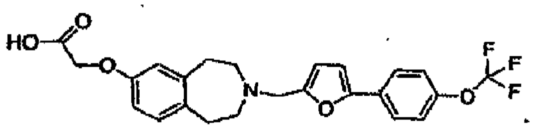

;

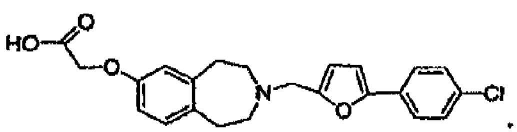

;

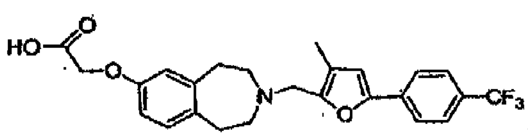

;

et

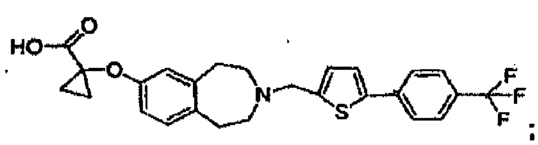

;

et les énantiomères, les diastéréosiomères, les tautomères, les solvates ou les sels pharmaceutiquement acceptables de celui-ci.

**18.** Composé selon la revendication 1, choisi dans le groupe constitué de

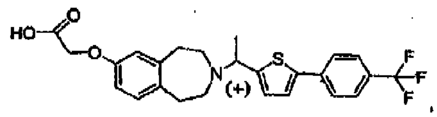
,

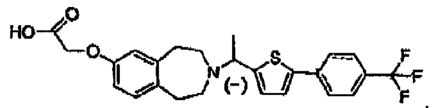
,

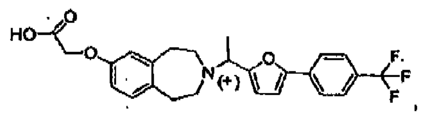
, et

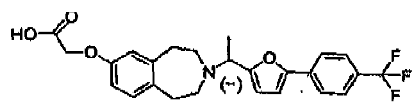
;

le composé étant sensiblement dépourvu de l'autre énantiomère correspondant.

**19.** Composé selon la revendication 1, de formule

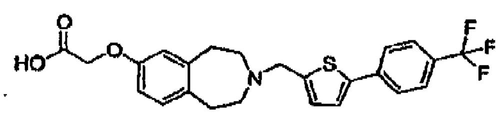

**20.** Composition pharmaceutique comprenant un composé, un sel ou un solvate selon l'une quelconque des revendications 1 à 19, en mélange avec un support, un excipient ou un diluant pharmaceutiquement acceptable.

**21.** Composé, sel ou solvate selon la revendication 1, destiné à être utilisé dans une méthode de traitement ou de prévention d'une maladie ou d'une affection chez un mammifère, maladie ou affection qui est affectée par la modulation de récepteurs PPAR.

**22.** Composé, sel ou solvate selon la revendication 1, destiné à être utilisé dans une méthode de traitement ou de prévention d'une maladie ou d'une affection chez un mammifère, maladie ou affection qui est affectée par la modulation de PPAR delta.

**23.** Composé, sel ou solvate selon la revendication 21 ou 22, **caractérisé en ce que** le composé, le sel ou le solvate de celui-ci est administré dans une gamme de doses d'environ 0,1 mg à environ 15 000 mg.

**24.** Composé, sel ou solvate selon la revendication 21 ou 22, **caractérisé en ce que** le composé, le sel ou le solvate de celui-ci est administré dans une gamme de doses d'environ 50 mg à environ 1000 mg.

**25.** Composé, sel ou solvate selon la revendication 21 ou 22, **caractérisé en ce que** le composé, le sel ou le solvate de celui-ci est administré dans une gamme de doses d'environ 100 mg à environ 1000 mg.

**26.** Composé, sel ou solvate selon la revendication 1, destiné à être utilisé dans une méthode de traitement ou de prévention d'une maladie ou d'une affection choisie dans le groupe constitué par le diabète, la néphropathie, la neuropathie, la rétinopathie, le syndrome des ovaires polykystiques, l'hypertension, l'ischémie, l'accident vasculaire

cérébral, le trouble de l'intestin irritable, l'inflammation, la cataracte, les maladies cardiovasculaires, le syndrome métabolique X, l'hyper-LDL-cholestérolémie, la dyslipidémie (y compris l'hypertriglycéridémie, l'hypercholestérolémie, l'hyperlipidémie mixte, et l'hypo-HDL-cholestérolémie), l'athérosclérose et l'obésité.

**27.** Composé, sel ou solvate selon la revendication 26, **caractérisé en ce que** le composé, le sel ou le solvate de celui-ci est administré dans une gamme de doses d'environ 0,1 mg à environ 15 000 mg.

**28.** Composé, sel ou solvate selon la revendication 26, **caractérisé en ce que** le composé, le sel ou le solvate de celui-ci est administré dans une gamme de doses d'environ 50 mg à environ 1000 mg.

**29.** composé, sel ou solvate selon la revendication 26, **caractérisé en ce que** le composé, le sel ou le solvate de celui-ci est administré dans une gamme de doses d'environ 100 mg à environ 1000 mg.

**30.** Kit comprenant, dans un ou plusieurs récipients, une quantité de la composition selon la revendication 1 efficace pour traiter ou prévenir une maladie ou une affection choisie dans le groupe constitué par le diabète, la néphropathie, la neuropathie, la rétinopathie, le syndrome des ovaires polykystiques, l'hypertension, l'ischémie, l'accident vasculaire cérébral, le trouble de l'intestin irritable, l'inflammation, la cataracte, les maladies cardiovasculaires, le syndrome métabolique X, l'hyper-LDL-cholestérolémie, la dyslipidémie (y compris l'hypertriglycéridémie, l'hypercholestérolémie, l'hyperlipidémie mixte, et l'hypo-HDL-cholestérolémie), l'athérosclérose et l'obésité.

**31.** Composé de formule (II)

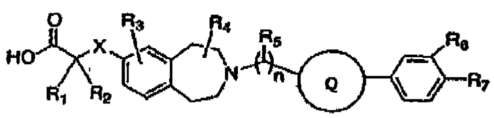

Formule (II)

dans laquelle :

X est une liaison covalent, O ou S ;
$R_1$ et $R_2$ s ont choisis indépendamment dans le groupe constitué de H, $C_{1-8}$-alkyle et $C_{1-8}$-alkyle substitué, ou $R_1$, $R_2$ et l'atome de carbone auquel ils sont liés forment ensemble $C_{3-7}$-cycloalkyle ;
$R_3$ est H ;
$R_4$ et $R_5$ sont choisis indépendamment dans le groupe constitué de H, halogéno, $C_{1-8}$-alkyle, $C_{3-7}$-cycloalkyle, $C_{3-7}$-cycloalkyl-$C_{1-4}$-alkyle, $C_{3-7}$-cycloalkyloxy-$C_{1-4}$-alkyle, $C_{1-6}$-alcoxy-$C_{1-4}$-alkyle, $C_{6-10}$-aryle, hétéroaryle, $C_{1-4}$-alkyle substitué par halogéno, $C_{1-4}$-alkyle substitué par amino, $C_{1-4}$-alkyle substitué par $C_{6-10}$-aryle, $C_{1-4}$-alkyle substitué par hétéroaryle, $C_{1-4}$-alkyle substitué par cyano et $C_{1-4}$-alkyle substitué par hydroxy ;
$R_6$ et $R_7$ sont choisis indépendamment parmi le groupe constitué de H, halogéno, $C_{1-3}$-alkyle, $C_{1-3}$-alkyle substitué par halogéno, $C_{1-3}$-alcoxy et $C_{1-3}$-alcoxy substitué par halogéno ;
n est 1 ; et
Q est $C_{6-10}$aryle ;

et les énantiomères, les diastéréosiomères, les tautomères, les solvates ou les sels pharmaceutiquement acceptables de celui-ci.

**32.** Composé de formule (III)

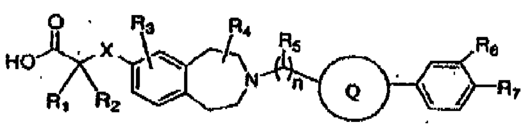

Formule (III)

dans laquelle :

X est une liaison covalente, O ou S ;

$R_1$ et $R_2$ sont choisis indépendamment dans le groupe constitué de H, $C_{1-8}$-alkyle et $C_{1-8}$-alkyle substitué, ou $R_1$, $R_2$ et l'atome de carbone auquel ils sont liés forment ensemble $C_{3-7}$-cycloalkyle ;
$R_3$ est H ;
$R_4$ et $R_5$ sont choisis indépendamment dans le groupe constitué de H, halogéno, $C_{1-8}$-alkyle, $C_{3-7}$-cycloalkyle, $C_{3-7}$-cycloalkyl-$C_{1-4}$-alkyle, $C_{3-7}$-cycloalkyloxy-$C_{1-4}$-alkyle, $C_{1-6}$-alcoxy-$C_{1-4}$-alkyle, $C_{6-10}$-aryle, hétéroaryle, $C_{1-4}$-alkyle substitué par halogéno, $C_{1-4}$-alkyle substitué par amino, $C_{1-4}$-alkyle substitué par $C_{5-10}$-aryle, $C_{1-4}$-alkyle substitué par hétéroaryle, $C_{1-4}$-alkyle substitué par cyano et $C_{1-4}$-alkyle substitué par hydroxy ;
$R_6$ et $R_7$ sont choisis indépendamment parmi le groupe constitué de H, halogéno, $C_{1-3}$-alkyle, $C_{1-3}$-alkyle substitué par halogéno, $C_{1-3}$-alcoxy et $C_{1-3}$-alcoxy substitué par halogéno ;
n est 1 ou 2 ; et
Q est choisi dans le groupe constitué de

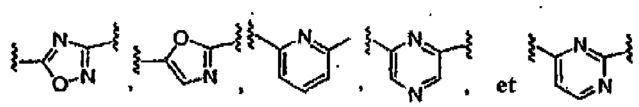 , , , , et ;

et les énantiomères, les diastéréosiomères, les tautomères, les solvates ou les sels pharmaceutiquement acceptables de celui-ci.

**33.** Composé de formule (IV)

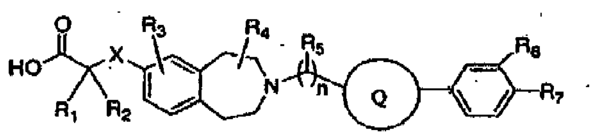

Formule (IV)

dans laquelle :

X est une liaison covalente, O ou S ;
$R_1$ et $R_2$ sont choisis indépendamment dans le groupe constitué de H, $C_{1-8}$-alkyle et $C_{1-8}$-alkyle substitué, ou $R_1$, $R_2$ et l'atome de carbone auquel ils sont liés forment ensemble $C_{3-7}$-cycloalkyle ;
$R_3$ est H ;
$R_4$ et $R_5$ sont choisis indépendamment dans le groupe constitué de H, halogène, $C_{1-8}$-alkyle, $C_{3-7}$-cycloalkyle, $C_{3-7}$-cycloalkyl-$C_{1-4}$-alkyle, $C_{3-7}$-cycloalkyloxy-$C_{1-4}$-alkyle, $C_{1-6}$-alcoxy-$C_{1-4}$-alkyle, $C_{6-10}$-aryle, hétéroaryle, $C_{1-4}$-alkyle substitué par halogéno, $C_{1-4}$-alkyle substitué par amino, $C_{1-4}$-alkyle substitué par $C_{6-10}$-aryle, $C_{1-4}$-alkyle substitué par cyano et $C_{1-4}$-alkyle substitué par hydroxy ;
$R_6$ et $R_7$ sont choisis indépendamment parmi le groupe constitué de H, halogéno, $C_{1-3}$-alkyle, $C_{1-3}$-alkyle substitué par halogéno, $C_{1-3}$-alcoxy et $C_{1-3}$-alcoxy substitué par halogéno ;
n est 2 ; et
Q est choisi dans le groupe constitué de

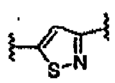 , 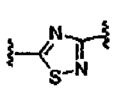 , 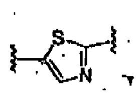 , 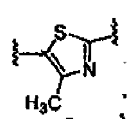 ,
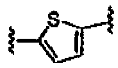 , 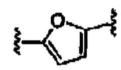 , 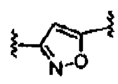 , 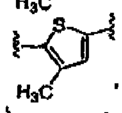 ,
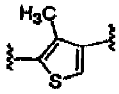 , 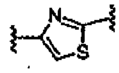 , 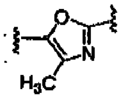 , 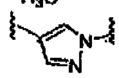 ,
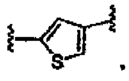 , 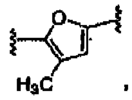 , et 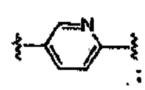 ;

et les énantiomères, les diastéréosiomères, les tautomères, les solvates ou les sels pharmaceutiquement acceptables de celui-ci.

**34.** composé selon la revendication 31, 32 ou 33, choisi dans le groupe constitué de

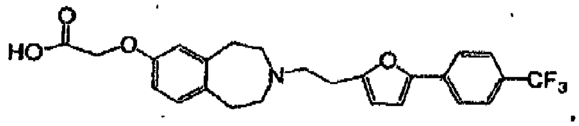

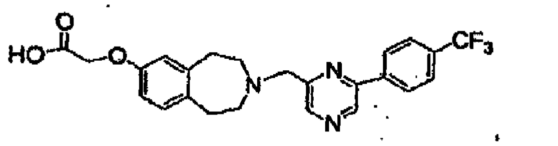

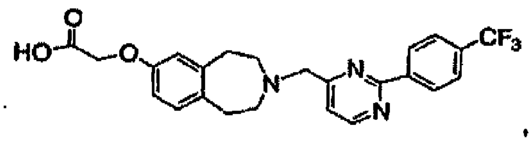

et

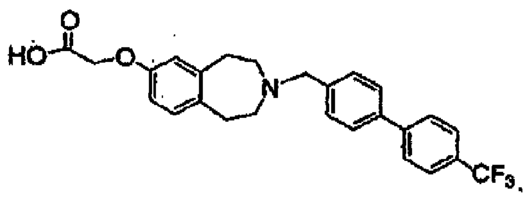

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005041959 A
- EP 1445258 A
- EP 1424330 A
- US 4659706 A
- EP 204349 A

### Non-patent literature cited in the description


- pharmaceutically acceptable salts. *Ref. International J. Pharm.,* 1986, vol. 33, 201-217
- *J. Pharm.Sci.,* January 1997, vol. 66, 1, 1
- Protective Groups in Organic Chemistry. Plenum Press, 1973
- **T.W. Greene ; P.G.M. Wuts.** Protective Groups in Organic Synthesis. John Wiley & Sons, 1991
- **Still, W. C.** *J. Org. Chem.,* 1978, vol. 43, 2921
- *Bioorg & Med. Chem. Lett.,* 2003, vol. 13 (9), 1517-1521
- *Bioorg. & Med. Chem. Lett,* 2003, vol. 13 (13), 2159-2161
- *J. Org. Chem.,* 1982, vol. 47, 2217-2218
- **Auboeuf et al.** *Diabetes,* 1997, vol. 46 (8), 1319-1327
- **Braissant et al.** *Endocrinology,* 1996, vol. 137 (1), 354-366
- **Barak et al.** *Proc. Natl. Acad. Sci. USA,* 2002, vol. 99 (1), 303-308
- **Lawn et al.** *J. Clin. Investigation,* 1999, vol. 104 (8), R25-R31
- **Leibowitz et al.** *FEBS Lett.,* 2000, vol. 473 (3), 333-336
- **Oliver et al.** *Proc. Natl. Acad. Sci. URSA,* 2001, vol. 98 (9), 5306-5311
- **Tanaka et al.** *Proc. Natl. Acad. Sci. USA,* 2003, vol. 100 (26), 15924-15929
- **Wang et al.** *Cell,* 2003, vol. 113, 159-170